(19) Europäisches Patentamt
European Patent Office
Office européen des brevets

(11) EP 2 066 177 B1

(12) **EUROPÄISCHE PATENTSCHRIFT**

(45) Veröffentlichungstag und Bekanntmachung des Hinweises auf die Patenterteilung:
**24.12.2014 Patentblatt 2014/52**

(21) Anmeldenummer: **08701553.3**

(22) Anmeldetag: **17.01.2008**

(51) Int Cl.:
***A01N 43/56*** *(2006.01)* ***A01N 43/90*** *(2006.01)*
***A01P 3/00*** *(2006.01)*

(86) Internationale Anmeldenummer:
**PCT/EP2008/050494**

(87) Internationale Veröffentlichungsnummer:
**WO 2008/087182 (24.07.2008 Gazette 2008/30)**

(54) **FUNGIZIDE MISCHUNGEN AUS 1-METHYLPYRAZOL-4-YLCARBONSÄUREANILIDEN UND AZOLOPYRIMIDINYLAMINEN**

FUNGICIDAL MIXTURES OF 1-METHYLPYRAZOLE-4-YLCARBOXYLIC ACID ANILIDES AND AZOLOPYRIMIDINYLAMINES

MÉLANGES FONGICIDES CONSTITUÉS D'ANILIDES DE L'ACIDE 1-METHYLPYRAZOL-4-YL CARBOXYLIQUE ET D'AZOLOPYRIMIDINYLAMINES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**MK RS**

(30) Priorität: **19.01.2007 EP 07100851**

(43) Veröffentlichungstag der Anmeldung:
**10.06.2009 Patentblatt 2009/24**

(73) Patentinhaber: **BASF SE**
**67056 Ludwigshafen (DE)**

(72) Erfinder:
- **DIETZ, Jochen**
**68167 Mannheim (DE)**
- **SCHÖFL, Ulrich**
**68782 Brühl (DE)**
- **HADEN, Egon**
**67259 Kleinniedesheim (DE)**

(56) Entgegenhaltungen:
**WO-A-2006/087343**
**WO-A-2007/000462**
**WO-A-2007/003540**
**WO-A-2007/012598**
**WO-A-2007/017416**

EP 2 066 177 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft fungizide Mischungen, enthaltend:

A) mindestens ein 1-Methylpyrazol-4-ylcarbonsäureanilid der Formel I

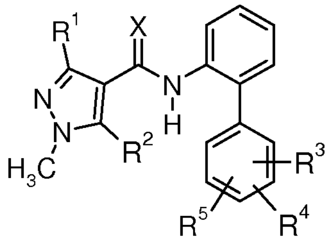

I

in der die Substituenten folgende Bedeutungen haben:

| | |
|---|---|
| X | Sauerstoff oder Schwefel; |
| $R^1$ | $C_1$-$C_4$-Alkyl oder $C_1$-$C_4$-Halogenalkyl; |
| $R^2$ | Wasserstoff oder Halogen; |
| $R^3$, $R^4$ und $R^5$ | unabhängig voneinander Cyano, Nitro, Halogen, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio; |

und

B) mindestens ein Azolopyrimidinylamin der Formel II

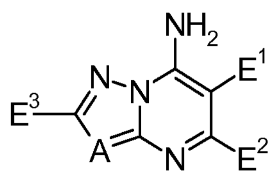

II

in der die Substituenten folgende Bedeutung haben:

| | |
|---|---|
| $E^1$ | $C_3$-$C_{12}$-Alkyl, $C_2$-$C_{12}$-Alkenyl, $C_5$-$C_{12}$-Alkoxyalkyl, $C_3$-$C_6$-Cycloalkyl, Phenyl oder Phenyl-$C_1$-$C_4$-alkyl; |
| $E^2$ | $C_1$-$C_{12}$-Alkyl, $C_2$-$C_{12}$-Alkenyl, $C_1$-$C_4$-Halogenalkyl oder $C_1$-$C_4$-Alkoxy-$C_1$-$C_4$-alkyl; wobei die aliphatischen Ketten in $E^1$ und/oder $E^2$ durch eine bis vier gleiche oder verschiedene Gruppen $R^a$ substituiert sein können: |
| $R^a$ | Halogen, Cyano, Hydroxy, Mercapto, $C_1$-$C_{10}$-Alkyl, $C_1$-$C_{10}$-Halogenalkyl, $C_3$-$C_8$-Cycloalkyl, $C_2$-$C_{10}$-Alkenyl, $C_2$-$C_{10}$-Alkinyl, $C_1$-$C_6$-Alkoxy, $C_1$-$C_6$-Alkylthio, $C_1$-$C_6$-Alkoxy-$C_1$-$C_6$-alkyl oder $NR^AR^B$; unabhängig voneinander Wasserstoff oder $C_1$-$C_6$-Alkyl; |
| $R^A$, $R^B$ | wobei die cyclischen Gruppen in $E^1$ und/oder $R^a$ durch eine bis vier Gruppen $R^b$ substituiert sein können: |
| $R^b$ | Halogen, Cyano, Hydroxy, Mercapto, Nitro, $NR^AR^B$, $C_1$-$C_{10}$-Alkyl, $C_1$-$C_6$-Halogenalkyl, $C_2$-$C_6$-Alkenyl, $C_2$-$C_6$-Alkinyl oder $C_1$-$C_6$-Alkoxy; |
| $E^3$ | Wasserstoff, Halogen, Cyano, $NR^AR^B$, Hydroxy, Mercapto, $C_1$-$C_6$-Alkyl, $C_1$-$C_6$-Halogenalkyl, $C_3$-$C_8$-Cycloalkyl, $C_1$-$C_6$-Alkoxy, $C_1$-$C_6$-Alkylthio, $C_3$-$C_8$-Cycloalkoxy, $C_3$-$C_8$-Cycloalkylthio, Carboxyl, Formyl, $C_1$-$C_{10}$-Alkylcarbonyl, $C_1$-$C_{10}$-Alkoxycarbonyl, $C_2$-$C_{10}$-Alkenyloxycarbonyl, $C_2$-$C_{10}$-Alkinyloxycarbonyl, Phenyl, Phenoxy, Phenylthio, Benzyloxy, Benzylthio oder $C_1$-$C_6$-Alkyl-$S(O)_m$-; |
| m | 0, 1 oder 2; |
| A | CH oder N; |

in einer synergistisch wirksamen Menge.

Außerdem betrifft die Erfindung ein Verfahren zur Bekämpfung von Schadpilzen mit einer Mischung enthaltend mindestens eine Verbindung I und mindestens eine Verbindung II, ein Verfahren zur Herstellung derartiger Mischungen sowie Mittel und Saatgut enthaltend diese Mischungen.

Die als Komponente A voranstehend bezeichneten 1-Methylpyrazol-4-ylcarbonsäure-anilide der Formel I und ihre Wirkung gegen Schadpilze, sowie deren Herstellung sind aus WO 06/087343 und PCT/EP2006/064907 und der darin zitierten Literatur bekannt, oder sind auf die dort beschriebene Weise herstellbar.

Mischungen von Verbindungen der Formel I mit anderen Wirkstoffen sind in PCT/EP2006/064907 allgemein beschrieben.

Die als Komponente B bezeichneten Azolopyrimidin-7-ylamine der Formel II und ihre Wirkung gegen Schadpilze, sowie deren Herstellung sind aus PCT/EP2006/064463 und der darin zitierten Literatur bekannt, oder sind auf die dort beschriebene Weise herstellbar.

Mischungen von Verbindungen der Formel II mit anderen Wirkstoffen sind in PCT/EP2006/064463 allgemein beschrieben.

Die fungizide Wirkung der bekannten Verbindungen der Formel I beziehungsweise Formel II sollte, insbesondere bei Verwendung niedriger Aufwandmengen, weiter gesteigert werden. Im Hinblick auf eine Senkung der Aufwandmengen und eine Verbreiterung des Wirkungsspektrums der Verbindungen der Formeln I und II lagen daher der vorliegenden Erfindung Mischungen als Aufgabe zugrunde, die bei verringerter Gesamtmenge an ausgebrachten Wirkstoffen eine verbesserte Wirkung gegen Schadpilze, insbesondere für bestimmte Indikationen, zeigen.

Demgemäss wurden die eingangs definierten Mischungen gefunden, die die Verbindungen der Formeln I und II enthalten. Es wurde außerdem gefunden, dass bei gleichzeitiger, und zwar gemeinsamer oder getrennter Anwendung mindestens einer Verbindung I und mindestens einer Verbindung II, oder mindestens einer Verbindung I und mindestens einer Verbindung II nacheinander, Schadpilze in überadditivem Maße bekämpft werden können und zwar besser als mit den Einzelverbindungen allein (synergistische Mischungen).

Bei den in den vorstehenden Formeln angegebenen Definitionen der Symbole wurden Sammelbegriffe verwendet, die allgemein repäsentativ für die folgenden Substituenten stehen:

Ausdrücke der Form $C_a$-$C_b$ bezeichnen im Rahmen dieser Erfindung chemische Verbindungen oder Substituenten mit einer bestimmten Anzahl von Kohlenstoffatomen. Die Anzahl an Kohlenstoffatomen kann aus dem gesamten Bereich von a bis b, einschließlich a und b gewählt werden, a ist mindestens 1 und b immer größer als a. Eine weitere Spezifizierung der chemischen Verbindungen oder der Substituenten erfolgt durch Ausdrücke der Form $C_a$-$C_b$-V. V steht hierbei für eine chemische Verbindungsklasse oder Substituentenklasse, beispielsweise für Alkylverbindungen oder Alkylsubstituenten.

Halogen: Fluor, Chlor, Brom oder Jod;

Alkyl: gesättigte, geradkettige oder verzweigte Kohlenwasserstoffreste mit 1 bis 4, 1 bis 6, 1 bis 10, 1 bis 12 beziehungsweise 3 bis 12 Kohlenstoffatomen, z.B. $C_1$-$C_6$-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl;

Halogenalkyl: geradkettige oder verzweigte Alkylreste mit 1 bis 4, 1 bis 6 beziehungsweise 1 bis 10 Kohlenstoffatomen (wie vorstehend genannt), wobei in diesen Resten teilweise oder vollständig die Wasserstoffatome durch Halogenatome, wie vorstehend genannt ersetzt sein können: insbesondere $C_1$-$C_2$-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl,
2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;

Alkenyl: ungesättigte, geradkettige oder verzweigte Kohlenwasserstoffreste mit 2 bis 6, 2 bis 10 beziehungsweise 2 bis 12 Kohlenstoffatomen und einer oder zwei Doppelbindungen in beliebiger Position, z.B. $C_2$-$C_6$-Alkenyl wie Ethenyl, 1-Propenyl, 2-Propenyl, 1-Methyl-ethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl oder 1-Ethyl-2-methyl-2-propenyl;

Alkinyl: geradkettige oder verzweigte Kohlenwasserstoffreste mit 2 bis 6 beziehungsweise 2 bis 10 Kohlenstoffatomen und einer oder zwei Dreifachbindungen in beliebiger Position, z.B. $C_2$-$C_6$-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl oder 1-Ethyl-1-methyl-2-propinyl;

Cycloalkyl: mono- oder bicyclische, gesättigte Kohlenwasserstoffreste mit 3 bis 6 oder 3 bis 8 Kohlenstoffringgliedern, z.B. $C_3$-$C_8$-Cycloalkyl wie Cyclopropyl, Cyclobutyl, Cyclopentyl, Cyclohexyl, Cycloheptyl oder Cyclooctyl;

Cycloalkoxy: mono- oder bicyclische, gesättigte Kohlenwasserstoffreste, welche über ein Sauerstoffatom (-O-) gebunden sind;

Cycloalkylthio: mono- oder bicyclische, gesättigte Kohlenwasserstoffreste, welche über ein Schwefelatom (-S-) gebunden sind;

Alkylthio: gesättigte, geradkettige oder verzweigte Kohlenwasserstoffreste, welche über ein Schwefelatom (-S-) gebunden sind;

Alkylcarbonyl: geradkettige oder verzweigte Alkylreste mit 1 bis 10 Kohlenstoffatomen, welche über eine Carbonylgruppe (-CO-) gebunden sind;

Alkoxy: geradkettige oder verzweigte Alkylreste, welche über ein Sauerstoffatom (-O-) gebunden sind;

Alkoxyalkyl: geradkettige oder verzweigte Alkoxyreste, welche an einen, Alkylrest gebunden sind;

Halogenalkoxy: geradkettige oder verzweigte Alkoxyreste, wobei in diesen Resten teilweise oder vollständig die Wasserstoffatome durch Halogen ersetzt sein können;

Alkoxycarbonyl: Alkoxyreste mit 1 bis 10 Kohlenstoffatomen, welche über eine Carbonylgruppe (-CO-) gebunden sind;

Alkenyloxycarbonyl: Alkenylreste, welche über ein Sauerstoffatom (-O-) an eine Carbonylgruppe (-CO-) gebunden sind;

Alkinyloxycarbonyl: Alkinylreste, welche über ein Sauerstoffatom (-O-) an eine Carbonylgruppe (-CO-) gebunden sind;

Phenylalkyl: Phenylgruppe, die über gesättigte, geradkettige oder verzweigte Alkylreste gebunden ist.

Im Hinblick auf die erfindungungsgemäßen fungiziden Mischungen, die die Verbindungen der Formel I und II enthalten, Verfahren zur Bekämpfung von pflanzenpathogenen Schadpilzen, Saatgut enthaltend fungizide Mischungen, fungiziden Mittel sowie Verfahren zur Herstellung dieser Mittel sind die folgenden Bedeutungen der Substituenten, und zwar jeweils für sich allein oder in Kombination, besonders bevorzugt:

Eine Ausgestaltung der Formel I betrifft solche, bei denen X Sauerstoff bedeutet.

Eine weitere Ausgestaltung der Formel I betrifft solche, bei denen X Schwefel bedeutet.
Für die erfindungsgemäßen Mischungen sind Verbindungen der Formel I bevorzugt, bei denen $R^1$ für Methyl

oder Halogenmethyl wie $CH_3$, $CHF_2$, $CH_2F$, $CF_3$, CHFCl oder $CF_2Cl$, insbesondere $CHF_2$ oder $CF_3$, steht.

Bevorzugt sind ferner Verbindungen I, bei denen $R^2$ für Wasserstoff, Fluor oder Chlor, insbesondere Wasserstoff, steht.

Des weiteren sind diejenigen Verbindungen I bevorzugt, bei denen $R^3$ für Halogen, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio, vorzugsweise für Halogen, Methyl, Halogenmethyl, Methoxy, Halogenmethoxy oder Methylthio, insbesondere F, Cl, $CH_3$, $CF_3$, $OCH_3$, $OCHF_2$, $OCF_3$ oder $SCH_3$, besonders bevorzugt Fluor, steht.

Außerdem sind diejenigen Verbindungen I bevorzugt, bei denen $R^4$ für Halogen, insbesondere Fluor steht.

Weiterhin sind diejenigen Verbindungen I bevorzugt, bei denen $R^5$ für Halogen, insbesondere Fluor steht.

Darüberhinaus sind Verbindungen der Formel I bevorzugt, die in der folgenden Tabelle 1 aufgelistet sind:

Tabelle 1: Verbindungen I mit X = Sauerstoff

| Nr. | $R^1$ | $R^2$ | $R^3$ | $R^4$ | $R^5$ |
|---|---|---|---|---|---|
| 1-1 | $CH_3$ | H | 2-F | 3-F | 4-F |
| 1-2 | $CH_3$ | H | 2-F | 3-F | 5-F |
| 1-3 | $CH_3$ | H | 2-F | 4-F | 5-F |
| 1-4 | $CH_3$ | H | 2-F | 4-F | 6-F |
| 1-5 | $CH_3$ | H | 3-F | 4-F | 5-F |
| 1-6 | $CH_3$ | H | 3-F | 5-F | 6-F |
| 1-7 | $CH_2F$ | H | 2-F | 3-F | 4-F |
| 1-8 | $CH_2F$ | H | 2-F | 3-F | 5-F |
| 1-9 | $CH_2F$ | H | 2-F | 4-F | 5-F |
| 1-10 | $CH_2F$ | H | 2-F | 4-F | 6-F |
| 1-11 | $CH_2F$ | H | 3-F | 4-F | 5-F |
| 1-12 | $CH_2F$ | H | 3-F | 5-F | 6-F |
| 1-13 | $CHF_2$ | H | 2-F | 3-F | 4-F |
| 1-14 | $CHF_2$ | H | 2-F | 3-F | 5-F |
| 1-15 | $CHF_2$ | H | 2-F | 4-F | 5-F |
| 1-16 | $CHF_2$ | H | 2-F | 4-F | 6-F |
| 1-17 | $CHF_2$ | H | 3-F | 4-F | 5-F |
| 1-18 | $CHF_2$ | H | 3-F | 5-F | 6-F |
| 1-19 | $CF_3$ | H | 2-F | 3-F | 4-F |
| 1-20 | $CF_3$ | H | 2-F | 3-F | 5-F |
| 1-21 | $CF_3$ | H | 2-F | 4-F | 5-F |
| 1-22 | $CF_3$ | H | 2-F | 4-F | 6-F |
| 1-23 | $CF_3$ | H | 3-F | 4-F | 5-F |
| 1-24 | $CF_3$ | H | 3-F | 5-F | 6-F |
| 1-25 | CHFCl | H | 2-F | 3-F | 4-F |
| 1-26 | CHFCl | H | 2-F | 3-F | 5-F |
| 1-27 | CHFCl | H | 2-F | 4-F | 5-F |
| 1-28 | CHFCl | H | 2-F | 4-F | 6-F |

| Nr. | $R^1$ | $R^2$ | $R^3$ | $R^4$ | $R^5$ |
|---|---|---|---|---|---|
| 1-29 | CHFCl | H | 3-F | 4-F | 5-F |
| 1-30 | CHFCl | H | 3-F | 5-F | 6-F |
| 1-31 | $CF_2Cl$ | H | 2-F | 3-F | 4-F |
| 1-32 | $CF_2Cl$ | H | 2-F | 3-F | 5-F |
| 1-33 | $CF_2Cl$ | H | 2-F | 4-F | 5-F |
| 1-34 | $CF_2Cl$ | H | 2-F | 4-F | 6-F |
| 1-35 | $CF_2Cl$ | H | 3-F | 4-F | 5-F |
| 1-36 | $CF_2Cl$ | H | 3-F | 5-F | 6-F |
| 1-37 | $CH_3$ | F | 2-F | 3-F | 4-F |
| 1-38 | $CH_3$ | F | 2-F | 3-F | 5-F |
| 1-39 | $CH_3$ | F | 2-F | 4-F | 5-F |
| 1-40 | $CH_3$ | F | 2-F | 4-F | 6-F |
| 1-41 | $CH_3$ | F | 3-F | 4-F | 5-F |
| 1-42 | $CH_3$ | F | 3-F | 5-F | 6-F |
| 1-43 | $CH_2F$ | F | 2-F | 3-F | 4-F |
| 1-44 | $CH_2F$ | F | 2-F | 3-F | 5-F |
| 1-45 | $CH_2F$ | F | 2-F | 4-F | 5-F |
| 1-46 | $CH_2F$ | F | 2-F | 4-F | 6-F |
| 1-47 | $CH_2F$ | F | 3-F | 4-F | 5-F |
| 1-48 | $CH_2F$ | F | 3-F | 5-F | 6-F |
| 1-49 | $CHF_2$ | F | 2-F | 3-F | 4-F |
| 1-50 | $CHF_2$ | F | 2-F | 3-F | 5-F |
| 1-51 | $CHF_2$ | F | 2-F | 4-F | 5-F |
| 1-52 | $CHF_2$ | F | 2-F | 4-F | 6-F |
| 1-53 | $CHF_2$ | F | 3-F | 4-F | 5-F |
| 1-54 | $CHF_2$ | F | 3-F | 5-F | 6-F |
| 1-55 | $CF_3$ | F | 2-F | 3-F | 4-F |
| 1-56 | $CF_3$ | F | 2-F | 3-F | 5-F |
| 1-57 | $CF_3$ | F | 2-F | 4-F | 5-F |
| 1-58 | $CF_3$ | F | 2-F | 4-F | 6-F |
| 1-59 | $CF_3$ | F | 3-F | 4-F | 5-F |
| 1-60 | $CF_3$ | F | 3-F | 5-F | 6-F |
| 1-61 | CHFCl | F | 2-F | 3-F | 4-F |
| 1-62 | CHFCl | F | 2-F | 3-F | 5-F |
| 1-63 | CHFCl | F | 2-F | 4-F | 5-F |
| 1-64 | CHFCl | F | 2-F | 4-F | 6-F |
| 1-65 | CHFCl | F | 3-F | 4-F | 5-F |
| 1-66 | CHFCl | F | 3-F | 5-F | 6-F |
| 1-67 | $CF_2Cl$ | F | 2-F | 3-F | 4-F |
| 1-68 | $CF_2Cl$ | F | 2-F | 3-F | 5-F |
| 1-69 | $CF_2Cl$ | F | 2-F | 4-F | 5-F |

| Nr. | $R^1$ | $R^2$ | $R^3$ | $R^4$ | $R^5$ |
|---|---|---|---|---|---|
| 1-70 | $CF_2Cl$ | F | 2-F | 4-F | 6-F |
| 1-71 | $CF_2Cl$ | F | 3-F | 4-F | 5-F |
| 1-72 | $CF_2Cl$ | F | 3-F | 5-F | 6-F |
| 1-73 | $CH_3$ | Cl | 2-F | 3-F | 4-F |
| 1-74 | $CH_3$ | Cl | 2-F | 3-F | 5-F |
| 1-75 | $CH_3$ | Cl | 2-F | 4-F | 5-F |
| 1-76 | $CH_3$ | Cl | 2-F | 4-F | 6-F |
| 1-77 | $CH_3$ | Cl | 3-F | 4-F | 5-F |
| 1-78 | $CH_3$ | Cl | 3-F | 5-F | 6-F |
| 1-79 | $CH_2F$ | Cl | 2-F | 3-F | 4-F |
| 1-80 | $CH_2F$ | Cl | 2-F | 3-F | 5-F |
| 1-81 | $CH_2F$ | Cl | 2-F | 4-F | 5-F |
| 1-82 | $CH_2F$ | Cl | 2-F | 4-F | 6-F |
| 1-83 | $CH_2F$ | Cl | 3-F | 4-F | 5-F |
| 1-84 | $CH_2F$ | Cl | 3-F | 5-F | 6-F |
| 1-85 | $CHF_2$ | Cl | 2-F | 3-F | 4-F |
| 1-86 | $CHF_2$ | Cl | 2-F | 3-F | 5-F |
| 1-87 | $CHF_2$ | Cl | 2-F | 4-F | 5-F |
| 1-88 | $CHF_2$ | Cl | 2-F | 4-F | 6-F |
| 1-89 | $CHF_2$ | Cl | 3-F | 4-F | 5-F |
| 1-90 | $CHF_2$ | Cl | 3-F | 5-F | 6-F |
| 1-91 | $CF_3$ | Cl | 2-F | 3-F | 4-F |
| 1-92 | $CF_3$ | Cl | 2-F | 3-F | 5-F |
| 1-93 | $CF_3$ | Cl | 2-F | 4-F | 5-F |
| 1-94 | $CF_3$ | Cl | 2-F | 4-F | 6-F |
| 1-95 | $CF_3$ | Cl | 3-F | 4-F | 5-F |
| 1-96 | $CF_3$ | Cl | 3-F | 5-F | 6-F |
| 1-97 | CHFCl | Cl | 2-F | 3-F | 4-F |
| 1-98 | CHFCl | Cl | 2-F | 3-F | 5-F |
| 1-99 | CHFCl | Cl | 2-F | 4-F | 5-F |
| 1-100 | CHFCl | Cl | 2-F | 4-F | 6-F |
| 1-101 | CHFCl | Cl | 3-F | 4-F | 5-F |
| 1-102 | CHFCl | Cl | 3-F | 5-F | 6-F |
| 1-103 | $CF_2Cl$ | Cl | 2-F | 3-F | 4-F |
| 1-104 | $CF_2Cl$ | Cl | 2-F | 3-F | 5-F |
| 1-105 | $CF_2Cl$ | Cl | 2-F | 4-F | 5-F |
| 1-106 | $CF_2Cl$ | Cl | 2-F | 4-F | 6-F |
| 1-107 | $CF_2Cl$ | Cl | 3-F | 4-F | 5-F |
| 1-108 | $CF_2Cl$ | Cl | 3-F | 5-F | 6-F |

Bevorzugt sind ferner Verbindungen der Formel I, in der X = O, $R^1 = CF_3$ und $R^2$ = H ist und in der die Variablen $R^3$ bis $R^5$ wie für Formel I definiert sind. Sie entsprechen der Formel Ia

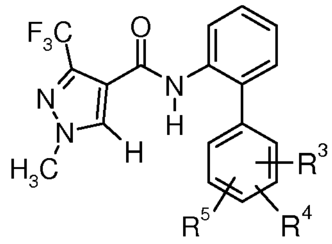

Ia

Bevorzugte Verbindungen Ia sind in Tabelle 2 aufgeführt (Verbindungen 2-1 bis 2-1029). Des Weiteren sind die Verbindungen der Formeln Ib bis If besonders bevorzugt, insbesondere:

- die Verbindungen Ib-1 bis Ib-1029, die sich von den entsprechenden Verbindungen 2-1 bis 2-1029 dadurch unterscheiden, daß $R^2$ für Fluor steht:

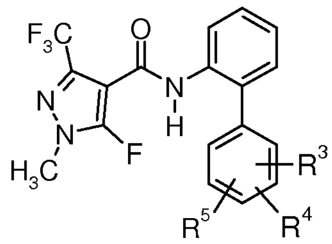

Ib

- die Verbindungen Ic-1 bis Ic-1029, die sich von den entsprechenden Verbindungen 2-1 bis 2-1029 dadurch unterscheiden, daß $R^2$ für Chlor steht:

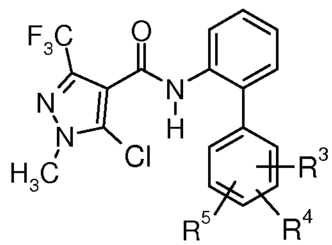

Ic

die Verbindungen Id-1 bis Id-1029, die sich von den entsprechenden Verbindungen 2-1 bis 2-1029 dadurch unterscheiden, daß $R^1$ für Difluormethyl steht:

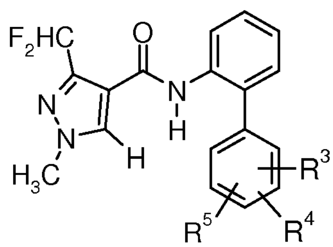

Id

| Verbindung | Fp. [°C] |
|---|---|
| Id-344 | 156-158 |
| Id-687 | 122-125 |
| Id-739 | 120-122 |
| Id-741 | 150-152 |

- die Verbindungen Ie-1 bis Ie-1029, die sich von den entsprechenden Verbindungen 2-1 bis 2-1029 dadurch unterscheiden, daß $R^1$ für Difluormethyl und $R^2$ für Fluor stehen:

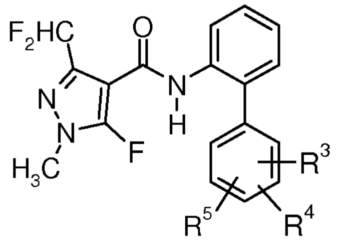

Ie

- die Verbindungen If-1 bis If-1029, die sich von den entsprechenden Verbindungen 2-1 bis 2-1029 dadurch unterscheiden, daß $R^1$ für Difluormethyl und $R^2$ für Chlor stehen:

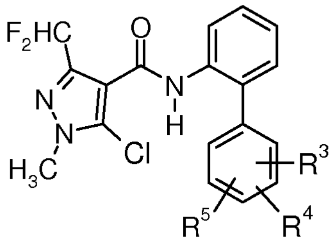

If

- die Verbindungen Ig-1 bis Ig-1029, die sich von den entsprechenden Verbindungen 2-1 bis 2-1029 dadurch unterscheiden, dass $R^1$ für Fluormethyl steht:

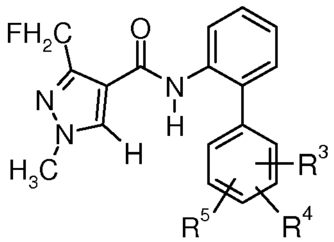

Ig

| Verbindung | Fp. [°C] |
|---|---|
| Ig-1 | 126-129 |
| Ig-344 | 152-156 |

die Verbindungen Ih-1 bis Ih-1029, die sich von den entsprechenden Verbindungen II-1 bis II-1029 dadurch unterscheiden, dass $R^1$ für $CF_2Cl$ steht:

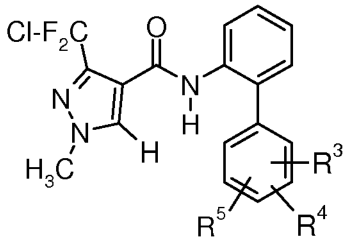

Ih

| Verbindung | Fp. [°C] |
|---|---|
| Ih-344 | 158-161 |

- die Verbindungen Ij-1 bis Ij-1029, die sich von den entsprechenden Verbindungen 2-1 bis 2-1029 dadurch unterscheiden, dass $R^1$ für Chlorfluormethyl steht:

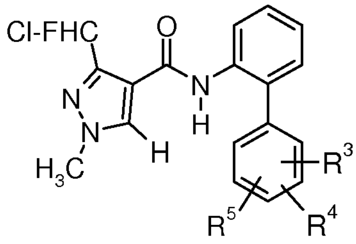

Ij

| Verbindung | Fp. [°C] |
|---|---|
| Ij-344 | 154-157 |

Tabelle 2: (Formel Ia bis Ij)

| Nr. | $R^1$ | $R^2$ | $R^3$ | $R^4$ | $R^5$ |
|---|---|---|---|---|---|
| 2-1 | $CF_3$ | H | 2-F | 3-F | 4-F |
| 2-2 | $CF_3$ | H | 2-F | 3-Cl | 4-F |
| 2-3 | $CF_3$ | H | 2-F | 3-CN | 4-F |
| 2-4 | $CF_3$ | H | 2-F | 3- $CH_3$ | 4-F |
| 2-5 | $CF_3$ | H | 2-F | 3-$CF_3$ | 4-F |
| 2-6 | $CF_3$ | H | 2-F | 3-$OCH_3$ | 4-F |
| 2-7 | $CF_3$ | H | 2-F | 3-$OCF_3$ | 4-F |
| 2-8 | $CF_3$ | H | 2-Cl | 3-F | 4-F |
| 2-9 | $CF_3$ | H | 2-Cl | 3-Cl | 4-F |
| 2-10 | $CF_3$ | H | 2-Cl | 3-CN | 4-F |
| 2-11 | $CF_3$ | H | 2-Cl | 3- $CH_3$ | 4-F |
| 2-12 | $CF_3$ | H | 2-Cl | 3-$CF_3$ | 4-F |
| 2-13 | $CF_3$ | H | 2-Cl | 3-$OCH_3$ | 4-F |
| 2-14 | $CF_3$ | H | 2-Cl | 3-$OCF_3$ | 4-F |
| 2-15 | $CF_3$ | H | 2-CN | 3-F | 4-F |
| 2-16 | $CF_3$ | H | 2-CN | 3-Cl | 4-F |
| 2-17 | $CF_3$ | H | 2-CN | 3-CN | 4-F |

| Nr. | $R^1$ | $R^2$ | $R^3$ | $R^4$ | $R^5$ |
|---|---|---|---|---|---|
| 2-18 | $CF_3$ | H | 2-CN | 3- $CH_3$ | 4-F |
| 2-19 | $CF_3$ | H | 2-CN | 3-$CF_3$ | 4-F |
| 2-20 | $CF_3$ | H | 2-CN | 3-$OCH_3$ | 4-F |
| 2-21 | $CF_3$ | H | 2-CN | 3-$OCF_3$ | 4-F |
| 2-22 | $CF_3$ | H | 2-$CH_3$ | 3-F | 4-F |
| 2-23 | $CF_3$ | H | 2-$CH_3$ | 3-Cl | 4-F |
| 2-24 | $CF_3$ | H | 2-$CH_3$ | 3-CN | 4-F |
| 2-25 | $CF_3$ | H | 2-$CH_3$ | 3- $CH_3$ | 4-F |
| 2-26 | $CF_3$ | H | 2-$CH_3$ | 3-$CF_3$ | 4-F |
| 2-27 | $CF_3$ | H | 2-$CH_3$ | 3-$OCH_3$ | 4-F |
| 2-28 | $CF_3$ | H | 2-$CH_3$ | 3-$OCF_3$ | 4-F |
| 2-29 | $CF_3$ | H | 2-$CF_3$ | 3-F | 4-F |
| 2-30 | $CF_3$ | H | 2-$CF_3$ | 3-Cl | 4-F |
| 2-31 | $CF_3$ | H | 2-$CF_3$ | 3-CN | 4-F |
| 2-32 | $CF_3$ | H | 2-$CF_3$ | 3- $CH_3$ | 4-F |
| 2-33 | $CF_3$ | H | 2-$CF_3$ | 3-$CF_3$ | 4-F |
| 2-34 | $CF_3$ | H | 2-$CF_3$ | 3-$OCH_3$ | 4-F |

| Nr. | $R^1$ | $R^2$ | $R^3$ | $R^4$ | $R^5$ |
|---|---|---|---|---|---|
| 2-35 | $CF_3$ | H | 2-$CF_3$ | 3-$OCF_3$ | 4-F |
| 2-36 | $CF_3$ | H | 2-$OCH_3$ | 3-F | 4-F |
| 2-37 | $CF_3$ | H | 2-$OCH_3$ | 3-Cl | 4-F |
| 2-38 | $CF_3$ | H | 2-$OCH_3$ | 3-CN | 4-F |
| 2-39 | $CF_3$ | H | 2-$OCH_3$ | 3- $CH_3$ | 4-F |
| 2-40 | $CF_3$ | H | 2-$OCH_3$ | 3-$CF_3$ | 4-F |
| 2-41 | $CF_3$ | H | 2-$OCH_3$ | 3-$OCH_3$ | 4-F |
| 2-42 | $CF_3$ | H | 2-$OCH_3$ | 3-$OCF_3$ | 4-F |
| 2-43 | $CF_3$ | H | 2-$OCF_3$ | 3-F | 4-F |
| 2-44 | $CF_3$ | H | 2-$OCF_3$ | 3-Cl | 4-F |
| 2-45 | $CF_3$ | H | 2-$OCF_3$ | 3-CN | 4-F |
| 2-46 | $CF_3$ | H | 2-$OCF_3$ | 3- $CH_3$ | 4-F |
| 2-47 | $CF_3$ | H | 2-$OCF_3$ | 3-$CF_3$ | 4-F |
| 2-48 | $CF_3$ | H | 2-$OCF_3$ | 3-$OCH_3$ | 4-F |
| 2-49 | $CF_3$ | H | 2-$OCF_3$ | 3-$OCF_3$ | 4-F |
| 2-50 | $CF_3$ | H | 2-F | 3-F | 4-Cl |
| 2-51 | $CF_3$ | H | 2-F | 3-Cl | 4-Cl |
| 2-52 | $CF_3$ | H | 2-F | 3-CN | 4-Cl |
| 2-53 | $CF_3$ | H | 2-F | 3- $CH_3$ | 4-Cl |
| 2-54 | $CF_3$ | H | 2-F | 3-$CF_3$ | 4-Cl |
| 2-55 | $CF_3$ | H | 2-F | 3-$OCH_3$ | 4-Cl |
| 2-56 | $CF_3$ | H | 2-F | 3-$OCF_3$ | 4-Cl |
| 2-57 | $CF_3$ | H | 2-Cl | 3-F | 4-Cl |
| 2-58 | $CF_3$ | H | 2-Cl | 3-Cl | 4-Cl |
| 2-59 | $CF_3$ | H | 2-Cl | 3-CN | 4-Cl |
| 2-60 | $CF_3$ | H | 2-Cl | 3- $CH_3$ | 4-Cl |
| 2-61 | $CF_3$ | H | 2-Cl | 3-$CF_3$ | 4-Cl |
| 2-62 | $CF_3$ | H | 2-Cl | 3-$OCH_3$ | 4-Cl |
| 2-63 | $CF_3$ | H | 2-Cl | 3-$OCF_3$ | 4-Cl |
| 2-64 | $CF_3$ | H | 2-CN | 3-F | 4-Cl |
| 2-65 | $CF_3$ | H | 2-CN | 3-Cl | 4-Cl |
| 2-66 | $CF_3$ | H | 2-CN | 3-CN | 4-Cl |
| 2-67 | $CF_3$ | H | 2-CN | 3- $CH_3$ | 4-Cl |
| 2-68 | $CF_3$ | H | 2-CN | 3-$CF_3$ | 4-Cl |
| 2-69 | $CF_3$ | H | 2-CN | 3-$OCH_3$ | 4-Cl |
| 2-70 | $CF_3$ | H | 2-CN | 3-$OCF_3$ | 4-Cl |
| 2-71 | $CF_3$ | H | 2-$CH_3$ | 3-F | 4-Cl |
| 2-72 | $CF_3$ | H | 2-$CH_3$ | 3-Cl | 4-Cl |
| 2-73 | $CF_3$ | H | 2-$CH_3$ | 3-CN | 4-Cl |

| Nr. | $R^1$ | $R^2$ | $R^3$ | $R^4$ | $R^5$ |
|---|---|---|---|---|---|
| 2-74 | $CF_3$ | H | 2-$CH_3$ | 3-$CH_3$ | 4-Cl |
| 2-75 | $CF_3$ | H | 2-$CH_3$ | 3-$CF_3$ | 4-Cl |
| 2-76 | $CF_3$ | H | 2-$CH_3$ | 3-$OCH_3$ | 4-Cl |
| 2-77 | $CF_3$ | H | 2-$CH_3$ | 3-$OCF_3$ | 4-Cl |
| 2-78 | $CF_3$ | H | 2-$CF_3$ | 3-F | 4-Cl |
| 2-79 | $CF_3$ | H | 2-$CF_3$ | 3-Cl | 4-Cl |
| 2-80 | $CF_3$ | H | 2-$CF_3$ | 3-CN | 4-Cl |
| 2-81 | $CF_3$ | H | 2-$CF_3$ | 3- $CH_3$ | 4-Cl |
| 2-82 | $CF_3$ | H | 2-$CF_3$ | 3-$CF_3$ | 4-Cl |
| 2-83 | $CF_3$ | H | 2-$CF_3$ | 3-$OCH_3$ | 4-Cl |
| 2-84 | $CF_3$ | H | 2-$CF_3$ | 3-$OCF_3$ | 4-Cl |
| 2-85 | $CF_3$ | H | 2-$OCH_3$ | 3-F | 4-Cl |
| 2-86 | $CF_3$ | H | 2-$OCH_3$ | 3-Cl | 4-Cl |
| 2-87 | $CF_3$ | H | 2-$OCH_3$ | 3-CN | 4-Cl |
| 2-88 | $CF_3$ | H | 2-$OCH_3$ | 3- $CH_3$ | 4-Cl |
| 2-89 | $CF_3$ | H | 2-$OCH_3$ | 3-$CF_3$ | 4-Cl |
| 2-90 | $CF_3$ | H | 2-$OCH_3$ | 3-$OCH_3$ | 4-Cl |
| 2-91 | $CF_3$ | H | 2-$OCH_3$ | 3-$OCF_3$ | 4-Cl |
| 2-92 | $CF_3$ | H | 2-$OCF_3$ | 3-F | 4-Cl |
| 2-93 | $CF_3$ | H | 2-$OCF_3$ | 3-Cl | 4-Cl |
| 2-94 | $CF_3$ | H | 2-$OCF_3$ | 3-CN | 4-Cl |
| 2-95 | $CF_3$ | H | 2-$OCF_3$ | 3-$CH_3$ | 4-Cl |
| 2-96 | $CF_3$ | H | 2-$OCF_3$ | 3-$CF_3$ | 4-Cl |
| 2-97 | $CF_3$ | H | 2-$OCF_3$ | 3-$OCH_3$ | 4-Cl |
| 2-98 | $CF_3$ | H | 2-$OCF_3$ | 3-$OCF_3$ | 4-Cl |
| 2-99 | $CF_3$ | H | 2-F | 3-F | 4-CN |
| 2-100 | $CF_3$ | H | 2-F | 3-Cl | 4-CN |
| 2-101 | $CF_3$ | H | 2-F | 3-CN | 4-CN |
| 2-102 | $CF_3$ | H | 2-F | 3-$CH_3$ | 4-CN |
| 2-103 | $CF_3$ | H | 2-F | 3-$CF_3$ | 4-CN |
| 2-104 | $CF_3$ | H | 2-F | 3-$OCH_3$ | 4-CN |
| 2-105 | $CF_3$ | H | 2-F | 3-$OCF_3$ | 4-CN |
| 2-106 | $CF_3$ | H | 2-Cl | 3-F | 4-CN |
| 2-107 | $CF_3$ | H | 2-Cl | 3-Cl | 4-CN |
| 2-108 | $CF_3$ | H | 2-Cl | 3-CN | 4-CN |
| 2-109 | $CF_3$ | H | 2-Cl | 3-$CH_3$ | 4-CN |
| 2-110 | $CF_3$ | H | 2-Cl | 3-$CF_3$ | 4-CN |
| 2-111 | $CF_3$ | H | 2-Cl | 3-$OCH_3$ | 4-CN |
| 2-112 | $CF_3$ | H | 2-Cl | 3-$OCF_3$ | 4-CN |

| Nr. | $R^1$ | $R^2$ | $R^3$ | $R^4$ | $R^5$ |
|---|---|---|---|---|---|
| 2-113 | $CF_3$ | H | 2-CN | 3-F | 4-CN |
| 2-114 | $CF_3$ | H | 2-CN | 3-Cl | 4-CN |
| 2-115 | $CF_3$ | H | 2-CN | 3-CN | 4-CN |
| 2-116 | $CF_3$ | H | 2-CN | 3- $CH_3$ | 4-CN |
| 2-117 | $CF_3$ | H | 2-CN | 3-$CF_3$ | 4-CN |
| 2-118 | $CF_3$ | H | 2-CN | 3-$OCH_3$ | 4-CN |
| 2-119 | $CF_3$ | H | 2-CN | 3-$OCF_3$ | 4-CN |
| 2-120 | $CF_3$ | H | 2-$CH_3$ | 3-F | 4-CN |
| 2-121 | $CF_3$ | H | 2-$CH_3$ | 3-Cl | 4-CN |
| 2-122 | $CF_3$ | H | 2-$CH_3$ | 3-CN | 4-CN |
| 2-123 | $CF_3$ | H | 2-$CH_3$ | 3- $CH_3$ | 4-CN |
| 2-124 | $CF_3$ | H | 2-$CH_3$ | 3-$CF_3$ | 4-CN |
| 2-125 | $CF_3$ | H | 2-$CH_3$ | 3-$OCH_3$ | 4-CN |
| 2-126 | $CF_3$ | H | 2-$CH_3$ | 3-$OCF_3$ | 4-CN |
| 2-127 | $CF_3$ | H | 2-$CF_3$ | 3-F | 4-CN |
| 2-128 | $CF_3$ | H | 2-$CF_3$ | 3-Cl | 4-CN |
| 2-129 | $CF_3$ | H | 2-$CF_3$ | 3-CN | 4-CN |
| 2-130 | $CF_3$ | H | 2-$CF_3$ | 3-$CH_3$ | 4-CN |
| 2-131 | $CF_3$ | H | 2-$CF_3$ | 3-$CF_3$ | 4-CN |
| 2-132 | $CF_3$ | H | 2-$CF_3$ | 3-$OCH_3$ | 4-CN |
| 2-133 | $CF_3$ | H | 2-$CF_3$ | 3-$OCF_3$ | 4-CN |
| 2-134 | $CF_3$ | H | 2-$OCH_3$ | 3-F | 4-CN |
| 2-135 | $CF_3$ | H | 2-$OCH_3$ | 3-Cl | 4-CN |
| 2-136 | $CF_3$ | H | 2-$OCH_3$ | 3-CN | 4-CN |
| 2-137 | $CF_3$ | H | 2-$OCH_3$ | 3- $CH_3$ | 4-CN |
| 2-138 | $CF_3$ | H | 2-$OCH_3$ | 3-$CF_3$ | 4-CN |
| 2-139 | $CF_3$ | H | 2-$OCH_3$ | 3-$OCH_3$ | 4-CN |
| 2-140 | $CF_3$ | H | 2-$OCH_3$ | 3-$OCF_3$ | 4-CN |
| 2-141 | $CF_3$ | H | 2-$OCF_3$ | 3-F | 4-CN |
| 2-142 | $CF_3$ | H | 2-$OCF_3$ | 3-Cl | 4-CN |
| 2-143 | $CF_3$ | H | 2-$OCF_3$ | 3-CN | 4-CN |
| 2-144 | $CF_3$ | H | 2-$OCF_3$ | 3-$CH_3$ | 4-CN |
| 2-145 | $CF_3$ | H | 2-$OCF_3$ | 3-$CF_3$ | 4-CN |
| 2-146 | $CF_3$ | H | 2-$OCF_3$ | 3-$OCH_3$ | 4-CN |
| 2-147 | $CF_3$ | H | 2-$OCF_3$ | 3-$OCF_3$ | 4-CN |
| 2-148 | $CF_3$ | H | 2-F | 3-F | 4- $CH_3$ |
| 2-149 | $CF_3$ | H | 2-F | 3-Cl | 4- $CH_3$ |
| 2-150 | $CF_3$ | H | 2-F | 3-CN | 4- $CH_3$ |
| 2-151 | $CF_3$ | H | 2-F | 3-$CH_3$ | 4- $CH_3$ |
| 2-152 | $CF_3$ | H | 2-F | 3-$CF_3$ | 4- $CH_3$ |
| 2-153 | $CF_3$ | H | 2-F | 3-$OCH_3$ | 4- $CH_3$ |
| 2-154 | $CF_3$ | H | 2-F | 3-$OCF_3$ | 4- $CH_3$ |
| 2-155 | $CF_3$ | H | 2-Cl | 3-F | 4- $CH_3$ |
| 2-156 | $CF_3$ | H | 2-Cl | 3-Cl | 4- $CH_3$ |
| 2-157 | $CF_3$ | H | 2-Cl | 3-CN | 4- $CH_3$ |
| 2-158 | $CF_3$ | H | 2-Cl | 3-$CH_3$ | 4- $CH_3$ |
| 2-159 | $CF_3$ | H | 2-Cl | 3-$CF_3$ | 4- $CH_3$ |
| 2-160 | $CF_3$ | H | 2-Cl | 3-$OCH_3$ | 4- $CH_3$ |
| 2-161 | $CF_3$ | H | 2-Cl | 3-$OCF_3$ | 4- $CH_3$ |
| 2-162 | $CF_3$ | H | 2-CN | 3-F | 4- $CH_3$ |
| 2-163 | $CF_3$ | H | 2-CN | 3-Cl | 4- $CH_3$ |
| 2-164 | $CF_3$ | H | 2-CN | 3-CN | 4- $CH_3$ |
| 2-165 | $CF_3$ | H | 2-CN | 3-$CH_3$ | 4- $CH_3$ |
| 2-166 | $CF_3$ | H | 2-CN | 3-$CF_3$ | 4- $CH_3$ |
| 2-167 | $CF_3$ | H | 2-CN | 3-$OCH_3$ | 4- $CH_3$ |
| 2-168 | $CF_3$ | H | 2-CN | 3-$OCF_3$ | 4- $CH_3$ |
| 2-169 | $CF_3$ | H | 2-$CH_3$ | 3-F | 4- $CH_3$ |
| 2-170 | $CF_3$ | H | 2-$CH_3$ | 3-Cl | 4- $CH_3$ |
| 2-171 | $CF_3$ | H | 2-$CH_3$ | 3-CN | 4- $CH_3$ |
| 2-172 | $CF_3$ | H | 2-$CH_3$ | 3-$CH_3$ | 4- $CH_3$ |
| 2-173 | $CF_3$ | H | 2-$CH_3$ | 3-$CF_3$ | 4-$CH_3$ |
| 2-174 | $CF_3$ | H | 2-$CH_3$ | 3-$OCH_3$ | 4- $CH_3$ |
| 2-175 | $CF_3$ | H | 2-$CH_3$ | 3-$OCF_3$ | 4- $CH_3$ |
| 2-176 | $CF_3$ | H | 2-$CF_3$ | 3-F | 4- $CH_3$ |
| 2-177 | $CF_3$ | H | 2-$CF_3$ | 3-Cl | 4- $CH_3$ |
| 2-178 | $CF_3$ | H | 2-$CF_3$ | 3-CN | 4- $CH_3$ |
| 2-179 | $CF_3$ | H | 2-$CF_3$ | 3-$CH_3$ | 4- $CH_3$ |
| 2-180 | $CF_3$ | H | 2-$CF_3$ | 3-$CF_3$ | 4- $CH_3$ |
| 2-181 | $CF_3$ | H | 2-$CF_3$ | 3-$OCH_3$ | 4- $CH_3$ |
| 2-182 | $CF_3$ | H | 2-$CF_3$ | 3-$OCF_3$ | 4- $CH_3$ |
| 2-183 | $CF_3$ | H | 2-$OCH_3$ | 3-F | 4- $CH_3$ |
| 2-184 | $CF_3$ | H | 2-$OCH_3$ | 3-Cl | 4- $CH_3$ |
| 2-185 | $CF_3$ | H | 2-$OCH_3$ | 3-CN | 4- $CH_3$ |
| 2-186 | $CF_3$ | H | 2-$OCH_3$ | 3-$CH_3$ | 4- $CH_3$ |
| 2-187 | $CF_3$ | H | 2-$OCH_3$ | 3-$CF_3$ | 4- $CH_3$ |
| 2-188 | $CF_3$ | H | 2-$OCH_3$ | 3-$OCH_3$ | 4- $CH_3$ |
| 2-189 | $CF_3$ | H | 2-$OCH_3$ | 3-$OCF_3$ | 4- $CH_3$ |
| 2-190 | $CF_3$ | H | 2-$OCF_3$ | 3-F | 4- $CH_3$ |

| Nr. | $R^1$ | $R^2$ | $R^3$ | $R^4$ | $R^5$ |
|---|---|---|---|---|---|
| 2-191 | $CF_3$ | H | 2-$OCF_3$ | 3-Cl | 4- $CH_3$ |
| 2-192 | $CF_3$ | H | 2-$OCF_3$ | 3-CN | 4- $CH_3$ |
| 2-193 | $CF_3$ | H | 2-$OCF_3$ | 3-$CH_3$ | 4- $CH_3$ |
| 2-194 | $CF_3$ | H | 2-$OCF_3$ | 3-$CF_3$ | 4- $CH_3$ |
| 2-195 | $CF_3$ | H | 2-$OCF_3$ | 3-$OCH_3$ | 4- $CH_3$ |
| 2-196 | $CF_3$ | H | 2-$OCF_3$ | 3-$OCF_3$ | 4- $CH_3$ |
| 2-197 | $CF_3$ | H | 2-F | 3-F | 4-CF3 |
| 2-198 | $CF_3$ | H | 2-F | 3-Cl | 4-CF3 |
| 2-199 | $CF_3$ | H | 2-F | 3-CN | 4-CF3 |
| 2-200 | $CF_3$ | H | 2-F | 3-$CH_3$ | 4-CF3 |
| 2-201 | $CF_3$ | H | 2-F | 3-$CF_3$ | 4-CF3 |
| 2-202 | $CF_3$ | H | 2-F | 3-$OCH_3$ | 4-CF3 |
| 2-203 | $CF_3$ | H | 2-F | 3-$OCF_3$ | 4-CF3 |
| 2-204 | $CF_3$ | H | 2-Cl | 3-F | 4-$CF_3$ |
| 2-205 | $CF_3$ | H | 2-Cl | 3-Cl | 4-$CF_3$ |
| 2-206 | $CF_3$ | H | 2-Cl | 3-CN | 4-$CF_3$ |
| 2-207 | $CF_3$ | H | 2-Cl | 3-$CH_3$ | 4-$CF_3$ |
| 2-208 | $CF_3$ | H | 2-Cl | 3-$CF_3$ | 4-$CF_3$ |
| 2-209 | $CF_3$ | H | 2-Cl | 3-$OCH_3$ | 4-$CF_3$ |
| 2-210 | $CF_3$ | H | 2-Cl | 3-$OCF_3$ | 4-$CF_3$ |
| 2-211 | $CF_3$ | H | 2-CN | 3-F | 4-$CF_3$ |
| 2-212 | $CF_3$ | H | 2-CN | 3-Cl | 4-$CF_3$ |
| 2-213 | $CF_3$ | H | 2-CN | 3-CN | 4-$CF_3$ |
| 2-214 | $CF_3$ | H | 2-CN | 3-$CH_3$ | 4-$CF_3$ |
| 2-215 | $CF_3$ | H | 2-CN | 3-$CF_3$ | 4-$CF_3$ |
| 2-216 | $CF_3$ | H | 2-CN | 3-$OCH_3$ | 4-$CF_3$ |
| 2-217 | $CF_3$ | H | 2-CN | 3-$OCF_3$ | 4-$CF_3$ |
| 2-218 | $CF_3$ | H | 2- $CH_3$ | 3-F | 4-CF3 |
| 2-219 | $CF_3$ | H | 2- $CH_3$ | 3-Cl | 4-CF3 |
| 2-220 | $CF_3$ | H | 2- $CH_3$ | 3-CN | 4-CF3 |
| 2-221 | $CF_3$ | H | 2- $CH_3$ | 3-$CH_3$ | 4-CF3 |
| 2-222 | $CF_3$ | H | 2- $CH_3$ | 3-$CF_3$ | 4-CF3 |
| 2-223 | $CF_3$ | H | 2- $CH_3$ | 3-$OCH_3$ | 4-CF3 |
| 2-224 | $CF_3$ | H | 2- $CH_3$ | 3-$OCF_3$ | 4-CF3 |
| 2-225 | $CF_3$ | H | 2-$CF_3$ | 3-F | 4-CF3 |
| 2-226 | $CF_3$ | H | 2-$CF_3$ | 3-Cl | 4-$CF_3$ |
| 2-227 | $CF_3$ | H | 2-$CF_3$ | 3-CN | 4-CF3 |
| 2-228 | $CF_3$ | H | 2-$CF_3$ | 3-$CH_3$ | 4-CF3 |
| 2-229 | $CF_3$ | H | 2-$CF_3$ | 3-$CF_3$ | 4-CF3 |
| 2-230 | $CF_3$ | H | 2-$CF_3$ | 3-$OCH_3$ | 4-CF3 |
| 2-231 | $CF_3$ | H | 2-$CF_3$ | 3-$OCF_3$ | 4-CF3 |
| 2-232 | $CF_3$ | H | 2-$OCH_3$ | 3-F | 4-CF3 |
| 2-233 | $CF_3$ | H | 2-$OCH_3$ | 3-Cl | 4-CF3 |
| 2-234 | $CF_3$ | H | 2-$OCH_3$ | 3-CN | 4-CF3 |
| 2-235 | $CF_3$ | H | 2-$OCH_3$ | 3-$CH_3$ | 4-CF3 |
| 2-236 | $CF_3$ | H | 2-$OCH_3$ | 3-$CF_3$ | 4-CF3 |
| 2-237 | $CF_3$ | H | 2-$OCH_3$ | 3-$OCH_3$ | 4-$CF_3$ |
| 2-238 | $CF_3$ | H | 2-$OCH_3$ | 3-$OCF_3$ | 4-$CF_3$ |
| 2-239 | $CF_3$ | H | 2-$OCF_3$ | 3-F | 4-$CF_3$ |
| 2-240 | $CF_3$ | H | 2-$OCF_3$ | 3-Cl | 4-$CF_3$ |
| 2-241 | $CF_3$ | H | 2-$OCF_3$ | 3-CN | 4-$CF_3$ |
| 2-242 | $CF_3$ | H | 2-$OCF_3$ | 3-$CH_3$ | 4-$CF_3$ |
| 2-243 | $CF_3$ | H | 2-$OCF_3$ | 3-$CF_3$ | 4-$CF_3$ |
| 2-244 | $CF_3$ | H | 2-$OCF_3$ | 3-$OCH_3$ | 4-$CF_3$ |
| 2-245 | $CF_3$ | H | 2-$OCF_3$ | 3-$OCF_3$ | 4-$CF_3$ |
| 2-246 | $CF_3$ | H | 2-F | 3-F | 4-$OCH_3$ |
| 2-247 | $CF_3$ | H | 2-F | 3-Cl | 4-$OCH_3$ |
| 2-248 | $CF_3$ | H | 2-F | 3-CN | 4-$OCH_3$ |
| 2-249 | $CF_3$ | H | 2-F | 3- $CH_3$ | 4-$OCH_3$ |
| 2-250 | $CF_3$ | H | 2-F | 3-$CF_3$ | 4-$OCH_3$ |
| 2-251 | $CF_3$ | H | 2-F | 3-$OCH_3$ | 4-$OCH_3$ |
| 2-252 | $CF_3$ | H | 2-F | 3-$OCF_3$ | 4-$OCH_3$ |
| 2-253 | $CF_3$ | H | 2-Cl | 3-F | 4-$OCH_3$ |
| 2-254 | $CF_3$ | H | 2-Cl | 3-Cl | 4-$OCH_3$ |
| 2-255 | $CF_3$ | H | 2-Cl | 3-CN | 4-$OCH_3$ |
| 2-256 | $CF_3$ | H | 2-Cl | 3- $CH_3$ | 4-$OCH_3$ |
| 2-257 | $CF_3$ | H | 2-Cl | 3-$CF_3$ | 4-$OCH_3$ |
| 2-258 | $CF_3$ | H | 2-Cl | 3-$OCH_3$ | 4-$OCH_3$ |
| 2-259 | $CF_3$ | H | 2-Cl | 3-$OCF_3$ | 4-$OCH_3$ |
| 2-260 | $CF_3$ | H | 2-CN | 3-F | 4-$OCH_3$ |
| 2-261 | $CF_3$ | H | 2-CN | 3-Cl | 4-$OCH_3$ |
| 2-262 | $CF_3$ | H | 2-CN | 3-CN | 4-$OCH_3$ |
| 2-263 | $CF_3$ | H | 2-CN | 3- $CH_3$ | 4-$OCH_3$ |
| 2-264 | $CF_3$ | H | 2-CN | 3-$CF_3$ | 4-$OCH_3$ |
| 2-265 | $CF_3$ | H | 2-CN | 3-$OCH_3$ | 4-$OCH_3$ |
| 2-266 | $CF_3$ | H | 2-CN | 3-$OCF_3$ | 4-$OCH_3$ |
| 2-267 | $CF_3$ | H | 2-$CH_3$ | 3-F | 4-$OCH_3$ |
| 2-268 | $CF_3$ | H | 2-$CH_3$ | 3-Cl | 4-$OCH_3$ |

| Nr. | $R^1$ | $R^2$ | $R^3$ | $R^4$ | $R^5$ |
|---|---|---|---|---|---|
| 2-269 | $CF_3$ | H | 2-$CH_3$ | 3-CN | 4-$OCH_3$ |
| 2-270 | $CF_3$ | H | 2-$CH_3$ | 3- $CH_3$ | 4-$OCH_3$ |
| 2-271 | $CF_3$ | H | 2-$CH_3$ | 3-$CF_3$ | 4-$OCH_3$ |
| 2-272 | $CF_3$ | H | 2-$CH_3$ | 3-$OCH_3$ | 4-$OCH_3$ |
| 2-273 | $CF_3$ | H | 2-$CH_3$ | 3-$OCF_3$ | 4-$OCH_3$ |
| 2-274 | $CF_3$ | H | 2-$CF_3$ | 3-F | 4-$OCH_3$ |
| 2-275 | $CF_3$ | H | 2-$CF_3$ | 3-Cl | 4-$OCH_3$ |
| 2-276 | $CF_3$ | H | 2-$CF_3$ | 3-CN | 4-$OCH_3$ |
| 2-277 | $CF_3$ | H | 2-$CF_3$ | 3-$CH_3$ | 4-$OCH_3$ |
| 2-278 | $CF_3$ | H | 2-$CF_3$ | 3-$CF_3$ | 4-$OCH_3$ |
| 2-279 | $CF_3$ | H | 2-$CF_3$ | 3-$OCH_3$ | 4-$OCH_3$ |
| 2-280 | $CF_3$ | H | 2-$CF_3$ | 3-$OCF_3$ | 4-$OCH_3$ |
| 2-281 | $CF_3$ | H | 2-$OCH_3$ | 3-F | 4-$OCH_3$ |
| 2-282 | $CF_3$ | H | 2-$OCH_3$ | 3-Cl | 4-$OCH_3$ |
| 2-283 | $CF_3$ | H | 2-$OCH_3$ | 3-CN | 4-$OCH_3$ |
| 2-284 | $CF_3$ | H | 2-$OCH_3$ | 3- $CH_3$ | 4-$OCH_3$ |
| 2-285 | $CF_3$ | H | 2-$OCH_3$ | 3-$CF_3$ | 4-$OCH_3$ |
| 2-286 | $CF_3$ | H | 2-$OCH_3$ | 3-$OCH_3$ | 4-$OCH_3$ |
| 2-287 | $CF_3$ | H | 2-$OCH_3$ | 3-$OCF_3$ | 4-$OCH_3$ |
| 2-288 | $CF_3$ | H | 2-$OCF_3$ | 3-F | 4-$OCH_3$ |
| 2-289 | $CF_3$ | H | 2-$OCF_3$ | 3-Cl | 4-$OCH_3$ |
| 2-290 | $CF_3$ | H | 2-$OCF_3$ | 3-CN | 4-$OCH_3$ |
| 2-291 | $CF_3$ | H | 2-$OCF_3$ | 3- $CH_3$ | 4-$OCH_3$ |
| 2-292 | $CF_3$ | H | 2-$OCF_3$ | 3-$CF_3$ | 4-$OCH_3$ |
| 2-293 | $CF_3$ | H | 2-$OCF_3$ | 3-$OCH_3$ | 4-$OCH_3$ |
| 2-294 | $CF_3$ | H | 2-$OCF_3$ | 3-$OCF_3$ | 4-$OCH_3$ |
| 2-295 | $CF_3$ | H | 2-F | 3-F | 4-$OCF_3$ |
| 2-296 | $CF_3$ | H | 2-F | 3-Cl | 4-$OCF_3$ |
| 2-297 | $CF_3$ | H | 2-F | 3-CN | 4-$OCF_3$ |
| 2-298 | $CF_3$ | H | 2-F | 3- $CH_3$ | 4-$OCF_3$ |
| 2-299 | $CF_3$ | H | 2-F | 3-$CF_3$ | 4-$OCF_3$ |
| 2-300 | $CF_3$ | H | 2-F | 3-$OCH_3$ | 4-$OCF_3$ |
| 2-301 | $CF_3$ | H | 2-F | 3-$OCF_3$ | 4-$OCF_3$ |
| 2-302 | $CF_3$ | H | 2-Cl | 3-F | 4-$OCF_3$ |
| 2-303 | $CF_3$ | H | 2-Cl | 3-Cl | 4-$OCF_3$ |
| 2-304 | $CF_3$ | H | 2-Cl | 3-CN | 4-$OCF_3$ |
| 2-305 | $CF_3$ | H | 2-Cl | 3- $CH_3$ | 4-$OCF_3$ |
| 2-306 | $CF_3$ | H | 2-Cl | 3-$CF_3$ | 4-$OCF_3$ |
| 2-307 | $CF_3$ | H | 2-Cl | 3-$OCH_3$ | 4-$OCF_3$ |

| Nr. | $R^1$ | $R^2$ | $R^3$ | $R^4$ | $R^5$ |
|---|---|---|---|---|---|
| 2-308 | $CF_3$ | H | 2-Cl | 3-$OCF_3$ | 4-$OCF_3$ |
| 2-309 | $CF_3$ | H | 2-CN | 3-F | 4-$OCF_3$ |
| 2-310 | $CF_3$ | H | 2-CN | 3-Cl | 4-$OCF_3$ |
| 2-311 | $CF_3$ | H | 2-CN | 3-CN | 4-$OCF_3$ |
| 2-312 | $CF_3$ | H | 2-CN | 3- $CH_3$ | 4-$OCF_3$ |
| 2-313 | $CF_3$ | H | 2-CN | 3-$CF_3$ | 4-$OCF_3$ |
| 2-314 | $CF_3$ | H | 2-CN | 3-$OCH_3$ | 4-$OCF_3$ |
| 2-315 | $CF_3$ | H | 2-CN | 3-$OCF_3$ | 4-$OCF_3$ |
| 2-316 | $CF_3$ | H | 2-$CH_3$ | 3-F | 4-$OCF_3$ |
| 2-317 | $CF_3$ | H | 2-$CH_3$ | 3-Cl | 4-$OCF_3$ |
| 2-318 | $CF_3$ | H | 2-$CH_3$ | 3-CN | 4-$OCF_3$ |
| 2-319 | $CF_3$ | H | 2-$CH_3$ | 3- $CH_3$ | 4-$OCF_3$ |
| 2-320 | $CF_3$ | H | 2-$CH_3$ | 3-$CF_3$ | 4-$OCF_3$ |
| 2-321 | $CF_3$ | H | 2-$CH_3$ | 3-$OCH_3$ | 4-$OCF_3$ |
| 2-322 | $CF_3$ | H | 2-$CH_3$ | 3-$OCF_3$ | 4-$OCF_3$ |
| 2-323 | $CF_3$ | H | 2-$CF_3$ | 3-F | 4-$OCF_3$ |
| 2-324 | $CF_3$ | H | 2-$CF_3$ | 3-Cl | 4-$OCF_3$ |
| 2-325 | $CF_3$ | H | 2-$CF_3$ | 3-CN | 4-$OCF_3$ |
| 2-326 | $CF_3$ | H | 2-$CF_3$ | 3- $CH_3$ | 4-$OCF_3$ |
| 2-327 | $CF_3$ | H | 2-$CF_3$ | 3-$CF_3$ | 4-$OCF_3$ |
| 2-328 | $CF_3$ | H | 2-$CF_3$ | 3-$OCH_3$ | 4-$OCF_3$ |
| 2-329 | $CF_3$ | H | 2-$CF_3$ | 3-$OCF_3$ | 4-$OCF_3$ |
| 2-330 | $CF_3$ | H | 2-$OCH_3$ | 3-F | 4-$OCF_3$ |
| 2-331 | $CF_3$ | H | 2-$OCH_3$ | 3-Cl | 4-$OCF_3$ |
| 2-332 | $CF_3$ | H | 2-$OCH_3$ | 3-CN | 4-$OCF_3$ |
| 2-333 | $CF_3$ | H | 2-$OCH_3$ | 3- $CH_3$ | 4-$OCF_3$ |
| 2-334 | $CF_3$ | H | 2-$OCH_3$ | 3-$CF_3$ | 4-$OCF_3$ |
| 2-335 | $CF_3$ | H | 2-$OCH_3$ | 3-$OCH_3$ | 4-$OCF_3$ |
| 2-336 | $CF_3$ | H | 2-$OCH_3$ | 3-$OCF_3$ | 4-$OCF_3$ |
| 2-337 | $CF_3$ | H | 2-$OCF_3$ | 3-F | 4-$OCF_3$ |
| 2-338 | $CF_3$ | H | 2-$OCF_3$ | 3-Cl | 4-$OCF_3$ |
| 2-339 | $CF_3$ | H | 2-$OCF_3$ | 3-CN | 4-$OCF_3$ |
| 2-340 | $CF_3$ | H | 2-$OCF_3$ | 3- $CH_3$ | 4-$OCF_3$ |
| 2-341 | $CF_3$ | H | 2-$OCF_3$ | 3-$CF_3$ | 4-$OCF_3$ |
| 2-342 | $CF_3$ | H | 2-$OCF_3$ | 3-$OCH_3$ | 4-$OCF_3$ |
| 2-343 | $CF_3$ | H | 2-$OCF_3$ | 3-$OCF_3$ | 4-$OCF_3$ |
| 2-344 | $CF_3$ | H | 3-F | 4-F | 5-F |
| 2-345 | $CF_3$ | H | 3-Cl | 4-F | 5-F |
| 2-346 | $CF_3$ | H | 3-CN | 4-F | 5-F |

| Nr. | $R^1$ | $R^2$ | $R^3$ | $R^4$ | $R^5$ |
|---|---|---|---|---|---|
| 2-347 | $CF_3$ | H | 3-$CH_3$ | 4-F | 5-F |
| 2-348 | $CF_3$ | H | 3-$CF_3$ | 4-F | 5-F |
| 2-349 | $CF_3$ | H | 3-$OCH_3$ | 4-F | 5-F |
| 2-350 | $CF_3$ | H | 3-$OCF_3$ | 4-F | 5-F |
| 2-351 | $CF_3$ | H | 3-F | 4-F | 5-Cl |
| 2-352 | $CF_3$ | H | 3-Cl | 4-F | 5-Cl |
| 2-353 | $CF_3$ | H | 3-CN | 4-F | 5-Cl |
| 2-354 | $CF_3$ | H | 3-$CH_3$ | 4-F | 5-Cl |
| 2-355 | $CF_3$ | H | 3-$CF_3$ | 4-F | 5-Cl |
| 2-356 | $CF_3$ | H | 3-$OCH_3$ | 4-F | 5-Cl |
| 2-357 | $CF_3$ | H | 3-$OCF_3$ | 4-F | 5-Cl |
| 2-358 | $CF_3$ | H | 3-F | 4-F | 5-CN |
| 2-359 | $CF_3$ | H | 3-Cl | 4-F | 5-CN |
| 2-360 | $CF_3$ | H | 3-CN | 4-F | 5-CN |
| 2-361 | $CF_3$ | H | 3-$CH_3$ | 4-F | 5-CN |
| 2-362 | $CF_3$ | H | 3-$CF_3$ | 4-F | 5-CN |
| 2-363 | $CF_3$ | H | 3-$OCH_3$ | 4-F | 5-CN |
| 2-364 | $CF_3$ | H | 3-$OCF_3$ | 4-F | 5-CN |
| 2-365 | $CF_3$ | H | 3-F | 4-F | 5-$CH_3$ |
| 2-366 | $CF_3$ | H | 3-Cl | 4-F | 5-$CH_3$ |
| 2-367 | $CF_3$ | H | 3-CN | 4-F | 5-$CH_3$ |
| 2-368 | $CF_3$ | H | 3-$CH_3$ | 4-F | 5-$CH_3$ |
| 2-369 | $CF_3$ | H | 3-$CF_3$ | 4-F | 5-$CH_3$ |
| 2-370 | $CF_3$ | H | 3-$OCH_3$ | 4-F | 5-$CH_3$ |
| 2-371 | $CF_3$ | H | 3-$OCF_3$ | 4-F | 5-$CH_3$ |
| 2-372 | $CF_3$ | H | 3-F | 4-F | 5-$CF_3$ |
| 2-373 | $CF_3$ | H | 3-Cl | 4-F | 5-$CF_3$ |
| 2-374 | $CF_3$ | H | 3-CN | 4-F | 5-$CF_3$ |
| 2-375 | $CF_3$ | H | 3-$CH_3$ | 4-F | 5-$CF_3$ |
| 2-376 | $CF_3$ | H | 3-$CF_3$ | 4-F | 5-$CF_3$ |
| 2-377 | $CF_3$ | H | 3-$OCH_3$ | 4-F | 5-$CF_3$ |
| 2-378 | $CF_3$ | H | 3-$OCF_3$ | 4-F | 5-$CF_3$ |
| 2-379 | $CF_3$ | H | 3-F | 4-F | 5-$OCH_3$ |
| 2-380 | $CF_3$ | H | 3-Cl | 4-F | 5-$OCH_3$ |
| 2-381 | $CF_3$ | H | 3-CN | 4-F | 5-$OCH_3$ |
| 2-382 | $CF_3$ | H | 3-$CH_3$ | 4-F | 5-$OCH_3$ |
| 2-383 | $CF_3$ | H | 3-$CF_3$ | 4-F | 5-$OCH_3$ |
| 2-384 | $CF_3$ | H | 3-$OCH_3$ | 4-F | 5-$OCH_3$ |
| 2-385 | $CF_3$ | H | 3-$OCF_3$ | 4-F | 5-$OCH_3$ |
| 2-386 | $CF_3$ | H | 3-F | 4-F | 5-$OCF_3$ |
| 2-387 | $CF_3$ | H | 3-Cl | 4-F | 5-$OCF_3$ |
| 2-388 | $CF_3$ | H | 3-CN | 4-F | 5-$OCF_3$ |
| 2-389 | $CF_3$ | H | 3-$CH_3$ | 4-F | 5-$OCF_3$ |
| 2-390 | $CF_3$ | H | 3-$CF_3$ | 4-F | 5-$OCF_3$ |
| 2-391 | $CF_3$ | H | 3-$OCH_3$ | 4-F | 5-$OCF_3$ |
| 2-392 | $CF_3$ | H | 3-$OCF_3$ | 4-F | 5-$OCF_3$ |
| 2-393 | $CF_3$ | H | 3-F | 4-Cl | 5-F |
| 2-394 | $CF_3$ | H | 3-Cl | 4-Cl | 5-F |
| 2-395 | $CF_3$ | H | 3-CN | 4-Cl | 5-F |
| 2-396 | $CF_3$ | H | 3-$CH_3$ | 4-Cl | 5-F |
| 2-397 | $CF_3$ | H | 3-$CF_3$ | 4-Cl | 5-F |
| 2-398 | $CF_3$ | H | 3-$OCH_3$ | 4-Cl | 5-F |
| 2-399 | $CF_3$ | H | 3-$OCF_3$ | 4-Cl | 5-F |
| 2-400 | $CF_3$ | H | 3-F | 4-Cl | 5-Cl |
| 2-401 | $CF_3$ | H | 3-Cl | 4-Cl | 5-Cl |
| 2-402 | $CF_3$ | H | 3-CN | 4-Cl | 5-Cl |
| 2-403 | $CF_3$ | H | 3-$CH_3$ | 4-Cl | 5-Cl |
| 2-404 | $CF_3$ | H | 3-$CF_3$ | 4-Cl | 5-Cl |
| 2-405 | $CF_3$ | H | 3-$OCH_3$ | 4-Cl | 5-Cl |
| 2-406 | $CF_3$ | H | 3-$OCF_3$ | 4-Cl | 5-Cl |
| 2-407 | $CF_3$ | H | 3-F | 4-Cl | 5-CN |
| 2-408 | $CF_3$ | H | 3-Cl | 4-Cl | 5-CN |
| 2-409 | $CF_3$ | H | 3-CN | 4-Cl | 5-CN |
| 2-410 | $CF_3$ | H | 3-$CH_3$ | 4-Cl | 5-CN |
| 2-411 | $CF_3$ | H | 3-$CF_3$ | 4-Cl | 5-CN |
| 2-412 | $CF_3$ | H | 3-$OCH_3$ | 4-Cl | 5-CN |
| 2-413 | $CF_3$ | H | 3-$OCF_3$ | 4-Cl | 5-CN |
| 2-414 | $CF_3$ | H | 3-F | 4-Cl | 5-$CH_3$ |
| 2-415 | $CF_3$ | H | 3-Cl | 4-Cl | 5-$CH_3$ |
| 2-416 | $CF_3$ | H | 3-CN | 4-Cl | 5-$CH_3$ |
| 2-417 | $CF_3$ | H | 3-$CH_3$ | 4-Cl | 5-$CH_3$ |
| 2-418 | $CF_3$ | H | 3-$CF_3$ | 4-Cl | 5-$CH_3$ |
| 2-419 | $CF_3$ | H | 3-$OCH_3$ | 4-Cl | 5-$CH_3$ |
| 2-420 | $CF_3$ | H | 3-$OCF_3$ | 4-Cl | 5-$CH_3$ |
| 2-421 | $CF_3$ | H | 3-F | 4-Cl | 5-$CF_3$ |
| 2-422 | $CF_3$ | H | 3-Cl | 4-Cl | 5-$CF_3$ |
| 2-423 | $CF_3$ | H | 3-CN | 4-Cl | 5-$CF_3$ |
| 2-424 | $CF_3$ | H | 3-$CH_3$ | 4-Cl | 5-$CF_3$ |

| Nr. | $R^1$ | $R^2$ | $R^3$ | $R^4$ | $R^5$ |
|---|---|---|---|---|---|
| 2-425 | $CF_3$ | H | 3-$CF_3$ | 4-Cl | 5-$CF_3$ |
| 2-426 | $CF_3$ | H | 3-$OCH_3$ | 4-Cl | 5-$CF_3$ |
| 2-427 | $CF_3$ | H | 3-$OCF_3$ | 4-Cl | 5-$CF_3$ |
| 2-428 | $CF_3$ | H | 3-F | 4-Cl | 5-$OCH_3$ |
| 2-429 | $CF_3$ | H | 3-Cl | 4-Cl | 5-$OCH_3$ |
| 2-430 | $CF_3$ | H | 3-CN | 4-Cl | 5-$OCH_3$ |
| 2-431 | $CF_3$ | H | 3-$CH_3$ | 4-Cl | 5-$OCH_3$ |
| 2-432 | $CF_3$ | H | 3-$CF_3$ | 4-Cl | 5-$OCH_3$ |
| 2-433 | $CF_3$ | H | 3-$OCH_3$ | 4-Cl | 5-$OCH_3$ |
| 2-434 | $CF_3$ | H | 3-$OCF_3$ | 4-Cl | 5-$OCH_3$ |
| 2-435 | $CF_3$ | H | 3-F | 4-Cl | 5-$OCF_3$ |
| 2-436 | $CF_3$ | H | 3-Cl | 4-Cl | 5-$OCF_3$ |
| 2-437 | $CF_3$ | H | 3-CN | 4-Cl | 5-$OCF_3$ |
| 2-438 | $CF_3$ | H | 3-$CH_3$ | 4-Cl | 5-$OCF_3$ |
| 2-439 | $CF_3$ | H | 3-$CF_3$ | 4-Cl | 5-$OCF_3$ |
| 2-440 | $CF_3$ | H | 3-$OCH_3$ | 4-Cl | 5-$OCF_3$ |
| 2-441 | $CF_3$ | H | 3-$OCF_3$ | 4-Cl | 5-$OCF_3$ |
| 2-442 | $CF_3$ | H | 3-F | 4-CN | 5-F |
| 2-443 | $CF_3$ | H | 3-Cl | 4-CN | 5-F |
| 2-444 | $CF_3$ | H | 3-CN | 4-CN | 5-F |
| 2-445 | $CF_3$ | H | 3-$CH_3$ | 4-CN | 5-F |
| 2-446 | $CF_3$ | H | 3-$CF_3$ | 4-CN | 5-F |
| 2-447 | $CF_3$ | H | 3-$OCH_3$ | 4-CN | 5-F |
| 2-448 | $CF_3$ | H | 3-$OCF_3$ | 4-CN | 5-F |
| 2-449 | $CF_3$ | H | 3-F | 4-CN | 5-Cl |
| 2-450 | $CF_3$ | H | 3-Cl | 4-CN | 5-Cl |
| 2-451 | $CF_3$ | H | 3-CN | 4-CN | 5-Cl |
| 2-452 | $CF_3$ | H | 3-$CH_3$ | 4-CN | 5-Cl |
| 2-453 | $CF_3$ | H | 3-$CF_3$ | 4-CN | 5-Cl |
| 2-454 | $CF_3$ | H | 3-$OCH_3$ | 4-CN | 5-Cl |
| 2-455 | $CF_3$ | H | 3-$OCF_3$ | 4-CN | 5-Cl |
| 2-456 | $CF_3$ | H | 3-F | 4-CN | 5-CN |
| 2-457 | $CF_3$ | H | 3-Cl | 4-CN | 5-CN |
| 2-458 | $CF_3$ | H | 3-CN | 4-CN | 5-CN |
| 2-459 | $CF_3$ | H | 3-$CH_3$ | 4-CN | 5-CN |
| 2-460 | $CF_3$ | H | 3-$CF_3$ | 4-CN | 5-CN |
| 2-461 | $CF_3$ | H | 3-$OCH_3$ | 4-CN | 5-CN |
| 2-462 | $CF_3$ | H | 3-$OCF_3$ | 4-CN | 5-CN |
| 2-463 | $CF_3$ | H | 3-F | 4-CN | 5-$CH_3$ |
| 2-464 | $CF_3$ | H | 3-Cl | 4-CN | 5-$CH_3$ |
| 2-465 | $CF_3$ | H | 3-CN | 4-CN | 5-$CH_3$ |
| 2-466 | $CF_3$ | H | 3-$CH_3$ | 4-CN | 5-$CH_3$ |
| 2-467 | $CF_3$ | H | 3-$CF_3$ | 4-CN | 5-$CH_3$ |
| 2-468 | $CF_3$ | H | 3-$OCH_3$ | 4-CN | 5-$CH_3$ |
| 2-469 | $CF_3$ | H | 3-$OCF_3$ | 4-CN | 5-$CH_3$ |
| 2-470 | $CF_3$ | H | 3-F | 4-CN | 5-$CF_3$ |
| 2-471 | $CF_3$ | H | 3-Cl | 4-CN | 5-$CF_3$ |
| 2-472 | $CF_3$ | H | 3-CN | 4-CN | 5-$CF_3$ |
| 2-473 | $CF_3$ | H | 3-$CH_3$ | 4-CN | 5-$CF_3$ |
| 2-474 | $CF_3$ | H | 3-$CF_3$ | 4-CN | 5-$CF_3$ |
| 2-475 | $CF_3$ | H | 3-$OCH_3$ | 4-CN | 5-$CF_3$ |
| 2-476 | $CF_3$ | H | 3-$OCF_3$ | 4-CN | 5-$CF_3$ |
| 2-477 | $CF_3$ | H | 3-F | 4-CN | 5-$OCH_3$ |
| 2-478 | $CF_3$ | H | 3-Cl | 4-CN | 5-$OCH_3$ |
| 2-479 | $CF_3$ | H | 3-CN | 4-CN | 5-$OCH_3$ |
| 2-480 | $CF_3$ | H | 3-$CH_3$ | 4-CN | 5-$OCH_3$ |
| 2-481 | $CF_3$ | H | 3-$CF_3$ | 4-CN | 5-$OCH_3$ |
| 2-482 | $CF_3$ | H | 3-$OCH_3$ | 4-CN | 5-$OCH_3$ |
| 2-483 | $CF_3$ | H | 3-$OCF_3$ | 4-CN | 5-$OCH_3$ |
| 2-484 | $CF_3$ | H | 3-F | 4-CN | 5-$OCF_3$ |
| 2-485 | $CF_3$ | H | 3-Cl | 4-CN | 5-$OCF_3$ |
| 2-486 | $CF_3$ | H | 3-CN | 4-CN | 5-$OCF_3$ |
| 2-487 | $CF_3$ | H | 3-$CH_3$ | 4-CN | 5-$OCF_3$ |
| 2-488 | $CF_3$ | H | 3-$CF_3$ | 4-CN | 5-$OCF_3$ |
| 2-489 | $CF_3$ | H | 3-$OCH_3$ | 4-CN | 5-$OCF_3$ |
| 2-490 | $CF_3$ | H | 3-$OCF_3$ | 4-CN | 5-$OCF_3$ |
| 2-491 | $CF_3$ | H | 3-F | 4-$CH_3$ | 5-F |
| 2-492 | $CF_3$ | H | 3-Cl | 4-$CH_3$ | 5-F |
| 2-493 | $CF_3$ | H | 3-CN | 4-$CH_3$ | 5-F |
| 2-494 | $CF_3$ | H | 3-$CH_3$ | 4-$CH_3$ | 5-F |
| 2-495 | $CF_3$ | H | 3-$CF_3$ | 4-$CH_3$ | 5-F |
| 2-496 | $CF_3$ | H | 3-$OCH_3$ | 4-$CH_3$ | 5-F |
| 2-497 | $CF_3$ | H | 3-$OCF_3$ | 4-$CH_3$ | 5-F |
| 2-498 | $CF_3$ | H | 3-F | 4-$CH_3$ | 5-Cl |
| 2-499 | $CF_3$ | H | 3-Cl | 4-$CH_3$ | 5-Cl |
| 2-500 | $CF_3$ | H | 3-CN | 4-$CH_3$ | 5-Cl |
| 2-501 | $CF_3$ | H | 3-$CH_3$ | 4-$CH_3$ | 5-Cl |
| 2-502 | $CF_3$ | H | 3-$CF_3$ | 4-$CH_3$ | 5-Cl |

| Nr. | $R^1$ | $R^2$ | $R^3$ | $R^4$ | $R^5$ |
|---|---|---|---|---|---|
| 2-503 | $CF_3$ | H | 3-$OCH_3$ | 4-$CH_3$ | 5-Cl |
| 2-504 | $CF_3$ | H | 3-$OCF_3$ | 4-$CH_3$ | 5-Cl |
| 2-505 | $CF_3$ | H | 3-F | 4-$CH_3$ | 5-CN |
| 2-506 | $CF_3$ | H | 3-Cl | 4-$CH_3$ | 5-CN |
| 2-507 | $CF_3$ | H | 3-CN | 4-$CH_3$ | 5-CN |
| 2-508 | $CF_3$ | H | 3-$CH_3$ | 4-$CH_3$ | 5-CN |
| 2-509 | $CF_3$ | H | 3-$CF_3$ | 4-$CH_3$ | 5-CN |
| 2-510 | $CF_3$ | H | 3-$OCH_3$ | 4-$CH_3$ | 5-CN |
| 2-511 | $CF_3$ | H | 3-$OCF_3$ | 4-$CH_3$ | 5-CN |
| 2-512 | $CF_3$ | H | 3-F | 4-$CH_3$ | 5-$CH_3$ |
| 2-513 | $CF_3$ | H | 3-Cl | 4-$CH_3$ | 5-$CH_3$ |
| 2-514 | $CF_3$ | H | 3-CN | 4-$CH_3$ | 5-$CH_3$ |
| 2-515 | $CF_3$ | H | 3-$CH_3$ | 4-$CH_3$ | 5-$CH_3$ |
| 2-516 | $CF_3$ | H | 3-$CF_3$ | 4-$CH_3$ | 5-$CH_3$ |
| 2-517 | $CF_3$ | H | 3-$OCH_3$ | 4-$CH_3$ | 5-$CH_3$ |
| 2-518 | $CF_3$ | H | 3-$OCF_3$ | 4-$CH_3$ | 5-$CH_3$ |
| 2-519 | $CF_3$ | H | 3-F | 4-$CH_3$ | 5-$CF_3$ |
| 2-520 | $CF_3$ | H | 3-Cl | 4-$CH_3$ | 5-$CF_3$ |
| 2-521 | $CF_3$ | H | 3-CN | 4-$CH_3$ | 5-$CF_3$ |
| 2-522 | $CF_3$ | H | 3-$CH_3$ | 4-$CH_3$ | 5-$CF_3$ |
| 2-523 | $CF_3$ | H | 3-$CF_3$ | 4-$CH_3$ | 5-$CF_3$ |
| 2-524 | $CF_3$ | H | 3-$OCH_3$ | 4-$CH_3$ | 5-$CF_3$ |
| 2-525 | $CF_3$ | H | 3-$OCF_3$ | 4-$CH_3$ | 5-$CF_3$ |
| 2-526 | $CF_3$ | H | 3-F | 4-$CH_3$ | 5-$OCH_3$ |
| 2-527 | $CF_3$ | H | 3-Cl | 4-$CH_3$ | 5-$OCH_3$ |
| 2-528 | $CF_3$ | H | 3-CN | 4-$CH_3$ | 5-$OCH_3$ |
| 2-529 | $CF_3$ | H | 3-$CH_3$ | 4-$CH_3$ | 5-$OCH_3$ |
| 2-530 | $CF_3$ | H | 3-$CF_3$ | 4-$CH_3$ | 5-$OCH_3$ |
| 2-531 | $CF_3$ | H | 3-$OCH_3$ | 4-$CH_3$ | 5-$OCH_3$ |
| 2-532 | $CF_3$ | H | 3-$OCF_3$ | 4-$CH_3$ | 5-$OCH_3$ |
| 2-533 | $CF_3$ | H | 3-F | 4-$CH_3$ | 5-$OCF_3$ |
| 2-534 | $CF_3$ | H | 3-Cl | 4-$CH_3$ | 5-$OCF_3$ |
| 2-535 | $CF_3$ | H | 3-CN | 4-$CH_3$ | 5-$OCF_3$ |
| 2-536 | $CF_3$ | H | 3-$CH_3$ | 4-$CH_3$ | 5-$OCF_3$ |
| 2-537 | $CF_3$ | H | 3-$CF_3$ | 4-$CH_3$ | 5-$OCF_3$ |
| 2-538 | $CF_3$ | H | 3-$OCH_3$ | 4-$CH_3$ | 5-$OCF_3$ |
| 2-539 | $CF_3$ | H | 3-$OCF_3$ | 4-$CH_3$ | 5-$OCF_3$ |
| 2-540 | $CF_3$ | H | 3-F | 4-$CF_3$ | 5-F |
| 2-541 | $CF_3$ | H | 3-Cl | 4-$CF_3$ | 5-F |

| Nr. | $R^1$ | $R^2$ | $R^3$ | $R^4$ | $R^5$ |
|---|---|---|---|---|---|
| 2-542 | $CF_3$ | H | 3-CN | 4-$CF_3$ | 5-F |
| 2-543 | $CF_3$ | H | 3-$CH_3$ | 4-$CF_3$ | 5-F |
| 2-544 | $CF_3$ | H | 3-$CF_3$ | 4-$CF_3$ | 5-F |
| 2-545 | $CF_3$ | H | 3-$OCH_3$ | 4-$CF_3$ | 5-F |
| 2-546 | $CF_3$ | H | 3-$OCF_3$ | 4-$CF_3$ | 5-F |
| 2-547 | $CF_3$ | H | 3-F | 4-$CF_3$ | 5-Cl |
| 2-548 | $CF_3$ | H | 3-Cl | 4-$CF_3$ | 5-Cl |
| 2-549 | $CF_3$ | H | 3-CN | 4-$CF_3$ | 5-Cl |
| 2-550 | $CF_3$ | H | 3-$CH_3$ | 4-$CF_3$ | 5-Cl |
| 2-551 | $CF_3$ | H | 3-$CF_3$ | 4-$CF_3$ | 5-Cl |
| 2-552 | $CF_3$ | H | 3-$OCH_3$ | 4-$CF_3$ | 5-Cl |
| 2-553 | $CF_3$ | H | 3-$OCF_3$ | 4-$CF_3$ | 5-Cl |
| 2-554 | $CF_3$ | H | 3-F | 4-$CF_3$ | 5-CN |
| 2-555 | $CF_3$ | H | 3-Cl | 4-$CF_3$ | 5-CN |
| 2-556 | $CF_3$ | H | 3-CN | 4-$CF_3$ | 5-CN |
| 2-557 | $CF_3$ | H | 3-$CH_3$ | 4-$CF_3$ | 5-CN |
| 2-558 | $CF_3$ | H | 3-$CF_3$ | 4-$CF_3$ | 5-CN |
| 2-559 | $CF_3$ | H | 3-$OCH_3$ | 4-$CF_3$ | 5-CN |
| 2-560 | $CF_3$ | H | 3-$OCF_3$ | 4-$CF_3$ | 5-CN |
| 2-561 | $CF_3$ | H | 3-F | 4-$CF_3$ | 5-$CH_3$ |
| 2-562 | $CF_3$ | H | 3-Cl | 4-$CF_3$ | 5-$CH_3$ |
| 2-563 | $CF_3$ | H | 3-CN | 4-$CF_3$ | 5-$CH_3$ |
| 2-564 | $CF_3$ | H | 3-$CH_3$ | 4-$CF_3$ | 5-$CH_3$ |
| 2-565 | $CF_3$ | H | 3-$CF_3$ | 4-$CF_3$ | 5-$CH_3$ |
| 2-566 | $CF_3$ | H | 3-$OCH_3$ | 4-$CF_3$ | 5-$CH_3$ |
| 2-567 | $CF_3$ | H | 3-$OCF_3$ | 4-$CF_3$ | 5-$CH_3$ |
| 2-568 | $CF_3$ | H | 3-F | 4-$CF_3$ | 5-$CF_3$ |
| 2-569 | $CF_3$ | H | 3-Cl | 4-$CF_3$ | 5-$CF_3$ |
| 2-570 | $CF_3$ | H | 3-CN | 4-$CF_3$ | 5-$CF_3$ |
| 2-571 | $CF_3$ | H | 3-$CH_3$ | 4-$CF_3$ | 5-$CF_3$ |
| 2-572 | $CF_3$ | H | 3-$CF_3$ | 4-$CF_3$ | 5-$CF_3$ |
| 2-573 | $CF_3$ | H | 3-$OCH_3$ | 4-$CF_3$ | 5-$CF_3$ |
| 2-574 | $CF_3$ | H | 3-$OCF_3$ | 4-$CF_3$ | 5-$CF_3$ |
| 2-575 | $CF_3$ | H | 3-F | 4-$CF_3$ | 5-$OCH_3$ |
| 2-576 | $CF_3$ | H | 3-Cl | 4-$CF_3$ | 5-$OCH_3$ |
| 2-577 | $CF_3$ | H | 3-CN | 4-$CF_3$ | 5-$OCH_3$ |
| 2-578 | $CF_3$ | H | 3-$CH_3$ | 4-$CF_3$ | 5-$OCH_3$ |
| 2-579 | $CF_3$ | H | 3-$CF_3$ | 4-$CF_3$ | 5-$OCH_3$ |
| 2-580 | $CF_3$ | H | 3-$OCH_3$ | 4-$CF_3$ | 5-$OCH_3$ |

| Nr. | $R^1$ | $R^2$ | $R^3$ | $R^4$ | $R^5$ |
|---|---|---|---|---|---|
| 2-581 | $CF_3$ | H | 3-$OCF_3$ | 4-$CF_3$ | 5-$OCH_3$ |
| 2-582 | $CF_3$ | H | 3-F | 4-$CF_3$ | 5-$OCF_3$ |
| 2-583 | $CF_3$ | H | 3-Cl | 4-$CF_3$ | 5-$OCF_3$ |
| 2-584 | $CF_3$ | H | 3-CN | 4-$CF_3$ | 5-$OCF_3$ |
| 2-585 | $CF_3$ | H | 3-$CH_3$ | 4-$CF_3$ | 5-$OCF_3$ |
| 2-586 | $CF_3$ | H | 3-$CF_3$ | 4-$CF_3$ | 5-$OCF_3$ |
| 2-587 | $CF_3$ | H | 3-$OCH_3$ | 4-$CF_3$ | 5-$OCF_3$ |
| 2-588 | $CF_3$ | H | 3-$OCF_3$ | 4-$CF_3$ | 5-$OCF_3$ |
| 2-589 | $CF_3$ | H | 3-F | 4-$OCH_3$ | 5-F |
| 2-590 | $CF_3$ | H | 3-Cl | 4-$OCH_3$ | 5-F |
| 2-591 | $CF_3$ | H | 3-CN | 4-$OCH_3$ | 5-F |
| 2-592 | $CF_3$ | H | 3-$CH_3$ | 4-$OCH_3$ | 5-F |
| 2-593 | $CF_3$ | H | 3-$CF_3$ | 4-$OCH_3$ | 5-F |
| 2-594 | $CF_3$ | H | 3-$OCH_3$ | 4-$OCH_3$ | 5-F |
| 2-595 | $CF_3$ | H | 3-$OCF_3$ | 4-$OCH_3$ | 5-F |
| 2-596 | $CF_3$ | H | 3-F | 4-$OCH_3$ | 5-Cl |
| 2-597 | $CF_3$ | H | 3-Cl | 4-$OCH_3$ | 5-Cl |
| 2-598 | $CF_3$ | H | 3-CN | 4-$OCH_3$ | 5-Cl |
| 2-599 | $CF_3$ | H | 3-$CH_3$ | 4-$OCH_3$ | 5-Cl |
| 2-600 | $CF_3$ | H | 3-$CF_3$ | 4-$OCH_3$ | 5-Cl |
| 2-601 | $CF_3$ | H | 3-$OCH_3$ | 4-$OCH_3$ | 5-Cl |
| 2-602 | $CF_3$ | H | 3-$OCF_3$ | 4-$OCH_3$ | 5-Cl |
| 2-603 | $CF_3$ | H | 3-F | 4-$OCH_3$ | 5-CN |
| 2-604 | $CF_3$ | H | 3-Cl | 4-$OCH_3$ | 5-CN |
| 2-605 | $CF_3$ | H | 3-CN | 4-$OCH_3$ | 5-CN |
| 2-606 | $CF_3$ | H | 3-$CH_3$ | 4-$OCH_3$ | 5-CN |
| 2-607 | $CF_3$ | H | 3-$CF_3$ | 4-$OCH_3$ | 5-CN |
| 2-608 | $CF_3$ | H | 3-$OCH_3$ | 4-$OCH_3$ | 5-CN |
| 2-609 | $CF_3$ | H | 3-$OCF_3$ | 4-$OCH_3$ | 5-CN |
| 2-610 | $CF_3$ | H | 3-F | 4-$OCH_3$ | 5-$CH_3$ |
| 2-611 | $CF_3$ | H | 3-Cl | 4-$OCH_3$ | 5-$CH_3$ |
| 2-612 | $CF_3$ | H | 3-CN | 4-$OCH_3$ | 5-$CH_3$ |
| 2-613 | $CF_3$ | H | 3-$CH_3$ | 4-$OCH_3$ | 5-$CH_3$ |
| 2-614 | $CF_3$ | H | 3-$CF_3$ | 4-$OCH_3$ | 5-$CH_3$ |
| 2-615 | $CF_3$ | H | 3-$OCH_3$ | 4-$OCH_3$ | 5-$CH_3$ |
| 2-616 | $CF_3$ | H | 3-$OCF_3$ | 4-$OCH_3$ | 5-$CH_3$ |
| 2-617 | $CF_3$ | H | 3-F | 4-$OCH_3$ | 5-$CF_3$ |
| 2-618 | $CF_3$ | H | 3-Cl | 4-$OCH_3$ | 5-$CF_3$ |
| 2-619 | $CF_3$ | H | 3-CN | 4-$OCH_3$ | 5-$CF_3$ |

| Nr. | $R^1$ | $R^2$ | $R^3$ | $R^4$ | $R^5$ |
|---|---|---|---|---|---|
| 2-620 | $CF_3$ | H | 3-$CH_3$ | 4-$OCH_3$ | 5-$CF_3$ |
| 2-621 | $CF_3$ | H | 3-$CF_3$ | 4-$OCH_3$ | 5-$CF_3$ |
| 2-622 | $CF_3$ | H | 3-$OCH_3$ | 4-$OCH_3$ | 5-$CF_3$ |
| 2-623 | $CF_3$ | H | 3-$OCF_3$ | 4-$OCH_3$ | 5-$CF_3$ |
| 2-624 | $CF_3$ | H | 3-F | 4-$OCH_3$ | 5-$OCH_3$ |
| 2-625 | $CF_3$ | H | 3-Cl | 4-$OCH_3$ | 5-$OCH_3$ |
| 2-626 | $CF_3$ | H | 3-CN | 4-$OCH_3$ | 5-$OCH_3$ |
| 2-627 | $CF_3$ | H | 3-$CH_3$ | 4-$OCH_3$ | 5-$OCH_3$ |
| 2-628 | $CF_3$ | H | 3-$CF_3$ | 4-$OCH_3$ | 5-$OCH_3$ |
| 2-629 | $CF_3$ | H | 3-$OCH_3$ | 4-$OCH_3$ | 5-$OCH_3$ |
| 2-630 | $CF_3$ | H | 3-$OCF_3$ | 4-$OCH_3$ | 5-$OCH_3$ |
| 2-631 | $CF_3$ | H | 3-F | 4-$OCH_3$ | 5-$OCF_3$ |
| 2-632 | $CF_3$ | H | 3-Cl | 4-$OCH_3$ | 5-$OCF_3$ |
| 2-633 | $CF_3$ | H | 3-CN | 4-$OCH_3$ | 5-$OCF_3$ |
| 2-634 | $CF_3$ | H | 3-$CH_3$ | 4-$OCH_3$ | 5-$OCF_3$ |
| 2-635 | $CF_3$ | H | 3-$CF_3$ | 4-$OCH_3$ | 5-$OCF_3$ |
| 2-636 | $CF_3$ | H | 3-$OCH_3$ | 4-$OCH_3$ | 5-$OCF_3$ |
| 2-637 | $CF_3$ | H | 3-$OCF_3$ | 4-$OCH_3$ | 5-$OCF_3$ |
| 2-638 | $CF_3$ | H | 3-F | 4-$OCF_3$ | 5-F |
| 2-639 | $CF_3$ | H | 3-Cl | 4-$OCF_3$ | 5-F |
| 2-640 | $CF_3$ | H | 3-CN | 4-$OCF_3$ | 5-F |
| 2-641 | $CF_3$ | H | 3-$CH_3$ | 4-$OCF_3$ | 5-F |
| 2-642 | $CF_3$ | H | 3-$CF_3$ | 4-$OCF_3$ | 5-F |
| 2-643 | $CF_3$ | H | 3-$OCH_3$ | 4-$OCF_3$ | 5-F |
| 2-644 | $CF_3$ | H | 3-$OCF_3$ | 4-$OCF_3$ | 5-F |
| 2-645 | $CF_3$ | H | 3-F | 4-$OCF_3$ | 5-Cl |
| 2-646 | $CF_3$ | H | 3-Cl | 4-$OCF_3$ | 5-Cl |
| 2-647 | $CF_3$ | H | 3-CN | 4-$OCF_3$ | 5-Cl |
| 2-648 | $CF_3$ | H | 3-$CH_3$ | 4-$OCF_3$ | 5-Cl |
| 2-649 | $CF_3$ | H | 3-$CF_3$ | 4-$OCF_3$ | 5-Cl |
| 2-650 | $CF_3$ | H | 3-$OCH_3$ | 4-$OCF_3$ | 5-Cl |
| 2-651 | $CF_3$ | H | 3-$OCF_3$ | 4-$OCF_3$ | 5-Cl |
| 2-652 | $CF_3$ | H | 3-F | 4-$OCF_3$ | 5-CN |
| 2-653 | $CF_3$ | H | 3-Cl | 4-$OCF_3$ | 5-CN |
| 2-654 | $CF_3$ | H | 3-CN | 4-$OCF_3$ | 5-CN |
| 2-655 | $CF_3$ | H | 3-$CH_3$ | 4-$OCF_3$ | 5-CN |
| 2-656 | $CF_3$ | H | 3-$CF_3$ | 4-$OCF_3$ | 5-CN |
| 2-657 | $CF_3$ | H | 3-$OCH_3$ | 4-$OCF_3$ | 5-CN |
| 2-658 | $CF_3$ | H | 3-$OCF_3$ | 4-$OCF_3$ | 5-CN |

| Nr. | $R^1$ | $R^2$ | $R^3$ | $R^4$ | $R^5$ |
|---|---|---|---|---|---|
| 2-659 | $CF_3$ | H | 3-F | 4-$OCF_3$ | 5-$CH_3$ |
| 2-660 | $CF_3$ | H | 3-Cl | 4-$OCF_3$ | 5-$CH_3$ |
| 2-661 | $CF_3$ | H | 3-CN | 4-$OCF_3$ | 5-$CH_3$ |
| 2-662 | $CF_3$ | H | 3-$CH_3$ | 4-$OCF_3$ | 5-$CH_3$ |
| 2-663 | $CF_3$ | H | 3-CF3 | 4-$OCF_3$ | 5-$CH_3$ |
| 2-664 | $CF_3$ | H | 3-$OCH_3$ | 4-$OCF_3$ | 5-$CH_3$ |
| 2-665 | $CF_3$ | H | 3-$OCF_3$ | 4-$OCF_3$ | 5-$CH_3$ |
| 2-666 | $CF_3$ | H | 3-F | 4-$OCF_3$ | 5-$CF_3$ |
| 2-667 | $CF_3$ | H | 3-Cl | 4-$OCF_3$ | 5-$CF_3$ |
| 2-668 | $CF_3$ | H | 3-CN | 4-$OCF_3$ | 5-$CF_3$ |
| 2-669 | $CF_3$ | H | 3-$CH_3$ | 4-$OCF_3$ | 5-$CF_3$ |
| 2-670 | $CF_3$ | H | 3-$CF_3$ | 4-$OCF_3$ | 5-$CF_3$ |
| 2-671 | $CF_3$ | H | 3-$OCH_3$ | 4-$OCF_3$ | 5-$CF_3$ |
| 2-672 | $CF_3$ | H | 3-$OCF_3$ | 4-$OCF_3$ | 5-$CF_3$ |
| 2-673 | $CF_3$ | H | 3-F | 4-$OCF_3$ | 5-$OCH_3$ |
| 2-674 | $CF_3$ | H | 3-Cl | 4-$OCF_3$ | 5-$OCH_3$ |
| 2-675 | $CF_3$ | H | 3-CN | 4-$OCF_3$ | 5-$OCH_3$ |
| 2-676 | $CF_3$ | H | 3-$CH_3$ | 4-$OCF_3$ | 5-$OCH_3$ |
| 2-677 | $CF_3$ | H | 3-$CF_3$ | 4-$OCF_3$ | 5-$OCH_3$ |
| 2-678 | $CF_3$ | H | 3-$OCH_3$ | 4-$OCF_3$ | 5-$OCH_3$ |
| 2-679 | $CF_3$ | H | 3-$OCF_3$ | 4-$OCF_3$ | 5-$OCH_3$ |
| 2-680 | $CF_3$ | H | 3-F | 4-$OCF_3$ | 5-$OCF_3$ |
| 2-681 | $CF_3$ | H | 3-Cl | 4-$OCF_3$ | 5-$OCF_3$ |
| 2-682 | $CF_3$ | H | 3-CN | 4-$OCF_3$ | 5-$OCF_3$ |
| 2-683 | $CF_3$ | H | 3-$CH_3$ | 4-$OCF_3$ | 5-$OCF_3$ |
| 2-684 | $CF_3$ | H | 3-$CF_3$ | 4-$OCF_3$ | 5-$OCF_3$ |
| 2-685 | $CF_3$ | H | 3-$OCH_3$ | 4-$OCF_3$ | 5-$OCF_3$ |
| 2-686 | $CF_3$ | H | 3-$OCF_3$ | 4-$OCF_3$ | 5-$OCF_3$ |
| 2-687 | $CF_3$ | H | 2-F | 4-F | 5-F |
| 2-688 | $CF_3$ | H | 2-F | 4-F | 5-Cl |
| 2-689 | $CF_3$ | H | 2-F | 4-F | 5-CN |
| 2-690 | $CF_3$ | H | 2-F | 4-F | 5-$CH_3$ |
| 2-691 | $CF_3$ | H | 2-F | 4-F | 5-$CF_3$ |
| 2-692 | $CF_3$ | H | 2-F | 4-F | 5-$OCH_3$ |
| 2-693 | $CF_3$ | H | 2-F | 4-F | 5-$OCF_3$ |
| 2-694 | $CF_3$ | H | 2-Cl | 4-F | 5-F |
| 2-695 | $CF_3$ | H | 2-Cl | 4-F | 5-Cl |
| 2-696 | $CF_3$ | H | 2-Cl | 4-F | 5-CN |
| 2-697 | $CF_3$ | H | 2-Cl | 4-F | 5-$CH_3$ |
| 2-698 | $CF_3$ | H | 2-Cl | 4-F | 5-$CF_3$ |
| 2-699 | $CF_3$ | H | 2-Cl | 4-F | 5-$OCH_3$ |
| 2-700 | $CF_3$ | H | 2-Cl | 4-F | 5-$OCF_3$ |
| 2-701 | $CF_3$ | H | 2-CN | 4-F | 5-F |
| 2-702 | $CF_3$ | H | 2-CN | 4-F | 5-Cl |
| 2-703 | $CF_3$ | H | 2-CN | 4-F | 5-CN |
| 2-704 | $CF_3$ | H | 2-CN | 4-F | 5-$CH_3$ |
| 2-705 | $CF_3$ | H | 2-CN | 4-F | 5-$CF_3$ |
| 2-706 | $CF_3$ | H | 2-CN | 4-F | 5-$OCH_3$ |
| 2-707 | $CF_3$ | H | 2-CN | 4-F | 5-$OCF_3$ |
| 2-708 | $CF_3$ | H | 2-$CH_3$ | 4-F | 5-F |
| 2-709 | $CF_3$ | H | 2-$CH_3$ | 4-F | 5-Cl |
| 2-710 | $CF_3$ | H | 2-$CH_3$ | 4-F | 5-CN |
| 2-711 | $CF_3$ | H | 2-$CH_3$ | 4-F | 5-$CH_3$ |
| 2-712 | $CF_3$ | H | 2-$CH_3$ | 4-F | 5-$CF_3$ |
| 2-713 | $CF_3$ | H | 2-$CH_3$ | 4-F | 5-$OCH_3$ |
| 2-714 | $CF_3$ | H | 2-$CH_3$ | 4-F | 5-$OCF_3$ |
| 2-715 | $CF_3$ | H | 2-$CF_3$ | 4-F | 5-F |
| 2-716 | $CF_3$ | H | 2-$CF_3$ | 4-F | 5-Cl |
| 2-717 | $CF_3$ | H | 2-$CF_3$ | 4-F | 5-CN |
| 2-718 | $CF_3$ | H | 2-$CF_3$ | 4-F | 5-$CH_3$ |
| 2-719 | $CF_3$ | H | 2-$CF_3$ | 4-F | 5-$CF_3$ |
| 2-720 | $CF_3$ | H | 2-$CF_3$ | 4-F | 5-$OCH_3$ |
| 2-721 | $CF_3$ | H | 2-$CF_3$ | 4-F | 5-$OCF_3$ |
| 2-722 | $CF_3$ | H | 2-$OCH_3$ | 4-F | 5-F |
| 2-723 | $CF_3$ | H | 2-$OCH_3$ | 4-F | 5-Cl |
| 2-724 | $CF_3$ | H | 2-$OCH_3$ | 4-F | 5-CN |
| 2-725 | $CF_3$ | H | 2-$OCH_3$ | 4-F | 5-$CH_3$ |
| 2-726 | $CF_3$ | H | 2-$OCH_3$ | 4-F | 5-$CF_3$ |
| 2-727 | $CF_3$ | H | 2-$OCH_3$ | 4-F | 5-$OCH_3$ |
| 2-728 | $CF_3$ | H | 2-$OCH_3$ | 4-F | 5-$OCF_3$ |
| 2-729 | $CF_3$ | H | 2-$OCF_3$ | 4-F | 5-F |
| 2-730 | $CF_3$ | H | 2-$OCF_3$ | 4-F | 5-Cl |
| 2-731 | $CF_3$ | H | 2-$OCF_3$ | 4-F | 5-CN |
| 2-732 | $CF_3$ | H | 2-$OCF_3$ | 4-F | 5-$CH_3$ |
| 2-733 | $CF_3$ | H | 2-$OCF_3$ | 4-F | 5-$CF_3$ |
| 2-734 | $CF_3$ | H | 2-$OCF_3$ | 4-F | 5-$OCH_3$ |
| 2-735 | $CF_3$ | H | 2-$OCF_3$ | 4-F | 5-$OCF_3$ |
| 2-736 | $CF_3$ | H | 2-F | 4-Cl | 5-F |

| Nr. | $R^1$ | $R^2$ | $R^3$ | $R^4$ | $R^5$ |
|---|---|---|---|---|---|
| 2-737 | $CF_3$ | H | 2-F | 4-Cl | 5-Cl |
| 2-738 | $CF_3$ | H | 2-F | 4-Cl | 5-CN |
| 2-739 | $CF_3$ | H | 2-F | 4-Cl | 5-$CH_3$ |
| 2-740 | $CF_3$ | H | 2-F | 4-Cl | 5-$CF_3$ |
| 2-741 | $CF_3$ | H | 2-F | 4-Cl | 5-$OCH_3$ |
| 2-742 | $CF_3$ | H | 2-F | 4-Cl | 5-$OCF_3$ |
| 2-743 | $CF_3$ | H | 2-Cl | 4-Cl | 5-F |
| 2-744 | $CF_3$ | H | 2-Cl | 4-Cl | 5-Cl |
| 2-745 | $CF_3$ | H | 2-Cl | 4-Cl | 5-CN |
| 2-746 | $CF_3$ | H | 2-Cl | 4-Cl | 5-$CH_3$ |
| 2-747 | $CF_3$ | H | 2-Cl | 4-Cl | 5-$CF_3$ |
| 2-748 | $CF_3$ | H | 2-Cl | 4-Cl | 5-$OCH_3$ |
| 2-749 | $CF_3$ | H | 2-Cl | 4-Cl | 5-$OCF_3$ |
| 2-750 | $CF_3$ | H | 2-CN | 4-Cl | 5-F |
| 2-751 | $CF_3$ | H | 2-CN | 4-Cl | 5-Cl |
| 2-752 | $CF_3$ | H | 2-CN | 4-Cl | 5-CN |
| 2-753 | $CF_3$ | H | 2-CN | 4-Cl | 5-$CH_3$ |
| 2-754 | $CF_3$ | H | 2-CN | 4-Cl | 5-$CF_3$ |
| 2-755 | $CF_3$ | H | 2-CN | 4-Cl | 5-$OCH_3$ |
| 2-756 | $CF_3$ | H | 2-CN | 4-Cl | 5-$OCF_3$ |
| 2-757 | $CF_3$ | H | 2- $CH_3$ | 4-Cl | 5-F |
| 2-758 | $CF_3$ | H | 2- $CH_3$ | 4-Cl | 5-Cl |
| 2-759 | $CF_3$ | H | 2- $CH_3$ | 4-Cl | 5-CN |
| 2-760 | $CF_3$ | H | 2- $CH_3$ | 4-Cl | 5-$CH_3$ |
| 2-761 | $CF_3$ | H | 2-$CH_3$ | 4-Cl | 5-$CF_3$ |
| 2-762 | $CF_3$ | H | 2-$CH_3$ | 4-Cl | 5-$OCH_3$ |
| 2-763 | $CF_3$ | H | 2-$CH_3$ | 4-Cl | 5-$OCF_3$ |
| 2-764 | $CF_3$ | H | 2-$CF_3$ | 4-Cl | 5-F |
| 2-765 | $CF_3$ | H | 2-$CF_3$ | 4-Cl | 5-Cl |
| 2-766 | $CF_3$ | H | 2-$CF_3$ | 4-Cl | 5-CN |
| 2-767 | $CF_3$ | H | 2-$CF_3$ | 4-Cl | 5-$CH_3$ |
| 2-768 | $CF_3$ | H | 2-$CF_3$ | 4-Cl | 5-$CF_3$ |
| 2-769 | $CF_3$ | H | 2-$CF_3$ | 4-Cl | 5-$OCH_3$ |
| 2-770 | $CF_3$ | H | 2-$CF_3$ | 4-Cl | 5-$OCF_3$ |
| 2-771 | $CF_3$ | H | 2-$OCH_3$ | 4-Cl | 5-F |
| 2-772 | $CF_3$ | H | 2-$OCH_3$ | 4-Cl | 5-Cl |
| 2-773 | $CF_3$ | H | 2-$OCH_3$ | 4-Cl | 5-CN |
| 2-774 | $CF_3$ | H | 2-$OCH_3$ | 4-Cl | 5-$CH_3$ |
| 2-775 | $CF_3$ | H | 2-$OCH_3$ | 4-Cl | 5-$CF_3$ |

| Nr. | $R^1$ | $R^2$ | $R^3$ | $R^4$ | $R^5$ |
|---|---|---|---|---|---|
| 2-776 | $CF_3$ | H | 2-$OCH_3$ | 4-Cl | 5-$OCH_3$ |
| 2-777 | $CF_3$ | H | 2-$OCH_3$ | 4-Cl | 5-$OCF_3$ |
| 2-778 | $CF_3$ | H | 2-$OCF_3$ | 4-Cl | 5-F |
| 2-779 | $CF_3$ | H | 2-$OCF_3$ | 4-Cl | 5-Cl |
| 2-780 | $CF_3$ | H | 2-$OCF_3$ | 4-Cl | 5-CN |
| 2-781 | $CF_3$ | H | 2-$OCF_3$ | 4-Cl | 5-$CH_3$ |
| 2-782 | $CF_3$ | H | 2-$OCF_3$ | 4-Cl | 5-$CF_3$ |
| 2-783 | $CF_3$ | H | 2-$OCF_3$ | 4-Cl | 5-$OCH_3$ |
| 2-784 | $CF_3$ | H | 2-$OCF_3$ | 4-Cl | 5-$OCF_3$ |
| 2-785 | $CF_3$ | H | 2-F | 4-CN | 5-F |
| 2-786 | $CF_3$ | H | 2-F | 4-CN | 5-Cl |
| 2-787 | $CF_3$ | H | 2-F | 4-CN | 5-CN |
| 2-788 | $CF_3$ | H | 2-F | 4-CN | 5-$CH_3$ |
| 2-789 | $CF_3$ | H | 2-F | 4-CN | 5-$CF_3$ |
| 2-790 | $CF_3$ | H | 2-F | 4-CN | 5-$OCH_3$ |
| 2-791 | $CF_3$ | H | 2-F | 4-CN | 5-$OCF_3$ |
| 2-792 | $CF_3$ | H | 2-Cl | 4-CN | 5-F |
| 2-793 | $CF_3$ | H | 2-Cl | 4-CN | 5-Cl |
| 2-794 | $CF_3$ | H | 2-Cl | 4-CN | 5-CN |
| 2-795 | $CF_3$ | H | 2-Cl | 4-CN | 5-$CH_3$ |
| 2-796 | $CF_3$ | H | 2-Cl | 4-CN | 5-$CF_3$ |
| 2-797 | $CF_3$ | H | 2-Cl | 4-CN | 5-$OCH_3$ |
| 2-798 | $CF_3$ | H | 2-Cl | 4-CN | 5-$OCF_3$ |
| 2-799 | $CF_3$ | H | 2-CN | 4-CN | 5-F |
| 2-800 | $CF_3$ | H | 2-CN | 4-CN | 5-Cl |
| 2-801 | $CF_3$ | H | 2-CN | 4-CN | 5-CN |
| 2-802 | $CF_3$ | H | 2-CN | 4-CN | 5-$CH_3$ |
| 2-803 | $CF_3$ | H | 2-CN | 4-CN | 5-$CF_3$ |
| 2-804 | $CF_3$ | H | 2-CN | 4-CN | 5-$OCH_3$ |
| 2-805 | $CF_3$ | H | 2-CN | 4-CN | 5-$OCF_3$ |
| 2-806 | $CF_3$ | H | 2- $CH_3$ | 4-CN | 5-F |
| 2-807 | $CF_3$ | H | 2- $CH_3$ | 4-CN | 5-Cl |
| 2-808 | $CF_3$ | H | 2- $CH_3$ | 4-CN | 5-CN |
| 2-809 | $CF_3$ | H | 2- $CH_3$ | 4-CN | 5-$CH_3$ |
| 2-810 | $CF_3$ | H | 2- $CH_3$ | 4-CN | 5-$CF_3$ |
| 2-811 | $CF_3$ | H | 2- $CH_3$ | 4-CN | 5-$OCH_3$ |
| 2-812 | $CF_3$ | H | 2- $CH_3$ | 4-CN | 5-$OCF_3$ |
| 2-813 | $CF_3$ | H | 2-$CF_3$ | 4-CN | 5-F |
| 2-814 | $CF_3$ | H | 2-$CF_3$ | 4-CN | 5-Cl |

| Nr. | $R^1$ | $R^2$ | $R^3$ | $R^4$ | $R^5$ |
|---|---|---|---|---|---|
| 2-815 | $CF_3$ | H | 2-$CF_3$ | 4-CN | 5-CN |
| 2-816 | $CF_3$ | H | 2-$CF_3$ | 4-CN | 5-$CH_3$ |
| 2-817 | $CF_3$ | H | 2-$CF_3$ | 4-CN | 5-$CF_3$ |
| 2-818 | $CF_3$ | H | 2-$CF_3$ | 4-CN | 5-$OCH_3$ |
| 2-819 | $CF_3$ | H | 2-$CF_3$ | 4-CN | 5-$OCF_3$ |
| 2-820 | $CF_3$ | H | 2-$OCH_3$ | 4-CN | 5-F |
| 2-821 | $CF_3$ | H | 2-$OCH_3$ | 4-CN | 5-Cl |
| 2-822 | $CF_3$ | H | 2-$OCH_3$ | 4-CN | 5-CN |
| 2-823 | $CF_3$ | H | 2-$OCH_3$ | 4-CN | 5-$CH_3$ |
| 2-824 | $CF_3$ | H | 2-$OCH_3$ | 4-CN | 5-$CF_3$ |
| 2-825 | $CF_3$ | H | 2-$OCH_3$ | 4-CN | 5-$OCH_3$ |
| 2-826 | $CF_3$ | H | 2-$OCH_3$ | 4-CN | 5-$OCF_3$ |
| 2-827 | $CF_3$ | H | 2-$OCF_3$ | 4-CN | 5-F |
| 2-828 | $CF_3$ | H | 2-$OCF_3$ | 4-CN | 5-Cl |
| 2-829 | $CF_3$ | H | 2-$OCF_3$ | 4-CN | 5-CN |
| 2-830 | $CF_3$ | H | 2-$OCF_3$ | 4-CN | 5-$CH_3$ |
| 2-831 | $CF_3$ | H | 2-$OCF_3$ | 4-CN | 5-$CF_3$ |
| 2-832 | $CF_3$ | H | 2-$OCF_3$ | 4-CN | 5-$OCH_3$ |
| 2-833 | $CF_3$ | H | 2-$OCF_3$ | 4-CN | 5-$OCF_3$ |
| 2-834 | $CF_3$ | H | 2-F | 4-$CH_3$ | 5-F |
| 2-835 | $CF_3$ | H | 2-F | 4-$CH_3$ | 5-Cl |
| 2-836 | $CF_3$ | H | 2-F | 4-$CH_3$ | 5-CN |
| 2-837 | $CF_3$ | H | 2-F | 4-$CH_3$ | 5-$CH_3$ |
| 2-838 | $CF_3$ | H | 2-F | 4-$CH_3$ | 5-$CF_3$ |
| 2-839 | $CF_3$ | H | 2-F | 4-$CH_3$ | 5-$OCH_3$ |
| 2-840 | $CF_3$ | H | 2-F | 4-$CH_3$ | 5-$OCF_3$ |
| 2-841 | $CF_3$ | H | 2-Cl | 4-$CH_3$ | 5-F |
| 2-842 | $CF_3$ | H | 2-Cl | 4-$CH_3$ | 5-Cl |
| 2-843 | $CF_3$ | H | 2-Cl | 4-$CH_3$ | 5-CN |
| 2-844 | $CF_3$ | H | 2-Cl | 4-$CH_3$ | 5-$CH_3$ |
| 2-845 | $CF_3$ | H | 2-Cl | 4-$CH_3$ | 5-$CF_3$ |
| 2-846 | $CF_3$ | H | 2-Cl | 4-$CH_3$ | 5-$OCH_3$ |
| 2-847 | $CF_3$ | H | 2-Cl | 4-$CH_3$ | 5-$OCF_3$ |
| 2-848 | $CF_3$ | H | 2-CN | 4-$CH_3$ | 5-F |
| 2-849 | $CF_3$ | H | 2-CN | 4-$CH_3$ | 5-Cl |
| 2-850 | $CF_3$ | H | 2-CN | 4-$CH_3$ | 5-CN |
| 2-851 | $CF_3$ | H | 2-CN | 4-$CH_3$ | 5-$CH_3$ |
| 2-852 | $CF_3$ | H | 2-CN | 4-$CH_3$ | 5-$CF_3$ |
| 2-853 | $CF_3$ | H | 2-CN | 4-$CH_3$ | 5-$OCH_3$ |

| Nr. | $R^1$ | $R^2$ | $R^3$ | $R^4$ | $R^5$ |
|---|---|---|---|---|---|
| 2-854 | $CF_3$ | H | 2-CN | 4-$CH_3$ | 5-$OCF_3$ |
| 2-855 | $CF_3$ | H | 2- $CH_3$ | 4-$CH_3$ | 5-F |
| 2-856 | $CF_3$ | H | 2- $CH_3$ | 4-$CH_3$ | 5-Cl |
| 2-857 | $CF_3$ | H | 2- $CH_3$ | 4-$CH_3$ | 5-CN |
| 2-858 | $CF_3$ | H | 2- $CH_3$ | 4-$CH_3$ | 5-$CH_3$ |
| 2-859 | $CF_3$ | H | 2- $CH_3$ | 4-$CH_3$ | 5-$CF_3$ |
| 2-860 | $CF_3$ | H | 2- $CH_3$ | 4-$CH_3$ | 5-$OCH_3$ |
| 2-861 | $CF_3$ | H | 2- $CH_3$ | 4-$CH_3$ | 5-$OCF_3$ |
| 2-862 | $CF_3$ | H | 2-$CF_3$ | 4-$CH_3$ | 5-F |
| 2-863 | $CF_3$ | H | 2-$CF_3$ | 4-$CH_3$ | 5-Cl |
| 2-864 | $CF_3$ | H | 2-$CF_3$ | 4-$CH_3$ | 5-CN |
| 2-865 | $CF_3$ | H | 2-$CF_3$ | 4-$CH_3$ | 5-$CH_3$ |
| 2-866 | $CF_3$ | H | 2-$CF_3$ | 4-$CH_3$ | 5-$CF_3$ |
| 2-867 | $CF_3$ | H | 2-$CF_3$ | 4-$CH_3$ | 5-$OCH_3$ |
| 2-868 | $CF_3$ | H | 2-$CF_3$ | 4-$CH_3$ | 5-$OCF_3$ |
| 2-869 | $CF_3$ | H | 2-$OCH_3$ | 4-$CH_3$ | 5-F |
| 2-870 | $CF_3$ | H | 2-$OCH_3$ | 4-$CH_3$ | 5-Cl |
| 2-871 | $CF_3$ | H | 2-$OCH_3$ | 4-$CH_3$ | 5-CN |
| 2-872 | $CF_3$ | H | 2-$OCH_3$ | 4-$CH_3$ | 5-$CH_3$ |
| 2-873 | $CF_3$ | H | 2-$OCH_3$ | 4-$CH_3$ | 5-$CF_3$ |
| 2-874 | $CF_3$ | H | 2-$OCH_3$ | 4-$CH_3$ | 5-$OCH_3$ |
| 2-875 | $CF_3$ | H | 2-$OCH_3$ | 4-$CH_3$ | 5-$OCF_3$ |
| 2-876 | $CF_3$ | H | 2-$OCF_3$ | 4-$CH_3$ | 5-F |
| 2-877 | $CF_3$ | H | 2-$OCF_3$ | 4-$CH_3$ | 5-Cl |
| 2-878 | $CF_3$ | H | 2-$OCF_3$ | 4-$CH_3$ | 5-CN |
| 2-879 | $CF_3$ | H | 2-$OCF_3$ | 4-$CH_3$ | 5-$CH_3$ |
| 2-880 | $CF_3$ | H | 2-$OCF_3$ | 4-$CH_3$ | 5-$CF_3$ |
| 2-881 | $CF_3$ | H | 2-$OCF_3$ | 4-$CH_3$ | 5-$OCH_3$ |
| 2-882 | $CF_3$ | H | 2-$OCF_3$ | 4-$CH_3$ | 5-$OCF_3$ |
| 2-883 | $CF_3$ | H | 2-F | 4-$CF_3$ | 5-F |
| 2-884 | $CF_3$ | H | 2-F | 4-$CF_3$ | 5-Cl |
| 2-885 | $CF_3$ | H | 2-F | 4-$CF_3$ | 5-CN |
| 2-886 | $CF_3$ | H | 2-F | 4-$CF_3$ | 5-$CH_3$ |
| 2-887 | $CF_3$ | H | 2-F | 4-$CF_3$ | 5-$CF_3$ |
| 2-888 | $CF_3$ | H | 2-F | 4-$CF_3$ | 5-$OCH_3$ |
| 2-889 | $CF_3$ | H | 2-F | 4-$CF_3$ | 5-$OCF_3$ |
| 2-890 | $CF_3$ | H | 2-Cl | 4-$CF_3$ | 5-F |
| 2-891 | $CF_3$ | H | 2-Cl | 4-$CF_3$ | 5-Cl |
| 2-892 | $CF_3$ | H | 2-Cl | 4-$CF_3$ | 5-CN |

| Nr. | $R^1$ | $R^2$ | $R^3$ | $R^4$ | $R^5$ |
|---|---|---|---|---|---|
| 2-893 | $CF_3$ | H | 2-Cl | 4-$CF_3$ | 5-$CH_3$ |
| 2-894 | $CF_3$ | H | 2-Cl | 4-$CF_3$ | 5-$CF_3$ |
| 2-895 | $CF_3$ | H | 2-Cl | 4-$CF_3$ | 5-$OCH_3$ |
| 2-896 | $CF_3$ | H | 2-Cl | 4-$CF_3$ | 5-$OCF_3$ |
| 2-897 | $CF_3$ | H | 2-CN | 4-$CF_3$ | 5-F |
| 2-898 | $CF_3$ | H | 2-CN | 4-$CF_3$ | 5-Cl |
| 2-899 | $CF_3$ | H | 2-CN | 4-$CF_3$ | 5-CN |
| 2-900 | $CF_3$ | H | 2-CN | 4-$CF_3$ | 5-$CH_3$ |
| 2-901 | $CF_3$ | H | 2-CN | 4-$CF_3$ | 5-$CF_3$ |
| 2-902 | $CF_3$ | H | 2-CN | 4-$CF_3$ | 5-$OCH_3$ |
| 2-903 | $CF_3$ | H | 2-CN | 4-$CF_3$ | 5-$OCF_3$ |
| 2-904 | $CF_3$ | H | 2- $CH_3$ | 4-$CF_3$ | 5-F |
| 2-905 | $CF_3$ | H | 2- $CH_3$ | 4-$CF_3$ | 5-Cl |
| 2-906 | $CF_3$ | H | 2- $CH_3$ | 4-$CF_3$ | 5-CN |
| 2-907 | $CF_3$ | H | 2- $CH_3$ | 4-$CF_3$ | 5-$CH_3$ |
| 2-908 | $CF_3$ | H | 2- $CH_3$ | 4-$CF_3$ | 5-$CF_3$ |
| 2-909 | $CF_3$ | H | 2- $CH_3$ | 4-$CF_3$ | 5-$OCH_3$ |
| 2-910 | $CF_3$ | H | 2- $CH_3$ | 4-$CF_3$ | 5-$OCF_3$ |
| 2-911 | $CF_3$ | H | 2-$CF_3$ | 4-$CF_3$ | 5-F |
| 2-912 | $CF_3$ | H | 2-$CF_3$ | 4-$CF_3$ | 5-Cl |
| 2-913 | $CF_3$ | H | 2-$CF_3$ | 4-$CF_3$ | 5-CN |
| 2-914 | $CF_3$ | H | 2-$CF_3$ | 4-$CF_3$ | 5-$CH_3$ |
| 2-915 | $CF_3$ | H | 2-$CF_3$ | 4-$CF_3$ | 5-$CF_3$ |
| 2-916 | $CF_3$ | H | 2-$CF_3$ | 4-$CF_3$ | 5-$OCH_3$ |
| 2-917 | $CF_3$ | H | 2-$CF_3$ | 4-$CF_3$ | 5-$OCF_3$ |
| 2-918 | $CF_3$ | H | 2-$OCH_3$ | 4-$CF_3$ | 5-F |
| 2-919 | $CF_3$ | H | 2-$OCH_3$ | 4-$CF_3$ | 5-Cl |
| 2-920 | $CF_3$ | H | 2-$OCH_3$ | 4-$CF_3$ | 5-CN |
| 2-921 | $CF_3$ | H | 2-$OCH_3$ | 4-$CF_3$ | 5-$CH_3$ |
| 2-922 | $CF_3$ | H | 2-$OCH_3$ | 4-$CF_3$ | 5-$CF_3$ |
| 2-923 | $CF_3$ | H | 2-$OCH_3$ | 4-$CF_3$ | 5-$OCH_3$ |
| 2-924 | $CF_3$ | H | 2-$OCH_3$ | 4-$CF_3$ | 5-$OCF_3$ |
| 2-925 | $CF_3$ | H | 2-$OCF_3$ | 4-$CF_3$ | 5-F |
| 2-926 | $CF_3$ | H | 2-$OCF_3$ | 4-$CF_3$ | 5-Cl |
| 2-927 | $CF_3$ | H | 2-$OCF_3$ | 4-$CF_3$ | 5-CN |
| 2-928 | $CF_3$ | H | 2-$OCF_3$ | 4-$CF_3$ | 5-$CH_3$ |
| 2-929 | $CF_3$ | H | 2-$OCF_3$ | 4-$CF_3$ | 5-$CF_3$ |
| 2-930 | $CF_3$ | H | 2-$OCF_3$ | 4-$CF_3$ | 5-$OCH_3$ |
| 2-931 | $CF_3$ | H | 2-$OCF_3$ | 4-$CF_3$ | 5-$OCF_3$ |

| Nr. | $R^1$ | $R^2$ | $R^3$ | $R^4$ | $R^5$ |
|---|---|---|---|---|---|
| 2-932 | $CF_3$ | H | 2-F | 4-$OCH_3$ | 5-F |
| 2-933 | $CF_3$ | H | 2-F | 4-$OCH_3$ | 5-Cl |
| 2-934 | $CF_3$ | H | 2-F | 4-$OCH_3$ | 5-CN |
| 2-935 | $CF_3$ | H | 2-F | 4-$OCH_3$ | 5-$CH_3$ |
| 2-936 | $CF_3$ | H | 2-F | 4-$OCH_3$ | 5-$CF_3$ |
| 2-937 | $CF_3$ | H | 2-F | 4-$OCH_3$ | 5-$OCH_3$ |
| 2-938 | $CF_3$ | H | 2-F | 4-$OCH_3$ | 5-$OCF_3$ |
| 2-939 | $CF_3$ | H | 2-Cl | 4-$OCH_3$ | 5-F |
| 2-940 | $CF_3$ | H | 2-Cl | 4-$OCH_3$ | 5-Cl |
| 2-941 | $CF_3$ | H | 2-Cl | 4-$OCH_3$ | 5-CN |
| 2-942 | $CF_3$ | H | 2-Cl | 4-$OCH_3$ | 5-$CH_3$ |
| 2-943 | $CF_3$ | H | 2-Cl | 4-$OCH_3$ | 5-$CF_3$ |
| 2-944 | $CF_3$ | H | 2-Cl | 4-$OCH_3$ | 5-$OCH_3$ |
| 2-945 | $CF_3$ | H | 2-Cl | 4-$OCH_3$ | 5-$OCF_3$ |
| 2-946 | $CF_3$ | H | 2-CN | 4-$OCH_3$ | 5-F |
| 2-947 | $CF_3$ | H | 2-CN | 4-$OCH_3$ | 5-Cl |
| 2-948 | $CF_3$ | H | 2-CN | 4-$OCH_3$ | 5-CN |
| 2-949 | $CF_3$ | H | 2-CN | 4-$OCH_3$ | 5-$CH_3$ |
| 2-950 | $CF_3$ | H | 2-CN | 4-$OCH_3$ | 5-$CF_3$ |
| 2-951 | $CF_3$ | H | 2-CN | 4-$OCH_3$ | 5-$OCH_3$ |
| 2-952 | $CF_3$ | H | 2-CN | 4-$OCH_3$ | 5-$OCF_3$ |
| 2-953 | $CF_3$ | H | 2- $CH_3$ | 4-$OCH_3$ | 5-F |
| 2-954 | $CF_3$ | H | 2- $CH_3$ | 4-$OCH_3$ | 5-Cl |
| 2-955 | $CF_3$ | H | 2- $CH_3$ | 4-$OCH_3$ | 5-CN |
| 2-956 | $CF_3$ | H | 2- $CH_3$ | 4-$OCH_3$ | 5-$CH_3$ |
| 2-957 | $CF_3$ | H | 2- $CH_3$ | 4-$OCH_3$ | 5-$CF_3$ |
| 2-958 | $CF_3$ | H | 2- $CH_3$ | 4-$OCH_3$ | 5-$OCH_3$ |
| 2-959 | $CF_3$ | H | 2- $CH_3$ | 4-$OCH_3$ | 5-$OCF_3$ |
| 2-960 | $CF_3$ | H | 2-$CF_3$ | 4-$OCH_3$ | 5-F |
| 2-961 | $CF_3$ | H | 2-$CF_3$ | 4-$OCH_3$ | 5-Cl |
| 2-962 | $CF_3$ | H | 2-$CF_3$ | 4-$OCH_3$ | 5-CN |
| 2-963 | $CF_3$ | H | 2-$CF_3$ | 4-$OCH_3$ | 5-$CH_3$ |
| 2-964 | $CF_3$ | H | 2-$CF_3$ | 4-$OCH_3$ | 5-$CF_3$ |
| 2-965 | $CF_3$ | H | 2-$CF_3$ | 4-$OCH_3$ | 5-$OCH_3$ |
| 2-966 | $CF_3$ | H | 2-$CF_3$ | 4-$OCH_3$ | 5-$OCF_3$ |
| 2-967 | $CF_3$ | H | 2-$OCH_3$ | 4-$OCH_3$ | 5-F |
| 2-968 | $CF_3$ | H | 2-$OCH_3$ | 4-$OCH_3$ | 5-Cl |
| 2-969 | $CF_3$ | H | 2-$OCH_3$ | 4-$OCH_3$ | 5-CN |
| 2-970 | $CF_3$ | H | 2-$OCH_3$ | 4-$OCH_3$ | 5-$CH_3$ |

| Nr. | $R^1$ | $R^2$ | $R^3$ | $R^4$ | $R^5$ |
|---|---|---|---|---|---|
| 2-971 | $CF_3$ | H | 2-$OCH_3$ | 4-$OCH_3$ | 5-$CF_3$ |
| 2-972 | $CF_3$ | H | 2-$OCH_3$ | 4-$OCH_3$ | 5-$OCH_3$ |
| 2-973 | $CF_3$ | H | 2-$OCH_3$ | 4-$OCH_3$ | 5-$OCF_3$ |
| 2-974 | $CF_3$ | H | 2-$OCF_3$ | 4-$OCH_3$ | 5-F |
| 2-975 | $CF_3$ | H | 2-$OCF_3$ | 4-$OCH_3$ | 5-Cl |
| 2-976 | $CF_3$ | H | 2-$OCF_3$ | 4-$OCH_3$ | 5-CN |
| 2-977 | $CF_3$ | H | 2-$OCF_3$ | 4-$OCH_3$ | 5-$CH_3$ |
| 2-978 | $CF_3$ | H | 2-$OCF_3$ | 4-$OCH_3$ | 5-$CF_3$ |
| 2-979 | $CF_3$ | H | 2-$OCF_3$ | 4-$OCH_3$ | 5-$OCH_3$ |
| 2-980 | $CF_3$ | H | 2-$OCF_3$ | 4-$OCH_3$ | 5-$OCF_3$ |
| 2-981 | $CF_3$ | H | 2-F | 4-$OCF_3$ | 5-F |
| 2-982 | $CF_3$ | H | 2-F | 4-$OCF_3$ | 5-Cl |
| 2-983 | $CF_3$ | H | 2-F | 4-$OCF_3$ | 5-CN |
| 2-984 | $CF_3$ | H | 2-F | 4-$OCF_3$ | 5-$CH_3$ |
| 2-985 | $CF_3$ | H | 2-F | 4-$OCF_3$ | 5-$CF_3$ |
| 2-986 | $CF_3$ | H | 2-F | 4-$OCF_3$ | 5-$OCH_3$ |
| 2-987 | $CF_3$ | H | 2-F | 4-$OCF_3$ | 5-$OCF_3$ |
| 2-988 | $CF_3$ | H | 2-Cl | 4-$OCF_3$ | 5-F |
| 2-989 | $CF_3$ | H | 2-Cl | 4-$OCF_3$ | 5-Cl |
| 2-990 | $CF_3$ | H | 2-Cl | 4-$OCF_3$ | 5-CN |
| 2-991 | $CF_3$ | H | 2-Cl | 4-$OCF_3$ | 5-$CH_3$ |
| 2-992 | $CF_3$ | H | 2-Cl | 4-$OCF_3$ | 5-$CF_3$ |
| 2-993 | $CF_3$ | H | 2-Cl | 4-$OCF_3$ | 5-$OCH_3$ |
| 2-994 | $CF_3$ | H | 2-Cl | 4-$OCF_3$ | 5-$OCF_3$ |
| 2-995 | $CF_3$ | H | 2-CN | 4-$OCF_3$ | 5-F |
| 2-996 | $CF_3$ | H | 2-CN | 4-$OCF_3$ | 5-Cl |
| 2-997 | $CF_3$ | H | 2-CN | 4-$OCF_3$ | 5-CN |
| 2-998 | $CF_3$ | H | 2-CN | 4-$OCF_3$ | 5-$CH_3$ |
| 2-999 | $CF_3$ | H | 2-CN | 4-$OCF_3$ | 5-$CF_3$ |
| 2-1000 | $CF_3$ | H | 2-CN | 4-$OCF_3$ | 5-$OCH_3$ |
| 2-1001 | $CF_3$ | H | 2-CN | 4-$OCF_3$ | 5-$OCF_3$ |
| 2-1002 | $CF_3$ | H | 2- $CH_3$ | 4-$OCF_3$ | 5-F |
| 2-1003 | $CF_3$ | H | 2- $CH_3$ | 4-$OCF_3$ | 5-Cl |
| 2-1004 | $CF_3$ | H | 2- $CH_3$ | 4-$OCF_3$ | 5-CN |
| 2-1005 | $CF_3$ | H | 2- $CH_3$ | 4-$OCF_3$ | 5-$CH_3$ |
| 2-1006 | $CF_3$ | H | 2- $CH_3$ | 4-$OCF_3$ | 5-$CF_3$ |
| 2-1007 | $CF_3$ | H | 2- $CH_3$ | 4-$OCF_3$ | 5-$OCH_3$ |
| 2-1008 | $CF_3$ | H | 2- $CH_3$ | 4-$OCF_3$ | 5-$OCF_3$ |
| 2-1009 | $CF_3$ | H | 2-$CF_3$ | 4-$OCF_3$ | 5-F |
| 2-1010 | $CF_3$ | H | 2-$CF_3$ | 4-$OCF_3$ | 5-Cl |
| 2-1011 | $CF_3$ | H | 2-$CF_3$ | 4-$OCF_3$ | 5-CN |
| 2-1012 | $CF_3$ | H | 2-$CF_3$ | 4-$OCF_3$ | 5-$CH_3$ |
| 2-1013 | $CF_3$ | H | 2-$CF_3$ | 4-$OCF_3$ | 5-$CF_3$ |
| 2-1014 | $CF_3$ | H | 2-$CF_3$ | 4-$OCF_3$ | 5-$OCH_3$ |
| 2-1015 | $CF_3$ | H | 2-$CF_3$ | 4-$OCF_3$ | 5-$OCF_3$ |
| 2-1016 | $CF_3$ | H | 2-$OCH_3$ | 4-$OCF_3$ | 5-F |
| 2-1017 | $CF_3$ | H | 2-$OCH_3$ | 4-$OCF_3$ | 5-Cl |
| 2-1018 | $CF_3$ | H | 2-$OCH_3$ | 4-$OCF_3$ | 5-CN |
| 2-1019 | $CF_3$ | H | 2-$OCH_3$ | 4-$OCF_3$ | 5-$CH_3$ |
| 2-1020 | $CF_3$ | H | 2-$OCH_3$ | 4-$OCF_3$ | 5-$CF_3$ |
| 2-1021 | $CF_3$ | H | 2-$OCH_3$ | 4-$OCF_3$ | 5-$OCH_3$ |
| 2-1022 | $CF_3$ | H | 2-$OCH_3$ | 4-$OCF_3$ | 5-$OCF_3$ |
| 2-1023 | $CF_3$ | H | 2-$OCF_3$ | 4-$OCF_3$ | 5-F |
| 2-1024 | $CF_3$ | H | 2-$OCF_3$ | 4-$OCF_3$ | 5-Cl |
| 2-1025 | $CF_3$ | H | 2-$OCF_3$ | 4-$OCF_3$ | 5-CN |
| 2-1026 | $CF_3$ | H | 2-$OCF_3$ | 4-$OCF_3$ | 5-$CH_3$ |
| 2-1027 | $CF_3$ | H | 2-$OCF_3$ | 4-$OCF_3$ | 5-$CF_3$ |
| 2-1028 | $CF_3$ | H | 2-$OCF_3$ | 4-$OCF_3$ | 5-$OCH_3$ |
| 2-1029 | $CF_3$ | H | 2-$OCF_3$ | 4-$OCF_3$ | 5-$OCF_3$ |

Alternativ zu oder in Kombination mit mindestens einer Verbindung 2-1 bis 2-1029 können auch mindestens eine Verbindung Ib-1 bis Ib-1029, Ic-1 bis Ic-1029, Id-1 bis Id-1029, Ie-1 bis Ie-1029, If-1 bis If-1029, Ig-1 bis Ig-1029, Ih-1 bis Ih-1029 oder Ij-1 bis Ij-1029 in den bevorzugten erfindungsgemäßen Verfahren zur Bekämpfung von pflanzenpathogenen Schadpilzen eingesetzt werden. Darüberhinaus, werden erfindungsgemäßen Verfahren zur Herstellung der erfindungsgemäßen Mittel und Saatgut, enthaltend mindestens eine Verbindung 2-1 bis 2-1029, verwendet.

Bevorzugte Ausgestaltungen der erfindungsgemäßen Mischungen enthalten als Komponente A mindestens eine, bevorzugt eine, Verbindung ausgewählt aus der Tabelle 3:

Tabelle 3:

| Nr. | Verbindung |
|---|---|
| 3-1 | 3-Trifluormethyl-1-methyl-1H-pyrazol-4-carbonsäure-N-(2'-fluor-4'-chlor-5'-methoxybiphenyl-2-yl)-amid |
| 3-2 | 3-Trifluormethyl-1-methyl-1H-pyrazol-4-carbonsäure-N-(2'-fluor-4'-chlor-5'-methylbiphenyl-2-yl)-amid |
| 3-3 | 3-Trifluormethyl-1-methyl-1H-pyrazol-4-carbonsäure-N-(3',4',5'-trifluorbiphenyl-2-yl)-amid |
| 3-4 | 3-Trifluormethyl-1-methyl-1H-pyrazol-4-carbonsäure-N-(2',4',5'-trifluorbiphenyl-2-yl)-amid |
| 3-5 | 3-Trifluormethyl-1-methyl-1H-pyrazol-4-carbonsäure-N-(2',4',5'-trifluorbiphenyl-2-yl)-amid |
| 3-6 | 3-Difluormethyl-1-methyl-1H-pyrazol-4-carbonsäure-N-(2'-fluor-4'-chlor-5'-methoxybiphenyl-2-yl)-amid |
| 3-7 | 3-Trifluormethyl-1-methyl-1H-pyrazol-4-carbonsäure-N-(2',3',4'-trifluorbiphenyl-2-yl)-amid |
| 3-8 | 3-Difluormethyl-1-methyl-1H-pyrazol-4-carbonsäure-N-(2'-fluor-4'-chlor-5'-methylbiphenyl-2-yl)-amid |
| 3-9 | 3-Difluormethyl-1-methyl-1H-pyrazol-4-carbonsäure-N-(3',4',5'-trifluorbiphenyl-2-yl)-amid |
| 3-10 | 3-Difluormethyl-1-methyl-1H-pyrazol-4-carbonsäure-N-(2',4',5'-trifluorbiphenyl-2-yl)-amid |
| 3-11 | 3-Fluormethyl-1-methyl-1H-pyrazol-4-carbonsäure-N-(3',4',5'-trifluorbiphenyl-2-yl)-amid |
| 3-12 | 3-Chlordifluormethyl-1-methyl-1H-pyrazol-4-carbonsäure-N-(3',4',5'-trifluorbiphenyl-2-yl)-amid |
| 3-13 | 3-Chlorfluormethyl-1-methyl-1H-pyrazol-4-carbonsäure-N-(3',4',5'-trifluorbiphenyl-2-yl)-amid |
| 3-14 | 3-Fluormethyl-1-methyl-1H-pyrazol-4-carbonsäure-N-(2',3',4'-trifluorbiphenyl-2-yl)-amid |
| 3-15 | 3-Fluormethyl-1-methyl-1H-pyrazol-4-carbonsäure-N-(2',4',5'-trifluorbiphenyl-2-yl)-amid |

In den erfindungsgemäßen Mischungen ist als Komponente B mindestens ein Azolopyrimidinylamin der Formel II enthalten, oder wird in den erfindungsgemäßen Verfahren verwendet.

Im Hinblick auf ihre bestimmungsgemäße Verwendung der Verbindungen der Formel II sind die folgenden Bedeutungen der Substituenten, und zwar jeweils für sich allein oder in Kombination, besonders bevorzugt:

Für die erfindungsgemäßen Mischungen kommen insbesondere Verbindungen der Formel II in Betracht, in welchen $E^1$ geradkettiges oder verzweigtes $C_3$-$C_{12}$-Alkyl oder Phenyl, welches durch eine bis drei Halogen- oder $C_1$-$C_4$-Alkylgruppen substituiert sein kann, bedeutet.

In einer Ausgestaltung der Verbindungen der Formel II sind die aliphatischen Ketten in $E^1$ und $E^2$ oder in $E^1$ oder $E^2$ nicht durch $R^a$ substituiert.

Eine bevorzugte Ausgestaltung betrifft Verbindungen der Formel II, in der $E^1$ geradkettiges oder verzweigtes $C_5$-$C_{10}$-Alkyl, insbesondere Ethyl, 3,5,5-Trimethyl-hexyl, n-Heptyl, n-Octyl, n-Nonyl oder n-Decyl bedeutet.

Eine weitere Ausgestaltung betrifft Verbindungen der Formel II, in der $E^1$ Phenyl bedeutet, welches unsubstituiert oder durch eine bis vier Reste $R^b$ substituiert ist.

Bevorzugte Verbindungen der Formel II sind solche, in denen $E^1$ für eine substituierte Phenylgruppe steht, welche einer Gruppe G

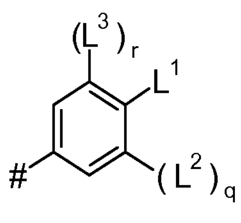

G

entspricht, in welcher

$L^1$ bis $L^3$ Halogen, Cyano, Hydroxy, Mercapto, Nitro, $NR^AR^B$, $C_1$-$C_{10}$-Alkyl, $C_1$-$C_6$-Halogenalkyl, $C_2$-$C_6$-Alkenyl, $C_2$-$C_6$-Alkinyl oder $C_1$-$C_6$-Alkoxy; r und q unabhängig voneinander 0 oder 1 sein können, wobei $NR^AR^B$ wie in Formel II definiert ist und # die Bindung zu dem Azolopyrimidin-Grundgerüst kennzeichnet.

In einer weiteren Ausgestaltung der Verbindungen der Formel II steht $L^1$ für Halogen, Cyano, Hydroxy, Mercapto,

Nitro, $NR^AR^B$, $C_1$-$C_6$-Alkyl, Halogenmethyl, und $C_1$-$C_2$-Alkoxy, bevorzugt für Halogen, Cyano, $C_1$-$C_6$-Alkyl, Halogenmethyl oder $C_1$-$C_2$-Alkoxy.

In einer weiteren Ausgestaltung der Verbindungen der Formel II steht q für 0 oder steht $L^2$ für eine der voranstehend genannten Gruppen und q für 1.

In einer weiteren Ausgestaltung der Verbindungen der Formel II steht r für 0 oder steht $L^3$ für Halogen, Cyano, Hydroxy, Mercapto, Nitro, $NR^AR^B$, $C_1$-$C_6$-Alkyl, Halogenmethyl oder $C_1$-$C_2$-Alkoxy und r für 1. Bevorzugt steht r für 0.

Bevorzugt sind Verbindungen der Formel II, in denen $E^2$ geradekettige oder verzweigtes $C_1$-$C_{12}$-Alkyl, $C_1$-$C_4$-Alkoxy-$C_1$-$C_4$-alkyl oder $C_1$-$C_4$-Halogenalkyl bedeutet.

In einer besonders bevorzugten Ausgestaltung der Verbindungen der Formel II steht $E^2$ für Methyl, Ethyl, n-Propyl, n-Octyl, Trifluormethyl oder Methoxymethyl, insbesondere Methyl, Ethyl, Trifluormethyl oder Methoxymethyl.

Weiterhin sind Verbindungen der Formel II bevorzugt, in denen $E^3$ Wasserstoff bedeutet.

In einer weiteren Ausgestaltung der Verbindungen der Formel I steht $E^3$ für Amino.

Eine Ausgestaltung der Verbindungen der Formel II betrifft solche, in denen A für N steht. Diese Verbindungen entsprechen der Formel IIa, in der die Variablen die Bedeutung gemäß Formel II aufweisen:

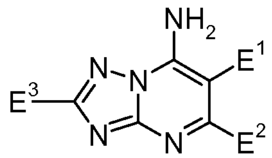

IIa

Eine andere Ausgestaltung der Verbindungen der Formel II betrifft solche, in denen A für CH steht. Diese Verbindungen entsprechen der Formel IIb, in der die Variablen die Bedeutung gemäß Formel II aufweisen:

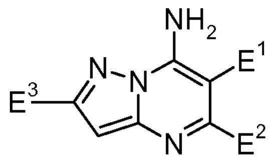

IIb

In einer weiteren Ausgestaltung bevorzugter Verbindungen II weisen die Kohlenstoffreste von $E^1$ und $E^2$ gemeinsam nicht mehr als 12 Kohlenstoffatome auf.

Insbesondere sind im Hinblick auf ihre bestimmungsgemäße Verwendung die in den folgenden Tabellen zusammengestellten Verbindungen II bevorzugt. Die in den Tabellen für einen Substituenten genannten Gruppen stellen außerdem für sich betrachtet, unabhängig von der Kombination in der sie genannt sind, eine besonders bevorzugte Ausgestaltung des betreffenden Substituenten dar.

Tabelle 4

Verbindungen der Formel IIa, in denen die Kombination von $E^1$, $E^2$, und $E^3$ für eine Verbindung jeweils einer Zeile der Tabelle 6 entspricht (Verbindungen 4a-1 bis 4a-298)

Tabelle 5

Verbindungen der Formel IIb, in denen die Kombination von $E^1$, $E^2$, und $E^3$ für eine Verbindung jeweils einer Zeile der Tabelle 6 entspricht (Verbindungen 5b-1 bis 5b-298)

Tabelle 6:

| Nr. | $E^1$ | $E^2$ | $E^3$ |
|---|---|---|---|
| 6-1 | $C_6H_5$ | $CH_3$ | H |
| 6-2 | 2-Cl-$C_6H_4$ | $CH_3$ | H |
| 6-3 | 3-Cl-$C_6H_4$ | $CH_3$ | H |
| 6-4 | 4-Cl-$C_6H_4$ | $CH_3$ | H |
| 6-5 | 2-F-$C_6H_4$ | $CH_3$ | H |

(fortgesetzt)

| Nr. | $E^1$ | $E^2$ | $E^3$ |
|---|---|---|---|
| 6-6 | $3\text{-F-}C_6H_4$ | $CH_3$ | H |
| 6-7 | $4\text{-F-}C_6H_4$ | $CH_3$ | H |
| 6-8 | $2,4\text{-}Cl_2\text{-}C_6H_3$ | $CH_3$ | H |
| 6-9 | $3,4\text{-}Cl_2\text{-}C_6H_3$ | $CH_3$ | H |
| 6-10 | $2,4\text{-}F_2\text{-}C_6H_3$ | $CH_3$ | H |
| 6-11 | $3,4\text{-}F_2\text{-}C_6H_3$ | $CH_3$ | H |
| 6-12 | $4\text{-}CH_3\text{-}C_6H_4$ | $CH_3$ | H |
| 6-13 | $4\text{-}CH_2CH_3\text{-}C_6H_4$ | $CH_3$ | H |
| 6-14 | $4\text{-}CH_2CH_2CH_3\text{-}C_6H_4$ | $CH_3$ | H |
| 6-15 | $4\text{-}CH(CH_3)_2\text{-}C_6H_4$ | $CH_3$ | H |
| 6-16 | $4\text{-}CH_2CH_2CH_2CH_3\text{-}C_6H_4$ | $CH_3$ | H |
| 6-17 | $4\text{-}C(CH_3)CH_2CH_3\text{-}C_6H_4$ | $CH_3$ | H |
| 6-18 | $4\text{-}C(CH_3)_3\text{-}C_6H_4$ | $CH_3$ | H |
| 6-19 | $CH_2CH_2CH_2CH_3$ | $CH_3$ | H |
| 6-20 | $CH_2CH_2CH_2CH_2CH_3$ | $CH_3$ | H |
| 6-21 | $CH_2CH_2CH_2CH_2CH_2CH_3$ | $CH_3$ | H |
| 6-22 | $CH_2CH(CH_3)CH_2CH_2CH_3$ | $CH_3$ | H |
| 6-23 | $CH_2CH(CH_2CH_3)_2$ | $CH_3$ | H |
| 6-24 | $CH_2CH_2CH_2CH_2CH_2CH_2CH_3$ | $CH_3$ | H |
| 6-25 | $CH_2CH_2CH_2CH_2CH_2CH_2CH_2CH_3$ | $CH_3$ | H |
| 6-26 | $CH_2CH_2CH_2CH_2CH_2CH_2CH_2CH_2CH_3$ | $CH_3$ | H |
| 6-27 | $CH_2CH_2CH_2CH_2CH_2CH_2CH_2CH_2CH_2CH_3$ | $CH_3$ | H |
| 6-28 | $CH_2CH_2CH(CH_3)CH_2CH(CH_3)_3$ | $CH_3$ | H |
| 6-29 | $CH_2CH_2CH_2CH_3$ | $CH_3$ | $NH_2$ |
| 6-30 | $CH_2CH_2CH_2CH_2CH_3$ | $CH_3$ | $NH_2$ |
| 6-31 | $CH_2CH_2CH_2CH_2CH_2CH_3$ | $CH_3$ | $NH_2$ |
| 6-32 | $CH_2CH(CH_3)CH_2CH_2CH_3$ | $CH_3$ | $NH_2$ |
| 6-33 | $CH_2CH(CH_2CH_3)_2$ | $CH_3$ | $NH_2$ |
| 6-34 | $CH_2CH_2CH_2CH_2CH_2CH_2CH_3$ | $CH_3$ | $NH_2$ |
| 6-35 | $CH_2CH_2CH_2CH_2CH_2CH_2CH_2CH_3$ | $CH_3$ | $NH_2$ |
| 6-36 | $CH_2CH_2CH_2CH_2CH_2CH_2CH_2CH_2CH_3$ | $CH_3$ | $NH_2$ |
| 6-37 | $CH_2CH_2CH_2CH_2CH_2CH_2CH_2CH_2CH_2CH_3$ | $CH_3$ | $NH_2$ |
| 6-38 | $CH_2CH_2CH(CH_3)CH_2CH(CH_3)_3$ | $CH_3$ | $NH_2$ |
| 6-39 | $CH_2CH_2CH_2CH_3$ | $CH_3$ | $CH_3$ |
| 6-40 | $CH_2CH_2CH_2CH_2CH_3$ | $CH_3$ | $CH_3$ |
| 6-41 | $CH_2CH_2CH_2CH_2CH_2CH_3$ | $CH_3$ | $CH_3$ |
| 6-42 | $CH_2CH(CH_3)CH_2CH_2CH_3$ | $CH_3$ | $CH_3$ |
| 6-43 | $CH_2CH(CH_2CH_3)_2$ | $CH_3$ | $CH_3$ |

(fortgesetzt)

| Nr. | $E^1$ | $E^2$ | $E^3$ |
|---|---|---|---|
| 6-44 | $CH_2CH_2CH_2CH_2CH_2CH_2CH_3$ | $CH_3$ | $CH_3$ |
| 6-45 | $CH_2CH_2CH_2CH_2CH_2CH_2CH_2CH_3$ | $CH_3$ | $CH_3$ |
| 6-46 | $CH_2CH_2CH_2CH_2CH_2CH_2CH_2CH_2CH_3$ | $CH_3$ | $CH_3$ |
| 6-47 | $CH_2CH_2CH_2CH_2CH_2CH_2CH_2CH_2CH_2CH_3$ | $CH_3$ | $CH_3$ |
| 6-48 | $CH_2CH_2CH(CH_3)CH_2CH(CH_3)_3$ | $CH_3$ | $CH_3$ |
| 6-49 | $(CH_2)_3$-O-$CH_3$ | $CH_3$ | H |
| 6-50 | $(CH_2)_3$-O-$CH_2CH_3$ | $CH_3$ | H |
| 6-51 | $(CH_2)_3$-O-$CH_2CH_2CH_3$ | $CH_3$ | H |
| 6-52 | $(CH_2)_3$-O-$CH_2CH_2CH_2CH_3$ | $CH_3$ | H |
| 6-53 | $(CH_2)_3$-O-$CH_2CH_2CH_2CH_2CH_3$ | $CH_3$ | H |
| 6-54 | $(CH_2)_3$-O-$CH_2CH_2CH_2CH_2CH_2CH_3$ | $CH_3$ | H |
| 6-55 | $(CH_2)_3$-O-$CH_2CH_2CH_2CH_2CH_2CH_2CH_3$ | $CH_3$ | H |
| 6-56 | $(CH_2)_3$-O-$CH_2CH_2CH_2CH_2CH_2CH_2CH_2CH_3$ | $CH_3$ | H |
| 6-57 | $(CH_2)_3$-O-$CH_2CH_2CH_2CH_2CH_2CH_2CH_2CH_2CH_3$ | $CH_3$ | H |
| 6-58 | $(CH_2)_3$-O-$CH(CH_3)_2$ | $CH_3$ | H |
| 6-59 | $(CH_2)_3$-O-$C(CH_3)_3$ | $CH_3$ | H |
| 6-60 | $(CH_2)_3$-O-$CH_2C(CH_3)_3$ | $CH_3$ | H |
| 6-61 | $(CH_2)_3$-O-$CH(CH_3)CH_2C(CH_3)_3$ | $CH_3$ | H |
| 6-62 | $(CH_2)_3$-O-$CH(CH_2CH_3)CH_2C(CH_3)_3$ | $CH_3$ | H |
| 6-63 | $(CH_2)_3$-O-$CH_2CH(CH_3)CH_2CH(CH_3)_2$ | $CH_3$ | H |
| 6-64 | $(CH_2)_3$-O-$CH_2CH(CH_2CH_3)CH_2CH_2CH_3$ | $CH_3$ | H |
| 6-65 | $(CH_2)_3$-O-$CH_2CH_2CH(CH_3)CH_2CH(CH_3)_2$ | $CH_3$ | H |
| 6-66 | $(CH_2)_3$-O-$CH_2CH_2CH(CH_3)CH_2C(CH_3)_3$ | $CH_3$ | H |
| 6-67 | $(CH_2)_3$-O-$CH_2CH_2CH(CH_3)CH_2CH_2CH(CH_3)_2$ | $CH_3$ | H |
| 6-68 | $(CH_2)_3$-O-$CH_2CH_2CH(CH_3)CH_2CH_2CH_2CH(CH_3)_2$ | $CH_3$ | H |
| 6-69 | $(CH_2)_3$-O-$CH_3$ | $CH_3$ | $CH_3$ |
| 6-70 | $(CH_2)_3$-O-$CH_2CH_3$ | $CH_3$ | $CH_3$ |
| 6-71 | $(CH_2)_3$-O-$CH_2CH_2CH_3$ | $CH_3$ | $CH_3$ |
| 6-72 | $(CH_2)_3$-O-$CH_2CH_2CH_2CH_3$ | $CH_3$ | $CH_3$ |
| 6-73 | $(CH_2)_3$-O-$CH_2CH_2CH_2CH_2CH_3$ | $CH_3$ | $CH_3$ |
| 6-74 | $(CH_2)_3$-O-$CH_2CH_2CH_2CH_2CH_2CH_3$ | $CH_3$ | $CH_3$ |
| 6-75 | $(CH_2)_3$-O-$CH_2CH_2CH_2CH_2CH_2CH_2CH_3$ | $CH_3$ | $CH_3$ |
| 6-76 | $(CH_2)_3$-O-$CH_2CH_2CH_2CH_2CH_2CH_2CH_2CH_3$ | $CH_3$ | $CH_3$ |
| 6-77 | $(CH_2)_3$-O-$CH_2CH_2CH_2CH_2CH_2CH_2CH_2CH_2CH_3$ | $CH_3$ | $CH_3$ |
| 6-78 | $(CH_2)_3$-O-$CH(CH_3)_2$ | $CH_3$ | $CH_3$ |
| 6-79 | $(CH_2)_3$-O-$C(CH_3)_3$ | $CH_3$ | $CH_3$ |
| 6-80 | $(CH_2)_3$-O-$CH_2C(CH_3)_3$ | $CH_3$ | $CH_3$ |
| 6-81 | $(CH_2)_3$-O-$CH(CH_3)CH_2C(CH_3)_3$ | $CH_3$ | $CH_3$ |

(fortgesetzt)

| Nr. | $E^1$ | $E^2$ | $E^3$ |
|---|---|---|---|
| 6-82 | $(CH_2)_3$-O-$CH(CH_2CH_3)CH_2C(CH_3)_3$ | $CH_3$ | $CH_3$ |
| 6-83 | $(CH_2)_3$-O-$CH_2CH(CH_3)CH_2CH(CH_3)_2$ | $CH_3$ | $CH_3$ |
| 6-84 | $(CH_2)_3$-O-$CH_2CH(CH_2CH_3)CH_2CH_2CH_3$ | $CH_3$ | $CH_3$ |
| 6-85 | $(CH_2)_3$-O-$CH_2CH_2CH(CH_3)CH_2CH(CH_3)_2$ | $CH_3$ | $CH_3$ |
| 6-86 | $(CH_2)_3$-O-$CH_2CH_2CH(CH_3)CH_2C(CH_3)_3$ | $CH_3$ | $CH_3$ |
| 6-87 | $(CH_2)_3$-O-$CH_2CH_2CH(CH_3)CH_2CH_2CH(CH_3)_2$ | $CH_3$ | $CH_3$ |
| 6-88 | $(CH_2)_3$-O-$CH_2CH_2CH(CH_3)CH_2CH_2CH_2CH(CH_3)_2$ | $CH_3$ | $CH_3$ |
| 6-89 | $CH_2$-$C_6H_5$ | $CF_3$ | H |
| 6-90 | $CH_2$-(4-Cl-$C_6H_4$) | $CF_3$ | H |
| 6-91 | $CH_2CH_2CH_3$ | $CF_3$ | H |
| 6-92 | $CH_2CH_2CH_2CH_3$ | $CF_3$ | H |
| 6-93 | $CH_2CH_2CH_2CH_2CH_3$ | $CF_3$ | H |
| 6-94 | $CH_2CH_2CH_2CH_2CH_2CH_3$ | $CF_3$ | H |
| 6-95 | $CH_2CH(CH_3)CH_2CH_2CH_3$ | $CF_3$ | H |
| 6-96 | $CH_2CH(CH_2CH_3)_2$ | $CF_3$ | H |
| 6-97 | $CH_2CH_2CH_2CH_2CH_2CH_2CH_3$ | $CF_3$ | H |
| 6-98 | $CH_2CH_2CH_2CH_2CH_2CH_2CH_2CH_3$ | $CF_3$ | H |
| 6-99 | $CH_2CH_2CH_2CH_2CH_2CH_2CH_2CH_2CH_3$ | $CF_3$ | H |
| 6-100 | $CH_2CH_2CH_2CH_2CH_2CH_2CH_2CH_2CH_2CH_3$ | $CF_3$ | H |
| 6-101 | $CH_2CH_2CH(CH_3)CH_2CH(CH_3)_3$ | $CF_3$ | H |
| 6-102 | cyclo-$C_5H_9$ | $CF_3$ | H |
| 6-103 | cyclo-$C_6H_{11}$ | $CF_3$ | H |
| 6-104 | $CH_2CH_2CH_2CH_3$ | $CH_2CH_3$ | H |
| 6-105 | $CH_2CH_2CH_2CH_2CH_3$ | $CH_2CH_3$ | H |
| 6-106 | $CH_2CH_2CH_2CH_2CH_2CH_3$ | $CH_2CH_3$ | H |
| 6-107 | $CH_2CH(CH_3)CH_2CH_2CH_3$ | $CH_2CH_3$ | H |
| 6-108 | $CH_2CH(CH_2CH_3)_2$ | $CH_2CH_3$ | H |
| 6-109 | $CH_2CH_2CH_2CH_2CH_2CH_2CH_3$ | $CH_2CH_3$ | H |
| 6-110 | $CH_2CH_2CH_2CH_2CH_2CH_2CH_2CH_3$ | $CH_2CH_3$ | H |
| 6-111 | $CH_2CH_2CH_2CH_2CH_2CH_2CH_2CH_2CH_3$ | $CH_2CH_3$ | H |
| 6-112 | $CH_2CH_2CH_2CH_2CH_2CH_2CH_2CH_2CH_2CH_3$ | $CH_2CH_3$ | H |
| 6-113 | $CH_2CH_2CH(CH_3)CH_2CH(CH_3)_3$ | $CH_2CH_3$ | H |
| 6-114 | $CH_2CH_2CH_2CH_3$ | $CH_2CH_3$ | $NH_2$ |
| 6-115 | $CH_2CH_2CH_2CH_2CH_3$ | $CH_2CH_3$ | $NH_2$ |
| 6-116 | $CH_2CH_2CH_2CH_2CH_2CH_3$ | $CH_2CH_3$ | $NH_2$ |
| 6-117 | $CH_2CH(CH_3)CH_2CH_2CH_3$ | $CH_2CH_3$ | $NH_2$ |
| 6-118 | $CH_2CH(CH_2CH_3)_2$ | $CH_2CH_3$ | $NH_2$ |
| 6-119 | $CH_2CH_2CH_2CH_2CH_2CH_2CH_3$ | $CH_2CH_3$ | $NH_2$ |

(fortgesetzt)

| Nr. | $E^1$ | $E^2$ | $E^3$ |
|---|---|---|---|
| 6-120 | $CH_2CH_2CH_2CH_2CH_2CH_2CH_2CH_3$ | $CH_2CH_3$ | $NH_2$ |
| 6-121 | $CH_2CH_2CH_2CH_2CH_2CH_2CH_2CH_2CH_3$ | $CH_2CH_3$ | $NH_2$ |
| 6-122 | $CH_2CH_2CH_2CH_2CH_2CH_2CH_2CH_2CH_2CH_3$ | $CH_2CH_3$ | $NH_2$ |
| 6-123 | $CH_2CH_2CH(CH_3)CH_2CH(CH_3)_3$ | $CH_2CH_3$ | $NH_2$ |
| 6-124 | $CH_2CH_2CH_2CH_3$ | $CH_2CH_3$ | $CH_3$ |
| 6-125 | $CH_2CH_2CH_2CH_2CH_3$ | $CH_2CH_3$ | $CH_3$ |
| 6-126 | $CH_2CH_2CH_2CH_2CH_2CH_3$ | $CH_2CH_3$ | $CH_3$ |
| 6-127 | $CH_2CH(CH_3)CH_2CH_2CH_3$ | $CH_2CH_3$ | $CH_3$ |
| 6-128 | $CH_2CH(CH_2CH_3)_2$ | $CH_2CH_3$ | $CH_3$ |
| 6-129 | $CH_2CH_2CH_2CH_2CH_2CH_2CH_3$ | $CH_2CH_3$ | $CH_3$ |
| 6-130 | $CH_2CH_2CH_2CH_2CH_2CH_2CH_2CH_3$ | $CH_2CH_3$ | $CH_3$ |
| 6-131 | $CH_2CH_2CH_2CH_2CH_2CH_2CH_2CH_2CH_3$ | $CH_2CH_3$ | $CH_3$ |
| 6-132 | $CH_2CH_2CH_2CH_2CH_2CH_2CH_2CH_2CH_2CH_3$ | $CH_2CH_3$ | $CH_3$ |
| 6-133 | $CH_2CH_2CH(CH_3)CH_2CH(CH_3)_3$ | $CH_2CH_3$ | $CH_3$ |
| 6-134 | $(CH_2)_3$-O-$CH_3$ | $CH_2CH_3$ | H |
| 6-135 | $(CH_2)_3$-O-$CH_2CH_3$ | $CH_2CH_3$ | H |
| 6-136 | $(CH_2)_3$-O-$CH_2CH_2CH_3$ | $CH_2CH_3$ | H |
| 6-137 | $(CH_2)_3$-O-$CH_2CH_2CH_2CH_3$ | $CH_2CH_3$ | H |
| 6-138 | $(CH_2)_3$-O-$CH_2CH_2CH_2CH_2CH_3$ | $CH_2CH_3$ | H |
| 6-139 | $(CH_2)_3$-O-$CH_2CH_2CH_2CH_2CH_2CH_3$ | $CH_2CH_3$ | H |
| 6-140 | $(CH_2)_3$-O-$CH_2CH_2CH_2CH_2CH_2CH_2CH_3$ | $CH_2CH_3$ | H |
| 6-141 | $(CH_2)_3$-O-$CH_2CH_2CH_2CH_2CH_2CH_2CH_2CH_3$ | $CH_2CH_3$ | H |
| 6-142 | $(CH_2)_3$-O-$CH_2CH_2CH_2CH_2CH_2CH_2CH_2CH_2CH_3$ | $CH_2CH_3$ | H |
| 6-143 | $(CH_2)_3$-O-$CH(CH_3)_2$ | $CH_2CH_3$ | H |
| 6-144 | $(CH_2)_3$-O-$C(CH_3)_3$ | $CH_2CH_3$ | H |
| 6-145 | $(CH_2)_3$-O-$CH_2C(CH_3)_3$ | $CH_2CH_3$ | H |
| 6-146 | $(CH_2)_3$-O-$CH(CH_3)CH_2C(CH_3)_3$ | $CH_2CH_3$ | H |
| 6-147 | $(CH_2)_3$-O-$CH(CH_2CH_3)CH_2C(CH_3)_3$ | $CH_2CH_3$ | H |
| 6-148 | $(CH_2)_3$-O-$CH_2CH(CH_3)CH_2CH(CH_3)_2$ | $CH_2CH_3$ | H |
| 6-149 | $(CH_2)_3$-O-$CH_2CH(CH_2CH_3)CH_2CH_2CH_3$ | $CH_2CH_3$ | H |
| 6-150 | $(CH_2)_3$-O-$CH_2CH_2CH(CH_3)CH_2CH(CH_3)_2$ | $CH_2CH_3$ | H |
| 6-151 | $(CH_2)_3$-O-$CH_2CH_2CH(CH_3)CH_2C(CH_3)_3$ | $CH_2CH_3$ | H |
| 6-152 | $(CH_2)_3$-O-$CH_2CH_2CH(CH_3)CH_2CH_2CH(CH_3)_2$ | $CH_2CH_3$ | H |
| 6-153 | $(CH_2)_3$-O-$CH_2CH_2CH(CH_3)CH_2CH_2CH_2CH(CH_3)_2$ | $CH_2CH_3$ | H |
| 6-154 | $(CH_2)_3$-O-$CH_3$ | $CH_2CH_3$ | $CH_3$ |
| 6-155 | $(CH_2)_3$-O-$CH_2CH_3$ | $CH_2CH_3$ | $CH_3$ |
| 6-156 | $(CH_2)_3$-O-$CH_2CH_2CH_3$ | $CH_2CH_3$ | $CH_3$ |
| 6-157 | $(CH_2)_3$-O-$CH_2CH_2CH_2CH_3$ | $CH_2CH_3$ | $CH_3$ |

(fortgesetzt)

| Nr. | $E^1$ | $E^2$ | $E^3$ |
|---|---|---|---|
| 6-158 | $(CH_2)_3$-O-$CH_2CH_2CH_2CH_2CH_3$ | $CH_2CH_3$ | $CH_3$ |
| 6-159 | $(CH_2)_3$-O-$CH_2CH_2CH_2CH_2CH_2CH_3$ | $CH_2CH_3$ | $CH_3$ |
| 6-160 | $(CH_2)_3$-O-$CH_2CH_2CH_2CH_2CH_2CH_2CH_3$ | $CH_2CH_3$ | $CH_3$ |
| 6-161 | $(CH_2)_3$-O-$CH_2CH_2CH_2CH_2CH_2CH_2CH_2CH_3$ | $CH_2CH_3$ | $CH_3$ |
| 6-162 | $(CH_2)_3$-O-$CH_2CH_2CH_2CH_2CH_2CH_2CH_2CH_2CH_3$ | $CH_2CH_3$ | $CH_3$ |
| 6-163 | $(CH_2)_3$-O-$CH(CH_3)_2$ | $CH_2CH_3$ | $CH_3$ |
| 6-164 | $(CH_2)_3$-O-$C(CH_3)_3$ | $CH_2CH_3$ | $CH_3$ |
| 6-165 | $(CH_2)_3$-O-$CH_2C(CH_3)_3$ | $CH_2CH_3$ | $CH_3$ |
| 6-166 | $(CH_2)_3$-O-$CH(CH_3)CH_2C(CH_3)_3$ | $CH_2CH_3$ | $CH_3$ |
| 6-167 | $(CH_2)_3$-O-$CH(CH_2CH_3)CH_2C(CH_3)_3$ | $CH_2CH_3$ | $CH_3$ |
| 6-168 | $(CH_2)_3$-O-$CH_2CH(CH_3)CH_2CH(CH_3)_2$ | $CH_2CH_3$ | $CH_3$ |
| 6-169 | $(CH_2)_3$-O-$CH_2CH(CH_2CH_3)CH_2CH_2CH_3$ | $CH_2CH_3$ | $CH_3$ |
| 6-170 | $(CH_2)_3$-O-$CH_2CH_2CH(CH_3)CH_2CH(CH_3)_2$ | $CH_2CH_3$ | $CH_3$ |
| 6-171 | $(CH_2)_3$-O-$CH_2CH_2CH(CH_3)CH_2C(CH_3)_3$ | $CH_2CH_3$ | $CH_3$ |
| 6-172 | $(CH_2)_3$-O-$CH_2CH_2CH(CH_3)CH_2CH_2CH(CH_3)_2$ | $CH_2CH_3$ | $CH_3$ |
| 6-173 | $(CH_2)_3$-O-$CH_2CH_2CH(CH_3)CH_2CH_2CH_2CH(CH_3)_2$ | $CH_2CH_3$ | $CH_3$ |
| 6-174 | $CH_2CH_2CH_2CH_3$ | $CH_2CH_2CH_3$ | H |
| 6-175 | $CH_2CH_2CH_2CH_2CH_3$ | $CH_2CH_2CH_3$ | H |
| 6-176 | $CH_2CH_2CH_2CH_2CH_2CH_3$ | $CH_2CH_2CH_3$ | H |
| 6-177 | $CH_2CH(CH_3)CH_2CH_2CH_3$ | $CH_2CH_2CH_3$ | H |
| 6-178 | $CH_2CH(CH_2CH_3)_2$ | $CH_2CH_2CH_3$ | H |
| 6-179 | $CH_2CH_2CH_2CH_2CH_2CH_2CH_3$ | $CH_2CH_2CH_3$ | H |
| 6-180 | $CH_2CH_2CH_2CH_2CH_2CH_2CH_2CH_3$ | $CH_2CH_2CH_3$ | H |
| 6-181 | $CH_2CH_2CH_2CH_2CH_2CH_2CH_2CH_2CH_3$ | $CH_2CH_2CH_3$ | H |
| 6-182 | $CH_2CH_2CH_2CH_2CH_2CH_2CH_2CH_2CH_2CH_3$ | $CH_2CH_2CH_3$ | H |
| 6-183 | $CH_2CH_2CH(CH_3)CH_2CH(CH_3)_3$ | $CH_2CH_2CH_3$ | H |
| 6-184 | $CH_2$-O-$CH_2CH_2CH_2CH_3$ | $CH_2CH_2CH_3$ | H |
| 6-185 | $CH_2$-O-$CH_2CH_2CH_2CH_2CH_3$ | $CH_2CH_2CH_3$ | H |
| 6-186 | $CH_2$-O-$CH_2CH_2CH_2CH_2CH_2CH_3$ | $CH_2CH_2CH_3$ | H |
| 6-187 | $CH_2$-O-$CH_2CH_2CH_2CH_2CH_2CH_2CH_3$ | $CH_2CH_2CH_3$ | H |
| 6-188 | $CH_2$-O-$CH_2CH_2CH_2CH_2CH_2CH_2CH_2CH_3$ | $CH_2CH_2CH_3$ | H |
| 6-189 | $CH_2$-O-$CH_2CH_2CH_2CH_2CH_2CH_2CH_2CH_2CH_3$ | $CH_2CH_2CH_3$ | H |
| 6-190 | $CH_2$-O-$C(CH_3)_3$ | $CH_2CH_2CH_3$ | H |
| 6-191 | $CH_2$-O-$CH_2C(CH_3)_3$ | $CH_2CH_2CH_3$ | H |
| 6-192 | $CH_2$-O-$CH(CH_3)CH_2C(CH_3)_3$ | $CH_2CH_2CH_3$ | H |
| 6-193 | $CH_2$-O-$CH(CH_2CH_3)CH_2C(CH_3)_3$ | $CH_2CH_2CH_3$ | H |
| 6-194 | $CH_2$-O-$CH_2CH(CH_3)CH_2CH(CH_3)_2$ | $CH_2CH_2CH_3$ | H |
| 6-195 | $CH_2$-O-$CH_2CH(CH_2CH_3)CH_2CH_2CH_3$ | $CH_2CH_2CH_3$ | H |

(fortgesetzt)

| Nr. | $E^1$ | $E^2$ | $E^3$ |
|---|---|---|---|
| 6-196 | $CH_2$-O-$CH_2CH_2CH(CH_3)CH_2CH(CH_3)_2$ | $CH_2CH_2CH_3$ | H |
| 6-197 | $CH_2$-O-$CH_2CH_2CH(CH_3)CH_2C(CH_3)_3$ | $CH_2CH_2CH_3$ | H |
| 6-198 | $CH_2$-O-$CH_2CH_2CH(CH_3)CH_2CH_2CH(CH_3)_2$ | $CH_2CH_2CH_3$ | H |
| 6-199 | $CH_2$-O-$CH_2CH_2CH(CH_2)CH_2CH_2CH_2CH(CH_3)_2$ | $CH_2CH_2CH_3$ | H |
| 6-200 | $(CH_2)_2$-O-$CH_2CH_2CH_3$ | $CH_2CH_2CH_3$ | H |
| 6-201 | $(CH_2)_2$-O-$CH_2CH_2CH_2CH_3$ | $CH_2CH_2CH_3$ | H |
| 6-202 | $(CH_2)_2$-O-$CH_2CH_2CH_2CH_2CH_3$ | $CH_2CH_2CH_3$ | H |
| 6-203 | $(CH_2)_2$-O-$CH_2CH_2CH_2CH_2CH_2CH_3$ | $CH_2CH_2CH_3$ | H |
| 6-204 | $(CH_2)_2$-O-$CH_2CH_2CH_2CH_2CH_2CH_2CH_3$ | $CH_2CH_2CH_3$ | H |
| 6-205 | $(CH_2)_2$-O-$CH_2CH_2CH_2CH_2CH_2CH_2CH_2CH_3$ | $CH_2CH_2CH_3$ | H |
| 6-206 | $(CH_2)_2$-O-$CH_2CH_2CH_2CH_2CH_2CH_2CH_2CH_2CH_3$ | $CH_2CH_2CH_3$ | H |
| 6-207 | $(CH_2)_2$-O-$CH(CH_3)_2$ | $CH_2CH_2CH_3$ | H |
| 6-208 | $(CH_2)_2$-O-$C(CH_3)_3$ | $CH_2CH_2CH_3$ | H |
| 6-209 | $(CH_2)_2$-O-$CH_2C(CH_3)_3$ | $CH_2CH_2CH_3$ | H |
| 6-210 | $(CH_2)_2$-O-$CH(CH_3)CH_2C(CH_3)_3$ | $CH_2CH_2CH_3$ | H |
| 6-211 | $(CH_2)_2$-O-$CH(CH_2CH_3)CH_2C(CH_3)_3$ | $CH_2CH_2CH_3$ | H |
| 6-212 | $(CH_2)_2$-O-$CH_2CH(CH_3)CH_2CH(CH_3)_2$ | $CH_2CH_2CH_3$ | H |
| 6-213 | $(CH_2)_2$-O-$CH_2CH(CH_2CH_3)CH_2CH_2CH_3$ | $CH_2CH_2CH_3$ | H |
| 6-214 | $(CH_2)_2$-O-$CH_2CH_2CH(CH_3)CH_2CH(CH_3)_2$ | $CH_2CH_2CH_3$ | H |
| 6-215 | $(CH_2)_2$-O-$CH_2CH_2CH(CH_3)CH_2C(CH_3)_3$ | $CH_2CH_2CH_3$ | H |
| 6-216 | $(CH_2)_2$-O-$CH_2CH_2CH(CH_3)CH_2CH_2CH(CH_3)_2$ | $CH_2CH_2CH_3$ | H |
| 6-217 | $(CH_2)_2$-O-$CH_2CH_2CH(CH_2)CH_2CH_2CH_2CH(CH_3)_2$ | $CH_2CH_2CH_3$ | H |
| 6-218 | $(CH_2)_3$-O-$CH_3$ | $CH_2CH_2CH_3$ | H |
| 6-219 | $(CH_2)_3$-O-$CH_2CH_3$ | $CH_2CH_2CH_3$ | H |
| 6-220 | $(CH_2)_3$-O-$CH_2CH_2CH_3$ | $CH_2CH_2CH_3$ | H |
| 6-221 | $(CH_2)_3$-O-$CH_2CH_2CH_2CH_3$ | $CH_2CH_2CH_3$ | H |
| 6-222 | $(CH_2)_3$-O-$CH_2CH_2CH_2CH_2CH_3$ | $CH_2CH_2CH_3$ | H |
| 6-223 | $(CH_2)_3$-O-$CH_2CH_2CH_2CH_2CH_2CH_3$ | $CH_2CH_2CH_3$ | H |
| 6-224 | $(CH_2)_3$-O-$CH_2CH_2CH_2CH_2CH_2CH_2CH_3$ | $CH_2CH_2CH_3$ | H |
| 6-225 | $(CH_2)_3$-O-$CH_2CH_2CH_2CH_2CH_2CH_2CH_2CH_3$ | $CH_2CH_2CH_3$ | H |
| 6-226 | $(CH_2)_3$-O-$CH_2CH_2CH_2CH_2CH_2CH_2CH_2CH_2CH_3$ | $CH_2CH_2CH_3$ | H |
| 6-227 | $(CH_2)_3$-O-$CH(CH_3)_2$ | $CH_2CH_2CH_3$ | H |
| 6-228 | $(CH_2)_3$-O-$C(CH_3)_3$ | $CH_2CH_2CH_3$ | H |
| 6-229 | $(CH_2)_3$-O-$CH_2C(CH_3)_3$ | $CH_2CH_2CH_3$ | H |
| 6-230 | $(CH_2)_3$-O-$CH(CH_3)CH_2C(CH_3)_3$ | $CH_2CH_2CH_3$ | H |
| 6-231 | $(CH_2)_3$-O-$CH(CH_2CH_3)CH_2C(CH_3)_3$ | $CH_2CH_2CH_3$ | H |
| 6-232 | $(CH_2)_3$-O-$CH_2CH(CH_3)CH_2CH(CH_3)_2$ | $CH_2CH_2CH_3$ | H |
| 6-233 | $(CH_2)_3$-O-$CH_2CH(CH_2CH_3)CH_2CH_2CH_3$ | $CH_2CH_2CH_3$ | H |

(fortgesetzt)

| Nr. | $E^1$ | $E^2$ | $E^3$ |
|---|---|---|---|
| 6-234 | $(CH_2)_3$-O-$CH_2CH_2CH(CH_3)CH_2CH(CH_3)_2$ | $CH_2CH_2CH_3$ | H |
| 6-235 | $(CH_2)_3$-O-$CH_2CH_2CH(CH_3)CH_2C(CH_3)_3$ | $CH_2CH_2CH_3$ | H |
| 6-236 | $(CH_2)_3$-O-$CH_2CH_2CH(CH_3)CH_2CH_2CH(CH_3)_2$ | $CH_2CH_2CH_3$ | H |
| 6-237 | $(CH_2)_3$-O-$CH_2CH_2CH(CH_3)CH_2CH_2CH_2CH(CH_3)_2$ | $CH_2CH_2CH_3$ | H |
| 6-238 | $CH_2CH_2CH_3$ | $CH_2OCH_3$ | H |
| 6-239 | $CH_2CH_2CH_2CH_3$ | $CH_2OCH_3$ | H |
| 6-240 | $CH_2CH_2CH_2CH_2CH_3$ | $CH_2OCH_3$ | H |
| 6-241 | $CH_2CH_2CH_2CH_2CH_2CH_3$ | $CH_2OCH_3$ | H |
| 6-242 | $CH_2CH(CH_3)CH_2CH_2CH_3$ | $CH_2OCH_3$ | H |
| 6-243 | $CH_2CH(CH_2CH_3)_2$ | $CH_2OCH_3$ | H |
| 6-244 | $CH_2CH_2CH_2CH_2CH_2CH_2CH_3$ | $CH_2OCH_3$ | H |
| 6-245 | $CH_2CH_2CH_2CH_2CH_2CH_2CH_2CH_3$ | $CH_2OCH_3$ | H |
| 6-246 | $CH_2CH_2CH_2CH_2CH_2CH_2CH_2CH_2CH_3$ | $CH_2OCH_3$ | H |
| 6-247 | $CH_2CH_2CH_2CH_2CH_2CH_2CH_2CH_2CH_2CH_3$ | $CH_2OCH_3$ | H |
| 6-248 | $CH_2CH_2CH(CH_3)CH_2CH(CH_3)_3$ | $CH_2OCH_3$ | H |
| 6-249 | $(CH_2)_3$-O-$CH_3$ | $CH_2OCH_3$ | H |
| 6-250 | $(CH_2)_3$-O-$CH_2CH_3$ | $CH_2OCH_3$ | H |
| 6-251 | $(CH_2)_3$-O-$CH_2CH_2CH_3$ | $CH_2CH_3$ | H |
| 6-252 | $(CH_2)_3$-O-$CH_2CH_2CH_2CH_3$ | $CH_2OCH_3$ | H |
| 6-253 | $(CH_2)_3$-O-$CH_2CH_2CH_2CH_2CH_3$ | $CH_2OCH_3$ | H |
| 6-254 | $(CH_2)_3$-O-$CH_2CH_2CH_2CH_2CH_2CH_3$ | $CH_2OCH_3$ | H |
| 6-255 | $(CH_2)_3$-O-$CH_2CH_2CH_2CH_2CH_2CH_2CH_3$ | $CH_2OCH_3$ | H |
| 6-256 | $(CH_2)_3$-O-$CH_2CH_2CH_2CH_2CH_2CH_2CH_2CH_3$ | $CH_2OCH_3$ | H |
| 6-257 | $(CH_2)_3$-O-$CH_2CH_2CH_2CH_2CH_2CH_2CH_2CH_2CH_3$ | $CH_2OCH_3$ | H |
| 6-258 | $(CH_2)_3$-O-$CH(CH_3)_2$ | $CH_2OCH_3$ | H |
| 6-259 | $(CH_2)_3$-O-$C(CH_3)_3$ | $CH_2OCH_3$ | H |
| 6-260 | $(CH_2)_3$-O-$CH_2C(CH_3)_3$ | $CH_2OCH_3$ | H |
| 6-261 | $(CH_2)_3$-O-$CH(CH_3)CH_2C(CH_3)_3$ | $CH_2OCH_3$ | H |
| 6-262 | $(CH_2)_3$-O-$CH(CH_2CH_3)CH_2C(CH_3)_3$ | $CH_2OCH_3$ | H |
| 6-263 | $(CH_2)_3$-O-$CH_2CH(CH_3)CH_2CH(CH_3)_2$ | $CH_2OCH_3$ | H |
| 6-264 | $(CH_2)_3$-O-$CH_2CH(CH_2CH_3)CH_2CH_2CH_3$ | $CH_2OCH_3$ | H |
| 6-265 | $(CH_2)_3$-O-$CH_2CH_2CH(CH_3)CH_2CH(CH_3)_2$ | $CH_2OCH_3$ | H |
| 6-266 | $(CH_2)_3$-O-$CH_2CH_2CH(CH_3)CH_2C(CH_3)_3$ | $CH_2OCH_3$ | H |
| 6-267 | $(CH_2)_3$-O-$CH_2CH_2CH(CH_3)CH_2CH_2CH(CH_3)_2$ | $CH_2OCH_3$ | H |
| 6-268 | $(CH_2)_3$-O-$CH_2CH_2CH(CH_3)CH_2CH_2CH_2CH(CH_3)_2$ | $CH_2OCH_3$ | H |
| 6-269 | $CH_3$ | $(CH_2)_3CH_3$ | H |
| 6-270 | $CH_2CH_3$ | $(CH_2)_3CH_3$ | H |
| 6-271 | $CH_2CH_2CH_3$ | $(CH_2)_3CH_3$ | H |

(fortgesetzt)

| Nr. | $E^1$ | $E^2$ | $E^3$ |
|---|---|---|---|
| 6-272 | $CH_2CH_2CH_2CH_3$ | $(CH_2)_3CH_3$ | H |
| 6-273 | $CH_2CH_2CH_2CH_2CH_3$ | $(CH_2)_3CH_3$ | H |
| 6-274 | $CH_3$ | $(CH_2)_4CH_3$ | H |
| 6-275 | $CH_2CH_3$ | $(CH_2)_4CH_3$ | H |
| 6-276 | $CH_2CH_2CH_3$ | $(CH_2)_4CH_3$ | H |
| 6-277 | $CH_2CH_2CH_2CH_3$ | $(CH_2)_4CH_3$ | H |
| 6-278 | $CH_2CH_2CH_2CH_2CH_3$ | $(CH_2)_4CH_3$ | H |
| 6-279 | $CH_3$ | $(CH_2)_5CH_3$ | H |
| 6-280 | $CH_2CH_3$ | $(CH_2)_5CH_3$ | H |
| 6-281 | $CH_2CH_2CH_3$ | $(CH_2)_5CH_3$ | H |
| 6-282 | $CH_2CH_2CH_2CH_3$ | $(CH_2)_5CH_3$ | H |
| 6-283 | $CH_2CH_2CH_2CH_2CH_3$ | $(CH_2)_5CH_3$ | H |
| 6-284 | $CH_3$ | $(CH_2)_6CH_3$ | H |
| 6-285 | $CH_2CH_3$ | $(CH_2)_6CH_3$ | H |
| 6-286 | $CH_2CH_2CH_3$ | $(CH_2)_6CH_3$ | H |
| 6-287 | $CH_2CH_2CH_2CH_3$ | $(CH_2)_6CH_3$ | H |
| 6-288 | $CH_2CH_2CH_2CH_2CH_3$ | $(CH_2)_6CH_3$ | H |
| 6-289 | $CH_3$ | $(CH_2)_7CH_3$ | H |
| 6-290 | $CH_2CH_3$ | $(CH_2)_7CH_3$ | H |
| 6-291 | $CH_2CH_2CH_3$ | $(CH_2)_7CH_3$ | H |
| 6-292 | $CH_2CH_2CH_2CH_3$ | $(CH_2)_7CH_3$ | H |
| 6-293 | $CH_2CH_2CH_2CH_2CH_3$ | $(CH_2)_7CH_3$ | H |
| 6-294 | $CH_3$ | $(CH_2)_8CH_3$ | H |
| 6-295 | $CH_2CH_3$ | $(CH_2)_8CH_3$ | H |
| 6-296 | $CH_2CH_2CH_3$ | $(CH_2)_8CH_3$ | H |
| 6-297 | $CH_2CH_2CH_2CH_3$ | $(CH_2)_8CH_3$ | H |
| 6-298 | $CH_2CH_2CH_2CH_2CH_3$ | $(CH_2)_8CH_3$ | H |

Alternativ zu oder in Kombination mit mindestens einer Verbindung 4a-1 bis 4a-298 kann auch mindestens eine Verbindung 5b-1 bis 5b-298 in den bevorzugten erfindungsgemäßen Verfahren zur Bekämpfung von pflanzenpathogenen Schadpilzen eingesetzt werden. Darüberhinaus, werden erfindungsgemäßen Verfahren zur Herstellung der erfindungsgemäßen Mittel und Saatgut enthaltend mindestens eine Verbindung der Tabellen 4 oder 5 verwendet.

Bevorzugte Ausgestaltungen der erfindungsgemäßen Mischungen enthalten als Komponente B mindestens eine, bevorzugt eine, Verbindung der Formel II ausgewählt aus Tabelle 7:

Tabelle 7:

| Nr. | Verbindung |
|---|---|
| 7-1 | 6-(3,4-Dichlor-phenyl)-5-methyl-[1,2,4]triazolo[1,5-a]pyrimidin-7-ylamin |
| 7-2 | 6-(4-tert-Butyl-phenyl)-5-methyl-[1,2,4]triazolo[1,5-a]pyrimidin-7-ylamin |
| 7-3 | 5-Methyl-6-(3,5,5-trimethyl-hexyl)-[1,2,4]triazolo[1,5-a]pyrimidin-7-ylamin |

(fortgesetzt)

| Nr. | Verbindung |
|---|---|
| 7-4 | 5-Methyl-6-octyl-[1,2,4]triazolo[1,5-a]pyrimidin-7-ylamin |
| 7-5 | 5-Ethyl-6-octyl-[1,2,4]triazolo[1,5-a]pyrimidin-2,7-diamin |
| 7-6 | 6-Ethyl-5-octyl-[1,2,4]triazolo[1,5-a]pyrimidin-7-ylamin |
| 7-7 | 5-Ethyl-6-octyl-[1,2,4]triazolo[1,5-a]pyrimidin-7-ylamin |
| 7-8 | 5-Ethyl-6-(3,5,5-trimethyl-hexyl)-[1,2,4]triazolo[1,5-a]pyrimidin-7-ylamin |
| 7-9 | 6-Octyl-5-propyl-[1,2,4]triazolo[1,5-a]pyrimidin-7-ylamin |
| 7-10 | 5-Methoxymethyl-6-octyl-[1,2,4]triazolo[1,5-a]pyrimidin-7-ylamin |
| 7-11 | 6-Octyl-5-trifluormethyl-[1,2,4]triazolo[1,5-a]pyrimidin-7-ylamin |
| 7-12 | 5-Trifluormethyl-6-(3,5,5-trimethyl-hexyl)-[1,2,4]triazolo[1,5-a]pyrimidin-7-ylamin |

Gemäß einer weiteren bevorzugten Ausgestaltung enthalten die erfindungsgemäßen Mischungen als Komponente A mindestens eine Verbindung I, ausgewählt aus Tabelle 3, und als Komponente B mindestens eine Verbindung II, ausgewählt aus Tabelle 7. Bevorzugte erfindungsgemäße Mischungen, enthalten jeweils eine Verbindung I und eine Verbindung II (binäre Kombination) als Komponente A und B. Diese binären Kombinationen sind in der folgenden Tabelle 8 aufgeführt:

Tabelle 8:

| Binäre Kombination | Verbindung I | Verbindung II | Binäre Kombination | Verbindung I | Verbindung II |
|---|---|---|---|---|---|
| 8-1 | 3-1 | 7-1 | 8-33 | 3-3 | 7-9 |
| 8-2 | 3-1 | 7-2 | 8-34 | 3-3 | 7-10 |
| 8-3 | 3-1 | 7-3 | 8-35 | 3-3 | 7-11 |
| 8-4 | 3-1 | 7-4 | 8-36 | 3-3 | 7-12 |
| 8-5 | 3-1 | 7-5 | 8-37 | 3-4 | 7-1 |
| 8-6 | 3-1 | 7-6 | 8-38 | 3-4 | 7-2 |
| 8-7 | 3-1 | 7-7 | 8-39 | 3-4 | 7-3 |
| 8-8 | 3-1 | 7-8 | 8-40 | 3-4 | 7-4 |
| 8-9 | 3-1 | 7-9 | 8-41 | 3-4 | 7-5 |
| 8-10 | 3-1 | 7-10 | 8-42 | 3-4 | 7-6 |
| 8-11 | 3-1 | 7-11 | 8-43 | 3-4 | 7-7 |
| 8-12 | 3-1 | 7-12 | 8-44 | 3-4 | 7-8 |
| 8-13 | 3-2 | 7-1 | 8-45 | 3-4 | 7-9 |
| 8-14 | 3-2 | 7-2 | 8-46 | 3-4 | 7-10 |
| 8-15 | 3-2 | 7-3 | 8-47 | 3-4 | 7-11 |
| 8-16 | 3-2 | 7-4 | 8-48 | 3-4 | 7-12 |
| 8-17 | 3-2 | 7-5 | 8-49 | 3-5 | 7-1 |
| 8-18 | 3-2 | 7-6 | 8-50 | 3-5 | 7-2 |
| 8-19 | 3-2 | 7-7 | 8-51 | 3-5 | 7-3 |
| 8-20 | 3-2 | 7-8 | 8-52 | 3-5 | 7-4 |
| 8-21 | 3-2 | 7-9 | 8-53 | 3-5 | 7-5 |
| 8-22 | 3-2 | 7-10 | 8-54 | 3-5 | 7-6 |
| 8-23 | 3-2 | 7-11 | 8-55 | 3-5 | 7-7 |
| 8-24 | 3-2 | 7-12 | 8-56 | 3-5 | 7-8 |
| 8-25 | 3-3 | 7-1 | 8-57 | 3-5 | 7-9 |
| 8-26 | 3-3 | 7-2 | 8-58 | 3-5 | 7-10 |
| 8-27 | 3-3 | 7-3 | 8-59 | 3-5 | 7-11 |
| 8-28 | 3-3 | 7-4 | 8-60 | 3-5 | 7-12 |
| 8-29 | 3-3 | 7-5 | 8-61 | 3-6 | 7-1 |
| 8-30 | 3-3 | 7-6 | 8-62 | 3-6 | 7-2 |
| 8-31 | 3-3 | 7-7 | 8-63 | 3-6 | 7-3 |
| 8-32 | 3-3 | 7-8 | 8-64 | 3-6 | 7-4 |

| Binäre Kombination | Verbindung I | Verbindung II |
|---|---|---|
| 8-65 | 3-6 | 7-5 |
| 8-66 | 3-6 | 7-6 |
| 8-67 | 3-6 | 7-7 |
| 8-68 | 3-6 | 7-8 |
| 8-69 | 3-6 | 7-9 |
| 8-70 | 3-6 | 7-10 |
| 8-71 | 3-6 | 7-11 |
| 8-72 | 3-6 | 7-12 |
| 8-73 | 3-7 | 7-1 |
| 8-74 | 3-7 | 7-2 |
| 8-75 | 3-7 | 7-3 |
| 8-76 | 3-7 | 7-4 |
| 8-77 | 3-7 | 7-5 |
| 8-78 | 3-7 | 7-6 |
| 8-79 | 3-7 | 7-7 |
| 8-80 | 3-7 | 7-8 |
| 8-81 | 3-7 | 7-9 |
| 8-82 | 3-7 | 7-10 |
| 8-83 | 3-7 | 7-11 |
| 8-84 | 3-7 | 7-12 |
| 8-85 | 3-8 | 7-1 |
| 8-86 | 3-8 | 7-2 |
| 8-87 | 3-8 | 7-3 |
| 8-88 | 3-8 | 7-4 |
| 8-89 | 3-8 | 7-5 |
| 8-90 | 3-8 | 7-6 |
| 8-91 | 3-8 | 7-7 |
| 8-92 | 3-8 | 7-8 |
| 8-93 | 3-8 | 7-9 |
| 8-94 | 3-8 | 7-10 |
| 8-95 | 3-8 | 7-11 |
| 8-96 | 3-8 | 7-12 |
| 8-97 | 3-9 | 7-1 |
| 8-98 | 3-9 | 7-2 |
| 8-99 | 3-9 | 7-3 |
| 8-100 | 3-9 | 7-4 |
| 8-101 | 3-9 | 7-5 |
| 8-102 | 3-9 | 7-6 |
| 8-103 | 3-9 | 7-7 |
| 8-104 | 3-9 | 7-8 |

| Binäre Kombination | Verbindung I | Verbindung II |
|---|---|---|
| 8-105 | 3-9 | 7-9 |
| 8-106 | 3-9 | 7-10 |
| 8-107 | 3-9 | 7-11 |
| 8-108 | 3-9 | 7-12 |
| 8-109 | 3-10 | 7-1 |
| 8-110 | 3-10 | 7-2 |
| 8-111 | 3-10 | 7-3 |
| 8-112 | 3-10 | 7-4 |
| 8-113 | 3-10 | 7-5 |
| 8-114 | 3-10 | 7-6 |
| 8-115 | 3-10 | 7-7 |
| 8-116 | 3-10 | 7-8 |
| 8-117 | 3-10 | 7-9 |
| 8-118 | 3-10 | 7-10 |
| 8-119 | 3-10 | 7-11 |
| 8-120 | 3-10 | 7-12 |
| 8-121 | 3-11 | 7-1 |
| 8-122 | 3-11 | 7-2 |
| 8-123 | 3-11 | 7-3 |
| 8-124 | 3-11 | 7-4 |
| 8-125 | 3-11 | 7-5 |
| 8-126 | 3-11 | 7-6 |
| 8-127 | 3-11 | 7-7 |
| 8-128 | 3-11 | 7-8 |
| 8-129 | 3-11 | 7-9 |
| 8-130 | 3-11 | 7-10 |
| 8-131 | 3-11 | 7-11 |
| 8-132 | 3-11 | 7-12 |
| 8-133 | 3-12 | 7-1 |
| 8-134 | 3-12 | 7-2 |
| 8-135 | 3-12 | 7-3 |
| 8-136 | 3-12 | 7-4 |
| 8-137 | 3-12 | 7-5 |
| 8-138 | 3-12 | 7-6 |
| 8-139 | 3-12 | 7-7 |
| 8-140 | 3-12 | 7-8 |
| 8-141 | 3-12 | 7-9 |
| 8-142 | 3-12 | 7-10 |
| 8-143 | 3-12 | 7-11 |
| 8-144 | 3-12 | 7-12 |

| Binäre Kombination | Verbindung I | Verbindung II |
|---|---|---|
| 8-145 | 3-13 | 7-1 |
| 8-146 | 3-13 | 7-2 |
| 8-147 | 3-13 | 7-3 |
| 8-148 | 3-13 | 7-4 |
| 8-149 | 3-13 | 7-5 |
| 8-150 | 3-13 | 7-6 |
| 8-151 | 3-13 | 7-7 |
| 8-152 | 3-13 | 7-8 |
| 8-153 | 3-13 | 7-9 |
| 8-154 | 3-13 | 7-10 |
| 8-155 | 3-13 | 7-11 |
| 8-156 | 3-13 | 7-12 |
| 8-157 | 3-14 | 7-1 |
| 8-158 | 3-14 | 7-2 |
| 8-159 | 3-14 | 7-3 |
| 8-160 | 3-14 | 7-4 |
| 8-161 | 3-14 | 7-5 |
| 8-162 | 3-14 | 7-6 |

| Binäre Kombination | Verbindung I | Verbindung II |
|---|---|---|
| 8-163 | 3-14 | 7-7 |
| 8-164 | 3-14 | 7-8 |
| 8-165 | 3-14 | 7-9 |
| 8-166 | 3-14 | 7-10 |
| 8-167 | 3-14 | 7-11 |
| 8-168 | 3-14 | 7-12 |
| 8-169 | 3-15 | 7-1 |
| 8-170 | 3-15 | 7-2 |
| 8-171 | 3-15 | 7-3 |
| 8-172 | 3-15 | 7-4 |
| 8-173 | 3-15 | 7-5 |
| 8-174 | 3-15 | 7-6 |
| 8-175 | 3-15 | 7-7 |
| 8-176 | 3-15 | 7-8 |
| 8-177 | 3-15 | 7-9 |
| 8-178 | 3-15 | 7-10 |
| 8-179 | 3-15 | 7-11 |
| 8-180 | 3-15 | 7-12 |

In Kombination mit mindestens einer binären Kombination 8-1 bis 8-180 kann auch mindestens eine weitere aktive Komponente C, ausgewählt aus Tabelle 9, in den bevorzugten erfindungsgemäßen Verfahren zur Bekämpfung von pflanzenpathogenen Schadpilzen eingesetzt werden. Darüberhinaus, werden erfindungsgemäße Verfahren zur Herstellung der erfindungsgemäßen Mittel und Saatgut, enthaltend mindestens eine binäre Kombination 8-1 bis 8-180 sowie als weitere aktive Komponente C mindestens einen weiteren Wirkstoff aus Tabelle 9, verwendet.

Gemäß einer weiteren bevorzugten Ausgestaltung enthalten die erfindungsgemäßen Mischungen als Komponente A mindestens eine Verbindung I, ausgewählt aus Tabelle 3, als Komponente B mindestens eine Verbindung II, ausgewählt aus Tabelle 7, sowie als weitere aktive Komponente C mindestens einen weiteren Wirkstoff aus Tabelle 9.

Tabelle 9:

| **Wirkstoffgruppe** | **Beispiele** |
|---|---|
| Azole | Bitertanol, Bromuconazole, Cyproconazole, Difenoconazole, Diniconazole, Enilconazol, Epoxiconazole, Fluquinconazole, Fenbuconazole, Flusilazole, Flutriafol, Hexaconazol, Imibenconazole, Ipconazole, Metconazol, Myclobutanil, Penconazole, Propiconazole, Prothioconazole, Simeconazole, Triadimefon, Triadimenol, Tebuconazole, Tetraconazole, Triticonazole, Prochloraz, Pefurazoate, Imazalil, Triflumizole, Cyazofamid, Benomyl, Car-bendazim, Thiabendazole, Fuberidazole, Ethaboxam, Etridiazole, Hymexazole |
| Strobilurine | Azoxystrobin, Dimoxystrobin, Enestroburin, Fluoxastrobin, Kresoxim-methyl, Metominostrobin, Orysastrobin, Picoxystrobin, Pyraclostrobin, Trifloxystrobin, oder (2-Chlor-5-[1-(3-methyl-benzyloxyimino)-ethyl]-benzyl)-carbaminsäuremethylester, (2-Chlor-5-[1-(6-methyl-pyridin-2-ylmethoxyimino)-ethyl]-benzyl)-carbaminsäuremethylester, 2-(ortho-((2,5-Dimethylphenyl-oxymethylen)phenyl)-3-methoxy-acrylsäuremethylester |

(fortgesetzt)

| Wirkstoffgruppe | Beispiele |
|---|---|
| Carbonsäureamide | Carboxin, Benalaxyl, Boscalid, Fenhexamid, Flutolanil, Furametpyr, Mepronil, Metalaxyl, Mefenoxam, Ofurace, Oxadixyl, Oxycarboxin, Penthiopyrad, Thifluzamide, Tiadinil, 4-Difluormethyl-2-methyl-thiazol-5-carbonsäure-(4'-brom-biphenyl-2-yl)-amid, 4-Difluormethyl-2-methyl-thiazol-5-carbonsäure-(4'-trifluormethyl-biphenyl-2-yl)-amid, 4-Difluormethyl-2-methyl-thiazol-5-carbonsäure-(4'-chlor-3'-fluor-biphenyl-2-yl)-amid, 3-Difluormethyl-1-methyl-pyrazol-4-carbonsäure-(3',4'-dichlor-4-fluorbiphenyl-2-yl)-amid, 3-Difluormethyl-1-methyl-pyrazol-4-carbonsäure-(3',4'-dichlor-5-fluor-biphenyl-2-yl)-amid, 3,4-Dichlor-N-(2-cyanophenyl)-isothiazol-5-carbonsäureamid, Dimethomorph, Flumorph, Flumetover, Fluopicolide (Picobezamid), Zoxamide,Carpropamid, Diclocymet, Mandipropamid, N-(2-{4-[3-(4-Chlor-phenyl)-prop-2-inyloxy]-3-methoxy-phenyl}-ethyl)-2-methansulfonylamino-3-methyl-butyramid, N-(2-{4-[3-(4-Chlorphenyl)-prop-2-inyloxy]-3-methoxy-phenyl}-ethyl)-2-ethansulfonylamino-3-methyl-butyramid |
| Heterocylische Verbindungen | Fluazinam, Pyrifenox, Bupirimate, Cyprodinil, Fenarimol, Ferimzone, Mepanipyrim, Nuarimol, Pyrimethanil, Triforine, Fenpiclonil, Fludioxonil, Aldimorph, Dodemorph, Fenpropimorph, Tridemorph, Fenpropidin, Iprodione, Procymidone, Vinclozolin, Famoxadone, Fenamidone, Octhilinone, Probenazole, Amisulbrom, Anilazin, Diclomezine, Pyroquilon, Proquinazid, Tricyclazole, 5-Chlor-7-(4-methyl-piperidin-1-yl)-6-(2,4,6-trifluorphenyl)-[1,2,4]triazolo[1,5-a]pyrimidin [AC1]; 2-Butoxy-6-iodo-3-propyl-chromen-4-on, Acibenzolar-S-methyl, Captafol, Captan, Dazomet, Folpet, Fenoxanil, Quinoxyfen, 3-[5-(4-Chlor-phenyl)-2,3-dimethyl-isoxazolidin-3-yl]-pyridin |
| Carbamate (I) | Mancozeb, Maneb, Metam, Metiram, Ferbam, Propineb, Thiram, Zineb, Ziram, Diethofencarb, Iprovalicarb, Flubenthiavalicarb, Propamocarb, 3-(4-Chlor-phenyl)-3-(2-isopropoxycarbonyl-amino-3-methyl-butyrylamino)-propansäuremethylester |
| Sonstige Wirkstoffe | Guanidine: Dodine, Iminoctadine, Guazatine Antibiotika: Kasugamycin, Streptomycin, Polyoxine, Validamycin A;<br>Nitrophenylderivate: Binapacryl, Dinocap, Dinobuton, Schwefelhaltige Heterocyclylverbindungen: Dithianon, Isoprothiolane;<br>Organometallverbindungen: Fentin Salze, wie Fentin-Acetat; Organophosphorverbindungen: Edifenphos, Iprobenfos, Fosetyl, Fosetyl-Aluminium, Phosphorige Säure und ihre Salze, Pyrazophos, Tolclofosmethyl;<br>Organochlorverbindungen: Chlorothalonil, Dichlofluanid, Flusulfamide, Hexachlorbenzol, Phthalid, Pencycuron, Quintozen, Thiophanate-Methyl, Tolylfluanid;<br>Anorganische Wirkstoffe: Schwefel oder Cu-Salze wie Bordeaux-Brühe, Kupferacetat, Kupferhydroxid, Kupferoxychlorid oder basisches Kupfersulfat;<br>Wachstumsretardantien: Prohexadion und seine Salze, Trinexapacethyl, Chlormequat, Mepiquat-chlorid und Diflufenzopyr; Sonstige: Cyflufenamid, Cymoxanil, Dimethirimol, Ethirimol, Furalaxyl, Metrafenone, Spiroxamine, N-(Cyclopropylmethoxyimino-(6-difluormethoxy-2,3-difluor-phenyl)-methyl)-2-phenylacetamid, N'-(4-(4-Chlor-3-trifluormethyl-phenoxy)-2,5-dimethylphenyl)-N-ethyl-N-methyl formamidin, N'-(4-(4-Fluor-3-trifluormethyl-phenoxy)-2,5-dimethyl-phenyl)-N-ethyl-N-methyl formamidin, N'-(2-Methyl-5-trifluormethyl-4-(3-trimethylsilanylpropoxy)-phenyl)-N-ethyl-N-methyl formamidin und N'-(5-Difluormethyl-2-methyl-4-(3-trimethylsilanyl-propoxy)-phenyl)-N-ethyl-N-methyl formamidin; |
| Organophosphate | acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon; |

(fortgesetzt)

| Wirkstoffgruppe | Beispiele |
|---|---|
| Carbamate (II) | alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate; |
| Pyrethroide | allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, taufluvalinate, tefluthrin, tetramethrin, tralomethrin, transfluthrin, profluthrin, dimefluthrin; |
| Arthropode Wachstumsregulatoren | a) Chitinsyntheseinhibitoren: Benzoylharnstoffe: chlorfluazuron, diflubenzuron, flucycloxuron, flufenoxuron, hexaflumuron, lufenuron, novaluron, teflubenzuron, triflumuron; buprofezin, diofenolan, hexythiazox, etoxazole, clofentazine;<br>b) Häutungsantagonisten: halofenozide, methoxyfenozide, tebufenozide, azadirachtin; c) Juvenoide: pyriproxyfen, methoprene, fenoxycarb; d) Lipidbiosyntheseinhibitoren: spirodiclofen, spiromesifen, spirotetramat; |
| Neonicotinoide | clothianidin, dinotefuran, imidacloprid, thiamethoxam, nitenpyram, acetamiprid, thiacloprid; das Thiazolderivat der Formel $\Gamma^1$<br>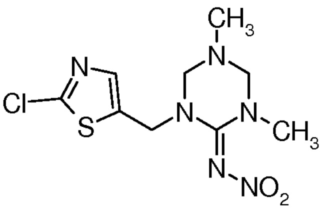 $(\Gamma^1)$ |
| Verschiedene | acetoprole, endosulfan, ethiprole, fipronil, vaniliprole, pyrafluprole, pyriprole, 5-Amino-1-(2,6-dichlor-4-trifluormethyl-phenyl)-4-trifluormethansulfinyl-1H-pyrazol-3-carbothionsäureamid; abamectin, emamectin, milbemectin, lepimectin, spinosad; fenazaquin, pyridaben, tebufenpyrad, tolfenpyrad, flufenerim; acequinocyl, fluacyprim, hydramethylnon; chlorfenapyr; cyhexatin, diafenthiuron, fenbutatin oxide, propargite; cyromazine; Piperonyl Butoxid; indoxacarb, metaflumizone, benclothiaz, bifenazate, cartap, flonicamid, pyridalyl, pymetrozine, sulfur, thiocyclam, flubendiamide, cyenopyrafen, flupyrazofos, cyflumetofen, amidoflumet, Anthranilamide der Formel $\Gamma^2$<br>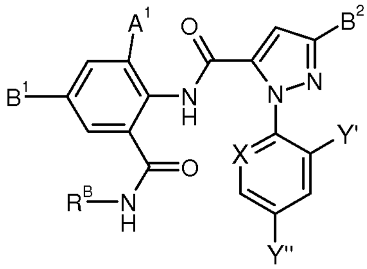 $(\Gamma^2)$<br>worin $A^1$ $CH_3$, Cl, Br, I; X C-H, C-Cl, C-F oder N; Y' F, Cl, oder Br; Y" Wasserstoff, F, Cl, $CF_3$; $B^1$ Wasserstoff, Cl, Br, I, CN; $B^2$ Cl, Br, $CF_3$, $OCH_2CF_3$, $OCF_2H$ und $R^B$ Wasserstoff, $CH_3$ oder $CH(CH_3)_2$ bedeuten;<br>$CF_2HCF_2CF_2CF_2CH_2C(CN)_2CH_2CH_2CF_3$;<br>$CF_3(CH_2)_2C(CN)_2CH_2(CF_2)_5CF_2H$;<br>$CF_3(CH_2)_2C(CN)_2(CH_2)_2C(CF_3)_2F$;<br>$CF_3(CH_2)_2C(CN)_2(CH_2)_2(CF_2)_3CF_3$;<br>$CF_2H(CF_2)_3CH_2C(CN)_2CH_2(CF_2)_3CF_2H$ ;<br>$CF_3(CH_2)_2C(CN)_2CH_2(CF_2)_3CF_3$;<br>$CF_3(CF_2)_2CH_2C(CN)_2CH_2(CF_2)_3CF_2H$ ; und<br>$CF_3CF_2CH_2C(CN)_2CH_2(CF_2)_3CF_2H$ |

Als Salze der phosphorigen Säure kommen beispielsweise Mn, Zn, Fe, Cu oder $NH_4$-Salze in Betracht. Die Salze können als Phosphite oder Hydrogenphosphite oder als Hydrate davon vorliegen.

Ternäre erfindungsgemäße Mischungen enthalten jeweils eine Verbindung I (als Komponente A), eine Verbindung II (als Komponente B) sowie eine weitere Komponente C. In einer Ausgestaltung enthalten die ternären erfindungsgemäßen Mischungen als Komponente C einen fungiziden Wirkstoff. In einer anderen Ausgestaltung enthalten sie als Komponente C einen Wirkstoff gegen tierische Schädlinge.
Einige ternäre Kombinationen sind in der folgenden Tabelle 10 aufgeführt:

Tabelle 10:

| Nr. | Binäre Kombination | Wirkstoff |
|---|---|---|
| 10-1 | 8-1 | Epoxiconazol |
| 10-2 | 8-2 | Epoxiconazol |
| 10-3 | 8-3 | Epoxiconazol |
| 10-4 | 8-4 | Epoxiconazol |
| 10-5 | 8-5 | Epoxiconazol |
| 10-6 | 8-6 | Epoxiconazol |
| 10-7 | 8-7 | Epoxiconazol |
| 10-8 | 8-8 | Epoxiconazol |
| 10-9 | 8-9 | Epoxiconazol |
| 10-10 | 8-10 | Epoxiconazol |
| 10-11 | 8-11 | Epoxiconazol |
| 10-12 | 8-12 | Epoxiconazol |
| 10-13 | 8-13 | Epoxiconazol |
| 10-14 | 8-14 | Epoxiconazol |
| 10-15 | 8-15 | Epoxiconazol |
| 10-16 | 8-16 | Epoxiconazol |
| 10-17 | 8-17 | Epoxiconazol |
| 10-18 | 8-18 | Epoxiconazol |
| 10-19 | 8-19 | Epoxiconazol |
| 10-20 | 8-20 | Epoxiconazol |

| Nr. | Binäre Kombination | Wirkstoff |
|---|---|---|
| 10-21 | 8-21 | Epoxiconazol |
| 10-22 | 8-22 | Epoxiconazol |
| 10-23 | 8-23 | Epoxiconazol |
| 10-24 | 8-24 | Epoxiconazol |
| 10-25 | 8-25 | Epoxiconazol |
| 10-26 | 8-26 | Epoxiconazol |
| 10-27 | 8-27 | Epoxiconazol |
| 10-28 | 8-28 | Epoxiconazol |
| 10-29 | 8-29 | Epoxiconazol |
| 10-30 | 8-30 | Epoxiconazol |
| 10-31 | 8-31 | Epoxiconazol |
| 10-32 | 8-32 | Epoxiconazol |
| 10-33 | 8-33 | Epoxiconazol |
| 10-34 | 8-34 | Epoxiconazol |
| 10-35 | 8-35 | Epoxiconazol |
| 10-36 | 8-36 | Epoxiconazol |
| 10-37 | 8-37 | Epoxiconazol |
| 10-38 | 8-38 | Epoxiconazol |
| 10-39 | 8-39 | Epoxiconazol |
| 10-40 | 8-40 | Epoxiconazol |

| Nr. | Binäre Kombination | Wirkstoff |
|---|---|---|
| 10-41 | 8-41 | Epoxiconazol |
| 10-42 | 8-42 | Epoxiconazol |
| 10-43 | 8-43 | Epoxiconazol |
| 10-44 | 8-44 | Epoxiconazol |
| 10-45 | 8-45 | Epoxiconazol |
| 10-46 | 8-46 | Epoxiconazol |
| 10-47 | 8-47 | Epoxiconazol |
| 10-48 | 8-48 | Epoxiconazol |
| 10-49 | 8-49 | Epoxiconazol |
| 10-50 | 8-50 | Epoxiconazol |
| 10-51 | 8-51 | Epoxiconazol |
| 10-52 | 8-52 | Epoxiconazol |
| 10-53 | 8-53 | Epoxiconazol |
| 10-54 | 8-54 | Epoxiconazol |
| 10-55 | 8-55 | Epoxiconazol |
| 10-56 | 8-56 | Epoxiconazol |
| 10-57 | 8-57 | Epoxiconazol |
| 10-58 | 8-58 | Epoxiconazol |
| 10-59 | 8-59 | Epoxiconazol |
| 10-60 | 8-60 | Epoxiconazol |
| 10-61 | 8-61 | Epoxiconazol |
| 10-62 | 8-62 | Epoxiconazol |
| 10-63 | 8-63 | Epoxiconazol |
| 10-64 | 8-64 | Epoxiconazol |
| 10-65 | 8-65 | Epoxiconazol |
| 10-66 | 8-66 | Epoxiconazol |
| 10-67 | 8-67 | Epoxiconazol |
| 10-68 | 8-68 | Epoxiconazol |
| 10-69 | 8-69 | Epoxiconazol |
| 10-70 | 8-70 | Epoxiconazol |
| 10-71 | 8-71 | Epoxiconazol |
| 10-72 | 8-72 | Epoxiconazol |
| 10-73 | 8-73 | Epoxiconazol |
| 10-74 | 8-74 | Epoxiconazol |
| 10-75 | 8-75 | Epoxiconazol |
| 10-76 | 8-76 | Epoxiconazol |
| 10-77 | 8-77 | Epoxiconazol |
| 10-78 | 8-78 | Epoxiconazol |
| 10-79 | 8-79 | Epoxiconazol |
| 10-80 | 8-80 | Epoxiconazol |

| Nr. | Binäre Kombination | Wirkstoff |
|---|---|---|
| 10-81 | 8-81 | Epoxiconazol |
| 10-82 | 8-82 | Epoxiconazol |
| 10-83 | 8-83 | Epoxiconazol |
| 10-84 | 8-84 | Epoxiconazol |
| 10-85 | 8-85 | Epoxiconazol |
| 10-86 | 8-86 | Epoxiconazol |
| 10-87 | 8-87 | Epoxiconazol |
| 10-88 | 8-88 | Epoxiconazol |
| 10-89 | 8-89 | Epoxiconazol |
| 10-90 | 8-90 | Epoxiconazol |
| 10-91 | 8-91 | Epoxiconazol |
| 10-92 | 8-92 | Epoxiconazol |
| 10-93 | 8-93 | Epoxiconazol |
| 10-94 | 8-94 | Epoxiconazol |
| 10-95 | 8-95 | Epoxiconazol |
| 10-96 | 8-96 | Epoxiconazol |
| 10-97 | 8-97 | Epoxiconazol |
| 10-98 | 8-98 | Epoxiconazol |
| 10-99 | 8-99 | Epoxiconazol |
| 10-100 | 8-100 | Epoxiconazol |
| 10-101 | 8-101 | Epoxiconazol |
| 10-102 | 8-102 | Epoxiconazol |
| 10-103 | 8-103 | Epoxiconazol |
| 10-104 | 8-104 | Epoxiconazol |
| 10-105 | 8-105 | Epoxiconazol |
| 10-106 | 8-106 | Epoxiconazol |
| 10-107 | 8-107 | Epoxiconazol |
| 10-108 | 8-108 | Epoxiconazol |
| 10-109 | 8-109 | Epoxiconazol |
| 10-110 | 8-110 | Epoxiconazol |
| 10-111 | 8-111 | Epoxiconazol |
| 10-112 | 8-112 | Epoxiconazol |
| 10-113 | 8-113 | Epoxiconazol |
| 10-114 | 8-114 | Epoxiconazol |
| 10-115 | 8-115 | Epoxiconazol |
| 10-116 | 8-116 | Epoxiconazol |
| 10-117 | 8-117 | Epoxiconazol |
| 10-118 | 8-118 | Epoxiconazol |
| 10-119 | 8-119 | Epoxiconazol |
| 10-120 | 8-120 | Epoxiconazol |

| Nr. | Binäre Kombination | Wirkstoff |
|---|---|---|
| 10-121 | 8-121 | Epoxiconazol |
| 10-122 | 8-122 | Epoxiconazol |
| 10-123 | 8-123 | Epoxiconazol |
| 10-124 | 8-124 | Epoxiconazol |
| 10-125 | 8-125 | Epoxiconazol |
| 10-126 | 8-126 | Epoxiconazol |
| 10-127 | 8-127 | Epoxiconazol |
| 10-128 | 8-128 | Epoxiconazol |
| 10-129 | 8-129 | Epoxiconazol |
| 10-130 | 8-130 | Epoxiconazol |
| 10-131 | 8-131 | Epoxiconazol |
| 10-132 | 8-132 | Epoxiconazol |
| 10-133 | 8-133 | Epoxiconazol |
| 10-134 | 8-134 | Epoxiconazol |
| 10-135 | 8-135 | Epoxiconazol |
| 10-136 | 8-136 | Epoxiconazol |
| 10-137 | 8-137 | Epoxiconazol |
| 10-138 | 8-138 | Epoxiconazol |
| 10-139 | 8-139 | Epoxiconazol |
| 10-140 | 8-140 | Epoxiconazol |
| 10-141 | 8-141 | Epoxiconazol |
| 10-142 | 8-142 | Epoxiconazol |
| 10-143 | 8-143 | Epoxiconazol |
| 10-144 | 8-144 | Epoxiconazol |
| 10-145 | 8-145 | Epoxiconazol |
| 10-146 | 8-146 | Epoxiconazol |
| 10-147 | 8-147 | Epoxiconazol |
| 10-148 | 8-148 | Epoxiconazol |
| 10-149 | 8-149 | Epoxiconazol |
| 10-150 | 8-150 | Epoxiconazol |
| 10-151 | 8-151 | Epoxiconazol |
| 10-152 | 8-152 | Epoxiconazol |
| 10-153 | 8-153 | Epoxiconazol |
| 10-154 | 8-154 | Epoxiconazol |
| 10-155 | 8-155 | Epoxiconazol |
| 10-156 | 8-156 | Epoxiconazol |
| 10-157 | 8-157 | Epoxiconazol |
| 10-158 | 8-158 | Epoxiconazol |
| 10-159 | 8-159 | Epoxiconazol |
| 10-160 | 8-160 | Epoxiconazol |

| Nr. | Binäre Kombination | Wirkstoff |
|---|---|---|
| 10-161 | 8-161 | Epoxiconazol |
| 10-162 | 8-162 | Epoxiconazol |
| 10-163 | 8-163 | Epoxiconazol |
| 10-164 | 8-164 | Epoxiconazol |
| 10-165 | 8-165 | Epoxiconazol |
| 10-166 | 8-166 | Epoxiconazol |
| 10-167 | 8-167 | Epoxiconazol |
| 10-168 | 8-168 | Epoxiconazol |
| 10-169 | 8-169 | Epoxiconazol |
| 10-170 | 8-170 | Epoxiconazol |
| 10-171 | 8-171 | Epoxiconazol |
| 10-172 | 8-172 | Epoxiconazol |
| 10-173 | 8-173 | Epoxiconazol |
| 10-174 | 8-174 | Epoxiconazol |
| 10-175 | 8-175 | Epoxiconazol |
| 10-176 | 8-176 | Epoxiconazol |
| 10-177 | 8-177 | Epoxiconazol |
| 10-178 | 8-178 | Epoxiconazol |
| 10-179 | 8-179 | Epoxiconazol |
| 10-180 | 8-180 | Epoxiconazol |
| 10-181 | 8-1 | Metconazol |
| 10-182 | 8-2 | Metconazol |
| 10-183 | 8-3 | Metconazol |
| 10-184 | 8-4 | Metconazol |
| 10-185 | 8-5 | Metconazol |
| 10-186 | 8-6 | Metconazol |
| 10-187 | 8-7 | Metconazol |
| 10-188 | 8-8 | Metconazol |
| 10-189 | 8-9 | Metconazol |
| 10-190 | 8-10 | Metconazol |
| 10-191 | 8-11 | Metconazol |
| 10-192 | 8-12 | Metconazol |
| 10-193 | 8-13 | Metconazol |
| 10-194 | 8-14 | Metconazol |
| 10-195 | 8-15 | Metconazol |
| 10-196 | 8-16 | Metconazol |
| 10-197 | 8-17 | Metconazol |
| 10-198 | 8-18 | Metconazol |
| 10-199 | 8-19 | Metconazol |
| 10-200 | 8-20 | Metconazol |

| Nr. | Binäre Kombination | Wirkstoff |
|---|---|---|
| 10-201 | 8-21 | Metconazol |
| 10-202 | 8-22 | Metconazol |
| 10-203 | 8-23 | Metconazol |
| 10-204 | 8-24 | Metconazol |
| 10-205 | 8-25 | Metconazol |
| 10-206 | 8-26 | Metconazol |
| 10-207 | 8-27 | Metconazol |
| 10-208 | 8-28 | Metconazol |
| 10-209 | 8-29 | Metconazol |
| 10-210 | 8-30 | Metconazol |
| 10-211 | 8-31 | Metconazol |
| 10-212 | 8-32 | Metconazol |
| 10-213 | 8-33 | Metconazol |
| 10-214 | 8-34 | Metconazol |
| 10-215 | 8-35 | Metconazol |
| 10-216 | 8-36 | Metconazol |
| 10-217 | 8-37 | Metconazol |
| 10-218 | 8-38 | Metconazol |
| 10-219 | 8-39 | Metconazol |
| 10-220 | 8-40 | Metconazol |
| 10-221 | 8-41 | Metconazol |
| 10-222 | 8-42 | Metconazol |
| 10-223 | 8-43 | Metconazol |
| 10-224 | 8-44 | Metconazol |
| 10-225 | 8-45 | Metconazol |
| 10-226 | 8-46 | Metconazol |
| 10-227 | 8-47 | Metconazol |
| 10-228 | 8-48 | Metconazol |
| 10-229 | 8-49 | Metconazol |
| 10-230 | 8-50 | Metconazol |
| 10-231 | 8-51 | Metconazol |
| 10-232 | 8-52 | Metconazol |
| 10-233 | 8-53 | Metconazol |
| 10-234 | 8-54 | Metconazol |
| 10-235 | 8-55 | Metconazol |
| 10-236 | 8-56 | Metconazol |
| 10-237 | 8-57 | Metconazol |
| 10-238 | 8-58 | Metconazol |
| 10-239 | 8-59 | Metconazol |
| 10-240 | 8-60 | Metconazol |

| Nr. | Binäre Kombination | Wirkstoff |
|---|---|---|
| 10-241 | 8-61 | Metconazol |
| 10-242 | 8-62 | Metconazol |
| 10-243 | 8-63 | Metconazol |
| 10-244 | 8-64 | Metconazol |
| 10-245 | 8-65 | Metconazol |
| 10-246 | 8-66 | Metconazol |
| 10-247 | 8-67 | Metconazol |
| 10-248 | 8-68 | Metconazol |
| 10-249 | 8-69 | Metconazol |
| 10-250 | 8-70 | Metconazol |
| 10-251 | 8-71 | Metconazol |
| 10-252 | 8-72 | Metconazol |
| 10-253 | 8-73 | Metconazol |
| 10-254 | 8-74 | Metconazol |
| 10-255 | 8-75 | Metconazol |
| 10-256 | 8-76 | Metconazol |
| 10-257 | 8-77 | Metconazol |
| 10-258 | 8-78 | Metconazol |
| 10-259 | 8-79 | Metconazol |
| 10-260 | 8-80 | Metconazol |
| 10-261 | 8-81 | Metconazol |
| 10-262 | 8-82 | Metconazol |
| 10-263 | 8-83 | Metconazol |
| 10-264 | 8-84 | Metconazol |
| 10-265 | 8-85 | Metconazol |
| 10-266 | 8-86 | Metconazol |
| 10-267 | 8-87 | Metconazol |
| 10-268 | 8-88 | Metconazol |
| 10-269 | 8-89 | Metconazol |
| 10-270 | 8-90 | Metconazol |
| 10-271 | 8-91 | Metconazol |
| 10-272 | 8-92 | Metconazol |
| 10-273 | 8-93 | Metconazol |
| 10-274 | 8-94 | Metconazol |
| 10-275 | 8-95 | Metconazol |
| 10-276 | 8-96 | Metconazol |
| 10-277 | 8-97 | Metconazol |
| 10-278 | 8-98 | Metconazol |
| 10-279 | 8-99 | Metconazol |
| 10-280 | 8-100 | Metconazol |

| Nr. | Binäre Kombination | Wirkstoff |
|---|---|---|
| 10-281 | 8-101 | Metconazol |
| 10-282 | 8-102 | Metconazol |
| 10-283 | 8-103 | Metconazol |
| 10-284 | 8-104 | Metconazol |
| 10-285 | 8-105 | Metconazol |
| 10-286 | 8-106 | Metconazol |
| 10-287 | 8-107 | Metconazol |
| 10-288 | 8-108 | Metconazol |
| 10-289 | 8-109 | Metconazol |
| 10-290 | 8-110 | Metconazol |
| 10-291 | 8-111 | Metconazol |
| 10-292 | 8-112 | Metconazol |
| 10-293 | 8-113 | Metconazol |
| 10-294 | 8-114 | Metconazol |
| 10-295 | 8-115 | Metconazol |
| 10-296 | 8-116 | Metconazol |
| 10-297 | 8-117 | Metconazol |
| 10-298 | 8-118 | Metconazol |
| 10-299 | 8-119 | Metconazol |
| 10-300 | 8-120 | Metconazol |
| 10-301 | 8-121 | Metconazol |
| 10-302 | 8-122 | Metconazol |
| 10-303 | 8-123 | Metconazol |
| 10-304 | 8-124 | Metconazol |
| 10-305 | 8-125 | Metconazol |
| 10-306 | 8-126 | Metconazol |
| 10-307 | 8-127 | Metconazol |
| 10-308 | 8-128 | Metconazol |
| 10-309 | 8-129 | Metconazol |
| 10-310 | 8-130 | Metconazol |
| 10-311 | 8-131 | Metconazol |
| 10-312 | 8-132 | Metconazol |
| 10-313 | 8-133 | Metconazol |
| 10-314 | 8-134 | Metconazol |
| 10-315 | 8-135 | Metconazol |
| 10-316 | 8-136 | Metconazol |
| 10-317 | 8-137 | Metconazol |
| 10-318 | 8-138 | Metconazol |
| 10-319 | 8-139 | Metconazol |
| 10-320 | 8-140 | Metconazol |
| 10-321 | 8-141 | Metconazol |
| 10-322 | 8-142 | Metconazol |
| 10-323 | 8-143 | Metconazol |
| 10-324 | 8-144 | Metconazol |
| 10-325 | 8-145 | Metconazol |
| 10-326 | 8-146 | Metconazol |
| 10-327 | 8-147 | Metconazol |
| 10-328 | 8-148 | Metconazol |
| 10-329 | 8-149 | Metconazol |
| 10-330 | 8-150 | Metconazol |
| 10-331 | 8-151 | Metconazol |
| 10-332 | 8-152 | Metconazol |
| 10-333 | 8-153 | Metconazol |
| 10-334 | 8-154 | Metconazol |
| 10-335 | 8-155 | Metconazol |
| 10-336 | 8-156 | Metconazol |
| 10-337 | 8-157 | Metconazol |
| 10-338 | 8-158 | Metconazol |
| 10-339 | 8-159 | Metconazol |
| 10-340 | 8-160 | Metconazol |
| 10-341 | 8-161 | Metconazol |
| 10-342 | 8-162 | Metconazol |
| 10-343 | 8-163 | Metconazol |
| 10-344 | 8-164 | Metconazol |
| 10-345 | 8-165 | Metconazol |
| 10-346 | 8-166 | Metconazol |
| 10-347 | 8-167 | Metconazol |
| 10-348 | 8-168 | Metconazol |
| 10-349 | 8-169 | Metconazol |
| 10-350 | 8-170 | Metconazol |
| 10-351 | 8-171 | Metconazol |
| 10-352 | 8-172 | Metconazol |
| 10-353 | 8-173 | Metconazol |
| 10-354 | 8-174 | Metconazol |
| 10-355 | 8-175 | Metconazol |
| 10-356 | 8-176 | Metconazol |
| 10-357 | 8-177 | Metconazol |
| 10-358 | 8-178 | Metconazol |
| 10-359 | 8-179 | Metconazol |
| 10-360 | 8-180 | Metconazol |

| Nr. | Binäre Kombination | Wirkstoff | Nr. | Binäre Kombination | Wirkstoff |
|---|---|---|---|---|---|
| 10-361 | 8-1 | Tebuconazol | 10-401 | 8-41 | Tebuconazol |
| 10-362 | 8-2 | Tebuconazol | 10-402 | 8-42 | Tebuconazol |
| 10-363 | 8-3 | Tebuconazol | 10-403 | 8-43 | Tebuconazol |
| 10-364 | 8-4 | Tebuconazol | 10-404 | 8-44 | Tebuconazol |
| 10-365 | 8-5 | Tebuconazol | 10-405 | 8-45 | Tebuconazol |
| 10-366 | 8-6 | Tebuconazol | 10-406 | 8-46 | Tebuconazol |
| 10-367 | 8-7 | Tebuconazol | 10-407 | 8-47 | Tebuconazol |
| 10-368 | 8-8 | Tebuconazol | 10-408 | 8-48 | Tebuconazol |
| 10-369 | 8-9 | Tebuconazol | 10-409 | 8-49 | Tebuconazol |
| 10-370 | 8-10 | Tebuconazol | 10-410 | 8-50 | Tebuconazol |
| 10-371 | 8-11 | Tebuconazol | 10-411 | 8-51 | Tebuconazol |
| 10-372 | 8-12 | Tebuconazol | 10-412 | 8-52 | Tebuconazol |
| 10-373 | 8-13 | Tebuconazol | 10-413 | 8-53 | Tebuconazol |
| 10-374 | 8-14 | Tebuconazol | 10-414 | 8-54 | Tebuconazol |
| 10-375 | 8-15 | Tebuconazol | 10-415 | 8-55 | Tebuconazol |
| 10-376 | 8-16 | Tebuconazol | 10-416 | 8-56 | Tebuconazol |
| 10-377 | 8-17 | Tebuconazol | 10-417 | 8-57 | Tebuconazol |
| 10-378 | 8-18 | Tebuconazol | 10-418 | 8-58 | Tebuconazol |
| 10-379 | 8-19 | Tebuconazol | 10-419 | 8-59 | Tebuconazol |
| 10-380 | 8-20 | Tebuconazol | 10-420 | 8-60 | Tebuconazol |
| 10-381 | 8-21 | Tebuconazol | 10-421 | 8-61 | Tebuconazol |
| 10-382 | 8-22 | Tebuconazol | 10-422 | 8-62 | Tebuconazol |
| 10-383 | 8-23 | Tebuconazol | 10-423 | 8-63 | Tebuconazol |
| 10-384 | 8-24 | Tebuconazol | 10-424 | 8-64 | Tebuconazol |
| 10-385 | 8-25 | Tebuconazol | 10-425 | 8-65 | Tebuconazol |
| 10-386 | 8-26 | Tebuconazol | 10-426 | 8-66 | Tebuconazol |
| 10-387 | 8-27 | Tebuconazol | 10-427 | 8-67 | Tebuconazol |
| 10-388 | 8-28 | Tebuconazol | 10-428 | 8-68 | Tebuconazol |
| 10-389 | 8-29 | Tebuconazol | 10-429 | 8-69 | Tebuconazol |
| 10-390 | 8-30 | Tebuconazol | 10-430 | 8-70 | Tebuconazol |
| 10-391 | 8-31 | Tebuconazol | 10-431 | 8-71 | Tebuconazol |
| 10-392 | 8-32 | Tebuconazol | 10-432 | 8-72 | Tebuconazol |
| 10-393 | 8-33 | Tebuconazol | 10-433 | 8-73 | Tebuconazol |
| 10-394 | 8-34 | Tebuconazol | 10-434 | 8-74 | Tebuconazol |
| 10-395 | 8-35 | Tebuconazol | 10-435 | 8-75 | Tebuconazol |
| 10-396 | 8-36 | Tebuconazol | 10-436 | 8-76 | Tebuconazol |
| 10-397 | 8-37 | Tebuconazol | 10-437 | 8-77 | Tebuconazol |
| 10-398 | 8-38 | Tebuconazol | 10-438 | 8-78 | Tebuconazol |
| 10-399 | 8-39 | Tebuconazol | 10-439 | 8-79 | Tebuconazol |
| 10-400 | 8-40 | Tebuconazol | 10-440 | 8-80 | Tebuconazol |

| Nr. | Binäre Kombination | Wirkstoff |
|---|---|---|
| 10-441 | 8-81 | Tebuconazol |
| 10-442 | 8-82 | Tebuconazol |
| 10-443 | 8-83 | Tebuconazol |
| 10-444 | 8-84 | Tebuconazol |
| 10-445 | 8-85 | Tebuconazol |
| 10-446 | 8-86 | Tebuconazol |
| 10-447 | 8-87 | Tebuconazol |
| 10-448 | 8-88 | Tebuconazol |
| 10-449 | 8-89 | Tebuconazol |
| 10-450 | 8-90 | Tebuconazol |
| 10-451 | 8-91 | Tebuconazol |
| 10-452 | 8-92 | Tebuconazol |
| 10-453 | 8-93 | Tebuconazol |
| 10-454 | 8-94 | Tebuconazol |
| 10-455 | 8-95 | Tebuconazol |
| 10-456 | 8-96 | Tebuconazol |
| 10-457 | 8-97 | Tebuconazol |
| 10-458 | 8-98 | Tebuconazol |
| 10-459 | 8-99 | Tebuconazol |
| 10-460 | 8-100 | Tebuconazol |
| 10-461 | 8-101 | Tebuconazol |
| 10-462 | 8-102 | Tebuconazol |
| 10-463 | 8-103 | Tebuconazol |
| 10-464 | 8-104 | Tebuconazol |
| 10-465 | 8-105 | Tebuconazol |
| 10-466 | 8-106 | Tebuconazol |
| 10-467 | 8-107 | Tebuconazol |
| 10-468 | 8-108 | Tebuconazol |
| 10-469 | 8-109 | Tebuconazol |
| 10-470 | 8-110 | Tebuconazol |
| 10-471 | 8-111 | Tebuconazol |
| 10-472 | 8-112 | Tebuconazol |
| 10-473 | 8-113 | Tebuconazol |
| 10-474 | 8-114 | Tebuconazol |
| 10-475 | 8-115 | Tebuconazol |
| 10-476 | 8-116 | Tebuconazol |
| 10-477 | 8-117 | Tebuconazol |
| 10-478 | 8-118 | Tebuconazol |
| 10-479 | 8-119 | Tebuconazol |
| 10-480 | 8-120 | Tebuconazol |

| Nr. | Binäre Kombination | Wirkstoff |
|---|---|---|
| 10-481 | 8-121 | Tebuconazol |
| 10-482 | 8-122 | Tebuconazol |
| 10-483 | 8-123 | Tebuconazol |
| 10-484 | 8-124 | Tebuconazol |
| 10-485 | 8-125 | Tebuconazol |
| 10-486 | 8-126 | Tebuconazol |
| 10-487 | 8-127 | Tebuconazol |
| 10-488 | 8-128 | Tebuconazol |
| 10-489 | 8-129 | Tebuconazol |
| 10-490 | 8-130 | Tebuconazol |
| 10-491 | 8-131 | Tebuconazol |
| 10-492 | 8-132 | Tebuconazol |
| 10-493 | 8-133 | Tebuconazol |
| 10-494 | 8-134 | Tebuconazol |
| 10-495 | 8-135 | Tebuconazol |
| 10-496 | 8-136 | Tebuconazol |
| 10-497 | 8-137 | Tebuconazol |
| 10-498 | 8-138 | Tebuconazol |
| 10-499 | 8-139 | Tebuconazol |
| 10-500 | 8-140 | Tebuconazol |
| 10-501 | 8-141 | Tebuconazol |
| 10-502 | 8-142 | Tebuconazol |
| 10-503 | 8-143 | Tebuconazol |
| 10-504 | 8-144 | Tebuconazol |
| 10-505 | 8-145 | Tebuconazol |
| 10-506 | 8-146 | Tebuconazol |
| 10-507 | 8-147 | Tebuconazol |
| 10-508 | 8-148 | Tebuconazol |
| 10-509 | 8-149 | Tebuconazol |
| 10-510 | 8-150 | Tebuconazol |
| 10-511 | 8-151 | Tebuconazol |
| 10-512 | 8-152 | Tebuconazol |
| 10-513 | 8-153 | Tebuconazol |
| 10-514 | 8-154 | Tebuconazol |
| 10-515 | 8-155 | Tebuconazol |
| 10-516 | 8-156 | Tebuconazol |
| 10-517 | 8-157 | Tebuconazol |
| 10-518 | 8-158 | Tebuconazol |
| 10-519 | 8-159 | Tebuconazol |
| 10-520 | 8-160 | Tebuconazol |

| Nr. | Binäre Kombination | Wirkstoff |
|---|---|---|
| 10-521 | 8-161 | Tebuconazol |
| 10-522 | 8-162 | Tebuconazol |
| 10-523 | 8-163 | Tebuconazol |
| 10-524 | 8-164 | Tebuconazol |
| 10-525 | 8-165 | Tebuconazol |
| 10-526 | 8-166 | Tebuconazol |
| 10-527 | 8-167 | Tebuconazol |
| 10-528 | 8-168 | Tebuconazol |
| 10-529 | 8-169 | Tebuconazol |
| 10-530 | 8-170 | Tebuconazol |
| 10-531 | 8-171 | Tebuconazol |
| 10-532 | 8-172 | Tebuconazol |
| 10-533 | 8-173 | Tebuconazol |
| 10-534 | 8-174 | Tebuconazol |
| 10-535 | 8-175 | Tebuconazol |
| 10-536 | 8-176 | Tebuconazol |
| 10-537 | 8-177 | Tebuconazol |
| 10-538 | 8-178 | Tebuconazol |
| 10-539 | 8-179 | Tebuconazol |
| 10-540 | 8-180 | Tebuconazol |
| 10-541 | 8-1 | Fluquinconazol |
| 10-542 | 8-2 | Fluquinconazol |
| 10-543 | 8-3 | Fluquinconazol |
| 10-544 | 8-4 | Fluquinconazol |
| 10-545 | 8-5 | Fluquinconazol |
| 10-546 | 8-6 | Fluquinconazol |
| 10-547 | 8-7 | Fluquinconazol |
| 10-548 | 8-8 | Fluquinconazol |
| 10-549 | 8-9 | Fluquinconazol |
| 10-550 | 8-10 | Fluquinconazol |
| 10-551 | 8-11 | Fluquinconazol |
| 10-552 | 8-12 | Fluquinconazol |
| 10-553 | 8-13 | Fluquinconazol |
| 10-554 | 8-14 | Fluquinconazol |
| 10-555 | 8-15 | Fluquinconazol |
| 10-556 | 8-16 | Fluquinconazol |
| 10-557 | 8-17 | Fluquinconazol |
| 10-558 | 8-18 | Fluquinconazol |
| 10-559 | 8-19 | Fluquinconazol |
| 10-560 | 8-20 | Fluquinconazol |

| Nr. | Binäre Kombination | Wirkstoff |
|---|---|---|
| 10-561 | 8-21 | Fluquinconazol |
| 10-562 | 8-22 | Fluquinconazol |
| 10-563 | 8-23 | Fluquinconazol |
| 10-564 | 8-24 | Fluquinconazol |
| 10-565 | 8-25 | Fluquinconazol |
| 10-566 | 8-26 | Fluquinconazol |
| 10-567 | 8-27 | Fluquinconazol |
| 10-568 | 8-28 | Fluquinconazol |
| 10-569 | 8-29 | Fluquinconazol |
| 10-570 | 8-30 | Fluquinconazol |
| 10-571 | 8-31 | Fluquinconazol |
| 10-572 | 8-32 | Fluquinconazol |
| 10-573 | 8-33 | Fluquinconazol |
| 10-574 | 8-34 | Fluquinconazol |
| 10-575 | 8-35 | Fluquinconazol |
| 10-576 | 8-36 | Fluquinconazol |
| 10-577 | 8-37 | Fluquinconazol |
| 10-578 | 8-38 | Fluquinconazol |
| 10-579 | 8-39 | Fluquinconazol |
| 10-580 | 8-40 | Fluquinconazol |
| 10-581 | 8-41 | Fluquinconazol |
| 10-582 | 8-42 | Fluquinconazol |
| 10-583 | 8-43 | Fluquinconazol |
| 10-584 | 8-44 | Fluquinconazol |
| 10-585 | 8-45 | Fluquinconazol |
| 10-586 | 8-46 | Fluquinconazol |
| 10-587 | 8-47 | Fluquinconazol |
| 10-588 | 8-48 | Fluquinconazol |
| 10-589 | 8-49 | Fluquinconazol |
| 10-590 | 8-50 | Fluquinconazol |
| 10-591 | 8-51 | Fluquinconazol |
| 10-592 | 8-52 | Fluquinconazol |
| 10-593 | 8-53 | Fluquinconazol |
| 10-594 | 8-54 | Fluquinconazol |
| 10-595 | 8-55 | Fluquinconazol |
| 10-596 | 8-56 | Fluquinconazol |
| 10-597 | 8-57 | Fluquinconazol |
| 10-598 | 8-58 | Fluquinconazol |
| 10-599 | 8-59 | Fluquinconazol |
| 10-600 | 8-60 | Fluquinconazol |

| Nr. | Binäre Kombination | Wirkstoff |
|---|---|---|
| 10-601 | 8-61 | Fluquinconazol |
| 10-602 | 8-62 | Fluquinconazol |
| 10-603 | 8-63 | Fluquinconazol |
| 10-604 | 8-64 | Fluquinconazol |
| 10-605 | 8-65 | Fluquinconazol |
| 10-606 | 8-66 | Fluquinconazol |
| 10-607 | 8-67 | Fluquinconazol |
| 10-608 | 8-68 | Fluquinconazol |
| 10-609 | 8-69 | Fluquinconazol |
| 10-610 | 8-70 | Fluquinconazol |
| 10-611 | 8-71 | Fluquinconazol |
| 10-612 | 8-72 | Fluquinconazol |
| 10-613 | 8-73 | Fluquinconazol |
| 10-614 | 8-74 | Fluquinconazol |
| 10-615 | 8-75 | Fluquinconazol |
| 10-616 | 8-76 | Fluquinconazol |
| 10-617 | 8-77 | Fluquinconazol |
| 10-618 | 8-78 | Fluquinconazol |
| 10-619 | 8-79 | Fluquinconazol |
| 10-620 | 8-80 | Fluquinconazol |
| 10-621 | 8-81 | Fluquinconazol |
| 10-622 | 8-82 | Fluquinconazol |
| 10-623 | 8-83 | Fluquinconazol |
| 10-624 | 8-84 | Fluquinconazol |
| 10-625 | 8-85 | Fluquinconazol |
| 10-626 | 8-86 | Fluquinconazol |
| 10-627 | 8-87 | Fluquinconazol |
| 10-628 | 8-88 | Fluquinconazol |
| 10-629 | 8-89 | Fluquinconazol |
| 10-630 | 8-90 | Fluquinconazol |
| 10-631 | 8-91 | Fluquinconazol |
| 10-632 | 8-92 | Fluquinconazol |
| 10-633 | 8-93 | Fluquinconazol |
| 10-634 | 8-94 | Fluquinconazol |
| 10-635 | 8-95 | Fluquinconazol |
| 10-636 | 8-96 | Fluquinconazol |
| 10-637 | 8-97 | Fluquinconazol |
| 10-638 | 8-98 | Fluquinconazol |
| 10-639 | 8-99 | Fluquinconazol |
| 10-640 | 8-100 | Fluquinconazol |

| Nr. | Binäre Kombination | Wirkstoff |
|---|---|---|
| 10-641 | 8-101 | Fluquinconazol |
| 10-642 | 8-102 | Fluquinconazol |
| 10-643 | 8-103 | Fluquinconazol |
| 10-644 | 8-104 | Fluquinconazol |
| 10-645 | 8-105 | Fluquinconazol |
| 10-646 | 8-106 | Fluquinconazol |
| 10-647 | 8-107 | Fluquinconazol |
| 10-648 | 8-108 | Fluquinconazol |
| 10-649 | 8-109 | Fluquinconazol |
| 10-650 | 8-110 | Fluquinconazol |
| 10-651 | 8-111 | Fluquinconazol |
| 10-652 | 8-112 | Fluquinconazol |
| 10-653 | 8-113 | Fluquinconazol |
| 10-654 | 8-114 | Fluquinconazol |
| 10-655 | 8-115 | Fluquinconazol |
| 10-656 | 8-116 | Fluquinconazol |
| 10-657 | 8-117 | Fluquinconazol |
| 10-658 | 8-118 | Fluquinconazol |
| 10-659 | 8-119 | Fluquinconazol |
| 10-660 | 8-120 | Fluquinconazol |
| 10-661 | 8-121 | Fluquinconazol |
| 10-662 | 8-122 | Fluquinconazol |
| 10-663 | 8-123 | Fluquinconazol |
| 10-664 | 8-124 | Fluquinconazol |
| 10-665 | 8-125 | Fluquinconazol |
| 10-666 | 8-126 | Fluquinconazol |
| 10-667 | 8-127 | Fluquinconazol |
| 10-668 | 8-128 | Fluquinconazol |
| 10-669 | 8-129 | Fluquinconazol |
| 10-670 | 8-130 | Fluquinconazol |
| 10-671 | 8-131 | Fluquinconazol |
| 10-672 | 8-132 | Fluquinconazol |
| 10-673 | 8-133 | Fluquinconazol |
| 10-674 | 8-134 | Fluquinconazol |
| 10-675 | 8-135 | Fluquinconazol |
| 10-676 | 8-136 | Fluquinconazol |
| 10-677 | 8-137 | Fluquinconazol |
| 10-678 | 8-138 | Fluquinconazol |
| 10-679 | 8-139 | Fluquinconazol |
| 10-680 | 8-140 | Fluquinconazol |

| Nr. | Binäre Kombination | Wirkstoff |
|---|---|---|
| 10-681 | 8-141 | Fluquinconazol |
| 10-682 | 8-142 | Fluquinconazol |
| 10-683 | 8-143 | Fluquinconazol |
| 10-684 | 8-144 | Fluquinconazol |
| 10-685 | 8-145 | Fluquinconazol |
| 10-686 | 8-146 | Fluquinconazol |
| 10-687 | 8-147 | Fluquinconazol |
| 10-688 | 8-148 | Fluquinconazol |
| 10-689 | 8-149 | Fluquinconazol |
| 10-690 | 8-150 | Fluquinconazol |
| 10-691 | 8-151 | Fluquinconazol |
| 10-692 | 8-152 | Fluquinconazol |
| 10-693 | 8-153 | Fluquinconazol |
| 10-694 | 8-154 | Fluquinconazol |
| 10-695 | 8-155 | Fluquinconazol |
| 10-696 | 8-156 | Fluquinconazol |
| 10-697 | 8-157 | Fluquinconazol |
| 10-698 | 8-158 | Fluquinconazol |
| 10-699 | 8-159 | Fluquinconazol |
| 10-700 | 8-160 | Fluquinconazol |
| 10-701 | 8-161 | Fluquinconazol |
| 10-702 | 8-162 | Fluquinconazol |
| 10-703 | 8-163 | Fluquinconazol |
| 10-704 | 8-164 | Fluquinconazol |
| 10-705 | 8-165 | Fluquinconazol |
| 10-706 | 8-166 | Fluquinconazol |
| 10-707 | 8-167 | Fluquinconazol |
| 10-708 | 8-168 | Fluquinconazol |
| 10-709 | 8-169 | Fluquinconazol |
| 10-710 | 8-170 | Fluquinconazol |
| 10-711 | 8-171 | Fluquinconazol |
| 10-712 | 8-172 | Fluquinconazol |
| 10-713 | 8-173 | Fluquinconazol |
| 10-714 | 8-174 | Fluquinconazol |
| 10-715 | 8-175 | Fluquinconazol |
| 10-716 | 8-176 | Fluquinconazol |
| 10-717 | 8-177 | Fluquinconazol |
| 10-718 | 8-178 | Fluquinconazol |
| 10-719 | 8-179 | Fluquinconazol |
| 10-720 | 8-180 | Fluquinconazol |

| Nr. | Binäre Kombination | Wirkstoff |
|---|---|---|
| 10-721 | 8-1 | Flutriafol |
| 10-722 | 8-2 | Flutriafol |
| 10-723 | 8-3 | Flutriafol |
| 10-724 | 8-4 | Flutriafol |
| 10-725 | 8-5 | Flutriafol |
| 10-726 | 8-6 | Flutriafol |
| 10-727 | 8-7 | Flutriafol |
| 10-728 | 8-8 | Flutriafol |
| 10-729 | 8-9 | Flutriafol |
| 10-730 | 8-10 | Flutriafol |
| 10-731 | 8-11 | Flutriafol |
| 10-732 | 8-12 | Flutriafol |
| 10-733 | 8-13 | Flutriafol |
| 10-734 | 8-14 | Flutriafol |
| 10-735 | 8-15 | Flutriafol |
| 10-736 | 8-16 | Flutriafol |
| 10-737 | 8-17 | Flutriafol |
| 10-738 | 8-18 | Flutriafol |
| 10-739 | 8-19 | Flutriafol |
| 10-740 | 8-20 | Flutriafol |
| 10-741 | 8-21 | Flutriafol |
| 10-742 | 8-22 | Flutriafol |
| 10-743 | 8-23 | Flutriafol |
| 10-744 | 8-24 | Flutriafol |
| 10-745 | 8-25 | Flutriafol |
| 10-746 | 8-26 | Flutriafol |
| 10-747 | 8-27 | Flutriafol |
| 10-748 | 8-28 | Flutriafol |
| 10-749 | 8-29 | Flutriafol |
| 10-750 | 8-30 | Flutriafol |
| 10-751 | 8-31 | Flutriafol |
| 10-752 | 8-32 | Flutriafol |
| 10-753 | 8-33 | Flutriafol |
| 10-754 | 8-34 | Flutriafol |
| 10-755 | 8-35 | Flutriafol |
| 10-756 | 8-36 | Flutriafol |
| 10-757 | 8-37 | Flutriafol |
| 10-758 | 8-38 | Flutriafol |
| 10-759 | 8-39 | Flutriafol |
| 10-760 | 8-40 | Flutriafol |

| Nr. | Binäre Kombination | Wirkstoff |
|---|---|---|
| 10-761 | 8-41 | Flutriafol |
| 10-762 | 8-42 | Flutriafol |
| 10-763 | 8-43 | Flutriafol |
| 10-764 | 8-44 | Flutriafol |
| 10-765 | 8-45 | Flutriafol |
| 10-766 | 8-46 | Flutriafol |
| 10-767 | 8-47 | Flutriafol |
| 10-768 | 8-48 | Flutriafol |
| 10-769 | 8-49 | Flutriafol |
| 10-770 | 8-50 | Flutriafol |
| 10-771 | 8-51 | Flutriafol |
| 10-772 | 8-52 | Flutriafol |
| 10-773 | 8-53 | Flutriafol |
| 10-774 | 8-54 | Flutriafol |
| 10-775 | 8-55 | Flutriafol |
| 10-776 | 8-56 | Flutriafol |
| 10-777 | 8-57 | Flutriafol |
| 10-778 | 8-58 | Flutriafol |
| 10-779 | 8-59 | Flutriafol |
| 10-780 | 8-60 | Flutriafol |
| 10-781 | 8-61 | Flutriafol |
| 10-782 | 8-62 | Flutriafol |
| 10-783 | 8-63 | Flutriafol |
| 10-784 | 8-64 | Flutriafol |
| 10-785 | 8-65 | Flutriafol |
| 10-786 | 8-66 | Flutriafol |
| 10-787 | 8-67 | Flutriafol |
| 10-788 | 8-68 | Flutriafol |
| 10-789 | 8-69 | Flutriafol |
| 10-790 | 8-70 | Flutriafol |
| 10-791 | 8-71 | Flutriafol |
| 10-792 | 8-72 | Flutriafol |
| 10-793 | 8-73 | Flutriafol |
| 10-794 | 8-74 | Flutriafol |
| 10-795 | 8-75 | Flutriafol |
| 10-796 | 8-76 | Flutriafol |
| 10-797 | 8-77 | Flutriafol |
| 10-798 | 8-78 | Flutriafol |
| 10-799 | 8-79 | Flutriafol |
| 10-800 | 8-80 | Flutriafol |

| Nr. | Binäre Kombination | Wirkstoff |
|---|---|---|
| 10-801 | 8-81 | Flutriafol |
| 10-802 | 8-82 | Flutriafol |
| 10-803 | 8-83 | Flutriafol |
| 10-804 | 8-84 | Flutriafol |
| 10-805 | 8-85 | Flutriafol |
| 10-806 | 8-86 | Flutriafol |
| 10-807 | 8-87 | Flutriafol |
| 10-808 | 8-88 | Flutriafol |
| 10-809 | 8-89 | Flutriafol |
| 10-810 | 8-90 | Flutriafol |
| 10-811 | 8-91 | Flutriafol |
| 10-812 | 8-92 | Flutriafol |
| 10-813 | 8-93 | Flutriafol |
| 10-814 | 8-94 | Flutriafol |
| 10-815 | 8-95 | Flutriafol |
| 10-816 | 8-96 | Flutriafol |
| 10-817 | 8-97 | Flutriafol |
| 10-818 | 8-98 | Flutriafol |
| 10-819 | 8-99 | Flutriafol |
| 10-820 | 8-100 | Flutriafol |
| 10-821 | 8-101 | Flutriafol |
| 10-822 | 8-102 | Flutriafol |
| 10-823 | 8-103 | Flutriafol |
| 10-824 | 8-104 | Flutriafol |
| 10-825 | 8-105 | Flutriafol |
| 10-826 | 8-106 | Flutriafol |
| 10-827 | 8-107 | Flutriafol |
| 10-828 | 8-108 | Flutriafol |
| 10-829 | 8-109 | Flutriafol |
| 10-830 | 8-110 | Flutriafol |
| 10-831 | 8-111 | Flutriafol |
| 10-832 | 8-112 | Flutriafol |
| 10-833 | 8-113 | Flutriafol |
| 10-834 | 8-114 | Flutriafol |
| 10-835 | 8-115 | Flutriafol |
| 10-836 | 8-116 | Flutriafol |
| 10-837 | 8-117 | Flutriafol |
| 10-838 | 8-118 | Flutriafol |
| 10-839 | 8-119 | Flutriafol |
| 10-840 | 8-120 | Flutriafol |

| Nr. | Binäre Kombination | Wirkstoff |
|---|---|---|
| 10-841 | 8-121 | Flutriafol |
| 10-842 | 8-122 | Flutriafol |
| 10-843 | 8-123 | Flutriafol |
| 10-844 | 8-124 | Flutriafol |
| 10-845 | 8-125 | Flutriafol |
| 10-846 | 8-126 | Flutriafol |
| 10-847 | 8-127 | Flutriafol |
| 10-848 | 8-128 | Flutriafol |
| 10-849 | 8-129 | Flutriafol |
| 10-850 | 8-130 | Flutriafol |
| 10-851 | 8-131 | Flutriafol |
| 10-852 | 8-132 | Flutriafol |
| 10-853 | 8-133 | Flutriafol |
| 10-854 | 8-134 | Flutriafol |
| 10-855 | 8-135 | Flutriafol |
| 10-856 | 8-136 | Flutriafol |
| 10-857 | 8-137 | Flutriafol |
| 10-858 | 8-138 | Flutriafol |
| 10-859 | 8-139 | Flutriafol |
| 10-860 | 8-140 | Flutriafol |
| 10-861 | 8-141 | Flutriafol |
| 10-862 | 8-142 | Flutriafol |
| 10-863 | 8-143 | Flutriafol |
| 10-864 | 8-144 | Flutriafol |
| 10-865 | 8-145 | Flutriafol |
| 10-866 | 8-146 | Flutriafol |
| 10-867 | 8-147 | Flutriafol |
| 10-868 | 8-148 | Flutriafol |
| 10-869 | 8-149 | Flutriafol |
| 10-870 | 8-150 | Flutriafol |
| 10-871 | 8-151 | Flutriafol |
| 10-872 | 8-152 | Flutriafol |
| 10-873 | 8-153 | Flutriafol |
| 10-874 | 8-154 | Flutriafol |
| 10-875 | 8-155 | Flutriafol |
| 10-876 | 8-156 | Flutriafol |
| 10-877 | 8-157 | Flutriafol |
| 10-878 | 8-158 | Flutriafol |
| 10-879 | 8-159 | Flutriafol |
| 10-880 | 8-160 | Flutriafol |

| Nr. | Binäre Kombination | Wirkstoff |
|---|---|---|
| 10-881 | 8-161 | Flutriafol |
| 10-882 | 8-162 | Flutriafol |
| 10-883 | 8-163 | Flutriafol |
| 10-884 | 8-164 | Flutriafol |
| 10-885 | 8-165 | Flutriafol |
| 10-886 | 8-166 | Flutriafol |
| 10-887 | 8-167 | Flutriafol |
| 10-888 | 8-168 | Flutriafol |
| 10-889 | 8-169 | Flutriafol |
| 10-890 | 8-170 | Flutriafol |
| 10-891 | 8-171 | Flutriafol |
| 10-892 | 8-172 | Flutriafol |
| 10-893 | 8-173 | Flutriafol |
| 10-894 | 8-174 | Flutriafol |
| 10-895 | 8-175 | Flutriafol |
| 10-896 | 8-176 | Flutriafol |
| 10-897 | 8-177 | Flutriafol |
| 10-898 | 8-178 | Flutriafol |
| 10-899 | 8-179 | Flutriafol |
| 10-900 | 8-180 | Flutriafol |
| 10-901 | 8-1 | Triticonazol |
| 10-902 | 8-2 | Triticonazol |
| 10-903 | 8-3 | Triticonazol |
| 10-904 | 8-4 | Triticonazol |
| 10-905 | 8-5 | Triticonazol |
| 10-906 | 8-6 | Triticonazol |
| 10-907 | 8-7 | Triticonazol |
| 10-908 | 8-8 | Triticonazol |
| 10-909 | 8-9 | Triticonazol |
| 10-910 | 8-10 | Triticonazol |
| 10-911 | 8-11 | Triticonazol |
| 10-912 | 8-12 | Triticonazol |
| 10-913 | 8-13 | Triticonazol |
| 10-914 | 8-14 | Triticonazol |
| 10-915 | 8-15 | Triticonazol |
| 10-916 | 8-16 | Triticonazol |
| 10-917 | 8-17 | Triticonazol |
| 10-918 | 8-18 | Triticonazol |
| 10-919 | 8-19 | Triticonazol |
| 10-920 | 8-20 | Triticonazol |

| Nr. | Binäre Kombination | Wirkstoff |
|---|---|---|
| 10-921 | 8-21 | Triticonazol |
| 10-922 | 8-22 | Triticonazol |
| 10-923 | 8-23 | Triticonazol |
| 10-924 | 8-24 | Triticonazol |
| 10-925 | 8-25 | Triticonazol |
| 10-926 | 8-26 | Triticonazol |
| 10-927 | 8-27 | Triticonazol |
| 10-928 | 8-28 | Triticonazol |
| 10-929 | 8-29 | Triticonazol |
| 10-930 | 8-30 | Triticonazol |
| 10-931 | 8-31 | Triticonazol |
| 10-932 | 8-32 | Triticonazol |
| 10-933 | 8-33 | Triticonazol |
| 10-934 | 8-34 | Triticonazol |
| 10-935 | 8-35 | Triticonazol |
| 10-936 | 8-36 | Triticonazol |
| 10-937 | 8-37 | Triticonazol |
| 10-938 | 8-38 | Triticonazol |
| 10-939 | 8-39 | Triticonazol |
| 10-940 | 8-40 | Triticonazol |
| 10-941 | 8-41 | Triticonazol |
| 10-942 | 8-42 | Triticonazol |
| 10-943 | 8-43 | Triticonazol |
| 10-944 | 8-44 | Triticonazol |
| 10-945 | 8-45 | Triticonazol |
| 10-946 | 8-46 | Triticonazol |
| 10-947 | 8-47 | Triticonazol |
| 10-948 | 8-48 | Triticonazol |
| 10-949 | 8-49 | Triticonazol |
| 10-950 | 8-50 | Triticonazol |
| 10-951 | 8-51 | Triticonazol |
| 10-952 | 8-52 | Triticonazol |
| 10-953 | 8-53 | Triticonazol |
| 10-954 | 8-54 | Triticonazol |
| 10-955 | 8-55 | Triticonazol |
| 10-956 | 8-56 | Triticonazol |
| 10-957 | 8-57 | Triticonazol |
| 10-958 | 8-58 | Triticonazol |
| 10-959 | 8-59 | Triticonazol |
| 10-960 | 8-60 | Triticonazol |

| Nr. | Binäre Kombination | Wirkstoff |
|---|---|---|
| 10-961 | 8-61 | Triticonazol |
| 10-962 | 8-62 | Triticonazol |
| 10-963 | 8-63 | Triticonazol |
| 10-964 | 8-64 | Triticonazol |
| 10-965 | 8-65 | Triticonazol |
| 10-966 | 8-66 | Triticonazol |
| 10-967 | 8-67 | Triticonazol |
| 10-968 | 8-68 | Triticonazol |
| 10-969 | 8-69 | Triticonazol |
| 10-970 | 8-70 | Triticonazol |
| 10-971 | 8-71 | Triticonazol |
| 10-972 | 8-72 | Triticonazol |
| 10-973 | 8-73 | Triticonazol |
| 10-974 | 8-74 | Triticonazol |
| 10-975 | 8-75 | Triticonazol |
| 10-976 | 8-76 | Triticonazol |
| 10-977 | 8-77 | Triticonazol |
| 10-978 | 8-78 | Triticonazol |
| 10-979 | 8-79 | Triticonazol |
| 10-980 | 8-80 | Triticonazol |
| 10-981 | 8-81 | Triticonazol |
| 10-982 | 8-82 | Triticonazol |
| 10-983 | 8-83 | Triticonazol |
| 10-984 | 8-84 | Triticonazol |
| 10-985 | 8-85 | Triticonazol |
| 10-986 | 8-86 | Triticonazol |
| 10-987 | 8-87 | Triticonazol |
| 10-988 | 8-88 | Triticonazol |
| 10-989 | 8-89 | Triticonazol |
| 10-990 | 8-90 | Triticonazol |
| 10-991 | 8-91 | Triticonazol |
| 10-992 | 8-92 | Triticonazol |
| 10-993 | 8-93 | Triticonazol |
| 10-994 | 8-94 | Triticonazol |
| 10-995 | 8-95 | Triticonazol |
| 10-996 | 8-96 | Triticonazol |
| 10-997 | 8-97 | Triticonazol |
| 10-998 | 8-98 | Triticonazol |
| 10-999 | 8-99 | Triticonazol |
| 10-1000 | 8-100 | Triticonazol |

| Nr. | Binäre Kombination | Wirkstoff |
|---|---|---|
| 10-1001 | 8-101 | Triticonazol |
| 10-1002 | 8-102 | Triticonazol |
| 10-1003 | 8-103 | Triticonazol |
| 10-1004 | 8-104 | Triticonazol |
| 10-1005 | 8-105 | Triticonazol |
| 10-1006 | 8-106 | Triticonazol |
| 10-1007 | 8-107 | Triticonazol |
| 10-1008 | 8-108 | Triticonazol |
| 10-1009 | 8-109 | Triticonazol |
| 10-1010 | 8-110 | Triticonazol |
| 10-1011 | 8-111 | Triticonazol |
| 10-1012 | 8-112 | Triticonazol |
| 10-1013 | 8-113 | Triticonazol |
| 10-1014 | 8-114 | Triticonazol |
| 10-1015 | 8-115 | Triticonazol |
| 10-1016 | 8-116 | Triticonazol |
| 10-1017 | 8-117 | Triticonazol |
| 10-1018 | 8-118 | Triticonazol |
| 10-1019 | 8-119 | Triticonazol |
| 10-1020 | 8-120 | Triticonazol |
| 10-1021 | 8-121 | Triticonazol |
| 10-1022 | 8-122 | Triticonazol |
| 10-1023 | 8-123 | Triticonazol |
| 10-1024 | 8-124 | Triticonazol |
| 10-1025 | 8-125 | Triticonazol |
| 10-1026 | 8-126 | Triticonazol |
| 10-1027 | 8-127 | Triticonazol |
| 10-1028 | 8-128 | Triticonazol |
| 10-1029 | 8-129 | Triticonazol |
| 10-1030 | 8-130 | Triticonazol |
| 10-1031 | 8-131 | Triticonazol |
| 10-1032 | 8-132 | Triticonazol |
| 10-1033 | 8-133 | Triticonazol |
| 10-1034 | 8-134 | Triticonazol |
| 10-1035 | 8-135 | Triticonazol |
| 10-1036 | 8-136 | Triticonazol |
| 10-1037 | 8-137 | Triticonazol |
| 10-1038 | 8-138 | Triticonazol |
| 10-1039 | 8-139 | Triticonazol |
| 10-1040 | 8-140 | Triticonazol |

| Nr. | Binäre Kombination | Wirkstoff |
|---|---|---|
| 10-1041 | 8-141 | Triticonazol |
| 10-1042 | 8-142 | Triticonazol |
| 10-1043 | 8-143 | Triticonazol |
| 10-1044 | 8-144 | Triticonazol |
| 10-1045 | 8-145 | Triticonazol |
| 10-1046 | 8-146 | Triticonazol |
| 10-1047 | 8-147 | Triticonazol |
| 10-1048 | 8-148 | Triticonazol |
| 10-1049 | 8-149 | Triticonazol |
| 10-1050 | 8-150 | Triticonazol |
| 10-1051 | 8-151 | Triticonazol |
| 10-1052 | 8-152 | Triticonazol |
| 10-1053 | 8-153 | Triticonazol |
| 10-1054 | 8-154 | Triticonazol |
| 10-1055 | 8-155 | Triticonazol |
| 10-1056 | 8-156 | Triticonazol |
| 10-1057 | 8-157 | Triticonazol |
| 10-1058 | 8-158 | Triticonazol |
| 10-1059 | 8-159 | Triticonazol |
| 10-1060 | 8-160 | Triticonazol |
| 10-1061 | 8-161 | Triticonazol |
| 10-1062 | 8-162 | Triticonazol |
| 10-1063 | 8-163 | Triticonazol |
| 10-1064 | 8-164 | Triticonazol |
| 10-1065 | 8-165 | Triticonazol |
| 10-1066 | 8-166 | Triticonazol |
| 10-1067 | 8-167 | Triticonazol |
| 10-1068 | 8-168 | Triticonazol |
| 10-1069 | 8-169 | Triticonazol |
| 10-1070 | 8-170 | Triticonazol |
| 10-1071 | 8-171 | Triticonazol |
| 10-1072 | 8-172 | Triticonazol |
| 10-1073 | 8-173 | Triticonazol |
| 10-1074 | 8-174 | Triticonazol |
| 10-1075 | 8-175 | Triticonazol |
| 10-1076 | 8-176 | Triticonazol |
| 10-1077 | 8-177 | Triticonazol |
| 10-1078 | 8-178 | Triticonazol |
| 10-1079 | 8-179 | Triticonazol |
| 10-1080 | 8-180 | Triticonazol |

| Nr. | Binäre Kombination | Wirkstoff |
|---|---|---|
| 10-1081 | 8-1 | Prochloraz |
| 10-1082 | 8-2 | Prochloraz |
| 10-1083 | 8-3 | Prochloraz |
| 10-1084 | 8-4 | Prochloraz |
| 10-1085 | 8-5 | Prochloraz |
| 10-1086 | 8-6 | Prochloraz |
| 10-1087 | 8-7 | Prochloraz |
| 10-1088 | 8-8 | Prochloraz |
| 10-1089 | 8-9 | Prochloraz |
| 10-1090 | 8-10 | Prochloraz |
| 10-1091 | 8-11 | Prochloraz |
| 10-1092 | 8-12 | Prochloraz |
| 10-1093 | 8-13 | Prochloraz |
| 10-1094 | 8-14 | Prochloraz |
| 10-1095 | 8-15 | Prochloraz |
| 10-1096 | 8-16 | Prochloraz |
| 10-1097 | 8-17 | Prochloraz |
| 10-1098 | 8-18 | Prochloraz |
| 10-1099 | 8-19 | Prochloraz |
| 10-1100 | 8-20 | Prochloraz |
| 10-1101 | 8-21 | Prochloraz |
| 10-1102 | 8-22 | Prochloraz |
| 10-1103 | 8-23 | Prochloraz |
| 10-1104 | 8-24 | Prochloraz |
| 10-1105 | 8-25 | Prochloraz |
| 10-1106 | 8-26 | Prochloraz |
| 10-1107 | 8-27 | Prochloraz |
| 10-1108 | 8-28 | Prochloraz |
| 10-1109 | 8-29 | Prochloraz |
| 10-1110 | 8-30 | Prochloraz |
| 10-1111 | 8-31 | Prochloraz |
| 10-1112 | 8-32 | Prochloraz |
| 10-1113 | 8-33 | Prochloraz |
| 10-1114 | 8-34 | Prochloraz |
| 10-1115 | 8-35 | Prochloraz |
| 10-1116 | 8-36 | Prochloraz |
| 10-1117 | 8-37 | Prochloraz |
| 10-1118 | 8-38 | Prochloraz |
| 10-1119 | 8-39 | Prochloraz |
| 10-1120 | 8-40 | Prochloraz |
| 10-1121 | 8-41 | Prochloraz |
| 10-1122 | 8-42 | Prochloraz |
| 10-1123 | 8-43 | Prochloraz |
| 10-1124 | 8-44 | Prochloraz |
| 10-1125 | 8-45 | Prochloraz |
| 10-1126 | 8-46 | Prochloraz |
| 10-1127 | 8-47 | Prochloraz |
| 10-1128 | 8-48 | Prochloraz |
| 10-1129 | 8-49 | Prochloraz |
| 10-1130 | 8-50 | Prochloraz |
| 10-1131 | 8-51 | Prochloraz |
| 10-1132 | 8-52 | Prochloraz |
| 10-1133 | 8-53 | Prochloraz |
| 10-1134 | 8-54 | Prochloraz |
| 10-1135 | 8-55 | Prochloraz |
| 10-1136 | 8-56 | Prochloraz |
| 10-1137 | 8-57 | Prochloraz |
| 10-1138 | 8-58 | Prochloraz |
| 10-1139 | 8-59 | Prochloraz |
| 10-1140 | 8-60 | Prochloraz |
| 10-1141 | 8-61 | Prochloraz |
| 10-1142 | 8-62 | Prochloraz |
| 10-1143 | 8-63 | Prochloraz |
| 10-1144 | 8-64 | Prochloraz |
| 10-1145 | 8-65 | Prochloraz |
| 10-1146 | 8-66 | Prochloraz |
| 10-1147 | 8-67 | Prochloraz |
| 10-1148 | 8-68 | Prochloraz |
| 10-1149 | 8-69 | Prochloraz |
| 10-1150 | 8-70 | Prochloraz |
| 10-1151 | 8-71 | Prochloraz |
| 10-1152 | 8-72 | Prochloraz |
| 10-1153 | 8-73 | Prochloraz |
| 10-1154 | 8-74 | Prochloraz |
| 10-1155 | 8-75 | Prochloraz |
| 10-1156 | 8-76 | Prochloraz |
| 10-1157 | 8-77 | Prochloraz |
| 10-1158 | 8-78 | Prochloraz |
| 10-1159 | 8-79 | Prochloraz |
| 10-1160 | 8-80 | Prochloraz |

| Nr. | Binäre Kombination | Wirkstoff |
|---|---|---|
| 10-1161 | 8-81 | Prochloraz |
| 10-1162 | 8-82 | Prochloraz |
| 10-1163 | 8-83 | Prochloraz |
| 10-1164 | 8-84 | Prochloraz |
| 10-1165 | 8-85 | Prochloraz |
| 10-1166 | 8-86 | Prochloraz |
| 10-1167 | 8-87 | Prochloraz |
| 10-1168 | 8-88 | Prochloraz |
| 10-1169 | 8-89 | Prochloraz |
| 10-1170 | 8-90 | Prochloraz |
| 10-1171 | 8-91 | Prochloraz |
| 10-1172 | 8-92 | Prochloraz |
| 10-1173 | 8-93 | Prochloraz |
| 10-1174 | 8-94 | Prochloraz |
| 10-1175 | 8-95 | Prochloraz |
| 10-1176 | 8-96 | Prochloraz |
| 10-1177 | 8-97 | Prochloraz |
| 10-1178 | 8-98 | Prochloraz |
| 10-1179 | 8-99 | Prochloraz |
| 10-1180 | 8-100 | Prochloraz |
| 10-1181 | 8-101 | Prochloraz |
| 10-1182 | 8-102 | Prochloraz |
| 10-1183 | 8-103 | Prochloraz |
| 10-1184 | 8-104 | Prochloraz |
| 10-1185 | 8-105 | Prochloraz |
| 10-1186 | 8-106 | Prochloraz |
| 10-1187 | 8-107 | Prochloraz |
| 10-1188 | 8-108 | Prochloraz |
| 10-1189 | 8-109 | Prochloraz |
| 10-1190 | 8-110 | Prochloraz |
| 10-1191 | 8-111 | Prochloraz |
| 10-1192 | 8-112 | Prochloraz |
| 10-1193 | 8-113 | Prochloraz |
| 10-1194 | 8-114 | Prochloraz |
| 10-1195 | 8-115 | Prochloraz |
| 10-1196 | 8-116 | Prochloraz |
| 10-1197 | 8-117 | Prochloraz |
| 10-1198 | 8-118 | Prochloraz |
| 10-1199 | 8-119 | Prochloraz |
| 10-1200 | 8-120 | Prochloraz |

| Nr. | Binäre Kombination | Wirkstoff |
|---|---|---|
| 10-1201 | 8-121 | Prochloraz |
| 10-1202 | 8-122 | Prochloraz |
| 10-1203 | 8-123 | Prochloraz |
| 10-1204 | 8-124 | Prochloraz |
| 10-1205 | 8-125 | Prochloraz |
| 10-1206 | 8-126 | Prochloraz |
| 10-1207 | 8-127 | Prochloraz |
| 10-1208 | 8-128 | Prochloraz |
| 10-1209 | 8-129 | Prochloraz |
| 10-1210 | 8-130 | Prochloraz |
| 10-1211 | 8-131 | Prochloraz |
| 10-1212 | 8-132 | Prochloraz |
| 10-1213 | 8-133 | Prochloraz |
| 10-1214 | 8-134 | Prochloraz |
| 10-1215 | 8-135 | Prochloraz |
| 10-1216 | 8-136 | Prochloraz |
| 10-1217 | 8-137 | Prochloraz |
| 10-1218 | 8-138 | Prochloraz |
| 10-1219 | 8-139 | Prochloraz |
| 10-1220 | 8-140 | Prochloraz |
| 10-1221 | 8-141 | Prochloraz |
| 10-1222 | 8-142 | Prochloraz |
| 10-1223 | 8-143 | Prochloraz |
| 10-1224 | 8-144 | Prochloraz |
| 10-1225 | 8-145 | Prochloraz |
| 10-1226 | 8-146 | Prochloraz |
| 10-1227 | 8-147 | Prochloraz |
| 10-1228 | 8-148 | Prochloraz |
| 10-1229 | 8-149 | Prochloraz |
| 10-1230 | 8-150 | Prochloraz |
| 10-1231 | 8-151 | Prochloraz |
| 10-1232 | 8-152 | Prochloraz |
| 10-1233 | 8-153 | Prochloraz |
| 10-1234 | 8-154 | Prochloraz |
| 10-1235 | 8-155 | Prochloraz |
| 10-1236 | 8-156 | Prochloraz |
| 10-1237 | 8-157 | Prochloraz |
| 10-1238 | 8-158 | Prochloraz |
| 10-1239 | 8-159 | Prochloraz |
| 10-1240 | 8-160 | Prochloraz |

| Nr. | Binäre Kombination | Wirkstoff |
|---|---|---|
| 10-1241 | 8-161 | Prochloraz |
| 10-1242 | 8-162 | Prochloraz |
| 10-1243 | 8-163 | Prochloraz |
| 10-1244 | 8-164 | Prochloraz |
| 10-1245 | 8-165 | Prochloraz |
| 10-1246 | 8-166 | Prochloraz |
| 10-1247 | 8-167 | Prochloraz |
| 10-1248 | 8-168 | Prochloraz |
| 10-1249 | 8-169 | Prochloraz |
| 10-1250 | 8-170 | Prochloraz |
| 10-1251 | 8-171 | Prochloraz |
| 10-1252 | 8-172 | Prochloraz |
| 10-1253 | 8-173 | Prochloraz |
| 10-1254 | 8-174 | Prochloraz |
| 10-1255 | 8-175 | Prochloraz |
| 10-1256 | 8-176 | Prochloraz |
| 10-1257 | 8-177 | Prochloraz |
| 10-1258 | 8-178 | Prochloraz |
| 10-1259 | 8-179 | Prochloraz |
| 10-1260 | 8-180 | Prochloraz |
| 10-1261 | 8-1 | Carbendazim |
| 10-1262 | 8-2 | Carbendazim |
| 10-1263 | 8-3 | Carbendazim |
| 10-1264 | 8-4 | Carbendazim |
| 10-1265 | 8-5 | Carbendazim |
| 10-1266 | 8-6 | Carbendazim |
| 10-1267 | 8-7 | Carbendazim |
| 10-1268 | 8-8 | Carbendazim |
| 10-1269 | 8-9 | Carbendazim |
| 10-1270 | 8-10 | Carbendazim |
| 10-1271 | 8-11 | Carbendazim |
| 10-1272 | 8-12 | Carbendazim |
| 10-1273 | 8-13 | Carbendazim |
| 10-1274 | 8-14 | Carbendazim |
| 10-1275 | 8-15 | Carbendazim |
| 10-1276 | 8-16 | Carbendazim |
| 10-1277 | 8-17 | Carbendazim |
| 10-1278 | 8-18 | Carbendazim |
| 10-1279 | 8-19 | Carbendazim |
| 10-1280 | 8-20 | Carbendazim |

| Nr. | Binäre Kombination | Wirkstoff |
|---|---|---|
| 10-1281 | 8-21 | Carbendazim |
| 10-1282 | 8-22 | Carbendazim |
| 10-1283 | 8-23 | Carbendazim |
| 10-1284 | 8-24 | Carbendazim |
| 10-1285 | 8-25 | Carbendazim |
| 10-1286 | 8-26 | Carbendazim |
| 10-1287 | 8-27 | Carbendazim |
| 10-1288 | 8-28 | Carbendazim |
| 10-1289 | 8-29 | Carbendazim |
| 10-1290 | 8-30 | Carbendazim |
| 10-1291 | 8-31 | Carbendazim |
| 10-1292 | 8-32 | Carbendazim |
| 10-1293 | 8-33 | Carbendazim |
| 10-1294 | 8-34 | Carbendazim |
| 10-1295 | 8-35 | Carbendazim |
| 10-1296 | 8-36 | Carbendazim |
| 10-1297 | 8-37 | Carbendazim |
| 10-1298 | 8-38 | Carbendazim |
| 10-1299 | 8-39 | Carbendazim |
| 10-1300 | 8-40 | Carbendazim |
| 10-1301 | 8-41 | Carbendazim |
| 10-1302 | 8-42 | Carbendazim |
| 10-1303 | 8-43 | Carbendazim |
| 10-1304 | 8-44 | Carbendazim |
| 10-1305 | 8-45 | Carbendazim |
| 10-1306 | 8-46 | Carbendazim |
| 10-1307 | 8-47 | Carbendazim |
| 10-1308 | 8-48 | Carbendazim |
| 10-1309 | 8-49 | Carbendazim |
| 10-1310 | 8-50 | Carbendazim |
| 10-1311 | 8-51 | Carbendazim |
| 10-1312 | 8-52 | Carbendazim |
| 10-1313 | 8-53 | Carbendazim |
| 10-1314 | 8-54 | Carbendazim |
| 10-1315 | 8-55 | Carbendazim |
| 10-1316 | 8-56 | Carbendazim |
| 10-1317 | 8-57 | Carbendazim |
| 10-1318 | 8-58 | Carbendazim |
| 10-1319 | 8-59 | Carbendazim |
| 10-1320 | 8-60 | Carbendazim |

| Nr. | Binäre Kombination | Wirkstoff |
|---|---|---|
| 10-1321 | 8-61 | Carbendazim |
| 10-1322 | 8-62 | Carbendazim |
| 10-1323 | 8-63 | Carbendazim |
| 10-1324 | 8-64 | Carbendazim |
| 10-1325 | 8-65 | Carbendazim |
| 10-1326 | 8-66 | Carbendazim |
| 10-1327 | 8-67 | Carbendazim |
| 10-1328 | 8-68 | Carbendazim |
| 10-1329 | 8-69 | Carbendazim |
| 10-1330 | 8-70 | Carbendazim |
| 10-1331 | 8-71 | Carbendazim |
| 10-1332 | 8-72 | Carbendazim |
| 10-1333 | 8-73 | Carbendazim |
| 10-1334 | 8-74 | Carbendazim |
| 10-1335 | 8-75 | Carbendazim |
| 10-1336 | 8-76 | Carbendazim |
| 10-1337 | 8-77 | Carbendazim |
| 10-1338 | 8-78 | Carbendazim |
| 10-1339 | 8-79 | Carbendazim |
| 10-1340 | 8-80 | Carbendazim |
| 10-1341 | 8-81 | Carbendazim |
| 10-1342 | 8-82 | Carbendazim |
| 10-1343 | 8-83 | Carbendazim |
| 10-1344 | 8-84 | Carbendazim |
| 10-1345 | 8-85 | Carbendazim |
| 10-1346 | 8-86 | Carbendazim |
| 10-1347 | 8-87 | Carbendazim |
| 10-1348 | 8-88 | Carbendazim |
| 10-1349 | 8-89 | Carbendazim |
| 10-1350 | 8-90 | Carbendazim |
| 10-1351 | 8-91 | Carbendazim |
| 10-1352 | 8-92 | Carbendazim |
| 10-1353 | 8-93 | Carbendazim |
| 10-1354 | 8-94 | Carbendazim |
| 10-1355 | 8-95 | Carbendazim |
| 10-1356 | 8-96 | Carbendazim |
| 10-1357 | 8-97 | Carbendazim |
| 10-1358 | 8-98 | Carbendazim |
| 10-1359 | 8-99 | Carbendazim |
| 10-1360 | 8-100 | Carbendazim |

| Nr. | Binäre Kombination | Wirkstoff |
|---|---|---|
| 10-1361 | 8-101 | Carbendazim |
| 10-1362 | 8-102 | Carbendazim |
| 10-1363 | 8-103 | Carbendazim |
| 10-1364 | 8-104 | Carbendazim |
| 10-1365 | 8-105 | Carbendazim |
| 10-1366 | 8-106 | Carbendazim |
| 10-1367 | 8-107 | Carbendazim |
| 10-1368 | 8-108 | Carbendazim |
| 10-1369 | 8-109 | Carbendazim |
| 10-1370 | 8-110 | Carbendazim |
| 10-1371 | 8-111 | Carbendazim |
| 10-1372 | 8-112 | Carbendazim |
| 10-1373 | 8-113 | Carbendazim |
| 10-1374 | 8-114 | Carbendazim |
| 10-1375 | 8-115 | Carbendazim |
| 10-1376 | 8-116 | Carbendazim |
| 10-1377 | 8-117 | Carbendazim |
| 10-1378 | 8-118 | Carbendazim |
| 10-1379 | 8-119 | Carbendazim |
| 10-1380 | 8-120 | Carbendazim |
| 10-1381 | 8-121 | Carbendazim |
| 10-1382 | 8-122 | Carbendazim |
| 10-1383 | 8-123 | Carbendazim |
| 10-1384 | 8-124 | Carbendazim |
| 10-1385 | 8-125 | Carbendazim |
| 10-1386 | 8-126 | Carbendazim |
| 10-1387 | 8-127 | Carbendazim |
| 10-1388 | 8-128 | Carbendazim |
| 10-1389 | 8-129 | Carbendazim |
| 10-1390 | 8-130 | Carbendazim |
| 10-1391 | 8-131 | Carbendazim |
| 10-1392 | 8-132 | Carbendazim |
| 10-1393 | 8-133 | Carbendazim |
| 10-1394 | 8-134 | Carbendazim |
| 10-1395 | 8-135 | Carbendazim |
| 10-1396 | 8-136 | Carbendazim |
| 10-1397 | 8-137 | Carbendazim |
| 10-1398 | 8-138 | Carbendazim |
| 10-1399 | 8-139 | Carbendazim |
| 10-1400 | 8-140 | Carbendazim |

| Nr. | Binäre Kombination | Wirkstoff |
|---|---|---|
| 10-1401 | 8-141 | Carbendazim |
| 10-1402 | 8-142 | Carbendazim |
| 10-1403 | 8-143 | Carbendazim |
| 10-1404 | 8-144 | Carbendazim |
| 10-1405 | 8-145 | Carbendazim |
| 10-1406 | 8-146 | Carbendazim |
| 10-1407 | 8-147 | Carbendazim |
| 10-1408 | 8-148 | Carbendazim |
| 10-1409 | 8-149 | Carbendazim |
| 10-1410 | 8-150 | Carbendazim |
| 10-1411 | 8-151 | Carbendazim |
| 10-1412 | 8-152 | Carbendazim |
| 10-1413 | 8-153 | Carbendazim |
| 10-1414 | 8-154 | Carbendazim |
| 10-1415 | 8-155 | Carbendazim |
| 10-1416 | 8-156 | Carbendazim |
| 10-1417 | 8-157 | Carbendazim |
| 10-1418 | 8-158 | Carbendazim |
| 10-1419 | 8-159 | Carbendazim |
| 10-1420 | 8-160 | Carbendazim |
| 10-1421 | 8-161 | Carbendazim |
| 10-1422 | 8-162 | Carbendazim |
| 10-1423 | 8-163 | Carbendazim |
| 10-1424 | 8-164 | Carbendazim |
| 10-1425 | 8-165 | Carbendazim |
| 10-1426 | 8-166 | Carbendazim |
| 10-1427 | 8-167 | Carbendazim |
| 10-1428 | 8-168 | Carbendazim |
| 10-1429 | 8-169 | Carbendazim |
| 10-1430 | 8-170 | Carbendazim |
| 10-1431 | 8-171 | Carbendazim |
| 10-1432 | 8-172 | Carbendazim |
| 10-1433 | 8-173 | Carbendazim |
| 10-1434 | 8-174 | Carbendazim |
| 10-1435 | 8-175 | Carbendazim |
| 10-1436 | 8-176 | Carbendazim |
| 10-1437 | 8-177 | Carbendazim |
| 10-1438 | 8-178 | Carbendazim |
| 10-1439 | 8-179 | Carbendazim |
| 10-1440 | 8-180 | Carbendazim |

| Nr. | Binäre Kombination | Wirkstoff |
|---|---|---|
| 10-1441 | 8-1 | Kresoxim-methyl |
| 10-1442 | 8-2 | Kresoxim-methyl |
| 10-1443 | 8-3 | Kresoxim-methyl |
| 10-1444 | 8-4 | Kresoxim-methyl |
| 10-1445 | 8-5 | Kresoxim-methyl |
| 10-1446 | 8-6 | Kresoxim-methyl |
| 10-1447 | 8-7 | Kresoxim-methyl |
| 10-1448 | 8-8 | Kresoxim-methyl |
| 10-1449 | 8-9 | Kresoxim-methyl |
| 10-1450 | 8-10 | Kresoxim-methyl |
| 10-1451 | 8-11 | Kresoxim-methyl |
| 10-1452 | 8-12 | Kresoxim-methyl |
| 10-1453 | 8-13 | Kresoxim-methyl |
| 10-1454 | 8-14 | Kresoxim-methyl |
| 10-1455 | 8-15 | Kresoxim-methyl |
| 10-1456 | 8-16 | Kresoxim-methyl |
| 10-1457 | 8-17 | Kresoxim-methyl |
| 10-1458 | 8-18 | Kresoxim-methyl |
| 10-1459 | 8-19 | Kresoxim-methyl |
| 10-1460 | 8-20 | Kresoxim-methyl |
| 10-1461 | 8-21 | Kresoxim- |

| Nr. | Binäre Kombination | Wirkstoff |
|---|---|---|
| | | methyl |
| 10-1462 | 8-22 | Kresoxim-methyl |
| 10-1463 | 8-23 | Kresoxim-methyl |
| 10-1464 | 8-24 | Kresoxim-methyl |
| 10-1465 | 8-25 | Kresoxim-methyl |
| 10-1466 | 8-26 | Kresoxim-methyl |
| 10-1467 | 8-27 | Kresoxim-methyl |
| 10-1468 | 8-28 | Kresoxim-methyl |
| 10-1469 | 8-29 | Kresoxim-methyl |
| 10-1470 | 8-30 | Kresoxim-methyl |
| 10-1471 | 8-31 | Kresoxim-methyl |
| 10-1472 | 8-32 | Kresoxim-methyl |
| 10-1473 | 8-33 | Kresoxim-methyl |
| 10-1474 | 8-34 | Kresoxim-methyl |
| 10-1475 | 8-35 | Kresoxim-methyl |
| 10-1476 | 8-36 | Kresoxim-methyl |
| 10-1477 | 8-37 | Kresoxim-methyl |
| 10-1478 | 8-38 | Kresoxim-methyl |
| 10-1479 | 8-39 | Kresoxim-methyl |
| 10-1480 | 8-40 | Kresoxim-methyl |
| 10-1481 | 8-41 | Kresoxim-methyl |

| Nr. | Binäre Kombination | Wirkstoff |
|---|---|---|
| 10-1482 | 8-42 | Kresoxim-methyl |
| 10-1483 | 8-43 | Kresoxim-methyl |
| 10-1484 | 8-44 | Kresoxim-methyl |
| 10-1485 | 8-45 | Kresoxim-methyl |
| 10-1486 | 8-46 | Kresoxim-methyl |
| 10-1487 | 8-47 | Kresoxim-methyl |
| 10-1488 | 8-48 | Kresoxim-methyl |
| 10-1489 | 8-49 | Kresoxim-methyl |
| 10-1490 | 8-50 | Kresoxim-methyl |
| 10-1491 | 8-51 | Kresoxim-methyl |
| 10-1492 | 8-52 | Kresoxim-methyl |
| 10-1493 | 8-53 | Kresoxim-methyl |
| 10-1494 | 8-54 | Kresoxim-methyl |
| 10-1495 | 8-55 | Kresoxim-methyl |
| 10-1496 | 8-56 | Kresoxim-methyl |
| 10-1497 | 8-57 | Kresoxim-methyl |
| 10-1498 | 8-58 | Kresoxim-methyl |
| 10-1499 | 8-59 | Kresoxim-methyl |
| 10-1500 | 8-60 | Kresoxim-methyl |
| 10-1501 | 8-61 | Kresoxim-methyl |
| 10-1502 | 8-62 | Kresoxim- |

| Nr. | Binäre Kombination | Wirkstoff |
|---|---|---|
| | | methyl |
| 10-1503 | 8-63 | Kresoxim-methyl |
| 10-1504 | 8-64 | Kresoxim-methyl |
| 10-1505 | 8-65 | Kresoxim-methyl |
| 10-1506 | 8-66 | Kresoxim-methyl |
| 10-1507 | 8-67 | Kresoxim-methyl |
| 10-1508 | 8-68 | Kresoxim-methyl |
| 10-1509 | 8-69 | Kresoxim-methyl |
| 10-1510 | 8-70 | Kresoxim-methyl |
| 10-1511 | 8-71 | Kresoxim-methyl |
| 10-1512 | 8-72 | Kresoxim-methyl |
| 10-1513 | 8-73 | Kresoxim-methyl |
| 10-1514 | 8-74 | Kresoxim-methyl |
| 10-1515 | 8-75 | Kresoxim-methyl |
| 10-1516 | 8-76 | Kresoxim-methyl |
| 10-1517 | 8-77 | Kresoxim-methyl |
| 10-1518 | 8-78 | Kresoxim-methyl |
| 10-1519 | 8-79 | Kresoxim-methyl |
| 10-1520 | 8-80 | Kresoxim-methyl |
| 10-1521 | 8-81 | Kresoxim-methyl |
| 10-1522 | 8-82 | Kresoxim-methyl |

| Nr. | Binäre Kombination | Wirkstoff |
|---|---|---|
| 10-1523 | 8-83 | Kresoxim-methyl |
| 10-1524 | 8-84 | Kresoxim-methyl |
| 10-1525 | 8-85 | Kresoxim-methyl |
| 10-1526 | 8-86 | Kresoxim-methyl |
| 10-1527 | 8-87 | Kresoxim-methyl |
| 10-1528 | 8-88 | Kresoxim-methyl |
| 10-1529 | 8-89 | Kresoxim-methyl |
| 10-1530 | 8-90 | Kresoxim-methyl |
| 10-1531 | 8-91 | Kresoxim-methyl |
| 10-1532 | 8-92 | Kresoxim-methyl |
| 10-1533 | 8-93 | Kresoxim-methyl |
| 10-1534 | 8-94 | Kresoxim-methyl |
| 10-1535 | 8-95 | Kresoxim-methyl |
| 10-1536 | 8-96 | Kresoxim-methyl |
| 10-1537 | 8-97 | Kresoxim-methyl |
| 10-1538 | 8-98 | Kresoxim-methyl |
| 10-1539 | 8-99 | Kresoxim-methyl |
| 10-1540 | 8-100 | Kresoxim-methyl |
| 10-1541 | 8-101 | Kresoxim-methyl |
| 10-1542 | 8-102 | Kresoxim-methyl |
| 10-1543 | 8-103 | Kresoxim- |

| Nr. | Binäre Kombination | Wirkstoff |
|---|---|---|
| | | methyl |
| 10-1544 | 8-104 | Kresoxim-methyl |
| 10-1545 | 8-105 | Kresoxim-methyl |
| 10-1546 | 8-106 | Kresoxim-methyl |
| 10-1547 | 8-107 | Kresoxim-methyl |
| 10-1548 | 8-108 | Kresoxim-methyl |
| 10-1549 | 8-109 | Kresoxim-methyl |
| 10-1550 | 8-110 | Kresoxim-methyl |
| 10-1551 | 8-111 | Kresoxim-methyl |
| 10-1552 | 8-112 | Kresoxim-methyl |
| 10-1553 | 8-113 | Kresoxim-methyl |
| 10-1554 | 8-114 | Kresoxim-methyl |
| 10-1555 | 8-115 | Kresoxim-methyl |
| 10-1556 | 8-116 | Kresoxim-methyl |
| 10-1557 | 8-117 | Kresoxim-methyl |
| 10-1558 | 8-118 | Kresoxim-methyl |
| 10-1559 | 8-119 | Kresoxim-methyl |
| 10-1560 | 8-120 | Kresoxim-methyl |
| 10-1561 | 8-121 | Kresoxim-methyl |
| 10-1562 | 8-122 | Kresoxim-methyl |
| 10-1563 | 8-123 | Kresoxim-methyl |

| Nr. | Binäre Kombination | Wirkstoff |
|---|---|---|
| 10-1564 | 8-124 | Kresoxim-methyl |
| 10-1565 | 8-125 | Kresoxim-methyl |
| 10-1566 | 8-126 | Kresoxim-methyl |
| 10-1567 | 8-127 | Kresoxim-methyl |
| 10-1568 | 8-128 | Kresoxim-methyl |
| 10-1569 | 8-129 | Kresoxim-methyl |
| 10-1570 | 8-130 | Kresoxim-methyl |
| 10-1571 | 8-131 | Kresoxim-methyl |
| 10-1572 | 8-132 | Kresoxim-methyl |
| 10-1573 | 8-133 | Kresoxim-methyl |
| 10-1574 | 8-134 | Kresoxim-methyl |
| 10-1575 | 8-135 | Kresoxim-methyl |
| 10-1576 | 8-136 | Kresoxim-methyl |
| 10-1577 | 8-137 | Kresoxim-methyl |
| 10-1578 | 8-138 | Kresoxim-methyl |
| 10-1579 | 8-139 | Kresoxim-methyl |
| 10-1580 | 8-140 | Kresoxim-methyl |
| 10-1581 | 8-141 | Kresoxim-methyl |
| 10-1582 | 8-142 | Kresoxim-methyl |
| 10-1583 | 8-143 | Kresoxim-methyl |
| 10-1584 | 8-144 | Kresoxim- |

| Nr. | Binäre Kombination | Wirkstoff |
|---|---|---|
| | | methyl |
| 10-1585 | 8-145 | Kresoxim-methyl |
| 10-1586 | 8-146 | Kresoxim-methyl |
| 10-1587 | 8-147 | Kresoxim-methyl |
| 10-1588 | 8-148 | Kresoxim-methyl |
| 10-1589 | 8-149 | Kresoxim-methyl |
| 10-1590 | 8-150 | Kresoxim-methyl |
| 10-1591 | 8-151 | Kresoxim-methyl |
| 10-1592 | 8-152 | Kresoxim-methyl |
| 10-1593 | 8-153 | Kresoxim-methyl |
| 10-1594 | 8-154 | Kresoxim-methyl |
| 10-1595 | 8-155 | Kresoxim-methyl |
| 10-1596 | 8-156 | Kresoxim-methyl |
| 10-1597 | 8-157 | Kresoxim-methyl |
| 10-1598 | 8-158 | Kresoxim-methyl |
| 10-1599 | 8-159 | Kresoxim-methyl |
| 10-1600 | 8-160 | Kresoxim-methyl |
| 10-1601 | 8-161 | Kresoxim-methyl |
| 10-1602 | 8-162 | Kresoxim-methyl |
| 10-1603 | 8-163 | Kresoxim-methyl |
| 10-1604 | 8-164 | Kresoxim-methyl |

| Nr. | Binäre Kombination | Wirkstoff |
|---|---|---|
| 10-1605 | 8-165 | Kresoxim-methyl |
| 10-1606 | 8-166 | Kresoxim-methyl |
| 10-1607 | 8-167 | Kresoxim-methyl |
| 10-1608 | 8-168 | Kresoxim-methyl |
| 10-1609 | 8-169 | Kresoxim-methyl |
| 10-1610 | 8-170 | Kresoxim-methyl |
| 10-1611 | 8-171 | Kresoxim-methyl |
| 10-1612 | 8-172 | Kresoxim-methyl |
| 10-1613 | 8-173 | Kresoxim-methyl |
| 10-1614 | 8-174 | Kresoxim-methyl |
| 10-1615 | 8-175 | Kresoxim-methyl |
| 10-1616 | 8-176 | Kresoxim-methyl |
| 10-1617 | 8-177 | Kresoxim-methyl |
| 10-1618 | 8-178 | Kresoxim-methyl |
| 10-1619 | 8-179 | Kresoxim-methyl |
| 10-1620 | 8-180 | Kresoxim-methyl |
| 10-1621 | 8-1 | Pyraclostrobin |
| 10-1622 | 8-2 | Pyraclostrobin |
| 10-1623 | 8-3 | Pyraclostrobin |
| 10-1624 | 8-4 | Pyraclostrobin |
| 10-1625 | 8-5 | Pyraclostrobin |
| 10-1626 | 8-6 | Pyraclostrobin |
| 10-1627 | 8-7 | Pyraclostrobin |
| 10-1628 | 8-8 | Pyraclostrobin |
| 10-1629 | 8-9 | Pyraclostrobin |

| Nr. | Binäre Kombination | Wirkstoff |
|---|---|---|
| 10-1630 | 8-10 | Pyraclostrobin |
| 10-1631 | 8-11 | Pyraclostrobin |
| 10-1632 | 8-12 | Pyraclostrobin |
| 10-1633 | 8-13 | Pyraclostrobin |
| 10-1634 | 8-14 | Pyraclostrobin |
| 10-1635 | 8-15 | Pyraclostrobin |
| 10-1636 | 8-16 | Pyraclostrobin |
| 10-1637 | 8-17 | Pyraclostrobin |
| 10-1638 | 8-18 | Pyraclostrobin |
| 10-1639 | 8-19 | Pyraclostrobin |
| 10-1640 | 8-20 | Pyraclostrobin |
| 10-1641 | 8-21 | Pyraclostrobin |
| 10-1642 | 8-22 | Pyraclostrobin |
| 10-1643 | 8-23 | Pyraclostrobin |
| 10-1644 | 8-24 | Pyraclostrobin |
| 10-1645 | 8-25 | Pyraclostrobin |
| 10-1646 | 8-26 | Pyraclostrobin |
| 10-1647 | 8-27 | Pyraclostrobin |
| 10-1648 | 8-28 | Pyraclostrobin |
| 10-1649 | 8-29 | Pyraclostrobin |
| 10-1650 | 8-30 | Pyraclostrobin |
| 10-1651 | 8-31 | Pyraclostrobin |
| 10-1652 | 8-32 | Pyraclostrobin |
| 10-1653 | 8-33 | Pyraclostrobin |
| 10-1654 | 8-34 | Pyraclostrobin |
| 10-1655 | 8-35 | Pyraclostrobin |
| 10-1656 | 8-36 | Pyraclostrobin |
| 10-1657 | 8-37 | Pyraclostrobin |
| 10-1658 | 8-38 | Pyraclostrobin |
| 10-1659 | 8-39 | Pyraclostrobin |
| 10-1660 | 8-40 | Pyraclostrobin |
| 10-1661 | 8-41 | Pyraclostrobin |
| 10-1662 | 8-42 | Pyraclostrobin |
| 10-1663 | 8-43 | Pyraclostrobin |
| 10-1664 | 8-44 | Pyraclostrobin |
| 10-1665 | 8-45 | Pyraclostrobin |
| 10-1666 | 8-46 | Pyraclostrobin |
| 10-1667 | 8-47 | Pyraclostrobin |
| 10-1668 | 8-48 | Pyraclostrobin |
| 10-1669 | 8-49 | Pyraclostrobin |

| Nr. | Binäre Kombination | Wirkstoff |
|---|---|---|
| 10-1670 | 8-50 | Pyraclostrobin |
| 10-1671 | 8-51 | Pyraclostrobin |
| 10-1672 | 8-52 | Pyraclostrobin |
| 10-1673 | 8-53 | Pyraclostrobin |
| 10-1674 | 8-54 | Pyraclostrobin |
| 10-1675 | 8-55 | Pyraclostrobin |
| 10-1676 | 8-56 | Pyraclostrobin |
| 10-1677 | 8-57 | Pyraclostrobin |
| 10-1678 | 8-58 | Pyraclostrobin |
| 10-1679 | 8-59 | Pyraclostrobin |
| 10-1680 | 8-60 | Pyraclostrobin |
| 10-1681 | 8-61 | Pyraclostrobin |
| 10-1682 | 8-62 | Pyraclostrobin |
| 10-1683 | 8-63 | Pyraclostrobin |
| 10-1684 | 8-64 | Pyraclostrobin |
| 10-1685 | 8-65 | Pyraclostrobin |
| 10-1686 | 8-66 | Pyraclostrobin |
| 10-1687 | 8-67 | Pyraclostrobin |
| 10-1688 | 8-68 | Pyraclostrobin |
| 10-1689 | 8-69 | Pyraclostrobin |
| 10-1690 | 8-70 | Pyraclostrobin |
| 10-1691 | 8-71 | Pyraclostrobin |
| 10-1692 | 8-72 | Pyraclostrobin |
| 10-1693 | 8-73 | Pyraclostrobin |
| 10-1694 | 8-74 | Pyraclostrobin |
| 10-1695 | 8-75 | Pyraclostrobin |
| 10-1696 | 8-76 | Pyraclostrobin |
| 10-1697 | 8-77 | Pyraclostrobin |
| 10-1698 | 8-78 | Pyraclostrobin |
| 10-1699 | 8-79 | Pyraclostrobin |
| 10-1700 | 8-80 | Pyraclostrobin |
| 10-1701 | 8-81 | Pyraclostrobin |
| 10-1702 | 8-82 | Pyraclostrobin |
| 10-1703 | 8-83 | Pyraclostrobin |
| 10-1704 | 8-84 | Pyraclostrobin |
| 10-1705 | 8-85 | Pyraclostrobin |
| 10-1706 | 8-86 | Pyraclostrobin |
| 10-1707 | 8-87 | Pyraclostrobin |
| 10-1708 | 8-88 | Pyraclostrobin |
| 10-1709 | 8-89 | Pyraclostrobin |

| Nr. | Binäre Kombination | Wirkstoff |
|---|---|---|
| 10-1710 | 8-90 | Pyraclostrobin |
| 10-1711 | 8-91 | Pyraclostrobin |
| 10-1712 | 8-92 | Pyraclostrobin |
| 10-1713 | 8-93 | Pyraclostrobin |
| 10-1714 | 8-94 | Pyraclostrobin |
| 10-1715 | 8-95 | Pyraclostrobin |
| 10-1716 | 8-96 | Pyraclostrobin |
| 10-1717 | 8-97 | Pyraclostrobin |
| 10-1718 | 8-98 | Pyraclostrobin |
| 10-1719 | 8-99 | Pyraclostrobin |
| 10-1720 | 8-100 | Pyraclostrobin |
| 10-1721 | 8-101 | Pyraclostrobin |
| 10-1722 | 8-102 | Pyraclostrobin |
| 10-1723 | 8-103 | Pyraclostrobin |
| 10-1724 | 8-104 | Pyraclostrobin |
| 10-1725 | 8-105 | Pyraclostrobin |
| 10-1726 | 8-106 | Pyraclostrobin |
| 10-1727 | 8-107 | Pyraclostrobin |
| 10-1728 | 8-108 | Pyraclostrobin |
| 10-1729 | 8-109 | Pyraclostrobin |
| 10-1730 | 8-110 | Pyraclostrobin |
| 10-1731 | 8-111 | Pyraclostrobin |
| 10-1732 | 8-112 | Pyraclostrobin |
| 10-1733 | 8-113 | Pyraclostrobin |
| 10-1734 | 8-114 | Pyraclostrobin |
| 10-1735 | 8-115 | Pyraclostrobin |
| 10-1736 | 8-116 | Pyraclostrobin |
| 10-1737 | 8-117 | Pyraclostrobin |
| 10-1738 | 8-118 | Pyraclostrobin |
| 10-1739 | 8-119 | Pyraclostrobin |
| 10-1740 | 8-120 | Pyraclostrobin |
| 10-1741 | 8-121 | Pyraclostrobin |
| 10-1742 | 8-122 | Pyraclostrobin |
| 10-1743 | 8-123 | Pyraclostrobin |
| 10-1744 | 8-124 | Pyraclostrobin |
| 10-1745 | 8-125 | Pyraclostrobin |
| 10-1746 | 8-126 | Pyraclostrobin |
| 10-1747 | 8-127 | Pyraclostrobin |
| 10-1748 | 8-128 | Pyraclostrobin |
| 10-1749 | 8-129 | Pyraclostrobin |

| Nr. | Binäre Kombination | Wirkstoff |
|---|---|---|
| 10-1750 | 8-130 | Pyraclostrobin |
| 10-1751 | 8-131 | Pyraclostrobin |
| 10-1752 | 8-132 | Pyraclostrobin |
| 10-1753 | 8-133 | Pyraclostrobin |
| 10-1754 | 8-134 | Pyraclostrobin |
| 10-1755 | 8-135 | Pyraclostrobin |
| 10-1756 | 8-136 | Pyraclostrobin |
| 10-1757 | 8-137 | Pyraclostrobin |
| 10-1758 | 8-138 | Pyraclostrobin |
| 10-1759 | 8-139 | Pyraclostrobin |
| 10-1760 | 8-140 | Pyraclostrobin |
| 10-1761 | 8-141 | Pyraclostrobin |
| 10-1762 | 8-142 | Pyraclostrobin |
| 10-1763 | 8-143 | Pyraclostrobin |
| 10-1764 | 8-144 | Pyraclostrobin |
| 10-1765 | 8-145 | Pyraclostrobin |
| 10-1766 | 8-146 | Pyraclostrobin |
| 10-1767 | 8-147 | Pyraclostrobin |
| 10-1768 | 8-148 | Pyraclostrobin |
| 10-1769 | 8-149 | Pyraclostrobin |
| 10-1770 | 8-150 | Pyraclostrobin |
| 10-1771 | 8-151 | Pyraclostrobin |
| 10-1772 | 8-152 | Pyraclostrobin |
| 10-1773 | 8-153 | Pyraclostrobin |
| 10-1774 | 8-154 | Pyraclostrobin |
| 10-1775 | 8-155 | Pyraclostrobin |
| 10-1776 | 8-156 | Pyraclostrobin |
| 10-1777 | 8-157 | Pyraclostrobin |
| 10-1778 | 8-158 | Pyraclostrobin |
| 10-1779 | 8-159 | Pyraclostrobin |
| 10-1780 | 8-160 | Pyraclostrobin |
| 10-1781 | 8-161 | Pyraclostrobin |
| 10-1782 | 8-162 | Pyraclostrobin |
| 10-1783 | 8-163 | Pyraclostrobin |
| 10-1784 | 8-164 | Pyraclostrobin |
| 10-1785 | 8-165 | Pyraclostrobin |
| 10-1786 | 8-166 | Pyraclostrobin |
| 10-1787 | 8-167 | Pyraclostrobin |
| 10-1788 | 8-168 | Pyraclostrobin |
| 10-1789 | 8-169 | Pyraclostrobin |

| Nr. | Binäre Kombination | Wirkstoff |
|---|---|---|
| 10-1790 | 8-170 | Pyraclostrobin |
| 10-1791 | 8-171 | Pyraclostrobin |
| 10-1792 | 8-172 | Pyraclostrobin |
| 10-1793 | 8-173 | Pyraclostrobin |
| 10-1794 | 8-174 | Pyraclostrobin |
| 10-1795 | 8-175 | Pyraclostrobin |
| 10-1796 | 8-176 | Pyraclostrobin |
| 10-1797 | 8-177 | Pyraclostrobin |
| 10-1798 | 8-178 | Pyraclostrobin |
| 10-1799 | 8-179 | Pyraclostrobin |
| 10-1800 | 8-180 | Pyraclostrobin |
| 10-1801 | 8-1 | Orysastrobin |
| 10-1802 | 8-2 | Orysastrobin |
| 10-1803 | 8-3 | Orysastrobin |
| 10-1804 | 8-4 | Orysastrobin |
| 10-1805 | 8-5 | Orysastrobin |
| 10-1806 | 8-6 | Orysastrobin |
| 10-1807 | 8-7 | Orysastrobin |
| 10-1808 | 8-8 | Orysastrobin |
| 10-1809 | 8-9 | Orysastrobin |
| 10-1810 | 8-10 | Orysastrobin |
| 10-1811 | 8-11 | Orysastrobin |
| 10-1812 | 8-12 | Orysastrobin |
| 10-1813 | 8-13 | Orysastrobin |
| 10-1814 | 8-14 | Orysastrobin |
| 10-1815 | 8-15 | Orysastrobin |
| 10-1816 | 8-16 | Orysastrobin |
| 10-1817 | 8-17 | Orysastrobin |
| 10-1818 | 8-18 | Orysastrobin |
| 10-1819 | 8-19 | Orysastrobin |
| 10-1820 | 8-20 | Orysastrobin |
| 10-1821 | 8-21 | Orysastrobin |
| 10-1822 | 8-22 | Orysastrobin |
| 10-1823 | 8-23 | Orysastrobin |
| 10-1824 | 8-24 | Orysastrobin |
| 10-1825 | 8-25 | Orysastrobin |
| 10-1826 | 8-26 | Orysastrobin |
| 10-1827 | 8-27 | Orysastrobin |
| 10-1828 | 8-28 | Orysastrobin |
| 10-1829 | 8-29 | Orysastrobin |

| Nr. | Binäre Kombination | Wirkstoff |
|---|---|---|
| 10-1830 | 8-30 | Orysastrobin |
| 10-1831 | 8-31 | Orysastrobin |
| 10-1832 | 8-32 | Orysastrobin |
| 10-1833 | 8-33 | Orysastrobin |
| 10-1834 | 8-34 | Orysastrobin |
| 10-1835 | 8-35 | Orysastrobin |
| 10-1836 | 8-36 | Orysastrobin |
| 10-1837 | 8-37 | Orysastrobin |
| 10-1838 | 8-38 | Orysastrobin |
| 10-1839 | 8-39 | Orysastrobin |
| 10-1840 | 8-40 | Orysastrobin |
| 10-1841 | 8-41 | Orysastrobin |
| 10-1842 | 8-42 | Orysastrobin |
| 10-1843 | 8-43 | Orysastrobin |
| 10-1844 | 8-44 | Orysastrobin |
| 10-1845 | 8-45 | Orysastrobin |
| 10-1846 | 8-46 | Orysastrobin |
| 10-1847 | 8-47 | Orysastrobin |
| 10-1848 | 8-48 | Orysastrobin |
| 10-1849 | 8-49 | Orysastrobin |
| 10-1850 | 8-50 | Orysastrobin |
| 10-1851 | 8-51 | Orysastrobin |
| 10-1852 | 8-52 | Orysastrobin |
| 10-1853 | 8-53 | Orysastrobin |
| 10-1854 | 8-54 | Orysastrobin |
| 10-1855 | 8-55 | Orysastrobin |
| 10-1856 | 8-56 | Orysastrobin |
| 10-1857 | 8-57 | Orysastrobin |
| 10-1858 | 8-58 | Orysastrobin |
| 10-1859 | 8-59 | Orysastrobin |
| 10-1860 | 8-60 | Orysastrobin |
| 10-1861 | 8-61 | Orysastrobin |
| 10-1862 | 8-62 | Orysastrobin |
| 10-1863 | 8-63 | Orysastrobin |
| 10-1864 | 8-64 | Orysastrobin |
| 10-1865 | 8-65 | Orysastrobin |
| 10-1866 | 8-66 | Orysastrobin |
| 10-1867 | 8-67 | Orysastrobin |
| 10-1868 | 8-68 | Orysastrobin |
| 10-1869 | 8-69 | Orysastrobin |

| Nr. | Binäre Kombination | Wirkstoff |
|---|---|---|
| 10-1870 | 8-70 | Orysastrobin |
| 10-1871 | 8-71 | Orysastrobin |
| 10-1872 | 8-72 | Orysastrobin |
| 10-1873 | 8-73 | Orysastrobin |
| 10-1874 | 8-74 | Orysastrobin |
| 10-1875 | 8-75 | Orysastrobin |
| 10-1876 | 8-76 | Orysastrobin |
| 10-1877 | 8-77 | Orysastrobin |
| 10-1878 | 8-78 | Orysastrobin |
| 10-1879 | 8-79 | Orysastrobin |
| 10-1880 | 8-80 | Orysastrobin |
| 10-1881 | 8-81 | Orysastrobin |
| 10-1882 | 8-82 | Orysastrobin |
| 10-1883 | 8-83 | Orysastrobin |
| 10-1884 | 8-84 | Orysastrobin |
| 10-1885 | 8-85 | Orysastrobin |
| 10-1886 | 8-86 | Orysastrobin |
| 10-1887 | 8-87 | Orysastrobin |
| 10-1888 | 8-88 | Orysastrobin |
| 10-1889 | 8-89 | Orysastrobin |
| 10-1890 | 8-90 | Orysastrobin |
| 10-1891 | 8-91 | Orysastrobin |
| 10-1892 | 8-92 | Orysastrobin |
| 10-1893 | 8-93 | Orysastrobin |
| 10-1894 | 8-94 | Orysastrobin |
| 10-1895 | 8-95 | Orysastrobin |
| 10-1896 | 8-96 | Orysastrobin |
| 10-1897 | 8-97 | Orysastrobin |
| 10-1898 | 8-98 | Orysastrobin |
| 10-1899 | 8-99 | Orysastrobin |
| 10-1900 | 8-100 | Orysastrobin |
| 10-1901 | 8-101 | Orysastrobin |
| 10-1902 | 8-102 | Orysastrobin |
| 10-1903 | 8-103 | Orysastrobin |
| 10-1904 | 8-104 | Orysastrobin |
| 10-1905 | 8-105 | Orysastrobin |
| 10-1906 | 8-106 | Orysastrobin |
| 10-1907 | 8-107 | Orysastrobin |
| 10-1908 | 8-108 | Orysastrobin |
| 10-1909 | 8-109 | Orysastrobin |

| Nr. | Binäre Kombination | Wirkstoff |
|---|---|---|
| 10-1910 | 8-110 | Orysastrobin |
| 10-1911 | 8-111 | Orysastrobin |
| 10-1912 | 8-112 | Orysastrobin |
| 10-1913 | 8-113 | Orysastrobin |
| 10-1914 | 8-114 | Orysastrobin |
| 10-1915 | 8-115 | Orysastrobin |
| 10-1916 | 8-116 | Orysastrobin |
| 10-1917 | 8-117 | Orysastrobin |
| 10-1918 | 8-118 | Orysastrobin |
| 10-1919 | 8-119 | Orysastrobin |
| 10-1920 | 8-120 | Orysastrobin |
| 10-1921 | 8-121 | Orysastrobin |
| 10-1922 | 8-122 | Orysastrobin |
| 10-1923 | 8-123 | Orysastrobin |
| 10-1924 | 8-124 | Orysastrobin |
| 10-1925 | 8-125 | Orysastrobin |
| 10-1926 | 8-126 | Orysastrobin |
| 10-1927 | 8-127 | Orysastrobin |
| 10-1928 | 8-128 | Orysastrobin |
| 10-1929 | 8-129 | Orysastrobin |
| 10-1930 | 8-130 | Orysastrobin |
| 10-1931 | 8-131 | Orysastrobin |
| 10-1932 | 8-132 | Orysastrobin |
| 10-1933 | 8-133 | Orysastrobin |
| 10-1934 | 8-134 | Orysastrobin |
| 10-1935 | 8-135 | Orysastrobin |
| 10-1936 | 8-136 | Orysastrobin |
| 10-1937 | 8-137 | Orysastrobin |
| 10-1938 | 8-138 | Orysastrobin |
| 10-1939 | 8-139 | Orysastrobin |
| 10-1940 | 8-140 | Orysastrobin |
| 10-1941 | 8-141 | Orysastrobin |
| 10-1942 | 8-142 | Orysastrobin |
| 10-1943 | 8-143 | Orysastrobin |
| 10-1944 | 8-144 | Orysastrobin |
| 10-1945 | 8-145 | Orysastrobin |
| 10-1946 | 8-146 | Orysastrobin |
| 10-1947 | 8-147 | Orysastrobin |
| 10-1948 | 8-148 | Orysastrobin |
| 10-1949 | 8-149 | Orysastrobin |

| Nr. | Binäre Kombination | Wirkstoff |
|---|---|---|
| 10-1950 | 8-150 | Orysastrobin |
| 10-1951 | 8-151 | Orysastrobin |
| 10-1952 | 8-152 | Orysastrobin |
| 10-1953 | 8-153 | Orysastrobin |
| 10-1954 | 8-154 | Orysastrobin |
| 10-1955 | 8-155 | Orysastrobin |
| 10-1956 | 8-156 | Orysastrobin |
| 10-1957 | 8-157 | Orysastrobin |
| 10-1958 | 8-158 | Orysastrobin |
| 10-1959 | 8-159 | Orysastrobin |
| 10-1960 | 8-160 | Orysastrobin |
| 10-1961 | 8-161 | Orysastrobin |
| 10-1962 | 8-162 | Orysastrobin |
| 10-1963 | 8-163 | Orysastrobin |
| 10-1964 | 8-164 | Orysastrobin |
| 10-1965 | 8-165 | Orysastrobin |
| 10-1966 | 8-166 | Orysastrobin |
| 10-1967 | 8-167 | Orysastrobin |
| 10-1968 | 8-168 | Orysastrobin |
| 10-1969 | 8-169 | Orysastrobin |
| 10-1970 | 8-170 | Orysastrobin |
| 10-1971 | 8-171 | Orysastrobin |
| 10-1972 | 8-172 | Orysastrobin |
| 10-1973 | 8-173 | Orysastrobin |
| 10-1974 | 8-174 | Orysastrobin |
| 10-1975 | 8-175 | Orysastrobin |
| 10-1976 | 8-176 | Orysastrobin |
| 10-1977 | 8-177 | Orysastrobin |
| 10-1978 | 8-178 | Orysastrobin |
| 10-1979 | 8-179 | Orysastrobin |
| 10-1980 | 8-180 | Orysastrobin |
| 10-1981 | 8-1 | Dimethomorph |
| 10-1982 | 8-2 | Dimethomorph |
| 10-1983 | 8-3 | Dimethomorph |
| 10-1984 | 8-4 | Dimethomorph |
| 10-1985 | 8-5 | Dimethomorph |
| 10-1986 | 8-6 | Dimethomorph |
| 10-1987 | 8-7 | Dimethomorph |
| 10-1988 | 8-8 | Dimethomorph |
| 10-1989 | 8-9 | Dimethomorph |

| Nr. | Binäre Kombination | Wirkstoff |
|---|---|---|
| 10-1990 | 8-10 | Dimethomorph |
| 10-1991 | 8-11 | Dimethomorph |
| 10-1992 | 8-12 | Dimethomorph |
| 10-1993 | 8-13 | Dimethomorph |
| 10-1994 | 8-14 | Dimethomorph |
| 10-1995 | 8-15 | Dimethomorph |
| 10-1996 | 8-16 | Dimethomorph |
| 10-1997 | 8-17 | Dimethomorph |
| 10-1998 | 8-18 | Dimethomorph |
| 10-1999 | 8-19 | Dimethomorph |
| 10-2000 | 8-20 | Dimethomorph |
| 10-2001 | 8-21 | Dimethomorph |
| 10-2002 | 8-22 | Dimethomorph |
| 10-2003 | 8-23 | Dimethomorph |
| 10-2004 | 8-24 | Dimethomorph |
| 10-2005 | 8-25 | Dimethomorph |
| 10-2006 | 8-26 | Dimethomorph |
| 10-2007 | 8-27 | Dimethomorph |
| 10-2008 | 8-28 | Dimethomorph |
| 10-2009 | 8-29 | Dimethomorph |
| 10-2010 | 8-30 | Dimethomorph |
| 10-2011 | 8-31 | Dimethomorph |
| 10-2012 | 8-32 | Dimethomorph |
| 10-2013 | 8-33 | Dimethomorph |
| 10-2014 | 8-34 | Dimethomorph |
| 10-2015 | 8-35 | Dimethomorph |
| 10-2016 | 8-36 | Dimethomorph |
| 10-2017 | 8-37 | Dimethomorph |
| 10-2018 | 8-38 | Dimethomorph |
| 10-2019 | 8-39 | Dimethomorph |
| 10-2020 | 8-40 | Dimethomorph |
| 10-2021 | 8-41 | Dimethomorph |
| 10-2022 | 8-42 | Dimethomorph |
| 10-2023 | 8-43 | Dimethomorph |
| 10-2024 | 8-44 | Dimethomorph |
| 10-2025 | 8-45 | Dimethomorph |
| 10-2026 | 8-46 | Dimethomorph |
| 10-2027 | 8-47 | Dimethomorph |
| 10-2028 | 8-48 | Dimethomorph |
| 10-2029 | 8-49 | Dimethomorph |

| Nr. | Binäre Kombination | Wirkstoff |
|---|---|---|
| 10-2030 | 8-50 | Dimethomorph |
| 10-2031 | 8-51 | Dimethomorph |
| 10-2032 | 8-52 | Dimethomorph |
| 10-2033 | 8-53 | Dimethomorph |
| 10-2034 | 8-54 | Dimethomorph |
| 10-2035 | 8-55 | Dimethomorph |
| 10-2036 | 8-56 | Dimethomorph |
| 10-2037 | 8-57 | Dimethomorph |
| 10-2038 | 8-58 | Dimethomorph |
| 10-2039 | 8-59 | Dimethomorph |
| 10-2040 | 8-60 | Dimethomorph |
| 10-2041 | 8-61 | Dimethomorph |
| 10-2042 | 8-62 | Dimethomorph |
| 10-2043 | 8-63 | Dimethomorph |
| 10-2044 | 8-64 | Dimethomorph |
| 10-2045 | 8-65 | Dimethomorph |
| 10-2046 | 8-66 | Dimethomorph |
| 10-2047 | 8-67 | Dimethomorph |
| 10-2048 | 8-68 | Dimethomorph |
| 10-2049 | 8-69 | Dimethomorph |
| 10-2050 | 8-70 | Dimethomorph |
| 10-2051 | 8-71 | Dimethomorph |
| 10-2052 | 8-72 | Dimethomorph |
| 10-2053 | 8-73 | Dimethomorph |
| 10-2054 | 8-74 | Dimethomorph |
| 10-2055 | 8-75 | Dimethomorph |
| 10-2056 | 8-76 | Dimethomorph |
| 10-2057 | 8-77 | Dimethomorph |
| 10-2058 | 8-78 | Dimethomorph |
| 10-2059 | 8-79 | Dimethomorph |
| 10-2060 | 8-80 | Dimethomorph |
| 10-2061 | 8-81 | Dimethomorph |
| 10-2062 | 8-82 | Dimethomorph |
| 10-2063 | 8-83 | Dimethomorph |
| 10-2064 | 8-84 | Dimethomorph |
| 10-2065 | 8-85 | Dimethomorph |
| 10-2066 | 8-86 | Dimethomorph |
| 10-2067 | 8-87 | Dimethomorph |
| 10-2068 | 8-88 | Dimethomorph |
| 10-2069 | 8-89 | Dimethomorph |

| Nr. | Binäre Kombination | Wirkstoff |
|---|---|---|
| 10-2070 | 8-90 | Dimethomorph |
| 10-2071 | 8-91 | Dimethomorph |
| 10-2072 | 8-92 | Dimethomorph |
| 10-2073 | 8-93 | Dimethomorph |
| 10-2074 | 8-94 | Dimethomorph |
| 10-2075 | 8-95 | Dimethomorph |
| 10-2076 | 8-96 | Dimethomorph |
| 10-2077 | 8-97 | Dimethomorph |
| 10-2078 | 8-98 | Dimethomorph |
| 10-2079 | 8-99 | Dimethomorph |
| 10-2080 | 8-100 | Dimethomorph |
| 10-2081 | 8-101 | Dimethomorph |
| 10-2082 | 8-102 | Dimethomorph |
| 10-2083 | 8-103 | Dimethomorph |
| 10-2084 | 8-104 | Dimethomorph |
| 10-2085 | 8-105 | Dimethomorph |
| 10-2086 | 8-106 | Dimethomorph |
| 10-2087 | 8-107 | Dimethomorph |
| 10-2088 | 8-108 | Dimethomorph |
| 10-2089 | 8-109 | Dimethomorph |
| 10-2090 | 8-110 | Dimethomorph |
| 10-2091 | 8-111 | Dimethomorph |
| 10-2092 | 8-112 | Dimethomorph |
| 10-2093 | 8-113 | Dimethomorph |
| 10-2094 | 8-114 | Dimethomorph |
| 10-2095 | 8-115 | Dimethomorph |
| 10-2096 | 8-116 | Dimethomorph |
| 10-2097 | 8-117 | Dimethomorph |
| 10-2098 | 8-118 | Dimethomorph |
| 10-2099 | 8-119 | Dimethomorph |
| 10-2100 | 8-120 | Dimethomorph |
| 10-2101 | 8-121 | Dimethomorph |
| 10-2102 | 8-122 | Dimethomorph |
| 10-2103 | 8-123 | Dimethomorph |
| 10-2104 | 8-124 | Dimethomorph |
| 10-2105 | 8-125 | Dimethomorph |
| 10-2106 | 8-126 | Dimethomorph |
| 10-2107 | 8-127 | Dimethomorph |
| 10-2108 | 8-128 | Dimethomorph |
| 10-2109 | 8-129 | Dimethomorph |

| Nr. | Binäre Kombination | Wirkstoff |
|---|---|---|
| 10-2110 | 8-130 | Dimethomorph |
| 10-2111 | 8-131 | Dimethomorph |
| 10-2112 | 8-132 | Dimethomorph |
| 10-2113 | 8-133 | Dimethomorph |
| 10-2114 | 8-134 | Dimethomorph |
| 10-2115 | 8-135 | Dimethomorph |
| 10-2116 | 8-136 | Dimethomorph |
| 10-2117 | 8-137 | Dimethomorph |
| 10-2118 | 8-138 | Dimethomorph |
| 10-2119 | 8-139 | Dimethomorph |
| 10-2120 | 8-140 | Dimethomorph |
| 10-2121 | 8-141 | Dimethomorph |
| 10-2122 | 8-142 | Dimethomorph |
| 10-2123 | 8-143 | Dimethomorph |
| 10-2124 | 8-144 | Dimethomorph |
| 10-2125 | 8-145 | Dimethomorph |
| 10-2126 | 8-146 | Dimethomorph |
| 10-2127 | 8-147 | Dimethomorph |
| 10-2128 | 8-148 | Dimethomorph |
| 10-2129 | 8-149 | Dimethomorph |
| 10-2130 | 8-150 | Dimethomorph |
| 10-2131 | 8-151 | Dimethomorph |
| 10-2132 | 8-152 | Dimethomorph |
| 10-2133 | 8-153 | Dimethomorph |
| 10-2134 | 8-154 | Dimethomorph |
| 10-2135 | 8-155 | Dimethomorph |
| 10-2136 | 8-156 | Dimethomorph |
| 10-2137 | 8-157 | Dimethomorph |
| 10-2138 | 8-158 | Dimethomorph |
| 10-2139 | 8-159 | Dimethomorph |
| 10-2140 | 8-160 | Dimethomorph |
| 10-2141 | 8-161 | Dimethomorph |
| 10-2142 | 8-162 | Dimethomorph |
| 10-2143 | 8-163 | Dimethomorph |
| 10-2144 | 8-164 | Dimethomorph |
| 10-2145 | 8-165 | Dimethomorph |
| 10-2146 | 8-166 | Dimethomorph |
| 10-2147 | 8-167 | Dimethomorph |
| 10-2148 | 8-168 | Dimethomorph |
| 10-2149 | 8-169 | Dimethomorph |
| 10-2150 | 8-170 | Dimethomorph |
| 10-2151 | 8-171 | Dimethomorph |
| 10-2152 | 8-172 | Dimethomorph |
| 10-2153 | 8-173 | Dimethomorph |
| 10-2154 | 8-174 | Dimethomorph |
| 10-2155 | 8-175 | Dimethomorph |
| 10-2156 | 8-176 | Dimethomorph |
| 10-2157 | 8-177 | Dimethomorph |
| 10-2158 | 8-178 | Dimethomorph |
| 10-2159 | 8-179 | Dimethomorph |
| 10-2160 | 8-180 | Dimethomorph |
| 10-2161 | 8-1 | Pyrimethanil |
| 10-2162 | 8-2 | Pyrimethanil |
| 10-2163 | 8-3 | Pyrimethanil |
| 10-2164 | 8-4 | Pyrimethanil |
| 10-2165 | 8-5 | Pyrimethanil |
| 10-2166 | 8-6 | Pyrimethanil |
| 10-2167 | 8-7 | Pyrimethanil |
| 10-2168 | 8-8 | Pyrimethanil |
| 10-2169 | 8-9 | Pyrimethanil |
| 10-2170 | 8-10 | Pyrimethanil |
| 10-2171 | 8-11 | Pyrimethanil |
| 10-2172 | 8-12 | Pyrimethanil |
| 10-2173 | 8-13 | Pyrimethanil |
| 10-2174 | 8-14 | Pyrimethanil |
| 10-2175 | 8-15 | Pyrimethanil |
| 10-2176 | 8-16 | Pyrimethanil |
| 10-2177 | 8-17 | Pyrimethanil |
| 10-2178 | 8-18 | Pyrimethanil |
| 10-2179 | 8-19 | Pyrimethanil |
| 10-2180 | 8-20 | Pyrimethanil |
| 10-2181 | 8-21 | Pyrimethanil |
| 10-2182 | 8-22 | Pyrimethanil |
| 10-2183 | 8-23 | Pyrimethanil |
| 10-2184 | 8-24 | Pyrimethanil |
| 10-2185 | 8-25 | Pyrimethanil |
| 10-2186 | 8-26 | Pyrimethanil |
| 10-2187 | 8-27 | Pyrimethanil |
| 10-2188 | 8-28 | Pyrimethanil |
| 10-2189 | 8-29 | Pyrimethanil |

| Nr. | Binäre Kombination | Wirkstoff |
|---|---|---|
| 10-2190 | 8-30 | Pyrimethanil |
| 10-2191 | 8-31 | Pyrimethanil |
| 10-2192 | 8-32 | Pyrimethanil |
| 10-2193 | 8-33 | Pyrimethanil |
| 10-2194 | 8-34 | Pyrimethanil |
| 10-2195 | 8-35 | Pyrimethanil |
| 10-2196 | 8-36 | Pyrimethanil |
| 10-2197 | 8-37 | Pyrimethanil |
| 10-2198 | 8-38 | Pyrimethanil |
| 10-2199 | 8-39 | Pyrimethanil |
| 10-2200 | 8-40 | Pyrimethanil |
| 10-2201 | 8-41 | Pyrimethanil |
| 10-2202 | 8-42 | Pyrimethanil |
| 10-2203 | 8-43 | Pyrimethanil |
| 10-2204 | 8-44 | Pyrimethanil |
| 10-2205 | 8-45 | Pyrimethanil |
| 10-2206 | 8-46 | Pyrimethanil |
| 10-2207 | 8-47 | Pyrimethanil |
| 10-2208 | 8-48 | Pyrimethanil |
| 10-2209 | 8-49 | Pyrimethanil |
| 10-2210 | 8-50 | Pyrimethanil |
| 10-2211 | 8-51 | Pyrimethanil |
| 10-2212 | 8-52 | Pyrimethanil |
| 10-2213 | 8-53 | Pyrimethanil |
| 10-2214 | 8-54 | Pyrimethanil |
| 10-2215 | 8-55 | Pyrimethanil |
| 10-2216 | 8-56 | Pyrimethanil |
| 10-2217 | 8-57 | Pyrimethanil |
| 10-2218 | 8-58 | Pyrimethanil |
| 10-2219 | 8-59 | Pyrimethanil |
| 10-2220 | 8-60 | Pyrimethanil |
| 10-2221 | 8-61 | Pyrimethanil |
| 10-2222 | 8-62 | Pyrimethanil |
| 10-2223 | 8-63 | Pyrimethanil |
| 10-2224 | 8-64 | Pyrimethanil |
| 10-2225 | 8-65 | Pyrimethanil |
| 10-2226 | 8-66 | Pyrimethanil |
| 10-2227 | 8-67 | Pyrimethanil |
| 10-2228 | 8-68 | Pyrimethanil |
| 10-2229 | 8-69 | Pyrimethanil |

| Nr. | Binäre Kombination | Wirkstoff |
|---|---|---|
| 10-2230 | 8-70 | Pyrimethanil |
| 10-2231 | 8-71 | Pyrimethanil |
| 10-2232 | 8-72 | Pyrimethanil |
| 10-2233 | 8-73 | Pyrimethanil |
| 10-2234 | 8-74 | Pyrimethanil |
| 10-2235 | 8-75 | Pyrimethanil |
| 10-2236 | 8-76 | Pyrimethanil |
| 10-2237 | 8-77 | Pyrimethanil |
| 10-2238 | 8-78 | Pyrimethanil |
| 10-2239 | 8-79 | Pyrimethanil |
| 10-2240 | 8-80 | Pyrimethanil |
| 10-2241 | 8-81 | Pyrimethanil |
| 10-2242 | 8-82 | Pyrimethanil |
| 10-2243 | 8-83 | Pyrimethanil |
| 10-2244 | 8-84 | Pyrimethanil |
| 10-2245 | 8-85 | Pyrimethanil |
| 10-2246 | 8-86 | Pyrimethanil |
| 10-2247 | 8-87 | Pyrimethanil |
| 10-2248 | 8-88 | Pyrimethanil |
| 10-2249 | 8-89 | Pyrimethanil |
| 10-2250 | 8-90 | Pyrimethanil |
| 10-2251 | 8-91 | Pyrimethanil |
| 10-2252 | 8-92 | Pyrimethanil |
| 10-2253 | 8-93 | Pyrimethanil |
| 10-2254 | 8-94 | Pyrimethanil |
| 10-2255 | 8-95 | Pyrimethanil |
| 10-2256 | 8-96 | Pyrimethanil |
| 10-2257 | 8-97 | Pyrimethanil |
| 10-2258 | 8-98 | Pyrimethanil |
| 10-2259 | 8-99 | Pyrimethanil |
| 10-2260 | 8-100 | Pyrimethanil |
| 10-2261 | 8-101 | Pyrimethanil |
| 10-2262 | 8-102 | Pyrimethanil |
| 10-2263 | 8-103 | Pyrimethanil |
| 10-2264 | 8-104 | Pyrimethanil |
| 10-2265 | 8-105 | Pyrimethanil |
| 10-2266 | 8-106 | Pyrimethanil |
| 10-2267 | 8-107 | Pyrimethanil |
| 10-2268 | 8-108 | Pyrimethanil |
| 10-2269 | 8-109 | Pyrimethanil |

| Nr. | Binäre Kombination | Wirkstoff |
|---|---|---|
| 10-2270 | 8-110 | Pyrimethanil |
| 10-2271 | 8-111 | Pyrimethanil |
| 10-2272 | 8-112 | Pyrimethanil |
| 10-2273 | 8-113 | Pyrimethanil |
| 10-2274 | 8-114 | Pyrimethanil |
| 10-2275 | 8-115 | Pyrimethanil |
| 10-2276 | 8-116 | Pyrimethanil |
| 10-2277 | 8-117 | Pyrimethanil |
| 10-2278 | 8-118 | Pyrimethanil |
| 10-2279 | 8-119 | Pyrimethanil |
| 10-2280 | 8-120 | Pyrimethanil |
| 10-2281 | 8-121 | Pyrimethanil |
| 10-2282 | 8-122 | Pyrimethanil |
| 10-2283 | 8-123 | Pyrimethanil |
| 10-2284 | 8-124 | Pyrimethanil |
| 10-2285 | 8-125 | Pyrimethanil |
| 10-2286 | 8-126 | Pyrimethanil |
| 10-2287 | 8-127 | Pyrimethanil |
| 10-2288 | 8-128 | Pyrimethanil |
| 10-2289 | 8-129 | Pyrimethanil |
| 10-2290 | 8-130 | Pyrimethanil |
| 10-2291 | 8-131 | Pyrimethanil |
| 10-2292 | 8-132 | Pyrimethanil |
| 10-2293 | 8-133 | Pyrimethanil |
| 10-2294 | 8-134 | Pyrimethanil |
| 10-2295 | 8-135 | Pyrimethanil |
| 10-2296 | 8-136 | Pyrimethanil |
| 10-2297 | 8-137 | Pyrimethanil |
| 10-2298 | 8-138 | Pyrimethanil |
| 10-2299 | 8-139 | Pyrimethanil |
| 10-2300 | 8-140 | Pyrimethanil |
| 10-2301 | 8-141 | Pyrimethanil |
| 10-2302 | 8-142 | Pyrimethanil |
| 10-2303 | 8-143 | Pyrimethanil |
| 10-2304 | 8-144 | Pyrimethanil |
| 10-2305 | 8-145 | Pyrimethanil |
| 10-2306 | 8-146 | Pyrimethanil |
| 10-2307 | 8-147 | Pyrimethanil |
| 10-2308 | 8-148 | Pyrimethanil |
| 10-2309 | 8-149 | Pyrimethanil |

| Nr. | Binäre Kombination | Wirkstoff |
|---|---|---|
| 10-2310 | 8-150 | Pyrimethanil |
| 10-2311 | 8-151 | Pyrimethanil |
| 10-2312 | 8-152 | Pyrimethanil |
| 10-2313 | 8-153 | Pyrimethanil |
| 10-2314 | 8-154 | Pyrimethanil |
| 10-2315 | 8-155 | Pyrimethanil |
| 10-2316 | 8-156 | Pyrimethanil |
| 10-2317 | 8-157 | Pyrimethanil |
| 10-2318 | 8-158 | Pyrimethanil |
| 10-2319 | 8-159 | Pyrimethanil |
| 10-2320 | 8-160 | Pyrimethanil |
| 10-2321 | 8-161 | Pyrimethanil |
| 10-2322 | 8-162 | Pyrimethanil |
| 10-2323 | 8-163 | Pyrimethanil |
| 10-2324 | 8-164 | Pyrimethanil |
| 10-2325 | 8-165 | Pyrimethanil |
| 10-2326 | 8-166 | Pyrimethanil |
| 10-2327 | 8-167 | Pyrimethanil |
| 10-2328 | 8-168 | Pyrimethanil |
| 10-2329 | 8-169 | Pyrimethanil |
| 10-2330 | 8-170 | Pyrimethanil |
| 10-2331 | 8-171 | Pyrimethanil |
| 10-2332 | 8-172 | Pyrimethanil |
| 10-2333 | 8-173 | Pyrimethanil |
| 10-2334 | 8-174 | Pyrimethanil |
| 10-2335 | 8-175 | Pyrimethanil |
| 10-2336 | 8-176 | Pyrimethanil |
| 10-2337 | 8-177 | Pyrimethanil |
| 10-2338 | 8-178 | Pyrimethanil |
| 10-2339 | 8-179 | Pyrimethanil |
| 10-2340 | 8-180 | Pyrimethanil |
| 10-2341 | 8-1 | AC1 |
| 10-2342 | 8-2 | AC1 |
| 10-2343 | 8-3 | AC1 |
| 10-2344 | 8-4 | AC1 |
| 10-2345 | 8-5 | AC1 |
| 10-2346 | 8-6 | AC1 |
| 10-2347 | 8-7 | AC1 |
| 10-2348 | 8-8 | AC1 |
| 10-2349 | 8-9 | AC1 |

| Nr. | Binäre Kombination | Wirkstoff |
|---|---|---|
| 10-2350 | 8-10 | AC1 |
| 10-2351 | 8-11 | AC1 |
| 10-2352 | 8-12 | AC1 |
| 10-2353 | 8-13 | AC1 |
| 10-2354 | 8-14 | AC1 |
| 10-2355 | 8-15 | AC1 |
| 10-2356 | 8-16 | AC1 |
| 10-2357 | 8-17 | AC1 |
| 10-2358 | 8-18 | AC1 |
| 10-2359 | 8-19 | AC1 |
| 10-2360 | 8-20 | AC1 |
| 10-2361 | 8-21 | AC1 |
| 10-2362 | 8-22 | AC1 |
| 10-2363 | 8-23 | AC1 |
| 10-2364 | 8-24 | AC1 |
| 10-2365 | 8-25 | AC1 |
| 10-2366 | 8-26 | AC1 |
| 10-2367 | 8-27 | AC1 |
| 10-2368 | 8-28 | AC1 |
| 10-2369 | 8-29 | AC1 |
| 10-2370 | 8-30 | AC1 |
| 10-2371 | 8-31 | AC1 |
| 10-2372 | 8-32 | AC1 |
| 10-2373 | 8-33 | AC1 |
| 10-2374 | 8-34 | AC1 |
| 10-2375 | 8-35 | AC1 |
| 10-2376 | 8-36 | AC1 |
| 10-2377 | 8-37 | AC1 |
| 10-2378 | 8-38 | AC1 |
| 10-2379 | 8-39 | AC1 |
| 10-2380 | 8-40 | AC1 |
| 10-2381 | 8-41 | AC1 |
| 10-2382 | 8-42 | AC1 |
| 10-2383 | 8-43 | AC1 |
| 10-2384 | 8-44 | AC1 |
| 10-2385 | 8-45 | AC1 |
| 10-2386 | 8-46 | AC1 |
| 10-2387 | 8-47 | AC1 |
| 10-2388 | 8-48 | AC1 |
| 10-2389 | 8-49 | AC1 |

| Nr. | Binäre Kombination | Wirkstoff |
|---|---|---|
| 10-2390 | 8-50 | AC1 |
| 10-2391 | 8-51 | AC1 |
| 10-2392 | 8-52 | AC1 |
| 10-2393 | 8-53 | AC1 |
| 10-2394 | 8-54 | AC1 |
| 10-2395 | 8-55 | AC1 |
| 10-2396 | 8-56 | AC1 |
| 10-2397 | 8-57 | AC1 |
| 10-2398 | 8-58 | AC1 |
| 10-2399 | 8-59 | AC1 |
| 10-2400 | 8-60 | AC1 |
| 10-2401 | 8-61 | AC1 |
| 10-2402 | 8-62 | AC1 |
| 10-2403 | 8-63 | AC1 |
| 10-2404 | 8-64 | AC1 |
| 10-2405 | 8-65 | AC1 |
| 10-2406 | 8-66 | AC1 |
| 10-2407 | 8-67 | AC1 |
| 10-2408 | 8-68 | AC1 |
| 10-2409 | 8-69 | AC1 |
| 10-2410 | 8-70 | AC1 |
| 10-2411 | 8-71 | AC1 |
| 10-2412 | 8-72 | AC1 |
| 10-2413 | 8-73 | AC1 |
| 10-2414 | 8-74 | AC1 |
| 10-2415 | 8-75 | AC1 |
| 10-2416 | 8-76 | AC1 |
| 10-2417 | 8-77 | AC1 |
| 10-2418 | 8-78 | AC1 |
| 10-2419 | 8-79 | AC1 |
| 10-2420 | 8-80 | AC1 |
| 10-2421 | 8-81 | AC1 |
| 10-2422 | 8-82 | AC1 |
| 10-2423 | 8-83 | AC1 |
| 10-2424 | 8-84 | AC1 |
| 10-2425 | 8-85 | AC1 |
| 10-2426 | 8-86 | AC1 |
| 10-2427 | 8-87 | AC1 |
| 10-2428 | 8-88 | AC1 |
| 10-2429 | 8-89 | AC1 |

| Nr. | Binäre Kombination | Wirkstoff |
|---|---|---|
| 10-2430 | 8-90 | AC1 |
| 10-2431 | 8-91 | AC1 |
| 10-2432 | 8-92 | AC1 |
| 10-2433 | 8-93 | AC1 |
| 10-2434 | 8-94 | AC1 |
| 10-2435 | 8-95 | AC1 |
| 10-2436 | 8-96 | AC1 |
| 10-2437 | 8-97 | AC1 |
| 10-2438 | 8-98 | AC1 |
| 10-2439 | 8-99 | AC1 |
| 10-2440 | 8-100 | AC1 |
| 10-2441 | 8-101 | AC1 |
| 10-2442 | 8-102 | AC1 |
| 10-2443 | 8-103 | AC1 |
| 10-2444 | 8-104 | AC1 |
| 10-2445 | 8-105 | AC1 |
| 10-2446 | 8-106 | AC1 |
| 10-2447 | 8-107 | AC1 |
| 10-2448 | 8-108 | AC1 |
| 10-2449 | 8-109 | AC1 |
| 10-2450 | 8-110 | AC1 |
| 10-2451 | 8-111 | AC1 |
| 10-2452 | 8-112 | AC1 |
| 10-2453 | 8-113 | AC1 |
| 10-2454 | 8-114 | AC1 |
| 10-2455 | 8-115 | AC1 |
| 10-2456 | 8-116 | AC1 |
| 10-2457 | 8-117 | AC1 |
| 10-2458 | 8-118 | AC1 |
| 10-2459 | 8-119 | AC1 |
| 10-2460 | 8-120 | AC1 |
| 10-2461 | 8-121 | AC1 |
| 10-2462 | 8-122 | AC1 |
| 10-2463 | 8-123 | AC1 |
| 10-2464 | 8-124 | AC1 |
| 10-2465 | 8-125 | AC1 |
| 10-2466 | 8-126 | AC1 |
| 10-2467 | 8-127 | AC1 |
| 10-2468 | 8-128 | AC1 |
| 10-2469 | 8-129 | AC1 |

| Nr. | Binäre Kombination | Wirkstoff |
|---|---|---|
| 10-2470 | 8-130 | AC1 |
| 10-2471 | 8-131 | AC1 |
| 10-2472 | 8-132 | AC1 |
| 10-2473 | 8-133 | AC1 |
| 10-2474 | 8-134 | AC1 |
| 10-2475 | 8-135 | AC1 |
| 10-2476 | 8-136 | AC1 |
| 10-2477 | 8-137 | AC1 |
| 10-2478 | 8-138 | AC1 |
| 10-2479 | 8-139 | AC1 |
| 10-2480 | 8-140 | AC1 |
| 10-2481 | 8-141 | AC1 |
| 10-2482 | 8-142 | AC1 |
| 10-2483 | 8-143 | AC1 |
| 10-2484 | 8-144 | AC1 |
| 10-2485 | 8-145 | AC1 |
| 10-2486 | 8-146 | AC1 |
| 10-2487 | 8-147 | AC1 |
| 10-2488 | 8-148 | AC1 |
| 10-2489 | 8-149 | AC1 |
| 10-2490 | 8-150 | AC1 |
| 10-2491 | 8-151 | AC1 |
| 10-2492 | 8-152 | AC1 |
| 10-2493 | 8-153 | AC1 |
| 10-2494 | 8-154 | AC1 |
| 10-2495 | 8-155 | AC1 |
| 10-2496 | 8-156 | AC1 |
| 10-2497 | 8-157 | AC1 |
| 10-2498 | 8-158 | AC1 |
| 10-2499 | 8-159 | AC1 |
| 10-2500 | 8-160 | AC1 |
| 10-2501 | 8-161 | AC1 |
| 10-2502 | 8-162 | AC1 |
| 10-2503 | 8-163 | AC1 |
| 10-2504 | 8-164 | AC1 |
| 10-2505 | 8-165 | AC1 |
| 10-2506 | 8-166 | AC1 |
| 10-2507 | 8-167 | AC1 |
| 10-2508 | 8-168 | AC1 |
| 10-2509 | 8-169 | AC1 |

| Nr. | Binäre Kombination | Wirkstoff |
|---|---|---|
| 10-2510 | 8-170 | AC1 |
| 10-2511 | 8-171 | AC1 |
| 10-2512 | 8-172 | AC1 |
| 10-2513 | 8-173 | AC1 |
| 10-2514 | 8-174 | AC1 |
| 10-2515 | 8-175 | AC1 |
| 10-2516 | 8-176 | AC1 |
| 10-2517 | 8-177 | AC1 |
| 10-2518 | 8-178 | AC1 |
| 10-2519 | 8-179 | AC1 |
| 10-2520 | 8-180 | AC1 |
| 10-2521 | 8-1 | Dodemorph |
| 10-2522 | 8-2 | Dodemorph |
| 10-2523 | 8-3 | Dodemorph |
| 10-2524 | 8-4 | Dodemorph |
| 10-2525 | 8-5 | Dodemorph |
| 10-2526 | 8-6 | Dodemorph |
| 10-2527 | 8-7 | Dodemorph |
| 10-2528 | 8-8 | Dodemorph |
| 10-2529 | 8-9 | Dodemorph |
| 10-2530 | 8-10 | Dodemorph |
| 10-2531 | 8-11 | Dodemorph |
| 10-2532 | 8-12 | Dodemorph |
| 10-2533 | 8-13 | Dodemorph |
| 10-2534 | 8-14 | Dodemorph |
| 10-2535 | 8-15 | Dodemorph |
| 10-2536 | 8-16 | Dodemorph |
| 10-2537 | 8-17 | Dodemorph |
| 10-2538 | 8-18 | Dodemorph |
| 10-2539 | 8-19 | Dodemorph |
| 10-2540 | 8-20 | Dodemorph |
| 10-2541 | 8-21 | Dodemorph |
| 10-2542 | 8-22 | Dodemorph |
| 10-2543 | 8-23 | Dodemorph |
| 10-2544 | 8-24 | Dodemorph |
| 10-2545 | 8-25 | Dodemorph |
| 10-2546 | 8-26 | Dodemorph |
| 10-2547 | 8-27 | Dodemorph |
| 10-2548 | 8-28 | Dodemorph |
| 10-2549 | 8-29 | Dodemorph |

| Nr. | Binäre Kombination | Wirkstoff |
|---|---|---|
| 10-2550 | 8-30 | Dodemorph |
| 10-2551 | 8-31 | Dodemorph |
| 10-2552 | 8-32 | Dodemorph |
| 10-2553 | 8-33 | Dodemorph |
| 10-2554 | 8-34 | Dodemorph |
| 10-2555 | 8-35 | Dodemorph |
| 10-2556 | 8-36 | Dodemorph |
| 10-2557 | 8-37 | Dodemorph |
| 10-2558 | 8-38 | Dodemorph |
| 10-2559 | 8-39 | Dodemorph |
| 10-2560 | 8-40 | Dodemorph |
| 10-2561 | 8-41 | Dodemorph |
| 10-2562 | 8-42 | Dodemorph |
| 10-2563 | 8-43 | Dodemorph |
| 10-2564 | 8-44 | Dodemorph |
| 10-2565 | 8-45 | Dodemorph |
| 10-2566 | 8-46 | Dodemorph |
| 10-2567 | 8-47 | Dodemorph |
| 10-2568 | 8-48 | Dodemorph |
| 10-2569 | 8-49 | Dodemorph |
| 10-2570 | 8-50 | Dodemorph |
| 10-2571 | 8-51 | Dodemorph |
| 10-2572 | 8-52 | Dodemorph |
| 10-2573 | 8-53 | Dodemorph |
| 10-2574 | 8-54 | Dodemorph |
| 10-2575 | 8-55 | Dodemorph |
| 10-2576 | 8-56 | Dodemorph |
| 10-2577 | 8-57 | Dodemorph |
| 10-2578 | 8-58 | Dodemorph |
| 10-2579 | 8-59 | Dodemorph |
| 10-2580 | 8-60 | Dodemorph |
| 10-2581 | 8-61 | Dodemorph |
| 10-2582 | 8-62 | Dodemorph |
| 10-2583 | 8-63 | Dodemorph |
| 10-2584 | 8-64 | Dodemorph |
| 10-2585 | 8-65 | Dodemorph |
| 10-2586 | 8-66 | Dodemorph |
| 10-2587 | 8-67 | Dodemorph |
| 10-2588 | 8-68 | Dodemorph |
| 10-2589 | 8-69 | Dodemorph |

| Nr. | Binäre Kombination | Wirkstoff |
|---|---|---|
| 10-2590 | 8-70 | Dodemorph |
| 10-2591 | 8-71 | Dodemorph |
| 10-2592 | 8-72 | Dodemorph |
| 10-2593 | 8-73 | Dodemorph |
| 10-2594 | 8-74 | Dodemorph |
| 10-2595 | 8-75 | Dodemorph |
| 10-2596 | 8-76 | Dodemorph |
| 10-2597 | 8-77 | Dodemorph |
| 10-2598 | 8-78 | Dodemorph |
| 10-2599 | 8-79 | Dodemorph |
| 10-2600 | 8-80 | Dodemorph |
| 10-2601 | 8-81 | Dodemorph |
| 10-2602 | 8-82 | Dodemorph |
| 10-2603 | 8-83 | Dodemorph |
| 10-2604 | 8-84 | Dodemorph |
| 10-2605 | 8-85 | Dodemorph |
| 10-2606 | 8-86 | Dodemorph |
| 10-2607 | 8-87 | Dodemorph |
| 10-2608 | 8-88 | Dodemorph |
| 10-2609 | 8-89 | Dodemorph |
| 10-2610 | 8-90 | Dodemorph |
| 10-2611 | 8-91 | Dodemorph |
| 10-2612 | 8-92 | Dodemorph |
| 10-2613 | 8-93 | Dodemorph |
| 10-2614 | 8-94 | Dodemorph |
| 10-2615 | 8-95 | Dodemorph |
| 10-2616 | 8-96 | Dodemorph |
| 10-2617 | 8-97 | Dodemorph |
| 10-2618 | 8-98 | Dodemorph |
| 10-2619 | 8-99 | Dodemorph |
| 10-2620 | 8-100 | Dodemorph |
| 10-2621 | 8-101 | Dodemorph |
| 10-2622 | 8-102 | Dodemorph |
| 10-2623 | 8-103 | Dodemorph |
| 10-2624 | 8-104 | Dodemorph |
| 10-2625 | 8-105 | Dodemorph |
| 10-2626 | 8-106 | Dodemorph |
| 10-2627 | 8-107 | Dodemorph |
| 10-2628 | 8-108 | Dodemorph |
| 10-2629 | 8-109 | Dodemorph |

| Nr. | Binäre Kombination | Wirkstoff |
|---|---|---|
| 10-2630 | 8-110 | Dodemorph |
| 10-2631 | 8-111 | Dodemorph |
| 10-2632 | 8-112 | Dodemorph |
| 10-2633 | 8-113 | Dodemorph |
| 10-2634 | 8-114 | Dodemorph |
| 10-2635 | 8-115 | Dodemorph |
| 10-2636 | 8-116 | Dodemorph |
| 10-2637 | 8-117 | Dodemorph |
| 10-2638 | 8-118 | Dodemorph |
| 10-2639 | 8-119 | Dodemorph |
| 10-2640 | 8-120 | Dodemorph |
| 10-2641 | 8-121 | Dodemorph |
| 10-2642 | 8-122 | Dodemorph |
| 10-2643 | 8-123 | Dodemorph |
| 10-2644 | 8-124 | Dodemorph |
| 10-2645 | 8-125 | Dodemorph |
| 10-2646 | 8-126 | Dodemorph |
| 10-2647 | 8-127 | Dodemorph |
| 10-2648 | 8-128 | Dodemorph |
| 10-2649 | 8-129 | Dodemorph |
| 10-2650 | 8-130 | Dodemorph |
| 10-2651 | 8-131 | Dodemorph |
| 10-2652 | 8-132 | Dodemorph |
| 10-2653 | 8-133 | Dodemorph |
| 10-2654 | 8-134 | Dodemorph |
| 10-2655 | 8-135 | Dodemorph |
| 10-2656 | 8-136 | Dodemorph |
| 10-2657 | 8-137 | Dodemorph |
| 10-2658 | 8-138 | Dodemorph |
| 10-2659 | 8-139 | Dodemorph |
| 10-2660 | 8-140 | Dodemorph |
| 10-2661 | 8-141 | Dodemorph |
| 10-2662 | 8-142 | Dodemorph |
| 10-2663 | 8-143 | Dodemorph |
| 10-2664 | 8-144 | Dodemorph |
| 10-2665 | 8-145 | Dodemorph |
| 10-2666 | 8-146 | Dodemorph |
| 10-2667 | 8-147 | Dodemorph |
| 10-2668 | 8-148 | Dodemorph |
| 10-2669 | 8-149 | Dodemorph |

| Nr. | Binäre Kombination | Wirkstoff |
|---|---|---|
| 10-2670 | 8-150 | Dodemorph |
| 10-2671 | 8-151 | Dodemorph |
| 10-2672 | 8-152 | Dodemorph |
| 10-2673 | 8-153 | Dodemorph |
| 10-2674 | 8-154 | Dodemorph |
| 10-2675 | 8-155 | Dodemorph |
| 10-2676 | 8-156 | Dodemorph |
| 10-2677 | 8-157 | Dodemorph |
| 10-2678 | 8-158 | Dodemorph |
| 10-2679 | 8-159 | Dodemorph |
| 10-2680 | 8-160 | Dodemorph |
| 10-2681 | 8-161 | Dodemorph |
| 10-2682 | 8-162 | Dodemorph |
| 10-2683 | 8-163 | Dodemorph |
| 10-2684 | 8-164 | Dodemorph |
| 10-2685 | 8-165 | Dodemorph |
| 10-2686 | 8-166 | Dodemorph |
| 10-2687 | 8-167 | Dodemorph |
| 10-2688 | 8-168 | Dodemorph |
| 10-2689 | 8-169 | Dodemorph |
| 10-2690 | 8-170 | Dodemorph |
| 10-2691 | 8-171 | Dodemorph |
| 10-2692 | 8-172 | Dodemorph |
| 10-2693 | 8-173 | Dodemorph |
| 10-2694 | 8-174 | Dodemorph |
| 10-2695 | 8-175 | Dodemorph |
| 10-2696 | 8-176 | Dodemorph |
| 10-2697 | 8-177 | Dodemorph |
| 10-2698 | 8-178 | Dodemorph |
| 10-2699 | 8-179 | Dodemorph |
| 10-2700 | 8-180 | Dodemorph |
| 10-2701 | 8-1 | Fenpropimorph |
| 10-2702 | 8-2 | Fenpropimorph |
| 10-2703 | 8-3 | Fenpropimorph |
| 10-2704 | 8-4 | Fenpropimorph |
| 10-2705 | 8-5 | Fenpropimorph |
| 10-2706 | 8-6 | Fenpropimorph |
| 10-2707 | 8-7 | Fenpropimorph |
| 10-2708 | 8-8 | Fenpropimorph |
| 10-2709 | 8-9 | Fenpropimorph |

| Nr. | Binäre Kombination | Wirkstoff |
|---|---|---|
| 10-2710 | 8-10 | Fenpropimorph |
| 10-2711 | 8-11 | Fenpropimorph |
| 10-2712 | 8-12 | Fenpropimorph |
| 10-2713 | 8-13 | Fenpropimorph |
| 10-2714 | 8-14 | Fenpropimorph |
| 10-2715 | 8-15 | Fenpropimorph |
| 10-2716 | 8-16 | Fenpropimorph |
| 10-2717 | 8-17 | Fenpropimorph |
| 10-2718 | 8-18 | Fenpropimorph |
| 10-2719 | 8-19 | Fenpropimorph |
| 10-2720 | 8-20 | Fenpropimorph |
| 10-2721 | 8-21 | Fenpropimorph |
| 10-2722 | 8-22 | Fenpropimorph |
| 10-2723 | 8-23 | Fenpropimorph |
| 10-2724 | 8-24 | Fenpropimorph |
| 10-2725 | 8-25 | Fenpropimorph |
| 10-2726 | 8-26 | Fenpropimorph |
| 10-2727 | 8-27 | Fenpropimorph |
| 10-2728 | 8-28 | Fenpropimorph |
| 10-2729 | 8-29 | Fenpropimorph |
| 10-2730 | 8-30 | Fenpropimorph |
| 10-2731 | 8-31 | Fenpropimorph |
| 10-2732 | 8-32 | Fenpropimorph |
| 10-2733 | 8-33 | Fenpropimorph |
| 10-2734 | 8-34 | Fenpropimorph |
| 10-2735 | 8-35 | Fenpropimorph |
| 10-2736 | 8-36 | Fenpropimorph |
| 10-2737 | 8-37 | Fenpropimorph |
| 10-2738 | 8-38 | Fenpropimorph |
| 10-2739 | 8-39 | Fenpropimorph |
| 10-2740 | 8-40 | Fenpropimorph |
| 10-2741 | 8-41 | Fenpropimorph |
| 10-2742 | 8-42 | Fenpropimorph |
| 10-2743 | 8-43 | Fenpropimorph |
| 10-2744 | 8-44 | Fenpropimorph |
| 10-2745 | 8-45 | Fenpropimorph |
| 10-2746 | 8-46 | Fenpropimorph |
| 10-2747 | 8-47 | Fenpropimorph |
| 10-2748 | 8-48 | Fenpropimorph |
| 10-2749 | 8-49 | Fenpropimorph |

| Nr. | Binäre Kombination | Wirkstoff |
|---|---|---|
| 10-2750 | 8-50 | Fenpropimorph |
| 10-2751 | 8-51 | Fenpropimorph |
| 10-2752 | 8-52 | Fenpropimorph |
| 10-2753 | 8-53 | Fenpropimorph |
| 10-2754 | 8-54 | Fenpropimorph |
| 10-2755 | 8-55 | Fenpropimorph |
| 10-2756 | 8-56 | Fenpropimorph |
| 10-2757 | 8-57 | Fenpropimorph |
| 10-2758 | 8-58 | Fenpropimorph |
| 10-2759 | 8-59 | Fenpropimorph |
| 10-2760 | 8-60 | Fenpropimorph |
| 10-2761 | 8-61 | Fenpropimorph |
| 10-2762 | 8-62 | Fenpropimorph |
| 10-2763 | 8-63 | Fenpropimorph |
| 10-2764 | 8-64 | Fenpropimorph |
| 10-2765 | 8-65 | Fenpropimorph |
| 10-2766 | 8-66 | Fenpropimorph |
| 10-2767 | 8-67 | Fenpropimorph |
| 10-2768 | 8-68 | Fenpropimorph |
| 10-2769 | 8-69 | Fenpropimorph |
| 10-2770 | 8-70 | Fenpropimorph |
| 10-2771 | 8-71 | Fenpropimorph |
| 10-2772 | 8-72 | Fenpropimorph |
| 10-2773 | 8-73 | Fenpropimorph |
| 10-2774 | 8-74 | Fenpropimorph |
| 10-2775 | 8-75 | Fenpropimorph |
| 10-2776 | 8-76 | Fenpropimorph |
| 10-2777 | 8-77 | Fenpropimorph |
| 10-2778 | 8-78 | Fenpropimorph |
| 10-2779 | 8-79 | Fenpropimorph |
| 10-2780 | 8-80 | Fenpropimorph |
| 10-2781 | 8-81 | Fenpropimorph |
| 10-2782 | 8-82 | Fenpropimorph |
| 10-2783 | 8-83 | Fenpropimorph |
| 10-2784 | 8-84 | Fenpropimorph |
| 10-2785 | 8-85 | Fenpropimorph |
| 10-2786 | 8-86 | Fenpropimorph |
| 10-2787 | 8-87 | Fenpropimorph |
| 10-2788 | 8-88 | Fenpropimorph |
| 10-2789 | 8-89 | Fenpropimorph |

| Nr. | Binäre Kombination | Wirkstoff |
|---|---|---|
| 10-2790 | 8-90 | Fenpropimorph |
| 10-2791 | 8-91 | Fenpropimorph |
| 10-2792 | 8-92 | Fenpropimorph |
| 10-2793 | 8-93 | Fenpropimorph |
| 10-2794 | 8-94 | Fenpropimorph |
| 10-2795 | 8-95 | Fenpropimorph |
| 10-2796 | 8-96 | Fenpropimorph |
| 10-2797 | 8-97 | Fenpropimorph |
| 10-2798 | 8-98 | Fenpropimorph |
| 10-2799 | 8-99 | Fenpropimorph |
| 10-2800 | 8-100 | Fenpropimorph |
| 10-2801 | 8-101 | Fenpropimorph |
| 10-2802 | 8-102 | Fenpropimorph |
| 10-2803 | 8-103 | Fenpropimorph |
| 10-2804 | 8-104 | Fenpropimorph |
| 10-2805 | 8-105 | Fenpropimorph |
| 10-2806 | 8-106 | Fenpropimorph |
| 10-2807 | 8-107 | Fenpropimorph |
| 10-2808 | 8-108 | Fenpropimorph |
| 10-2809 | 8-109 | Fenpropimorph |
| 10-2810 | 8-110 | Fenpropimorph |
| 10-2811 | 8-111 | Fenpropimorph |
| 10-2812 | 8-112 | Fenpropimorph |
| 10-2813 | 8-113 | Fenpropimorph |
| 10-2814 | 8-114 | Fenpropimorph |
| 10-2815 | 8-115 | Fenpropimorph |
| 10-2816 | 8-116 | Fenpropimorph |
| 10-2817 | 8-117 | Fenpropimorph |
| 10-2818 | 8-118 | Fenpropimorph |
| 10-2819 | 8-119 | Fenpropimorph |
| 10-2820 | 8-120 | Fenpropimorph |
| 10-2821 | 8-121 | Fenpropimorph |
| 10-2822 | 8-122 | Fenpropimorph |
| 10-2823 | 8-123 | Fenpropimorph |
| 10-2824 | 8-124 | Fenpropimorph |
| 10-2825 | 8-125 | Fenpropimorph |
| 10-2826 | 8-126 | Fenpropimorph |
| 10-2827 | 8-127 | Fenpropimorph |
| 10-2828 | 8-128 | Fenpropimorph |
| 10-2829 | 8-129 | Fenpropimorph |

| Nr. | Binäre Kombination | Wirkstoff |
|---|---|---|
| 10-2830 | 8-130 | Fenpropimorph |
| 10-2831 | 8-131 | Fenpropimorph |
| 10-2832 | 8-132 | Fenpropimorph |
| 10-2833 | 8-133 | Fenpropimorph |
| 10-2834 | 8-134 | Fenpropimorph |
| 10-2835 | 8-135 | Fenpropimorph |
| 10-2836 | 8-136 | Fenpropimorph |
| 10-2837 | 8-137 | Fenpropimorph |
| 10-2838 | 8-138 | Fenpropimorph |
| 10-2839 | 8-139 | Fenpropimorph |
| 10-2840 | 8-140 | Fenpropimorph |
| 10-2841 | 8-141 | Fenpropimorph |
| 10-2842 | 8-142 | Fenpropimorph |
| 10-2843 | 8-143 | Fenpropimorph |
| 10-2844 | 8-144 | Fenpropimorph |
| 10-2845 | 8-145 | Fenpropimorph |
| 10-2846 | 8-146 | Fenpropimorph |
| 10-2847 | 8-147 | Fenpropimorph |
| 10-2848 | 8-148 | Fenpropimorph |
| 10-2849 | 8-149 | Fenpropimorph |
| 10-2850 | 8-150 | Fenpropimorph |
| 10-2851 | 8-151 | Fenpropimorph |
| 10-2852 | 8-152 | Fenpropimorph |
| 10-2853 | 8-153 | Fenpropimorph |
| 10-2854 | 8-154 | Fenpropimorph |
| 10-2855 | 8-155 | Fenpropimorph |
| 10-2856 | 8-156 | Fenpropimorph |
| 10-2857 | 8-157 | Fenpropimorph |
| 10-2858 | 8-158 | Fenpropimorph |
| 10-2859 | 8-159 | Fenpropimorph |
| 10-2860 | 8-160 | Fenpropimorph |
| 10-2861 | 8-161 | Fenpropimorph |
| 10-2862 | 8-162 | Fenpropimorph |
| 10-2863 | 8-163 | Fenpropimorph |
| 10-2864 | 8-164 | Fenpropimorph |
| 10-2865 | 8-165 | Fenpropimorph |
| 10-2866 | 8-166 | Fenpropimorph |
| 10-2867 | 8-167 | Fenpropimorph |
| 10-2868 | 8-168 | Fenpropimorph |
| 10-2869 | 8-169 | Fenpropimorph |

| Nr. | Binäre Kombination | Wirkstoff |
|---|---|---|
| 10-2870 | 8-170 | Fenpropimorph |
| 10-2871 | 8-171 | Fenpropimorph |
| 10-2872 | 8-172 | Fenpropimorph |
| 10-2873 | 8-173 | Fenpropimorph |
| 10-2874 | 8-174 | Fenpropimorph |
| 10-2875 | 8-175 | Fenpropimorph |
| 10-2876 | 8-176 | Fenpropimorph |
| 10-2877 | 8-177 | Fenpropimorph |
| 10-2878 | 8-178 | Fenpropimorph |
| 10-2879 | 8-179 | Fenpropimorph |
| 10-2880 | 8-180 | Fenpropimorph |
| 10-2881 | 8-1 | Tridemorph |
| 10-2882 | 8-2 | Tridemorph |
| 10-2883 | 8-3 | Tridemorph |
| 10-2884 | 8-4 | Tridemorph |
| 10-2885 | 8-5 | Tridemorph |
| 10-2886 | 8-6 | Tridemorph |
| 10-2887 | 8-7 | Tridemorph |
| 10-2888 | 8-8 | Tridemorph |
| 10-2889 | 8-9 | Tridemorph |
| 10-2890 | 8-10 | Tridemorph |
| 10-2891 | 8-11 | Tridemorph |
| 10-2892 | 8-12 | Tridemorph |
| 10-2893 | 8-13 | Tridemorph |
| 10-2894 | 8-14 | Tridemorph |
| 10-2895 | 8-15 | Tridemorph |
| 10-2896 | 8-16 | Tridemorph |
| 10-2897 | 8-17 | Tridemorph |
| 10-2898 | 8-18 | Tridemorph |
| 10-2899 | 8-19 | Tridemorph |
| 10-2900 | 8-20 | Tridemorph |
| 10-2901 | 8-21 | Tridemorph |
| 10-2902 | 8-22 | Tridemorph |
| 10-2903 | 8-23 | Tridemorph |
| 10-2904 | 8-24 | Tridemorph |
| 10-2905 | 8-25 | Tridemorph |
| 10-2906 | 8-26 | Tridemorph |
| 10-2907 | 8-27 | Tridemorph |
| 10-2908 | 8-28 | Tridemorph |
| 10-2909 | 8-29 | Tridemorph |

| Nr. | Binäre Kombination | Wirkstoff |
|---|---|---|
| 10-2910 | 8-30 | Tridemorph |
| 10-2911 | 8-31 | Tridemorph |
| 10-2912 | 8-32 | Tridemorph |
| 10-2913 | 8-33 | Tridemorph |
| 10-2914 | 8-34 | Tridemorph |
| 10-2915 | 8-35 | Tridemorph |
| 10-2916 | 8-36 | Tridemorph |
| 10-2917 | 8-37 | Tridemorph |
| 10-2918 | 8-38 | Tridemorph |
| 10-2919 | 8-39 | Tridemorph |
| 10-2920 | 8-40 | Tridemorph |
| 10-2921 | 8-41 | Tridemorph |
| 10-2922 | 8-42 | Tridemorph |
| 10-2923 | 8-43 | Tridemorph |
| 10-2924 | 8-44 | Tridemorph |
| 10-2925 | 8-45 | Tridemorph |
| 10-2926 | 8-46 | Tridemorph |
| 10-2927 | 8-47 | Tridemorph |
| 10-2928 | 8-48 | Tridemorph |
| 10-2929 | 8-49 | Tridemorph |
| 10-2930 | 8-50 | Tridemorph |
| 10-2931 | 8-51 | Tridemorph |
| 10-2932 | 8-52 | Tridemorph |
| 10-2933 | 8-53 | Tridemorph |
| 10-2934 | 8-54 | Tridemorph |
| 10-2935 | 8-55 | Tridemorph |
| 10-2936 | 8-56 | Tridemorph |
| 10-2937 | 8-57 | Tridemorph |
| 10-2938 | 8-58 | Tridemorph |
| 10-2939 | 8-59 | Tridemorph |
| 10-2940 | 8-60 | Tridemorph |
| 10-2941 | 8-61 | Tridemorph |
| 10-2942 | 8-62 | Tridemorph |
| 10-2943 | 8-63 | Tridemorph |
| 10-2944 | 8-64 | Tridemorph |
| 10-2945 | 8-65 | Tridemorph |
| 10-2946 | 8-66 | Tridemorph |
| 10-2947 | 8-67 | Tridemorph |
| 10-2948 | 8-68 | Tridemorph |
| 10-2949 | 8-69 | Tridemorph |

| Nr. | Binäre Kombination | Wirkstoff |
|---|---|---|
| 10-2950 | 8-70 | Tridemorph |
| 10-2951 | 8-71 | Tridemorph |
| 10-2952 | 8-72 | Tridemorph |
| 10-2953 | 8-73 | Tridemorph |
| 10-2954 | 8-74 | Tridemorph |
| 10-2955 | 8-75 | Tridemorph |
| 10-2956 | 8-76 | Tridemorph |
| 10-2957 | 8-77 | Tridemorph |
| 10-2958 | 8-78 | Tridemorph |
| 10-2959 | 8-79 | Tridemorph |
| 10-2960 | 8-80 | Tridemorph |
| 10-2961 | 8-81 | Tridemorph |
| 10-2962 | 8-82 | Tridemorph |
| 10-2963 | 8-83 | Tridemorph |
| 10-2964 | 8-84 | Tridemorph |
| 10-2965 | 8-85 | Tridemorph |
| 10-2966 | 8-86 | Tridemorph |
| 10-2967 | 8-87 | Tridemorph |
| 10-2968 | 8-88 | Tridemorph |
| 10-2969 | 8-89 | Tridemorph |
| 10-2970 | 8-90 | Tridemorph |
| 10-2971 | 8-91 | Tridemorph |
| 10-2972 | 8-92 | Tridemorph |
| 10-2973 | 8-93 | Tridemorph |
| 10-2974 | 8-94 | Tridemorph |
| 10-2975 | 8-95 | Tridemorph |
| 10-2976 | 8-96 | Tridemorph |
| 10-2977 | 8-97 | Tridemorph |
| 10-2978 | 8-98 | Tridemorph |
| 10-2979 | 8-99 | Tridemorph |
| 10-2980 | 8-100 | Tridemorph |
| 10-2981 | 8-101 | Tridemorph |
| 10-2982 | 8-102 | Tridemorph |
| 10-2983 | 8-103 | Tridemorph |
| 10-2984 | 8-104 | Tridemorph |
| 10-2985 | 8-105 | Tridemorph |
| 10-2986 | 8-106 | Tridemorph |
| 10-2987 | 8-107 | Tridemorph |
| 10-2988 | 8-108 | Tridemorph |
| 10-2989 | 8-109 | Tridemorph |

| Nr. | Binäre Kombination | Wirkstoff |
|---|---|---|
| 10-2990 | 8-110 | Tridemorph |
| 10-2991 | 8-111 | Tridemorph |
| 10-2992 | 8-112 | Tridemorph |
| 10-2993 | 8-113 | Tridemorph |
| 10-2994 | 8-114 | Tridemorph |
| 10-2995 | 8-115 | Tridemorph |
| 10-2996 | 8-116 | Tridemorph |
| 10-2997 | 8-117 | Tridemorph |
| 10-2998 | 8-118 | Tridemorph |
| 10-2999 | 8-119 | Tridemorph |
| 10-3000 | 8-120 | Tridemorph |
| 10-3001 | 8-121 | Tridemorph |
| 10-3002 | 8-122 | Tridemorph |
| 10-3003 | 8-123 | Tridemorph |
| 10-3004 | 8-124 | Tridemorph |
| 10-3005 | 8-125 | Tridemorph |
| 10-3006 | 8-126 | Tridemorph |
| 10-3007 | 8-127 | Tridemorph |
| 10-3008 | 8-128 | Tridemorph |
| 10-3009 | 8-129 | Tridemorph |
| 10-3010 | 8-130 | Tridemorph |
| 10-3011 | 8-131 | Tridemorph |
| 10-3012 | 8-132 | Tridemorph |
| 10-3013 | 8-133 | Tridemorph |
| 10-3014 | 8-134 | Tridemorph |
| 10-3015 | 8-135 | Tridemorph |
| 10-3016 | 8-136 | Tridemorph |
| 10-3017 | 8-137 | Tridemorph |
| 10-3018 | 8-138 | Tridemorph |
| 10-3019 | 8-139 | Tridemorph |
| 10-3020 | 8-140 | Tridemorph |
| 10-3021 | 8-141 | Tridemorph |
| 10-3022 | 8-142 | Tridemorph |
| 10-3023 | 8-143 | Tridemorph |
| 10-3024 | 8-144 | Tridemorph |
| 10-3025 | 8-145 | Tridemorph |
| 10-3026 | 8-146 | Tridemorph |
| 10-3027 | 8-147 | Tridemorph |
| 10-3028 | 8-148 | Tridemorph |
| 10-3029 | 8-149 | Tridemorph |

| Nr. | Binäre Kombination | Wirkstoff |
|---|---|---|
| 10-3030 | 8-150 | Tridemorph |
| 10-3031 | 8-151 | Tridemorph |
| 10-3032 | 8-152 | Tridemorph |
| 10-3033 | 8-153 | Tridemorph |
| 10-3034 | 8-154 | Tridemorph |
| 10-3035 | 8-155 | Tridemorph |
| 10-3036 | 8-156 | Tridemorph |
| 10-3037 | 8-157 | Tridemorph |
| 10-3038 | 8-158 | Tridemorph |
| 10-3039 | 8-159 | Tridemorph |
| 10-3040 | 8-160 | Tridemorph |
| 10-3041 | 8-161 | Tridemorph |
| 10-3042 | 8-162 | Tridemorph |
| 10-3043 | 8-163 | Tridemorph |
| 10-3044 | 8-164 | Tridemorph |
| 10-3045 | 8-165 | Tridemorph |
| 10-3046 | 8-166 | Tridemorph |
| 10-3047 | 8-167 | Tridemorph |
| 10-3048 | 8-168 | Tridemorph |
| 10-3049 | 8-169 | Tridemorph |
| 10-3050 | 8-170 | Tridemorph |
| 10-3051 | 8-171 | Tridemorph |
| 10-3052 | 8-172 | Tridemorph |
| 10-3053 | 8-173 | Tridemorph |
| 10-3054 | 8-174 | Tridemorph |
| 10-3055 | 8-175 | Tridemorph |
| 10-3056 | 8-176 | Tridemorph |
| 10-3057 | 8-177 | Tridemorph |
| 10-3058 | 8-178 | Tridemorph |
| 10-3059 | 8-179 | Tridemorph |
| 10-3060 | 8-180 | Tridemorph |
| 10-3061 | 8-1 | Iprodion |
| 10-3062 | 8-2 | Iprodion |
| 10-3063 | 8-3 | Iprodion |
| 10-3064 | 8-4 | Iprodion |
| 10-3065 | 8-5 | Iprodion |
| 10-3066 | 8-6 | Iprodion |
| 10-3067 | 8-7 | Iprodion |
| 10-3068 | 8-8 | Iprodion |
| 10-3069 | 8-9 | Iprodion |

| Nr. | Binäre Kombination | Wirkstoff |
|---|---|---|
| 10-3070 | 8-10 | Iprodion |
| 10-3071 | 8-11 | Iprodion |
| 10-3072 | 8-12 | Iprodion |
| 10-3073 | 8-13 | Iprodion |
| 10-3074 | 8-14 | Iprodion |
| 10-3075 | 8-15 | Iprodion |
| 10-3076 | 8-16 | Iprodion |
| 10-3077 | 8-17 | Iprodion |
| 10-3078 | 8-18 | Iprodion |
| 10-3079 | 8-19 | Iprodion |
| 10-3080 | 8-20 | Iprodion |
| 10-3081 | 8-21 | Iprodion |
| 10-3082 | 8-22 | Iprodion |
| 10-3083 | 8-23 | Iprodion |
| 10-3084 | 8-24 | Iprodion |
| 10-3085 | 8-25 | Iprodion |
| 10-3086 | 8-26 | Iprodion |
| 10-3087 | 8-27 | Iprodion |
| 10-3088 | 8-28 | Iprodion |
| 10-3089 | 8-29 | Iprodion |
| 10-3090 | 8-30 | Iprodion |
| 10-3091 | 8-31 | Iprodion |
| 10-3092 | 8-32 | Iprodion |
| 10-3093 | 8-33 | Iprodion |
| 10-3094 | 8-34 | Iprodion |
| 10-3095 | 8-35 | Iprodion |
| 10-3096 | 8-36 | Iprodion |
| 10-3097 | 8-37 | Iprodion |
| 10-3098 | 8-38 | Iprodion |
| 10-3099 | 8-39 | Iprodion |
| 10-3100 | 8-40 | Iprodion |
| 10-3101 | 8-41 | Iprodion |
| 10-3102 | 8-42 | Iprodion |
| 10-3103 | 8-43 | Iprodion |
| 10-3104 | 8-44 | Iprodion |
| 10-3105 | 8-45 | Iprodion |
| 10-3106 | 8-46 | Iprodion |
| 10-3107 | 8-47 | Iprodion |
| 10-3108 | 8-48 | Iprodion |
| 10-3109 | 8-49 | Iprodion |

| Nr. | Binäre Kombination | Wirkstoff |
|---|---|---|
| 10-3110 | 8-50 | Iprodion |
| 10-3111 | 8-51 | Iprodion |
| 10-3112 | 8-52 | Iprodion |
| 10-3113 | 8-53 | Iprodion |
| 10-3114 | 8-54 | Iprodion |
| 10-3115 | 8-55 | Iprodion |
| 10-3116 | 8-56 | Iprodion |
| 10-3117 | 8-57 | Iprodion |
| 10-3118 | 8-58 | Iprodion |
| 10-3119 | 8-59 | Iprodion |
| 10-3120 | 8-60 | Iprodion |
| 10-3121 | 8-61 | Iprodion |
| 10-3122 | 8-62 | Iprodion |
| 10-3123 | 8-63 | Iprodion |
| 10-3124 | 8-64 | Iprodion |
| 10-3125 | 8-65 | Iprodion |
| 10-3126 | 8-66 | Iprodion |
| 10-3127 | 8-67 | Iprodion |
| 10-3128 | 8-68 | Iprodion |
| 10-3129 | 8-69 | Iprodion |
| 10-3130 | 8-70 | Iprodion |
| 10-3131 | 8-71 | Iprodion |
| 10-3132 | 8-72 | Iprodion |
| 10-3133 | 8-73 | Iprodion |
| 10-3134 | 8-74 | Iprodion |
| 10-3135 | 8-75 | Iprodion |
| 10-3136 | 8-76 | Iprodion |
| 10-3137 | 8-77 | Iprodion |
| 10-3138 | 8-78 | Iprodion |
| 10-3139 | 8-79 | Iprodion |
| 10-3140 | 8-80 | Iprodion |
| 10-3141 | 8-81 | Iprodion |
| 10-3142 | 8-82 | Iprodion |
| 10-3143 | 8-83 | Iprodion |
| 10-3144 | 8-84 | Iprodion |
| 10-3145 | 8-85 | Iprodion |
| 10-3146 | 8-86 | Iprodion |
| 10-3147 | 8-87 | Iprodion |
| 10-3148 | 8-88 | Iprodion |
| 10-3149 | 8-89 | Iprodion |

| Nr. | Binäre Kombination | Wirkstoff |
|---|---|---|
| 10-3150 | 8-90 | Iprodion |
| 10-3151 | 8-91 | Iprodion |
| 10-3152 | 8-92 | Iprodion |
| 10-3153 | 8-93 | Iprodion |
| 10-3154 | 8-94 | Iprodion |
| 10-3155 | 8-95 | Iprodion |
| 10-3156 | 8-96 | Iprodion |
| 10-3157 | 8-97 | Iprodion |
| 10-3158 | 8-98 | Iprodion |
| 10-3159 | 8-99 | Iprodion |
| 10-3160 | 8-100 | Iprodion |
| 10-3161 | 8-101 | Iprodion |
| 10-3162 | 8-102 | Iprodion |
| 10-3163 | 8-103 | Iprodion |
| 10-3164 | 8-104 | Iprodion |
| 10-3165 | 8-105 | Iprodion |
| 10-3166 | 8-106 | Iprodion |
| 10-3167 | 8-107 | Iprodion |
| 10-3168 | 8-108 | Iprodion |
| 10-3169 | 8-109 | Iprodion |
| 10-3170 | 8-110 | Iprodion |
| 10-3171 | 8-111 | Iprodion |
| 10-3172 | 8-112 | Iprodion |
| 10-3173 | 8-113 | Iprodion |
| 10-3174 | 8-114 | Iprodion |
| 10-3175 | 8-115 | Iprodion |
| 10-3176 | 8-116 | Iprodion |
| 10-3177 | 8-117 | Iprodion |
| 10-3178 | 8-118 | Iprodion |
| 10-3179 | 8-119 | Iprodion |
| 10-3180 | 8-120 | Iprodion |
| 10-3181 | 8-121 | Iprodion |
| 10-3182 | 8-122 | Iprodion |
| 10-3183 | 8-123 | Iprodion |
| 10-3184 | 8-124 | Iprodion |
| 10-3185 | 8-125 | Iprodion |
| 10-3186 | 8-126 | Iprodion |
| 10-3187 | 8-127 | Iprodion |
| 10-3188 | 8-128 | Iprodion |
| 10-3189 | 8-129 | Iprodion |

| Nr. | Binäre Kombination | Wirkstoff |
|---|---|---|
| 10-3190 | 8-130 | Iprodion |
| 10-3191 | 8-131 | Iprodion |
| 10-3192 | 8-132 | Iprodion |
| 10-3193 | 8-133 | Iprodion |
| 10-3194 | 8-134 | Iprodion |
| 10-3195 | 8-135 | Iprodion |
| 10-3196 | 8-136 | Iprodion |
| 10-3197 | 8-137 | Iprodion |
| 10-3198 | 8-138 | Iprodion |
| 10-3199 | 8-139 | Iprodion |
| 10-3200 | 8-140 | Iprodion |
| 10-3201 | 8-141 | Iprodion |
| 10-3202 | 8-142 | Iprodion |
| 10-3203 | 8-143 | Iprodion |
| 10-3204 | 8-144 | Iprodion |
| 10-3205 | 8-145 | Iprodion |
| 10-3206 | 8-146 | Iprodion |
| 10-3207 | 8-147 | Iprodion |
| 10-3208 | 8-148 | Iprodion |
| 10-3209 | 8-149 | Iprodion |
| 10-3210 | 8-150 | Iprodion |
| 10-3211 | 8-151 | Iprodion |
| 10-3212 | 8-152 | Iprodion |
| 10-3213 | 8-153 | Iprodion |
| 10-3214 | 8-154 | Iprodion |
| 10-3215 | 8-155 | Iprodion |
| 10-3216 | 8-156 | Iprodion |
| 10-3217 | 8-157 | Iprodion |
| 10-3218 | 8-158 | Iprodion |
| 10-3219 | 8-159 | Iprodion |
| 10-3220 | 8-160 | Iprodion |
| 10-3221 | 8-161 | Iprodion |
| 10-3222 | 8-162 | Iprodion |
| 10-3223 | 8-163 | Iprodion |
| 10-3224 | 8-164 | Iprodion |
| 10-3225 | 8-165 | Iprodion |
| 10-3226 | 8-166 | Iprodion |
| 10-3227 | 8-167 | Iprodion |
| 10-3228 | 8-168 | Iprodion |
| 10-3229 | 8-169 | Iprodion |

| Nr. | Binäre Kombination | Wirkstoff |
|---|---|---|
| 10-3230 | 8-170 | Iprodion |
| 10-3231 | 8-171 | Iprodion |
| 10-3232 | 8-172 | Iprodion |
| 10-3233 | 8-173 | Iprodion |
| 10-3234 | 8-174 | Iprodion |
| 10-3235 | 8-175 | Iprodion |
| 10-3236 | 8-176 | Iprodion |
| 10-3237 | 8-177 | Iprodion |
| 10-3238 | 8-178 | Iprodion |
| 10-3239 | 8-179 | Iprodion |
| 10-3240 | 8-180 | Iprodion |
| 10-3241 | 8-1 | Vinclozolin |
| 10-3242 | 8-2 | Vinclozolin |
| 10-3243 | 8-3 | Vinclozolin |
| 10-3244 | 8-4 | Vinclozolin |
| 10-3245 | 8-5 | Vinclozolin |
| 10-3246 | 8-6 | Vinclozolin |
| 10-3247 | 8-7 | Vinclozolin |
| 10-3248 | 8-8 | Vinclozolin |
| 10-3249 | 8-9 | Vinclozolin |
| 10-3250 | 8-10 | Vinclozolin |
| 10-3251 | 8-11 | Vinclozolin |
| 10-3252 | 8-12 | Vinclozolin |
| 10-3253 | 8-13 | Vinclozolin |
| 10-3254 | 8-14 | Vinclozolin |
| 10-3255 | 8-15 | Vinclozolin |
| 10-3256 | 8-16 | Vinclozolin |
| 10-3257 | 8-17 | Vinclozolin |
| 10-3258 | 8-18 | Vinclozolin |
| 10-3259 | 8-19 | Vinclozolin |
| 10-3260 | 8-20 | Vinclozolin |
| 10-3261 | 8-21 | Vinclozolin |
| 10-3262 | 8-22 | Vinclozolin |
| 10-3263 | 8-23 | Vinclozolin |
| 10-3264 | 8-24 | Vinclozolin |
| 10-3265 | 8-25 | Vinclozolin |
| 10-3266 | 8-26 | Vinclozolin |
| 10-3267 | 8-27 | Vinclozolin |
| 10-3268 | 8-28 | Vinclozolin |
| 10-3269 | 8-29 | Vinclozolin |

| Nr. | Binäre Kombination | Wirkstoff |
|---|---|---|
| 10-3270 | 8-30 | Vinclozolin |
| 10-3271 | 8-31 | Vinclozolin |
| 10-3272 | 8-32 | Vinclozolin |
| 10-3273 | 8-33 | Vinclozolin |
| 10-3274 | 8-34 | Vinclozolin |
| 10-3275 | 8-35 | Vinclozolin |
| 10-3276 | 8-36 | Vinclozolin |
| 10-3277 | 8-37 | Vinclozolin |
| 10-3278 | 8-38 | Vinclozolin |
| 10-3279 | 8-39 | Vinclozolin |
| 10-3280 | 8-40 | Vinclozolin |
| 10-3281 | 8-41 | Vinclozolin |
| 10-3282 | 8-42 | Vinclozolin |
| 10-3283 | 8-43 | Vinclozolin |
| 10-3284 | 8-44 | Vinclozolin |
| 10-3285 | 8-45 | Vinclozolin |
| 10-3286 | 8-46 | Vinclozolin |
| 10-3287 | 8-47 | Vinclozolin |
| 10-3288 | 8-48 | Vinclozolin |
| 10-3289 | 8-49 | Vinclozolin |
| 10-3290 | 8-50 | Vinclozolin |
| 10-3291 | 8-51 | Vinclozolin |
| 10-3292 | 8-52 | Vinclozolin |
| 10-3293 | 8-53 | Vinclozolin |
| 10-3294 | 8-54 | Vinclozolin |
| 10-3295 | 8-55 | Vinclozolin |
| 10-3296 | 8-56 | Vinclozolin |
| 10-3297 | 8-57 | Vinclozolin |
| 10-3298 | 8-58 | Vinclozolin |
| 10-3299 | 8-59 | Vinclozolin |
| 10-3300 | 8-60 | Vinclozolin |
| 10-3301 | 8-61 | Vinclozolin |
| 10-3302 | 8-62 | Vinclozolin |
| 10-3303 | 8-63 | Vinclozolin |
| 10-3304 | 8-64 | Vinclozolin |
| 10-3305 | 8-65 | Vinclozolin |
| 10-3306 | 8-66 | Vinclozolin |
| 10-3307 | 8-67 | Vinclozolin |
| 10-3308 | 8-68 | Vinclozolin |
| 10-3309 | 8-69 | Vinclozolin |

| Nr. | Binäre Kombination | Wirkstoff |
|---|---|---|
| 10-3310 | 8-70 | Vinclozolin |
| 10-3311 | 8-71 | Vinclozolin |
| 10-3312 | 8-72 | Vinclozolin |
| 10-3313 | 8-73 | Vinclozolin |
| 10-3314 | 8-74 | Vinclozolin |
| 10-3315 | 8-75 | Vinclozolin |
| 10-3316 | 8-76 | Vinclozolin |
| 10-3317 | 8-77 | Vinclozolin |
| 10-3318 | 8-78 | Vinclozolin |
| 10-3319 | 8-79 | Vinclozolin |
| 10-3320 | 8-80 | Vinclozolin |
| 10-3321 | 8-81 | Vinclozolin |
| 10-3322 | 8-82 | Vinclozolin |
| 10-3323 | 8-83 | Vinclozolin |
| 10-3324 | 8-84 | Vinclozolin |
| 10-3325 | 8-85 | Vinclozolin |
| 10-3326 | 8-86 | Vinclozolin |
| 10-3327 | 8-87 | Vinclozolin |
| 10-3328 | 8-88 | Vinclozolin |
| 10-3329 | 8-89 | Vinclozolin |
| 10-3330 | 8-90 | Vinclozolin |
| 10-3331 | 8-91 | Vinclozolin |
| 10-3332 | 8-92 | Vinclozolin |
| 10-3333 | 8-93 | Vinclozolin |
| 10-3334 | 8-94 | Vinclozolin |
| 10-3335 | 8-95 | Vinclozolin |
| 10-3336 | 8-96 | Vinclozolin |
| 10-3337 | 8-97 | Vinclozolin |
| 10-3338 | 8-98 | Vinclozolin |
| 10-3339 | 8-99 | Vinclozolin |
| 10-3340 | 8-100 | Vinclozolin |
| 10-3341 | 8-101 | Vinclozolin |
| 10-3342 | 8-102 | Vinclozolin |
| 10-3343 | 8-103 | Vinclozolin |
| 10-3344 | 8-104 | Vinclozolin |
| 10-3345 | 8-105 | Vinclozolin |
| 10-3346 | 8-106 | Vinclozolin |
| 10-3347 | 8-107 | Vinclozolin |
| 10-3348 | 8-108 | Vinclozolin |
| 10-3349 | 8-109 | Vinclozolin |

| Nr. | Binäre Kombination | Wirkstoff |
|---|---|---|
| 10-3350 | 8-110 | Vinclozolin |
| 10-3351 | 8-111 | Vinclozolin |
| 10-3352 | 8-112 | Vinclozolin |
| 10-3353 | 8-113 | Vinclozolin |
| 10-3354 | 8-114 | Vinclozolin |
| 10-3355 | 8-115 | Vinclozolin |
| 10-3356 | 8-116 | Vinclozolin |
| 10-3357 | 8-117 | Vinclozolin |
| 10-3358 | 8-118 | Vinclozolin |
| 10-3359 | 8-119 | Vinclozolin |
| 10-3360 | 8-120 | Vinclozolin |
| 10-3361 | 8-121 | Vinclozolin |
| 10-3362 | 8-122 | Vinclozolin |
| 10-3363 | 8-123 | Vinclozolin |
| 10-3364 | 8-124 | Vinclozolin |
| 10-3365 | 8-125 | Vinclozolin |
| 10-3366 | 8-126 | Vinclozolin |
| 10-3367 | 8-127 | Vinclozolin |
| 10-3368 | 8-128 | Vinclozolin |
| 10-3369 | 8-129 | Vinclozolin |
| 10-3370 | 8-130 | Vinclozolin |
| 10-3371 | 8-131 | Vinclozolin |
| 10-3372 | 8-132 | Vinclozolin |
| 10-3373 | 8-133 | Vinclozolin |
| 10-3374 | 8-134 | Vinclozolin |
| 10-3375 | 8-135 | Vinclozolin |
| 10-3376 | 8-136 | Vinclozolin |
| 10-3377 | 8-137 | Vinclozolin |
| 10-3378 | 8-138 | Vinclozolin |
| 10-3379 | 8-139 | Vinclozolin |
| 10-3380 | 8-140 | Vinclozolin |
| 10-3381 | 8-141 | Vinclozolin |
| 10-3382 | 8-142 | Vinclozolin |
| 10-3383 | 8-143 | Vinclozolin |
| 10-3384 | 8-144 | Vinclozolin |
| 10-3385 | 8-145 | Vinclozolin |
| 10-3386 | 8-146 | Vinclozolin |
| 10-3387 | 8-147 | Vinclozolin |
| 10-3388 | 8-148 | Vinclozolin |
| 10-3389 | 8-149 | Vinclozolin |

| Nr. | Binäre Kombination | Wirkstoff |
|---|---|---|
| 10-3390 | 8-150 | Vinclozolin |
| 10-3391 | 8-151 | Vinclozolin |
| 10-3392 | 8-152 | Vinclozolin |
| 10-3393 | 8-153 | Vinclozolin |
| 10-3394 | 8-154 | Vinclozolin |
| 10-3395 | 8-155 | Vinclozolin |
| 10-3396 | 8-156 | Vinclozolin |
| 10-3397 | 8-157 | Vinclozolin |
| 10-3398 | 8-158 | Vinclozolin |
| 10-3399 | 8-159 | Vinclozolin |
| 10-3400 | 8-160 | Vinclozolin |
| 10-3401 | 8-161 | Vinclozolin |
| 10-3402 | 8-162 | Vinclozolin |
| 10-3403 | 8-163 | Vinclozolin |
| 10-3404 | 8-164 | Vinclozolin |
| 10-3405 | 8-165 | Vinclozolin |
| 10-3406 | 8-166 | Vinclozolin |
| 10-3407 | 8-167 | Vinclozolin |
| 10-3408 | 8-168 | Vinclozolin |
| 10-3409 | 8-169 | Vinclozolin |
| 10-3410 | 8-170 | Vinclozolin |
| 10-3411 | 8-171 | Vinclozolin |
| 10-3412 | 8-172 | Vinclozolin |
| 10-3413 | 8-173 | Vinclozolin |
| 10-3414 | 8-174 | Vinclozolin |
| 10-3415 | 8-175 | Vinclozolin |
| 10-3416 | 8-176 | Vinclozolin |
| 10-3417 | 8-177 | Vinclozolin |
| 10-3418 | 8-178 | Vinclozolin |
| 10-3419 | 8-179 | Vinclozolin |
| 10-3420 | 8-180 | Vinclozolin |
| 10-3421 | 8-1 | Mancozeb |
| 10-3422 | 8-2 | Mancozeb |
| 10-3423 | 8-3 | Mancozeb |
| 10-3424 | 8-4 | Mancozeb |
| 10-3425 | 8-5 | Mancozeb |
| 10-3426 | 8-6 | Mancozeb |
| 10-3427 | 8-7 | Mancozeb |
| 10-3428 | 8-8 | Mancozeb |
| 10-3429 | 8-9 | Mancozeb |

| Nr. | Binäre Kombination | Wirkstoff |
|---|---|---|
| 10-3430 | 8-10 | Mancozeb |
| 10-3431 | 8-11 | Mancozeb |
| 10-3432 | 8-12 | Mancozeb |
| 10-3433 | 8-13 | Mancozeb |
| 10-3434 | 8-14 | Mancozeb |
| 10-3435 | 8-15 | Mancozeb |
| 10-3436 | 8-16 | Mancozeb |
| 10-3437 | 8-17 | Mancozeb |
| 10-3438 | 8-18 | Mancozeb |
| 10-3439 | 8-19 | Mancozeb |
| 10-3440 | 8-20 | Mancozeb |
| 10-3441 | 8-21 | Mancozeb |
| 10-3442 | 8-22 | Mancozeb |
| 10-3443 | 8-23 | Mancozeb |
| 10-3444 | 8-24 | Mancozeb |
| 10-3445 | 8-25 | Mancozeb |
| 10-3446 | 8-26 | Mancozeb |
| 10-3447 | 8-27 | Mancozeb |
| 10-3448 | 8-28 | Mancozeb |
| 10-3449 | 8-29 | Mancozeb |
| 10-3450 | 8-30 | Mancozeb |
| 10-3451 | 8-31 | Mancozeb |
| 10-3452 | 8-32 | Mancozeb |
| 10-3453 | 8-33 | Mancozeb |
| 10-3454 | 8-34 | Mancozeb |
| 10-3455 | 8-35 | Mancozeb |
| 10-3456 | 8-36 | Mancozeb |
| 10-3457 | 8-37 | Mancozeb |
| 10-3458 | 8-38 | Mancozeb |
| 10-3459 | 8-39 | Mancozeb |
| 10-3460 | 8-40 | Mancozeb |
| 10-3461 | 8-41 | Mancozeb |
| 10-3462 | 8-42 | Mancozeb |
| 10-3463 | 8-43 | Mancozeb |
| 10-3464 | 8-44 | Mancozeb |
| 10-3465 | 8-45 | Mancozeb |
| 10-3466 | 8-46 | Mancozeb |
| 10-3467 | 8-47 | Mancozeb |
| 10-3468 | 8-48 | Mancozeb |
| 10-3469 | 8-49 | Mancozeb |

| Nr. | Binäre Kombination | Wirkstoff |
|---|---|---|
| 10-3470 | 8-50 | Mancozeb |
| 10-3471 | 8-51 | Mancozeb |
| 10-3472 | 8-52 | Mancozeb |
| 10-3473 | 8-53 | Mancozeb |
| 10-3474 | 8-54 | Mancozeb |
| 10-3475 | 8-55 | Mancozeb |
| 10-3476 | 8-56 | Mancozeb |
| 10-3477 | 8-57 | Mancozeb |
| 10-3478 | 8-58 | Mancozeb |
| 10-3479 | 8-59 | Mancozeb |
| 10-3480 | 8-60 | Mancozeb |
| 10-3481 | 8-61 | Mancozeb |
| 10-3482 | 8-62 | Mancozeb |
| 10-3483 | 8-63 | Mancozeb |
| 10-3484 | 8-64 | Mancozeb |
| 10-3485 | 8-65 | Mancozeb |
| 10-3486 | 8-66 | Mancozeb |
| 10-3487 | 8-67 | Mancozeb |
| 10-3488 | 8-68 | Mancozeb |
| 10-3489 | 8-69 | Mancozeb |
| 10-3490 | 8-70 | Mancozeb |
| 10-3491 | 8-71 | Mancozeb |
| 10-3492 | 8-72 | Mancozeb |
| 10-3493 | 8-73 | Mancozeb |
| 10-3494 | 8-74 | Mancozeb |
| 10-3495 | 8-75 | Mancozeb |
| 10-3496 | 8-76 | Mancozeb |
| 10-3497 | 8-77 | Mancozeb |
| 10-3498 | 8-78 | Mancozeb |
| 10-3499 | 8-79 | Mancozeb |
| 10-3500 | 8-80 | Mancozeb |
| 10-3501 | 8-81 | Mancozeb |
| 10-3502 | 8-82 | Mancozeb |
| 10-3503 | 8-83 | Mancozeb |
| 10-3504 | 8-84 | Mancozeb |
| 10-3505 | 8-85 | Mancozeb |
| 10-3506 | 8-86 | Mancozeb |
| 10-3507 | 8-87 | Mancozeb |
| 10-3508 | 8-88 | Mancozeb |
| 10-3509 | 8-89 | Mancozeb |

| Nr. | Binäre Kombination | Wirkstoff |
|---|---|---|
| 10-3510 | 8-90 | Mancozeb |
| 10-3511 | 8-91 | Mancozeb |
| 10-3512 | 8-92 | Mancozeb |
| 10-3513 | 8-93 | Mancozeb |
| 10-3514 | 8-94 | Mancozeb |
| 10-3515 | 8-95 | Mancozeb |
| 10-3516 | 8-96 | Mancozeb |
| 10-3517 | 8-97 | Mancozeb |
| 10-3518 | 8-98 | Mancozeb |
| 10-3519 | 8-99 | Mancozeb |
| 10-3520 | 8-100 | Mancozeb |
| 10-3521 | 8-101 | Mancozeb |
| 10-3522 | 8-102 | Mancozeb |
| 10-3523 | 8-103 | Mancozeb |
| 10-3524 | 8-104 | Mancozeb |
| 10-3525 | 8-105 | Mancozeb |
| 10-3526 | 8-106 | Mancozeb |
| 10-3527 | 8-107 | Mancozeb |
| 10-3528 | 8-108 | Mancozeb |
| 10-3529 | 8-109 | Mancozeb |
| 10-3530 | 8-110 | Mancozeb |
| 10-3531 | 8-111 | Mancozeb |
| 10-3532 | 8-112 | Mancozeb |
| 10-3533 | 8-113 | Mancozeb |
| 10-3534 | 8-114 | Mancozeb |
| 10-3535 | 8-115 | Mancozeb |
| 10-3536 | 8-116 | Mancozeb |
| 10-3537 | 8-117 | Mancozeb |
| 10-3538 | 8-118 | Mancozeb |
| 10-3539 | 8-119 | Mancozeb |
| 10-3540 | 8-120 | Mancozeb |
| 10-3541 | 8-121 | Mancozeb |
| 10-3542 | 8-122 | Mancozeb |
| 10-3543 | 8-123 | Mancozeb |
| 10-3544 | 8-124 | Mancozeb |
| 10-3545 | 8-125 | Mancozeb |
| 10-3546 | 8-126 | Mancozeb |
| 10-3547 | 8-127 | Mancozeb |
| 10-3548 | 8-128 | Mancozeb |
| 10-3549 | 8-129 | Mancozeb |

| Nr. | Binäre Kombination | Wirkstoff |
|---|---|---|
| 10-3550 | 8-130 | Mancozeb |
| 10-3551 | 8-131 | Mancozeb |
| 10-3552 | 8-132 | Mancozeb |
| 10-3553 | 8-133 | Mancozeb |
| 10-3554 | 8-134 | Mancozeb |
| 10-3555 | 8-135 | Mancozeb |
| 10-3556 | 8-136 | Mancozeb |
| 10-3557 | 8-137 | Mancozeb |
| 10-3558 | 8-138 | Mancozeb |
| 10-3559 | 8-139 | Mancozeb |
| 10-3560 | 8-140 | Mancozeb |
| 10-3561 | 8-141 | Mancozeb |
| 10-3562 | 8-142 | Mancozeb |
| 10-3563 | 8-143 | Mancozeb |
| 10-3564 | 8-144 | Mancozeb |
| 10-3565 | 8-145 | Mancozeb |
| 10-3566 | 8-146 | Mancozeb |
| 10-3567 | 8-147 | Mancozeb |
| 10-3568 | 8-148 | Mancozeb |
| 10-3569 | 8-149 | Mancozeb |
| 10-3570 | 8-150 | Mancozeb |
| 10-3571 | 8-151 | Mancozeb |
| 10-3572 | 8-152 | Mancozeb |
| 10-3573 | 8-153 | Mancozeb |
| 10-3574 | 8-154 | Mancozeb |
| 10-3575 | 8-155 | Mancozeb |
| 10-3576 | 8-156 | Mancozeb |
| 10-3577 | 8-157 | Mancozeb |
| 10-3578 | 8-158 | Mancozeb |
| 10-3579 | 8-159 | Mancozeb |
| 10-3580 | 8-160 | Mancozeb |
| 10-3581 | 8-161 | Mancozeb |
| 10-3582 | 8-162 | Mancozeb |
| 10-3583 | 8-163 | Mancozeb |
| 10-3584 | 8-164 | Mancozeb |
| 10-3585 | 8-165 | Mancozeb |
| 10-3586 | 8-166 | Mancozeb |
| 10-3587 | 8-167 | Mancozeb |
| 10-3588 | 8-168 | Mancozeb |
| 10-3589 | 8-169 | Mancozeb |

| Nr. | Binäre Kombination | Wirkstoff |
|---|---|---|
| 10-3590 | 8-170 | Mancozeb |
| 10-3591 | 8-171 | Mancozeb |
| 10-3592 | 8-172 | Mancozeb |
| 10-3593 | 8-173 | Mancozeb |
| 10-3594 | 8-174 | Mancozeb |
| 10-3595 | 8-175 | Mancozeb |
| 10-3596 | 8-176 | Mancozeb |
| 10-3597 | 8-177 | Mancozeb |
| 10-3598 | 8-178 | Mancozeb |
| 10-3599 | 8-179 | Mancozeb |
| 10-3600 | 8-180 | Mancozeb |
| 10-3601 | 8-1 | Metiram |
| 10-3602 | 8-2 | Metiram |
| 10-3603 | 8-3 | Metiram |
| 10-3604 | 8-4 | Metiram |
| 10-3605 | 8-5 | Metiram |
| 10-3606 | 8-6 | Metiram |
| 10-3607 | 8-7 | Metiram |
| 10-3608 | 8-8 | Metiram |
| 10-3609 | 8-9 | Metiram |
| 10-3610 | 8-10 | Metiram |
| 10-3611 | 8-11 | Metiram |
| 10-3612 | 8-12 | Metiram |
| 10-3613 | 8-13 | Metiram |
| 10-3614 | 8-14 | Metiram |
| 10-3615 | 8-15 | Metiram |
| 10-3616 | 8-16 | Metiram |
| 10-3617 | 8-17 | Metiram |
| 10-3618 | 8-18 | Metiram |
| 10-3619 | 8-19 | Metiram |
| 10-3620 | 8-20 | Metiram |
| 10-3621 | 8-21 | Metiram |
| 10-3622 | 8-22 | Metiram |
| 10-3623 | 8-23 | Metiram |
| 10-3624 | 8-24 | Metiram |
| 10-3625 | 8-25 | Metiram |
| 10-3626 | 8-26 | Metiram |
| 10-3627 | 8-27 | Metiram |
| 10-3628 | 8-28 | Metiram |
| 10-3629 | 8-29 | Metiram |

| Nr. | Binäre Kombination | Wirkstoff |
|---|---|---|
| 10-3630 | 8-30 | Metiram |
| 10-3631 | 8-31 | Metiram |
| 10-3632 | 8-32 | Metiram |
| 10-3633 | 8-33 | Metiram |
| 10-3634 | 8-34 | Metiram |
| 10-3635 | 8-35 | Metiram |
| 10-3636 | 8-36 | Metiram |
| 10-3637 | 8-37 | Metiram |
| 10-3638 | 8-38 | Metiram |
| 10-3639 | 8-39 | Metiram |
| 10-3640 | 8-40 | Metiram |
| 10-3641 | 8-41 | Metiram |
| 10-3642 | 8-42 | Metiram |
| 10-3643 | 8-43 | Metiram |
| 10-3644 | 8-44 | Metiram |
| 10-3645 | 8-45 | Metiram |
| 10-3646 | 8-46 | Metiram |
| 10-3647 | 8-47 | Metiram |
| 10-3648 | 8-48 | Metiram |
| 10-3649 | 8-49 | Metiram |
| 10-3650 | 8-50 | Metiram |
| 10-3651 | 8-51 | Metiram |
| 10-3652 | 8-52 | Metiram |
| 10-3653 | 8-53 | Metiram |
| 10-3654 | 8-54 | Metiram |
| 10-3655 | 8-55 | Metiram |
| 10-3656 | 8-56 | Metiram |
| 10-3657 | 8-57 | Metiram |
| 10-3658 | 8-58 | Metiram |
| 10-3659 | 8-59 | Metiram |
| 10-3660 | 8-60 | Metiram |
| 10-3661 | 8-61 | Metiram |
| 10-3662 | 8-62 | Metiram |
| 10-3663 | 8-63 | Metiram |
| 10-3664 | 8-64 | Metiram |
| 10-3665 | 8-65 | Metiram |
| 10-3666 | 8-66 | Metiram |
| 10-3667 | 8-67 | Metiram |
| 10-3668 | 8-68 | Metiram |
| 10-3669 | 8-69 | Metiram |
| 10-3670 | 8-70 | Metiram |
| 10-3671 | 8-71 | Metiram |
| 10-3672 | 8-72 | Metiram |
| 10-3673 | 8-73 | Metiram |
| 10-3674 | 8-74 | Metiram |
| 10-3675 | 8-75 | Metiram |
| 10-3676 | 8-76 | Metiram |
| 10-3677 | 8-77 | Metiram |
| 10-3678 | 8-78 | Metiram |
| 10-3679 | 8-79 | Metiram |
| 10-3680 | 8-80 | Metiram |
| 10-3681 | 8-81 | Metiram |
| 10-3682 | 8-82 | Metiram |
| 10-3683 | 8-83 | Metiram |
| 10-3684 | 8-84 | Metiram |
| 10-3685 | 8-85 | Metiram |
| 10-3686 | 8-86 | Metiram |
| 10-3687 | 8-87 | Metiram |
| 10-3688 | 8-88 | Metiram |
| 10-3689 | 8-89 | Metiram |
| 10-3690 | 8-90 | Metiram |
| 10-3691 | 8-91 | Metiram |
| 10-3692 | 8-92 | Metiram |
| 10-3693 | 8-93 | Metiram |
| 10-3694 | 8-94 | Metiram |
| 10-3695 | 8-95 | Metiram |
| 10-3696 | 8-96 | Metiram |
| 10-3697 | 8-97 | Metiram |
| 10-3698 | 8-98 | Metiram |
| 10-3699 | 8-99 | Metiram |
| 10-3700 | 8-100 | Metiram |
| 10-3701 | 8-101 | Metiram |
| 10-3702 | 8-102 | Metiram |
| 10-3703 | 8-103 | Metiram |
| 10-3704 | 8-104 | Metiram |
| 10-3705 | 8-105 | Metiram |
| 10-3706 | 8-106 | Metiram |
| 10-3707 | 8-107 | Metiram |
| 10-3708 | 8-108 | Metiram |
| 10-3709 | 8-109 | Metiram |

| Nr. | Binäre Kombination | Wirkstoff |
|---|---|---|
| 10-3710 | 8-110 | Metiram |
| 10-3711 | 8-111 | Metiram |
| 10-3712 | 8-112 | Metiram |
| 10-3713 | 8-113 | Metiram |
| 10-3714 | 8-114 | Metiram |
| 10-3715 | 8-115 | Metiram |
| 10-3716 | 8-116 | Metiram |
| 10-3717 | 8-117 | Metiram |
| 10-3718 | 8-118 | Metiram |
| 10-3719 | 8-119 | Metiram |
| 10-3720 | 8-120 | Metiram |
| 10-3721 | 8-121 | Metiram |
| 10-3722 | 8-122 | Metiram |
| 10-3723 | 8-123 | Metiram |
| 10-3724 | 8-124 | Metiram |
| 10-3725 | 8-125 | Metiram |
| 10-3726 | 8-126 | Metiram |
| 10-3727 | 8-127 | Metiram |
| 10-3728 | 8-128 | Metiram |
| 10-3729 | 8-129 | Metiram |
| 10-3730 | 8-130 | Metiram |
| 10-3731 | 8-131 | Metiram |
| 10-3732 | 8-132 | Metiram |
| 10-3733 | 8-133 | Metiram |
| 10-3734 | 8-134 | Metiram |
| 10-3735 | 8-135 | Metiram |
| 10-3736 | 8-136 | Metiram |
| 10-3737 | 8-137 | Metiram |
| 10-3738 | 8-138 | Metiram |
| 10-3739 | 8-139 | Metiram |
| 10-3740 | 8-140 | Metiram |
| 10-3741 | 8-141 | Metiram |
| 10-3742 | 8-142 | Metiram |
| 10-3743 | 8-143 | Metiram |
| 10-3744 | 8-144 | Metiram |
| 10-3745 | 8-145 | Metiram |
| 10-3746 | 8-146 | Metiram |
| 10-3747 | 8-147 | Metiram |
| 10-3748 | 8-148 | Metiram |
| 10-3749 | 8-149 | Metiram |

| Nr. | Binäre Kombination | Wirkstoff |
|---|---|---|
| 10-3750 | 8-150 | Metiram |
| 10-3751 | 8-151 | Metiram |
| 10-3752 | 8-152 | Metiram |
| 10-3753 | 8-153 | Metiram |
| 10-3754 | 8-154 | Metiram |
| 10-3755 | 8-155 | Metiram |
| 10-3756 | 8-156 | Metiram |
| 10-3757 | 8-157 | Metiram |
| 10-3758 | 8-158 | Metiram |
| 10-3759 | 8-159 | Metiram |
| 10-3760 | 8-160 | Metiram |
| 10-3761 | 8-161 | Metiram |
| 10-3762 | 8-162 | Metiram |
| 10-3763 | 8-163 | Metiram |
| 10-3764 | 8-164 | Metiram |
| 10-3765 | 8-165 | Metiram |
| 10-3766 | 8-166 | Metiram |
| 10-3767 | 8-167 | Metiram |
| 10-3768 | 8-168 | Metiram |
| 10-3769 | 8-169 | Metiram |
| 10-3770 | 8-170 | Metiram |
| 10-3771 | 8-171 | Metiram |
| 10-3772 | 8-172 | Metiram |
| 10-3773 | 8-173 | Metiram |
| 10-3774 | 8-174 | Metiram |
| 10-3775 | 8-175 | Metiram |
| 10-3776 | 8-176 | Metiram |
| 10-3777 | 8-177 | Metiram |
| 10-3778 | 8-178 | Metiram |
| 10-3779 | 8-179 | Metiram |
| 10-3780 | 8-180 | Metiram |
| 10-3781 | 8-1 | Chlorothalonil |
| 10-3782 | 8-2 | Chlorothalonil |
| 10-3783 | 8-3 | Chlorothalonil |
| 10-3784 | 8-4 | Chlorothalonil |
| 10-3785 | 8-5 | Chlorothalonil |
| 10-3786 | 8-6 | Chlorothalonil |
| 10-3787 | 8-7 | Chlorothalonil |
| 10-3788 | 8-8 | Chlorothalonil |
| 10-3789 | 8-9 | Chlorothalonil |

| Nr. | Binäre Kombination | Wirkstoff |
|---|---|---|
| 10-3790 | 8-10 | Chlorothalonil |
| 10-3791 | 8-11 | Chlorothalonil |
| 10-3792 | 8-12 | Chlorothalonil |
| 10-3793 | 8-13 | Chlorothalonil |
| 10-3794 | 8-14 | Chlorothalonil |
| 10-3795 | 8-15 | Chlorothalonil |
| 10-3796 | 8-16 | Chlorothalonil |
| 10-3797 | 8-17 | Chlorothalonil |
| 10-3798 | 8-18 | Chlorothalonil |
| 10-3799 | 8-19 | Chlorothalonil |
| 10-3800 | 8-20 | Chlorothalonil |
| 10-3801 | 8-21 | Chlorothalonil |
| 10-3802 | 8-22 | Chlorothalonil |
| 10-3803 | 8-23 | Chlorothalonil |
| 10-3804 | 8-24 | Chlorothalonil |
| 10-3805 | 8-25 | Chlorothalonil |
| 10-3806 | 8-26 | Chlorothalonil |
| 10-3807 | 8-27 | Chlorothalonil |
| 10-3808 | 8-28 | Chlorothalonil |
| 10-3809 | 8-29 | Chlorothalonil |
| 10-3810 | 8-30 | Chlorothalonil |
| 10-3811 | 8-31 | Chlorothalonil |
| 10-3812 | 8-32 | Chlorothalonil |
| 10-3813 | 8-33 | Chlorothalonil |
| 10-3814 | 8-34 | Chlorothalonil |
| 10-3815 | 8-35 | Chlorothalonil |
| 10-3816 | 8-36 | Chlorothalonil |
| 10-3817 | 8-37 | Chlorothalonil |
| 10-3818 | 8-38 | Chlorothalonil |
| 10-3819 | 8-39 | Chlorothalonil |
| 10-3820 | 8-40 | Chlorothalonil |
| 10-3821 | 8-41 | Chlorothalonil |
| 10-3822 | 8-42 | Chlorothalonil |
| 10-3823 | 8-43 | Chlorothalonil |
| 10-3824 | 8-44 | Chlorothalonil |
| 10-3825 | 8-45 | Chlorothalonil |
| 10-3826 | 8-46 | Chlorothalonil |
| 10-3827 | 8-47 | Chlorothalonil |
| 10-3828 | 8-48 | Chlorothalonil |
| 10-3829 | 8-49 | Chlorothalonil |

| Nr. | Binäre Kombination | Wirkstoff |
|---|---|---|
| 10-3830 | 8-50 | Chlorothalonil |
| 10-3831 | 8-51 | Chlorothalonil |
| 10-3832 | 8-52 | Chlorothalonil |
| 10-3833 | 8-53 | Chlorothalonil |
| 10-3834 | 8-54 | Chlorothalonil |
| 10-3835 | 8-55 | Chlorothalonil |
| 10-3836 | 8-56 | Chlorothalonil |
| 10-3837 | 8-57 | Chlorothalonil |
| 10-3838 | 8-58 | Chlorothalonil |
| 10-3839 | 8-59 | Chlorothalonil |
| 10-3840 | 8-60 | Chlorothalonil |
| 10-3841 | 8-61 | Chlorothalonil |
| 10-3842 | 8-62 | Chlorothalonil |
| 10-3843 | 8-63 | Chlorothalonil |
| 10-3844 | 8-64 | Chlorothalonil |
| 10-3845 | 8-65 | Chlorothalonil |
| 10-3846 | 8-66 | Chlorothalonil |
| 10-3847 | 8-67 | Chlorothalonil |
| 10-3848 | 8-68 | Chlorothalonil |
| 10-3849 | 8-69 | Chlorothalonil |
| 10-3850 | 8-70 | Chlorothalonil |
| 10-3851 | 8-71 | Chlorothalonil |
| 10-3852 | 8-72 | Chlorothalonil |
| 10-3853 | 8-73 | Chlorothalonil |
| 10-3854 | 8-74 | Chlorothalonil |
| 10-3855 | 8-75 | Chlorothalonil |
| 10-3856 | 8-76 | Chlorothalonil |
| 10-3857 | 8-77 | Chlorothalonil |
| 10-3858 | 8-78 | Chlorothalonil |
| 10-3859 | 8-79 | Chlorothalonil |
| 10-3860 | 8-80 | Chlorothalonil |
| 10-3861 | 8-81 | Chlorothalonil |
| 10-3862 | 8-82 | Chlorothalonil |
| 10-3863 | 8-83 | Chlorothalonil |
| 10-3864 | 8-84 | Chlorothalonil |
| 10-3865 | 8-85 | Chlorothalonil |
| 10-3866 | 8-86 | Chlorothalonil |
| 10-3867 | 8-87 | Chlorothalonil |
| 10-3868 | 8-88 | Chlorothalonil |
| 10-3869 | 8-89 | Chlorothalonil |

| Nr. | Binäre Kombination | Wirkstoff |
|---|---|---|
| 10-3870 | 8-90 | Chlorothalonil |
| 10-3871 | 8-91 | Chlorothalonil |
| 10-3872 | 8-92 | Chlorothalonil |
| 10-3873 | 8-93 | Chlorothalonil |
| 10-3874 | 8-94 | Chlorothalonil |
| 10-3875 | 8-95 | Chlorothalonil |
| 10-3876 | 8-96 | Chlorothalonil |
| 10-3877 | 8-97 | Chlorothalonil |
| 10-3878 | 8-98 | Chlorothalonil |
| 10-3879 | 8-99 | Chlorothalonil |
| 10-3880 | 8-100 | Chlorothalonil |
| 10-3881 | 8-101 | Chlorothalonil |
| 10-3882 | 8-102 | Chlorothalonil |
| 10-3883 | 8-103 | Chlorothalonil |
| 10-3884 | 8-104 | Chlorothalonil |
| 10-3885 | 8-105 | Chlorothalonil |
| 10-3886 | 8-106 | Chlorothalonil |
| 10-3887 | 8-107 | Chlorothalonil |
| 10-3888 | 8-108 | Chlorothalonil |
| 10-3889 | 8-109 | Chlorothalonil |
| 10-3890 | 8-110 | Chlorothalonil |
| 10-3891 | 8-111 | Chlorothalonil |
| 10-3892 | 8-112 | Chlorothalonil |
| 10-3893 | 8-113 | Chlorothalonil |
| 10-3894 | 8-114 | Chlorothalonil |
| 10-3895 | 8-115 | Chlorothalonil |
| 10-3896 | 8-116 | Chlorothalonil |
| 10-3897 | 8-117 | Chlorothalonil |
| 10-3898 | 8-118 | Chlorothalonil |
| 10-3899 | 8-119 | Chlorothalonil |
| 10-3900 | 8-120 | Chlorothalonil |
| 10-3901 | 8-121 | Chlorothalonil |
| 10-3902 | 8-122 | Chlorothalonil |
| 10-3903 | 8-123 | Chlorothalonil |
| 10-3904 | 8-124 | Chlorothalonil |
| 10-3905 | 8-125 | Chlorothalonil |
| 10-3906 | 8-126 | Chlorothalonil |
| 10-3907 | 8-127 | Chlorothalonil |
| 10-3908 | 8-128 | Chlorothalonil |
| 10-3909 | 8-129 | Chlorothalonil |

| Nr. | Binäre Kombination | Wirkstoff |
|---|---|---|
| 10-3910 | 8-130 | Chlorothalonil |
| 10-3911 | 8-131 | Chlorothalonil |
| 10-3912 | 8-132 | Chlorothalonil |
| 10-3913 | 8-133 | Chlorothalonil |
| 10-3914 | 8-134 | Chlorothalonil |
| 10-3915 | 8-135 | Chlorothalonil |
| 10-3916 | 8-136 | Chlorothalonil |
| 10-3917 | 8-137 | Chlorothalonil |
| 10-3918 | 8-138 | Chlorothalonil |
| 10-3919 | 8-139 | Chlorothalonil |
| 10-3920 | 8-140 | Chlorothalonil |
| 10-3921 | 8-141 | Chlorothalonil |
| 10-3922 | 8-142 | Chlorothalonil |
| 10-3923 | 8-143 | Chlorothalonil |
| 10-3924 | 8-144 | Chlorothalonil |
| 10-3925 | 8-145 | Chlorothalonil |
| 10-3926 | 8-146 | Chlorothalonil |
| 10-3927 | 8-147 | Chlorothalonil |
| 10-3928 | 8-148 | Chlorothalonil |
| 10-3929 | 8-149 | Chlorothalonil |
| 10-3930 | 8-150 | Chlorothalonil |
| 10-3931 | 8-151 | Chlorothalonil |
| 10-3932 | 8-152 | Chlorothalonil |
| 10-3933 | 8-153 | Chlorothalonil |
| 10-3934 | 8-154 | Chlorothalonil |
| 10-3935 | 8-155 | Chlorothalonil |
| 10-3936 | 8-156 | Chlorothalonil |
| 10-3937 | 8-157 | Chlorothalonil |
| 10-3938 | 8-158 | Chlorothalonil |
| 10-3939 | 8-159 | Chlorothalonil |
| 10-3940 | 8-160 | Chlorothalonil |
| 10-3941 | 8-161 | Chlorothalonil |
| 10-3942 | 8-162 | Chlorothalonil |
| 10-3943 | 8-163 | Chlorothalonil |
| 10-3944 | 8-164 | Chlorothalonil |
| 10-3945 | 8-165 | Chlorothalonil |
| 10-3946 | 8-166 | Chlorothalonil |
| 10-3947 | 8-167 | Chlorothalonil |
| 10-3948 | 8-168 | Chlorothalonil |
| 10-3949 | 8-169 | Chlorothalonil |

| Nr. | Binäre Kombination | Wirkstoff |
|---|---|---|
| 10-3950 | 8-170 | Chlorothalonil |
| 10-3951 | 8-171 | Chlorothalonil |
| 10-3952 | 8-172 | Chlorothalonil |
| 10-3953 | 8-173 | Chlorothalonil |
| 10-3954 | 8-174 | Chlorothalonil |
| 10-3955 | 8-175 | Chlorothalonil |
| 10-3956 | 8-176 | Chlorothalonil |
| 10-3957 | 8-177 | Chlorothalonil |
| 10-3958 | 8-178 | Chlorothalonil |
| 10-3959 | 8-179 | Chlorothalonil |
| 10-3960 | 8-180 | Chlorothalonil |
| 10-3961 | 8-1 | Metrafenon |
| 10-3962 | 8-2 | Metrafenon |
| 10-3963 | 8-3 | Metrafenon |
| 10-3964 | 8-4 | Metrafenon |
| 10-3965 | 8-5 | Metrafenon |
| 10-3966 | 8-6 | Metrafenon |
| 10-3967 | 8-7 | Metrafenon |
| 10-3968 | 8-8 | Metrafenon |
| 10-3969 | 8-9 | Metrafenon |
| 10-3970 | 8-10 | Metrafenon |
| 10-3971 | 8-11 | Metrafenon |
| 10-3972 | 8-12 | Metrafenon |
| 10-3973 | 8-13 | Metrafenon |
| 10-3974 | 8-14 | Metrafenon |
| 10-3975 | 8-15 | Metrafenon |
| 10-3976 | 8-16 | Metrafenon |
| 10-3977 | 8-17 | Metrafenon |
| 10-3978 | 8-18 | Metrafenon |
| 10-3979 | 8-19 | Metrafenon |
| 10-3980 | 8-20 | Metrafenon |
| 10-3981 | 8-21 | Metrafenon |
| 10-3982 | 8-22 | Metrafenon |
| 10-3983 | 8-23 | Metrafenon |
| 10-3984 | 8-24 | Metrafenon |
| 10-3985 | 8-25 | Metrafenon |
| 10-3986 | 8-26 | Metrafenon |
| 10-3987 | 8-27 | Metrafenon |
| 10-3988 | 8-28 | Metrafenon |
| 10-3989 | 8-29 | Metrafenon |

| Nr. | Binäre Kombination | Wirkstoff |
|---|---|---|
| 10-3990 | 8-30 | Metrafenon |
| 10-3991 | 8-31 | Metrafenon |
| 10-3992 | 8-32 | Metrafenon |
| 10-3993 | 8-33 | Metrafenon |
| 10-3994 | 8-34 | Metrafenon |
| 10-3995 | 8-35 | Metrafenon |
| 10-3996 | 8-36 | Metrafenon |
| 10-3997 | 8-37 | Metrafenon |
| 10-3998 | 8-38 | Metrafenon |
| 10-3999 | 8-39 | Metrafenon |
| 10-4000 | 8-40 | Metrafenon |
| 10-4001 | 8-41 | Metrafenon |
| 10-4002 | 8-42 | Metrafenon |
| 10-4003 | 8-43 | Metrafenon |
| 10-4004 | 8-44 | Metrafenon |
| 10-4005 | 8-45 | Metrafenon |
| 10-4006 | 8-46 | Metrafenon |
| 10-4007 | 8-47 | Metrafenon |
| 10-4008 | 8-48 | Metrafenon |
| 10-4009 | 8-49 | Metrafenon |
| 10-4010 | 8-50 | Metrafenon |
| 10-4011 | 8-51 | Metrafenon |
| 10-4012 | 8-52 | Metrafenon |
| 10-4013 | 8-53 | Metrafenon |
| 10-4014 | 8-54 | Metrafenon |
| 10-4015 | 8-55 | Metrafenon |
| 10-4016 | 8-56 | Metrafenon |
| 10-4017 | 8-57 | Metrafenon |
| 10-4018 | 8-58 | Metrafenon |
| 10-4019 | 8-59 | Metrafenon |
| 10-4020 | 8-60 | Metrafenon |
| 10-4021 | 8-61 | Metrafenon |
| 10-4022 | 8-62 | Metrafenon |
| 10-4023 | 8-63 | Metrafenon |
| 10-4024 | 8-64 | Metrafenon |
| 10-4025 | 8-65 | Metrafenon |
| 10-4026 | 8-66 | Metrafenon |
| 10-4027 | 8-67 | Metrafenon |
| 10-4028 | 8-68 | Metrafenon |
| 10-4029 | 8-69 | Metrafenon |

| Nr. | Binäre Kombination | Wirkstoff |
|---|---|---|
| 10-4030 | 8-70 | Metrafenon |
| 10-4031 | 8-71 | Metrafenon |
| 10-4032 | 8-72 | Metrafenon |
| 10-4033 | 8-73 | Metrafenon |
| 10-4034 | 8-74 | Metrafenon |
| 10-4035 | 8-75 | Metrafenon |
| 10-4036 | 8-76 | Metrafenon |
| 10-4037 | 8-77 | Metrafenon |
| 10-4038 | 8-78 | Metrafenon |
| 10-4039 | 8-79 | Metrafenon |
| 10-4040 | 8-80 | Metrafenon |
| 10-4041 | 8-81 | Metrafenon |
| 10-4042 | 8-82 | Metrafenon |
| 10-4043 | 8-83 | Metrafenon |
| 10-4044 | 8-84 | Metrafenon |
| 10-4045 | 8-85 | Metrafenon |
| 10-4046 | 8-86 | Metrafenon |
| 10-4047 | 8-87 | Metrafenon |
| 10-4048 | 8-88 | Metrafenon |
| 10-4049 | 8-89 | Metrafenon |
| 10-4050 | 8-90 | Metrafenon |
| 10-4051 | 8-91 | Metrafenon |
| 10-4052 | 8-92 | Metrafenon |
| 10-4053 | 8-93 | Metrafenon |
| 10-4054 | 8-94 | Metrafenon |
| 10-4055 | 8-95 | Metrafenon |
| 10-4056 | 8-96 | Metrafenon |
| 10-4057 | 8-97 | Metrafenon |
| 10-4058 | 8-98 | Metrafenon |
| 10-4059 | 8-99 | Metrafenon |
| 10-4060 | 8-100 | Metrafenon |
| 10-4061 | 8-101 | Metrafenon |
| 10-4062 | 8-102 | Metrafenon |
| 10-4063 | 8-103 | Metrafenon |
| 10-4064 | 8-104 | Metrafenon |
| 10-4065 | 8-105 | Metrafenon |
| 10-4066 | 8-106 | Metrafenon |
| 10-4067 | 8-107 | Metrafenon |
| 10-4068 | 8-108 | Metrafenon |
| 10-4069 | 8-109 | Metrafenon |

| Nr. | Binäre Kombination | Wirkstoff |
|---|---|---|
| 10-4070 | 8-110 | Metrafenon |
| 10-4071 | 8-111 | Metrafenon |
| 10-4072 | 8-112 | Metrafenon |
| 10-4073 | 8-113 | Metrafenon |
| 10-4074 | 8-114 | Metrafenon |
| 10-4075 | 8-115 | Metrafenon |
| 10-4076 | 8-116 | Metrafenon |
| 10-4077 | 8-117 | Metrafenon |
| 10-4078 | 8-118 | Metrafenon |
| 10-4079 | 8-119 | Metrafenon |
| 10-4080 | 8-120 | Metrafenon |
| 10-4081 | 8-121 | Metrafenon |
| 10-4082 | 8-122 | Metrafenon |
| 10-4083 | 8-123 | Metrafenon |
| 10-4084 | 8-124 | Metrafenon |
| 10-4085 | 8-125 | Metrafenon |
| 10-4086 | 8-126 | Metrafenon |
| 10-4087 | 8-127 | Metrafenon |
| 10-4088 | 8-128 | Metrafenon |
| 10-4089 | 8-129 | Metrafenon |
| 10-4090 | 8-130 | Metrafenon |
| 10-4091 | 8-131 | Metrafenon |
| 10-4092 | 8-132 | Metrafenon |
| 10-4093 | 8-133 | Metrafenon |
| 10-4094 | 8-134 | Metrafenon |
| 10-4095 | 8-135 | Metrafenon |
| 10-4096 | 8-136 | Metrafenon |
| 10-4097 | 8-137 | Metrafenon |
| 10-4098 | 8-138 | Metrafenon |
| 10-4099 | 8-139 | Metrafenon |
| 10-4100 | 8-140 | Metrafenon |
| 10-4101 | 8-141 | Metrafenon |
| 10-4102 | 8-142 | Metrafenon |
| 10-4103 | 8-143 | Metrafenon |
| 10-4104 | 8-144 | Metrafenon |
| 10-4105 | 8-145 | Metrafenon |
| 10-4106 | 8-146 | Metrafenon |
| 10-4107 | 8-147 | Metrafenon |
| 10-4108 | 8-148 | Metrafenon |
| 10-4109 | 8-149 | Metrafenon |

| Nr. | Binäre Kombination | Wirkstoff |
|---|---|---|
| 10-4110 | 8-150 | Metrafenon |
| 10-4111 | 8-151 | Metrafenon |
| 10-4112 | 8-152 | Metrafenon |
| 10-4113 | 8-153 | Metrafenon |
| 10-4114 | 8-154 | Metrafenon |
| 10-4115 | 8-155 | Metrafenon |
| 10-4116 | 8-156 | Metrafenon |
| 10-4117 | 8-157 | Metrafenon |
| 10-4118 | 8-158 | Metrafenon |
| 10-4119 | 8-159 | Metrafenon |
| 10-4120 | 8-160 | Metrafenon |
| 10-4121 | 8-161 | Metrafenon |
| 10-4122 | 8-162 | Metrafenon |
| 10-4123 | 8-163 | Metrafenon |
| 10-4124 | 8-164 | Metrafenon |
| 10-4125 | 8-165 | Metrafenon |
| 10-4126 | 8-166 | Metrafenon |
| 10-4127 | 8-167 | Metrafenon |
| 10-4128 | 8-168 | Metrafenon |
| 10-4129 | 8-169 | Metrafenon |
| 10-4130 | 8-170 | Metrafenon |
| 10-4131 | 8-171 | Metrafenon |
| 10-4132 | 8-172 | Metrafenon |
| 10-4133 | 8-173 | Metrafenon |
| 10-4134 | 8-174 | Metrafenon |
| 10-4135 | 8-175 | Metrafenon |
| 10-4136 | 8-176 | Metrafenon |
| 10-4137 | 8-177 | Metrafenon |
| 10-4138 | 8-178 | Metrafenon |
| 10-4139 | 8-179 | Metrafenon |
| 10-4140 | 8-180 | Metrafenon |
| 10-4141 | 8-1 | $H_3PO_3$ |
| 10-4142 | 8-2 | $H_3PO_3$ |
| 10-4143 | 8-3 | $H_3PO_3$ |
| 10-4144 | 8-4 | $H_3PO_3$ |
| 10-4145 | 8-5 | $H_3PO_3$ |
| 10-4146 | 8-6 | $H_3PO_3$ |
| 10-4147 | 8-7 | $H_3PO_3$ |
| 10-4148 | 8-8 | $H_3PO_3$ |
| 10-4149 | 8-9 | $H_3PO_3$ |

| Nr. | Binäre Kombination | Wirkstoff |
|---|---|---|
| 10-4150 | 8-10 | $H_3PO_3$ |
| 10-4151 | 8-11 | $H_3PO_3$ |
| 10-4152 | 8-12 | $H_3PO_3$ |
| 10-4153 | 8-13 | $H_3PO_3$ |
| 10-4154 | 8-14 | $H_3PO_3$ |
| 10-4155 | 8-15 | $H_3PO_3$ |
| 10-4156 | 8-16 | $H_3PO_3$ |
| 10-4157 | 8-17 | $H_3PO_3$ |
| 10-4158 | 8-18 | $H_3PO_3$ |
| 10-4159 | 8-19 | $H_3PO_3$ |
| 10-4160 | 8-20 | $H_3PO_3$ |
| 10-4161 | 8-21 | $H_3PO_3$ |
| 10-4162 | 8-22 | $H_3PO_3$ |
| 10-4163 | 8-23 | $H_3PO_3$ |
| 10-4164 | 8-24 | $H_3PO_3$ |
| 10-4165 | 8-25 | $H_3PO_3$ |
| 10-4166 | 8-26 | $H_3PO_3$ |
| 10-4167 | 8-27 | $H_3PO_3$ |
| 10-4168 | 8-28 | $H_3PO_3$ |
| 10-4169 | 8-29 | $H_3PO_3$ |
| 10-4170 | 8-30 | $H_3PO_3$ |
| 10-4171 | 8-31 | $H_3PO_3$ |
| 10-4172 | 8-32 | $H_3PO_3$ |
| 10-4173 | 8-33 | $H_3PO_3$ |
| 10-4174 | 8-34 | $H_3PO_3$ |
| 10-4175 | 8-35 | $H_3PO_3$ |
| 10-4176 | 8-36 | $H_3PO_3$ |
| 10-4177 | 8-37 | $H_3PO_3$ |
| 10-4178 | 8-38 | $H_3PO_3$ |
| 10-4179 | 8-39 | $H_3PO_3$ |
| 10-4180 | 8-40 | $H_3PO_3$ |
| 10-4181 | 8-41 | $H_3PO_3$ |
| 10-4182 | 8-42 | $H_3PO_3$ |
| 10-4183 | 8-43 | $H_3PO_3$ |
| 10-4184 | 8-44 | $H_3PO_3$ |
| 10-4185 | 8-45 | $H_3PO_3$ |
| 10-4186 | 8-46 | $H_3PO_3$ |
| 10-4187 | 8-47 | $H_3PO_3$ |
| 10-4188 | 8-48 | $H_3PO_3$ |
| 10-4189 | 8-49 | $H_3PO_3$ |

| Nr. | Binäre Kombination | Wirkstoff |
|---|---|---|
| 10-4190 | 8-50 | $H_3PO_3$ |
| 10-4191 | 8-51 | $H_3PO_3$ |
| 10-4192 | 8-52 | $H_3PO_3$ |
| 10-4193 | 8-53 | $H_3PO_3$ |
| 10-4194 | 8-54 | $H_3PO_3$ |
| 10-4195 | 8-55 | $H_3PO_3$ |
| 10-4196 | 8-56 | $H_3PO_3$ |
| 10-4197 | 8-57 | $H_3PO_3$ |
| 10-4198 | 8-58 | $H_3PO_3$ |
| 10-4199 | 8-59 | $H_3PO_3$ |
| 10-4200 | 8-60 | $H_3PO_3$ |
| 10-4201 | 8-61 | $H_3PO_3$ |
| 10-4202 | 8-62 | $H_3PO_3$ |
| 10-4203 | 8-63 | $H_3PO_3$ |
| 10-4204 | 8-64 | $H_3PO_3$ |
| 10-4205 | 8-65 | $H_3PO_3$ |
| 10-4206 | 8-66 | $H_3PO_3$ |
| 10-4207 | 8-67 | $H_3PO_3$ |
| 10-4208 | 8-68 | $H_3PO_3$ |
| 10-4209 | 8-69 | $H_3PO_3$ |
| 10-4210 | 8-70 | $H_3PO_3$ |
| 10-4211 | 8-71 | $H_3PO_3$ |
| 10-4212 | 8-72 | $H_3PO_3$ |
| 10-4213 | 8-73 | $H_3PO_3$ |
| 10-4214 | 8-74 | $H_3PO_3$ |
| 10-4215 | 8-75 | $H_3PO_3$ |
| 10-4216 | 8-76 | $H_3PO_3$ |
| 10-4217 | 8-77 | $H_3PO_3$ |
| 10-4218 | 8-78 | $H_3PO_3$ |
| 10-4219 | 8-79 | $H_3PO_3$ |
| 10-4220 | 8-80 | $H_3PO_3$ |
| 10-4221 | 8-81 | $H_3PO_3$ |
| 10-4222 | 8-82 | $H_3PO_3$ |
| 10-4223 | 8-83 | $H_3PO_3$ |
| 10-4224 | 8-84 | $H_3PO_3$ |
| 10-4225 | 8-85 | $H_3PO_3$ |
| 10-4226 | 8-86 | $H_3PO_3$ |
| 10-4227 | 8-87 | $H_3PO_3$ |
| 10-4228 | 8-88 | $H_3PO_3$ |
| 10-4229 | 8-89 | $H_3PO_3$ |

| Nr. | Binäre Kombination | Wirkstoff |
|---|---|---|
| 10-4230 | 8-90 | $H_3PO_3$ |
| 10-4231 | 8-91 | $H_3PO_3$ |
| 10-4232 | 8-92 | $H_3PO_3$ |
| 10-4233 | 8-93 | $H_3PO_3$ |
| 10-4234 | 8-94 | $H_3PO_3$ |
| 10-4235 | 8-95 | $H_3PO_3$ |
| 10-4236 | 8-96 | $H_3PO_3$ |
| 10-4237 | 8-97 | $H_3PO_3$ |
| 10-4238 | 8-98 | $H_3PO_3$ |
| 10-4239 | 8-99 | $H_3PO_3$ |
| 10-4240 | 8-100 | $H_3PO_3$ |
| 10-4241 | 8-101 | $H_3PO_3$ |
| 10-4242 | 8-102 | $H_3PO_3$ |
| 10-4243 | 8-103 | $H_3PO_3$ |
| 10-4244 | 8-104 | $H_3PO_3$ |
| 10-4245 | 8-105 | $H_3PO_3$ |
| 10-4246 | 8-106 | $H_3PO_3$ |
| 10-4247 | 8-107 | $H_3PO_3$ |
| 10-4248 | 8-108 | $H_3PO_3$ |
| 10-4249 | 8-109 | $H_3PO_3$ |
| 10-4250 | 8-110 | $H_3PO_3$ |
| 10-4251 | 8-111 | $H_3PO_3$ |
| 10-4252 | 8-112 | $H_3PO_3$ |
| 10-4253 | 8-113 | $H_3PO_3$ |
| 10-4254 | 8-114 | $H_3PO_3$ |
| 10-4255 | 8-115 | $H_3PO_3$ |
| 10-4256 | 8-116 | $H_3PO_3$ |
| 10-4257 | 8-117 | $H_3PO_3$ |
| 10-4258 | 8-118 | $H_3PO_3$ |
| 10-4259 | 8-119 | $H_3PO_3$ |
| 10-4260 | 8-120 | $H_3PO_3$ |
| 10-4261 | 8-121 | $H_3PO_3$ |
| 10-4262 | 8-122 | $H_3PO_3$ |
| 10-4263 | 8-123 | $H_3PO_3$ |
| 10-4264 | 8-124 | $H_3PO_3$ |
| 10-4265 | 8-125 | $H_3PO_3$ |
| 10-4266 | 8-126 | $H_3PO_3$ |
| 10-4267 | 8-127 | $H_3PO_3$ |
| 10-4268 | 8-128 | $H_3PO_3$ |
| 10-4269 | 8-129 | $H_3PO_3$ |

| Nr. | Binäre Kombination | Wirkstoff |
|---|---|---|
| 10-4270 | 8-130 | $H_3PO_3$ |
| 10-4271 | 8-131 | $H_3PO_3$ |
| 10-4272 | 8-132 | $H_3PO_3$ |
| 10-4273 | 8-133 | $H_3PO_3$ |
| 10-4274 | 8-134 | $H_3PO_3$ |
| 10-4275 | 8-135 | $H_3PO_3$ |
| 10-4276 | 8-136 | $H_3PO_3$ |
| 10-4277 | 8-137 | $H_3PO_3$ |
| 10-4278 | 8-138 | $H_3PO_3$ |
| 10-4279 | 8-139 | $H_3PO_3$ |
| 10-4280 | 8-140 | $H_3PO_3$ |
| 10-4281 | 8-141 | $H_3PO_3$ |
| 10-4282 | 8-142 | $H_3PO_3$ |
| 10-4283 | 8-143 | $H_3PO_3$ |
| 10-4284 | 8-144 | $H_3PO_3$ |
| 10-4285 | 8-145 | $H_3PO_3$ |
| 10-4286 | 8-146 | $H_3PO_3$ |
| 10-4287 | 8-147 | $H_3PO_3$ |
| 10-4288 | 8-148 | $H_3PO_3$ |
| 10-4289 | 8-149 | $H_3PO_3$ |
| 10-4290 | 8-150 | $H_3PO_3$ |
| 10-4291 | 8-151 | $H_3PO_3$ |
| 10-4292 | 8-152 | $H_3PO_3$ |
| 10-4293 | 8-153 | $H_3PO_3$ |
| 10-4294 | 8-154 | $H_3PO_3$ |
| 10-4295 | 8-155 | $H_3PO_3$ |
| 10-4296 | 8-156 | $H_3PO_3$ |
| 10-4297 | 8-157 | $H_3PO_3$ |
| 10-4298 | 8-158 | $H_3PO_3$ |
| 10-4299 | 8-159 | $H_3PO_3$ |
| 10-4300 | 8-160 | $H_3PO_3$ |
| 10-4301 | 8-161 | $H_3PO_3$ |
| 10-4302 | 8-162 | $H_3PO_3$ |
| 10-4303 | 8-163 | $H_3PO_3$ |
| 10-4304 | 8-164 | $H_3PO_3$ |
| 10-4305 | 8-165 | $H_3PO_3$ |
| 10-4306 | 8-166 | $H_3PO_3$ |
| 10-4307 | 8-167 | $H_3PO_3$ |
| 10-4308 | 8-168 | $H_3PO_3$ |
| 10-4309 | 8-169 | $H_3PO_3$ |

| Nr. | Binäre Kombination | Wirkstoff |
|---|---|---|
| 10-4310 | 8-170 | $H_3PO_3$ |
| 10-4311 | 8-171 | $H_3PO_3$ |
| 10-4312 | 8-172 | $H_3PO_3$ |
| 10-4313 | 8-173 | $H_3PO_3$ |
| 10-4314 | 8-174 | $H_3PO_3$ |
| 10-4315 | 8-175 | $H_3PO_3$ |
| 10-4316 | 8-176 | $H_3PO_3$ |
| 10-4317 | 8-177 | $H_3PO_3$ |
| 10-4318 | 8-178 | $H_3PO_3$ |
| 10-4319 | 8-179 | $H_3PO_3$ |
| 10-4320 | 8-180 | $H_3PO_3$ |
| 10-4321 | 8-1 | Dithianon |
| 10-4322 | 8-2 | Dithianon |
| 10-4323 | 8-3 | Dithianon |
| 10-4324 | 8-4 | Dithianon |
| 10-4325 | 8-5 | Dithianon |
| 10-4326 | 8-6 | Dithianon |
| 10-4327 | 8-7 | Dithianon |
| 10-4328 | 8-8 | Dithianon |
| 10-4329 | 8-9 | Dithianon |
| 10-4330 | 8-10 | Dithianon |
| 10-4331 | 8-11 | Dithianon |
| 10-4332 | 8-12 | Dithianon |
| 10-4333 | 8-13 | Dithianon |
| 10-4334 | 8-14 | Dithianon |
| 10-4335 | 8-15 | Dithianon |
| 10-4336 | 8-16 | Dithianon |
| 10-4337 | 8-17 | Dithianon |
| 10-4338 | 8-18 | Dithianon |
| 10-4339 | 8-19 | Dithianon |
| 10-4340 | 8-20 | Dithianon |
| 10-4341 | 8-21 | Dithianon |
| 10-4342 | 8-22 | Dithianon |
| 10-4343 | 8-23 | Dithianon |
| 10-4344 | 8-24 | Dithianon |
| 10-4345 | 8-25 | Dithianon |
| 10-4346 | 8-26 | Dithianon |
| 10-4347 | 8-27 | Dithianon |
| 10-4348 | 8-28 | Dithianon |
| 10-4349 | 8-29 | Dithianon |

| Nr. | Binäre Kombination | Wirkstoff |
|---|---|---|
| 10-4350 | 8-30 | Dithianon |
| 10-4351 | 8-31 | Dithianon |
| 10-4352 | 8-32 | Dithianon |
| 10-4353 | 8-33 | Dithianon |
| 10-4354 | 8-34 | Dithianon |
| 10-4355 | 8-35 | Dithianon |
| 10-4356 | 8-36 | Dithianon |
| 10-4357 | 8-37 | Dithianon |
| 10-4358 | 8-38 | Dithianon |
| 10-4359 | 8-39 | Dithianon |
| 10-4360 | 8-40 | Dithianon |
| 10-4361 | 8-41 | Dithianon |
| 10-4362 | 8-42 | Dithianon |
| 10-4363 | 8-43 | Dithianon |
| 10-4364 | 8-44 | Dithianon |
| 10-4365 | 8-45 | Dithianon |
| 10-4366 | 8-46 | Dithianon |
| 10-4367 | 8-47 | Dithianon |
| 10-4368 | 8-48 | Dithianon |
| 10-4369 | 8-49 | Dithianon |
| 10-4370 | 8-50 | Dithianon |
| 10-4371 | 8-51 | Dithianon |
| 10-4372 | 8-52 | Dithianon |
| 10-4373 | 8-53 | Dithianon |
| 10-4374 | 8-54 | Dithianon |
| 10-4375 | 8-55 | Dithianon |
| 10-4376 | 8-56 | Dithianon |
| 10-4377 | 8-57 | Dithianon |
| 10-4378 | 8-58 | Dithianon |
| 10-4379 | 8-59 | Dithianon |
| 10-4380 | 8-60 | Dithianon |
| 10-4381 | 8-61 | Dithianon |
| 10-4382 | 8-62 | Dithianon |
| 10-4383 | 8-63 | Dithianon |
| 10-4384 | 8-64 | Dithianon |
| 10-4385 | 8-65 | Dithianon |
| 10-4386 | 8-66 | Dithianon |
| 10-4387 | 8-67 | Dithianon |
| 10-4388 | 8-68 | Dithianon |
| 10-4389 | 8-69 | Dithianon |

| Nr. | Binäre Kombination | Wirkstoff |
|---|---|---|
| 10-4390 | 8-70 | Dithianon |
| 10-4391 | 8-71 | Dithianon |
| 10-4392 | 8-72 | Dithianon |
| 10-4393 | 8-73 | Dithianon |
| 10-4394 | 8-74 | Dithianon |
| 10-4395 | 8-75 | Dithianon |
| 10-4396 | 8-76 | Dithianon |
| 10-4397 | 8-77 | Dithianon |
| 10-4398 | 8-78 | Dithianon |
| 10-4399 | 8-79 | Dithianon |
| 10-4400 | 8-80 | Dithianon |
| 10-4401 | 8-81 | Dithianon |
| 10-4402 | 8-82 | Dithianon |
| 10-4403 | 8-83 | Dithianon |
| 10-4404 | 8-84 | Dithianon |
| 10-4405 | 8-85 | Dithianon |
| 10-4406 | 8-86 | Dithianon |
| 10-4407 | 8-87 | Dithianon |
| 10-4408 | 8-88 | Dithianon |
| 10-4409 | 8-89 | Dithianon |
| 10-4410 | 8-90 | Dithianon |
| 10-4411 | 8-91 | Dithianon |
| 10-4412 | 8-92 | Dithianon |
| 10-4413 | 8-93 | Dithianon |
| 10-4414 | 8-94 | Dithianon |
| 10-4415 | 8-95 | Dithianon |
| 10-4416 | 8-96 | Dithianon |
| 10-4417 | 8-97 | Dithianon |
| 10-4418 | 8-98 | Dithianon |
| 10-4419 | 8-99 | Dithianon |
| 10-4420 | 8-100 | Dithianon |
| 10-4421 | 8-101 | Dithianon |
| 10-4422 | 8-102 | Dithianon |
| 10-4423 | 8-103 | Dithianon |
| 10-4424 | 8-104 | Dithianon |
| 10-4425 | 8-105 | Dithianon |
| 10-4426 | 8-106 | Dithianon |
| 10-4427 | 8-107 | Dithianon |
| 10-4428 | 8-108 | Dithianon |
| 10-4429 | 8-109 | Dithianon |

| Nr. | Binäre Kombination | Wirkstoff |
|---|---|---|
| 10-4430 | 8-110 | Dithianon |
| 10-4431 | 8-111 | Dithianon |
| 10-4432 | 8-112 | Dithianon |
| 10-4433 | 8-113 | Dithianon |
| 10-4434 | 8-114 | Dithianon |
| 10-4435 | 8-115 | Dithianon |
| 10-4436 | 8-116 | Dithianon |
| 10-4437 | 8-117 | Dithianon |
| 10-4438 | 8-118 | Dithianon |
| 10-4439 | 8-119 | Dithianon |
| 10-4440 | 8-120 | Dithianon |
| 10-4441 | 8-121 | Dithianon |
| 10-4442 | 8-122 | Dithianon |
| 10-4443 | 8-123 | Dithianon |
| 10-4444 | 8-124 | Dithianon |
| 10-4445 | 8-125 | Dithianon |
| 10-4446 | 8-126 | Dithianon |
| 10-4447 | 8-127 | Dithianon |
| 10-4448 | 8-128 | Dithianon |
| 10-4449 | 8-129 | Dithianon |
| 10-4450 | 8-130 | Dithianon |
| 10-4451 | 8-131 | Dithianon |
| 10-4452 | 8-132 | Dithianon |
| 10-4453 | 8-133 | Dithianon |
| 10-4454 | 8-134 | Dithianon |
| 10-4455 | 8-135 | Dithianon |
| 10-4456 | 8-136 | Dithianon |
| 10-4457 | 8-137 | Dithianon |
| 10-4458 | 8-138 | Dithianon |
| 10-4459 | 8-139 | Dithianon |
| 10-4460 | 8-140 | Dithianon |
| 10-4461 | 8-141 | Dithianon |
| 10-4462 | 8-142 | Dithianon |
| 10-4463 | 8-143 | Dithianon |
| 10-4464 | 8-144 | Dithianon |
| 10-4465 | 8-145 | Dithianon |
| 10-4466 | 8-146 | Dithianon |
| 10-4467 | 8-147 | Dithianon |
| 10-4468 | 8-148 | Dithianon |
| 10-4469 | 8-149 | Dithianon |

| Nr. | Binäre Kombination | Wirkstoff |
|---|---|---|
| 10-4470 | 8-150 | Dithianon |
| 10-4471 | 8-151 | Dithianon |
| 10-4472 | 8-152 | Dithianon |
| 10-4473 | 8-153 | Dithianon |
| 10-4474 | 8-154 | Dithianon |
| 10-4475 | 8-155 | Dithianon |
| 10-4476 | 8-156 | Dithianon |
| 10-4477 | 8-157 | Dithianon |
| 10-4478 | 8-158 | Dithianon |
| 10-4479 | 8-159 | Dithianon |
| 10-4480 | 8-160 | Dithianon |
| 10-4481 | 8-161 | Dithianon |
| 10-4482 | 8-162 | Dithianon |
| 10-4483 | 8-163 | Dithianon |
| 10-4484 | 8-164 | Dithianon |
| 10-4485 | 8-165 | Dithianon |
| 10-4486 | 8-166 | Dithianon |
| 10-4487 | 8-167 | Dithianon |
| 10-4488 | 8-168 | Dithianon |
| 10-4489 | 8-169 | Dithianon |
| 10-4490 | 8-170 | Dithianon |
| 10-4491 | 8-171 | Dithianon |
| 10-4492 | 8-172 | Dithianon |
| 10-4493 | 8-173 | Dithianon |
| 10-4494 | 8-174 | Dithianon |
| 10-4495 | 8-175 | Dithianon |
| 10-4496 | 8-176 | Dithianon |
| 10-4497 | 8-177 | Dithianon |
| 10-4498 | 8-178 | Dithianon |
| 10-4499 | 8-179 | Dithianon |
| 10-4500 | 8-180 | Dithianon |
| 10-4501 | 8-1 | Cu-Salze |
| 10-4502 | 8-2 | Cu-Salze |
| 10-4503 | 8-3 | Cu-Salze |
| 10-4504 | 8-4 | Cu-Salze |
| 10-4505 | 8-5 | Cu-Salze |
| 10-4506 | 8-6 | Cu-Salze |
| 10-4507 | 8-7 | Cu-Salze |
| 10-4508 | 8-8 | Cu-Salze |
| 10-4509 | 8-9 | Cu-Salze |

| Nr. | Binäre Kombination | Wirkstoff |
|---|---|---|
| 10-4510 | 8-10 | Cu-Salze |
| 10-4511 | 8-11 | Cu-Salze |
| 10-4512 | 8-12 | Cu-Salze |
| 10-4513 | 8-13 | Cu-Salze |
| 10-4514 | 8-14 | Cu-Salze |
| 10-4515 | 8-15 | Cu-Salze |
| 10-4516 | 8-16 | Cu-Salze |
| 10-4517 | 8-17 | Cu-Salze |
| 10-4518 | 8-18 | Cu-Salze |
| 10-4519 | 8-19 | Cu-Salze |
| 10-4520 | 8-20 | Cu-Salze |
| 10-4521 | 8-21 | Cu-Salze |
| 10-4522 | 8-22 | Cu-Salze |
| 10-4523 | 8-23 | Cu-Salze |
| 10-4524 | 8-24 | Cu-Salze |
| 10-4525 | 8-25 | Cu-Salze |
| 10-4526 | 8-26 | Cu-Salze |
| 10-4527 | 8-27 | Cu-Salze |
| 10-4528 | 8-28 | Cu-Salze |
| 10-4529 | 8-29 | Cu-Salze |
| 10-4530 | 8-30 | Cu-Salze |
| 10-4531 | 8-31 | Cu-Salze |
| 10-4532 | 8-32 | Cu-Salze |
| 10-4533 | 8-33 | Cu-Salze |
| 10-4534 | 8-34 | Cu-Salze |
| 10-4535 | 8-35 | Cu-Salze |
| 10-4536 | 8-36 | Cu-Salze |
| 10-4537 | 8-37 | Cu-Salze |
| 10-4538 | 8-38 | Cu-Salze |
| 10-4539 | 8-39 | Cu-Salze |
| 10-4540 | 8-40 | Cu-Salze |
| 10-4541 | 8-41 | Cu-Salze |
| 10-4542 | 8-42 | Cu-Salze |
| 10-4543 | 8-43 | Cu-Salze |
| 10-4544 | 8-44 | Cu-Salze |
| 10-4545 | 8-45 | Cu-Salze |
| 10-4546 | 8-46 | Cu-Salze |
| 10-4547 | 8-47 | Cu-Salze |
| 10-4548 | 8-48 | Cu-Salze |
| 10-4549 | 8-49 | Cu-Salze |

| Nr. | Binäre Kombination | Wirkstoff |
|---|---|---|
| 10-4550 | 8-50 | Cu-Salze |
| 10-4551 | 8-51 | Cu-Salze |
| 10-4552 | 8-52 | Cu-Salze |
| 10-4553 | 8-53 | Cu-Salze |
| 10-4554 | 8-54 | Cu-Salze |
| 10-4555 | 8-55 | Cu-Salze |
| 10-4556 | 8-56 | Cu-Salze |
| 10-4557 | 8-57 | Cu-Salze |
| 10-4558 | 8-58 | Cu-Salze |
| 10-4559 | 8-59 | Cu-Salze |
| 10-4560 | 8-60 | Cu-Salze |
| 10-4561 | 8-61 | Cu-Salze |
| 10-4562 | 8-62 | Cu-Salze |
| 10-4563 | 8-63 | Cu-Salze |
| 10-4564 | 8-64 | Cu-Salze |
| 10-4565 | 8-65 | Cu-Salze |
| 10-4566 | 8-66 | Cu-Salze |
| 10-4567 | 8-67 | Cu-Salze |
| 10-4568 | 8-68 | Cu-Salze |
| 10-4569 | 8-69 | Cu-Salze |
| 10-4570 | 8-70 | Cu-Salze |
| 10-4571 | 8-71 | Cu-Salze |
| 10-4572 | 8-72 | Cu-Salze |
| 10-4573 | 8-73 | Cu-Salze |
| 10-4574 | 8-74 | Cu-Salze |
| 10-4575 | 8-75 | Cu-Salze |
| 10-4576 | 8-76 | Cu-Salze |
| 10-4577 | 8-77 | Cu-Salze |
| 10-4578 | 8-78 | Cu-Salze |
| 10-4579 | 8-79 | Cu-Salze |
| 10-4580 | 8-80 | Cu-Salze |
| 10-4581 | 8-81 | Cu-Salze |
| 10-4582 | 8-82 | Cu-Salze |
| 10-4583 | 8-83 | Cu-Salze |
| 10-4584 | 8-84 | Cu-Salze |
| 10-4585 | 8-85 | Cu-Salze |
| 10-4586 | 8-86 | Cu-Salze |
| 10-4587 | 8-87 | Cu-Salze |
| 10-4588 | 8-88 | Cu-Salze |
| 10-4589 | 8-89 | Cu-Salze |

| Nr. | Binäre Kombination | Wirkstoff |
|---|---|---|
| 10-4590 | 8-90 | Cu-Salze |
| 10-4591 | 8-91 | Cu-Salze |
| 10-4592 | 8-92 | Cu-Salze |
| 10-4593 | 8-93 | Cu-Salze |
| 10-4594 | 8-94 | Cu-Salze |
| 10-4595 | 8-95 | Cu-Salze |
| 10-4596 | 8-96 | Cu-Salze |
| 10-4597 | 8-97 | Cu-Salze |
| 10-4598 | 8-98 | Cu-Salze |
| 10-4599 | 8-99 | Cu-Salze |
| 10-4600 | 8-100 | Cu-Salze |
| 10-4601 | 8-101 | Cu-Salze |
| 10-4602 | 8-102 | Cu-Salze |
| 10-4603 | 8-103 | Cu-Salze |
| 10-4604 | 8-104 | Cu-Salze |
| 10-4605 | 8-105 | Cu-Salze |
| 10-4606 | 8-106 | Cu-Salze |
| 10-4607 | 8-107 | Cu-Salze |
| 10-4608 | 8-108 | Cu-Salze |
| 10-4609 | 8-109 | Cu-Salze |
| 10-4610 | 8-110 | Cu-Salze |
| 10-4611 | 8-111 | Cu-Salze |
| 10-4612 | 8-112 | Cu-Salze |
| 10-4613 | 8-113 | Cu-Salze |
| 10-4614 | 8-114 | Cu-Salze |
| 10-4615 | 8-115 | Cu-Salze |
| 10-4616 | 8-116 | Cu-Salze |
| 10-4617 | 8-117 | Cu-Salze |
| 10-4618 | 8-118 | Cu-Salze |
| 10-4619 | 8-119 | Cu-Salze |
| 10-4620 | 8-120 | Cu-Salze |
| 10-4621 | 8-121 | Cu-Salze |
| 10-4622 | 8-122 | Cu-Salze |
| 10-4623 | 8-123 | Cu-Salze |
| 10-4624 | 8-124 | Cu-Salze |
| 10-4625 | 8-125 | Cu-Salze |
| 10-4626 | 8-126 | Cu-Salze |
| 10-4627 | 8-127 | Cu-Salze |
| 10-4628 | 8-128 | Cu-Salze |
| 10-4629 | 8-129 | Cu-Salze |

| Nr. | Binäre Kombination | Wirkstoff |
|---|---|---|
| 10-4630 | 8-130 | Cu-Salze |
| 10-4631 | 8-131 | Cu-Salze |
| 10-4632 | 8-132 | Cu-Salze |
| 10-4633 | 8-133 | Cu-Salze |
| 10-4634 | 8-134 | Cu-Salze |
| 10-4635 | 8-135 | Cu-Salze |
| 10-4636 | 8-136 | Cu-Salze |
| 10-4637 | 8-137 | Cu-Salze |
| 10-4638 | 8-138 | Cu-Salze |
| 10-4639 | 8-139 | Cu-Salze |
| 10-4640 | 8-140 | Cu-Salze |
| 10-4641 | 8-141 | Cu-Salze |
| 10-4642 | 8-142 | Cu-Salze |
| 10-4643 | 8-143 | Cu-Salze |
| 10-4644 | 8-144 | Cu-Salze |
| 10-4645 | 8-145 | Cu-Salze |
| 10-4646 | 8-146 | Cu-Salze |
| 10-4647 | 8-147 | Cu-Salze |
| 10-4648 | 8-148 | Cu-Salze |
| 10-4649 | 8-149 | Cu-Salze |
| 10-4650 | 8-150 | Cu-Salze |
| 10-4651 | 8-151 | Cu-Salze |
| 10-4652 | 8-152 | Cu-Salze |
| 10-4653 | 8-153 | Cu-Salze |
| 10-4654 | 8-154 | Cu-Salze |
| 10-4655 | 8-155 | Cu-Salze |
| 10-4656 | 8-156 | Cu-Salze |
| 10-4657 | 8-157 | Cu-Salze |
| 10-4658 | 8-158 | Cu-Salze |
| 10-4659 | 8-159 | Cu-Salze |
| 10-4660 | 8-160 | Cu-Salze |
| 10-4661 | 8-161 | Cu-Salze |
| 10-4662 | 8-162 | Cu-Salze |
| 10-4663 | 8-163 | Cu-Salze |
| 10-4664 | 8-164 | Cu-Salze |
| 10-4665 | 8-165 | Cu-Salze |
| 10-4666 | 8-166 | Cu-Salze |
| 10-4667 | 8-167 | Cu-Salze |
| 10-4668 | 8-168 | Cu-Salze |
| 10-4669 | 8-169 | Cu-Salze |

| Nr. | Binäre Kombination | Wirkstoff |
|---|---|---|
| 10-4670 | 8-170 | Cu-Salze |
| 10-4671 | 8-171 | Cu-Salze |
| 10-4672 | 8-172 | Cu-Salze |
| 10-4673 | 8-173 | Cu-Salze |
| 10-4674 | 8-174 | Cu-Salze |
| 10-4675 | 8-175 | Cu-Salze |

| Nr. | Binäre Kombination | Wirkstoff |
|---|---|---|
| 10-4676 | 8-176 | Cu-Salze |
| 10-4677 | 8-177 | Cu-Salze |
| 10-4678 | 8-178 | Cu-Salze |
| 10-4679 | 8-179 | Cu-Salze |
| 10-4680 | 8-180 | Cu-Salze |

Die erfindungsgemäßen Mischungen, insbesondere solche, die eine Verbindung I und eine Verbindung II enthalten, zeichnen sich durch eine hervorragende Wirksamkeit gegen ein breites Spektrum von pflanzenpathogenen Pilzen, insbesondere aus der Klasse der *Ascomyceten, Basidiomyceten, Deuteromyceten* und *Peronosporomyceten* (syn. *Oomyceten*) aus. Sie sind zum Teil systemisch wirksam und können im Pflanzenschutz als Blatt-, Beiz- und Bodenfungizide eingesetzt werden. Darüberhinaus können sie auch zur Saatgutbehandlung verwendet werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbissen, sowie an den Samen dieser Pflanzen. Darüber hinaus können sie auch in Kulturen, die durch Züchtung, einschließlich gentechnischer Methoden, gegen Insekten- oder Pilzbefall tolerant sind, verwendet werden.

Besondere Bedeutung haben die erfindungsgemäßen Mischungen für die Bekämpfung von *Botryosphaeria* Arten, *Cylindrocarpon* Arten, *Eutypa lata, Neonectria liriodendri* und *Stereum hirsutum,* die unter anderem das Holz oder die Wurzeln von Weinreben befallen.

Speziell eignen sich die erfindungsgemäßen Mischungen zur Bekämpfung folgender Pflanzenkrankheiten:

*Alternaria* Arten an Gemüse, Raps, Zuckerrüben, Obst, Reis, Sojabohnen sowie an Kartoffeln (z.B. *A. solani* oder *A. alternata)* und Tomaten (z.B. *A. solani* oder *A. alternata)* und *Alternaria* ssp. (Ährenschwärze) an Weizen,
*Aphanomyces* Arten an Zuckerrüben und Gemüse,
*Ascochyta* Arten an Getreide and Gemüse z.B. *Ascochyta tritici* (Blattdürre) an Weizen, *Bipolaris-* und *Drechslera* Arten an Mais, Getreide, Reis und Rasen und Mais (z.B. *D. maydis),*
*Blumeria graminis* (Echter Mehltau) an Getreide (z.B. Weizen oder Gerste),
*Botrytis cinerea* (Grauschimmel) an Erdbeeren, Gemüse, Blumen, Weinreben und Weizen (Ährenschimmel),
*Bremia lactucae* an Salat,
*Cercospora Arten* an Mais, Reis, Zuckerrüben und z.B. *Cercospora sojina* (Blattflecken) oder *Cercospora kikuchii* (Blattflecken) an Sojabohnen,
*Cladosporium herbarum* (Ährenschwärze) an Weizen,
*Cochliobolus* Arten an Mais, Getreide (z.B. *Cochliobolus sativus)* und Reis (z.B. *Cochliobolus miyabeanus),*
*Colletotricum* Arten an Baumwolle und z.B. *Colletotrichum truncatum* (Antracnose) an Sojabohnen
*Corynespora cassiicola* (Blattflecken) an Sojabohnen,
*Dematophora necatrix* (Wurzel-/Stengelfäule) an Sojabohnen,
*Diaporthe phaseolorum* (Stengelkrankheit) an Sojabohnen,
*Drechslera* Arten, *PyrenophoraArten* an Mais, Getreide, Reis und Rasen, an Gerste (z.B. *D. teres)* und an Weizen (z.B. *D. tritici-repentis),*
*Esca* an Weinrebe, verursacht durch *Phaeoacremonium chlamydosporium, Ph. Aleophilum,* und *Formitipora punctata (syn. Phellinus punctatus),*
*Elsinoe ampelina* an Weinrebe,
*Epicoccum spp.* (Ährenschwärze) an Weizen,
*Exserohilum* Arten an Mais,
*Erysiphe cichoracearum* und *Sphaerotheca fuliginea* an Gurkengewächsen,
*Fusarium* und *Verticillium* Arten an verschiedenen Pflanzen: z.B. *F. graminearum* oder *F. culmorum* (Wurzelfäule) an Getreide (z.B. Weizen oder Gerste) oder *z.B. F. oxysporum an* Tomaten und *Fusarium solani* (Stengelkrankheit) an Sojabohnen *Gaeumanomyces graminis* (Wurzelschwärze) an Getreide (z.B. Weizen oder Gerste), *Gibberella* Arten an Getreide und Reis (z.B. *Gibberella fujikuroi*),
*Glomerella cingulata* an Weinrebe und anderen Pflanzen,
*Grainstaining complex* an Reis,

*Guignardia budwelli an* Weinrebe,
*Helminthosporium* Arten an Mais und Reis,
*Isariopsis clavispora* an Weinrebe,
*Macrophomina phaseolina* (Wurzel-/Stengelfäule) an Sojabohnen,
*Michrodochium nivale* (Schneeschimmel) an Getreide (z.B. Weizen oder Gerste), *Microsphaera diffusa* (Echter Mehltau) an Sojabohnen,
*Mycosphaerella* Arten an Getreide, Bananen und Erdnüssen, wie z.B. *M. graminicola* an Weizen oder *M. fijiensis* an Bananen,
*PeronosporaArten* an Kohl (z.B. *P. brassicae*), Zwiebelgewächsen (z.B. *P. destructor*) und z.B. *Peronospora manshurica* (Falscher Mehltau) an Sojabohnen *Phakopsara pachyrhizi* (Soja-Rost) und *Phakopsara meibomiae* (Soja-Rost) an Sojabohnen,
*Phialophora gregata* (Stengelkrankheit) an Sojabohnen
*PhomopsisArten* an Sonnenblumen, Weinrebe (z.B. *P. viticola*) und Sojabohnen (z.B. *Phomopsis phaseoli*),
*PhytophthoraArten* an verschiedenen Pflanzen z.B. *P. capsici* an Paprika, *Phytopthora megasperma* (Blatt-/Stengelfäule) an Sojabohnen, *Phytophthora infestans* an Kartoffeln und Tomaten,
*Plasmopara viticola an* Weinreben,
*Podosphaera leucotricha* an Apfel,
*Pseudocercosporella herpotrichoides* (Halmbruch) an Getreide (Weizen oder Gerste), *Pseudoperonospora* an verschiedenen Pflanzen z.B. *P. cubensis* an Gurke oder
*P. humili* an Hopfen,
*Pseudopezicula tracheiphilai* an Weinrebe,
*Puccinia Arten* an verschiedenen Pflanzen z.B. *P. triticina, P. striformins, P. hordei* oder *P. graminis* an Getreide (z.B. Weizen oder Gerste) oder an Spargel (z.B. *P. asparagi),*
*Pyricularia oryzae, Corticium sasakii, Sarocladium oryzae, S. attenuatum,*
*Pyrenophora tritici-repentis* (Blattdürre) an Weizen oder *Pyrenophora teres* (Netzflecken) an Gerste,
*Entyloma oryzae* an Reis,
*Pyricularia grisea* an Rasen und Getreide,
*Pythium spp.* an Rasen, Reis, Mais, Baumwolle, Raps, Sonnenblumen, Zuckerrüben, Gemüse und anderen Pflanzen (z.B. *P. ultiumum* oder *P. aphanidermatum*),
*Ramularia collo-cygni* (Ramularia/Sonnenbrand-Komplex/Physiological leaf spots) an Gerste,
*Rhizoctonia* Arten an Baumwolle, Reis, Kartoffeln, Rasen, Mais, Raps, Kartoffeln, Zuckerrüben, Gemüse und an verschiedenen weiteren Pflanzen z.B. *Rhizoctonia solani* (Wurzel-/Stengelfäule) an Sojabohnen oder *Rhizoctonia cerealis* (Spitzer Augenfleck) an Weizen oder Gerste,
*Rhynchosporium secalis* an Gerste (Blattflecken), Roggen und Triticale,
*Sclerotinia* Arten an Raps, Sonnenblumen und z.B. *Sclerotinia sclerotiorum* (Stengelkrankheit) oder *Sclerotinia rolfsii* (Stengelkrankheit) an Sojabohnen,
*Septoria glycines* (Blattflecken) an Sojabohnen,
*Septoria tritici* (Blattseptoria) und *Stagonospora nodorum* an Weizen,
*Erysiphe* (syn. *Uncinula) necator* an Weinrebe,
*Setospaeria* Arten an Mais und Rasen,
*Sphacelotheca reilinia* an Mais,
*Stagonospora nodorum* (Ährenseptoria) an Weizen,
*Thievaliopsis* Arten an Sojabohnen und Baumwolle,
*Tilletia* Arten an Getreide,
*Typhula incarnata* (Schneefäule) an Weizen oder Gerste,
*Ustilago* Arten an Getreide, Mais (z.B. *U. maydis)* und Zuckerrohr,
*Venturia* Arten (Schorf) an Äpfeln (z.B. *V. inaequalis)* und Birnen.

Die erfindungsgemäßen Mischungen, insbesondere diejenigen, die eine Verbindung I und eine Verbindung II enthalten, eignen sich besonders zur Bekämpfung von Schadpilzen aus der Klasse der *Peronosporomycetes* (syn. *Oomyceten),* wie *Peronospora*Arten, *Phytophthora*-Arten, *Plasmopara viticola* und *Pseudoperonospora*-Arten, insbesondere der entsprechenden voranstehend genannten.

Die erfindungsgemäßen Mischungen, insbesondere diejenigen, die eine Verbindung I und eine Verbindung II enthalten, eignen sich außerdem zur Bekämpfung von Schadpilzen im Materialschutz (z.B. Holz, Papier, Dispersionen für den Anstrich, Fasern oder Gewebe) und im Vorratsschutz. Im Holzschutz finden insbesondere folgende Schadpilze Beachtung: Ascomyceten wie *Ophiostoma* spp., *Ceratocystis* spp., *Aureobasidium pullulans, Sclerophoma* spp., *Chaetomium* spp., *Humicola* spp., *Petriella* spp., *Trichu* spp.; Basidiomyceten wie *Coniophora* spp., *Coriolus* spp., *Gloeophyllum* spp., *Lentinus* spp., *Pleurotus* spp., *Poria* spp., *Serpula* spp. und *Tyromyces* spp., Deuteromyceten wie *Asper-*

*gillus* spp., *Cladosporium* spp., *Penicillium* spp., *Trichoderma spp., Alternaria* spp., *Paecilomyces* spp. und Zygomyceten wie *Mucorspp.,* darüber hinaus im Materialschutz folgende Hefepilze: *Candida* spp. und *Saccharomyces cerevisae.*

Die Art und Weise, wie die erfindungsgemäßen Mischungen appliziert werden, kann in weiten Bereichen variieren. So können die Verbindungen I und die Verbindungen II gleichzeitig, und zwar gemeinsam oder getrennt, oder nacheinander appliziert werden, wobei die Reihenfolge bei getrennter Applikation im allgemeinen keine Auswirkung auf den Bekämpfungserfolg hat.

Üblicherweise kommen Mischungen aus einer Verbindung I und einer Verbindung II zur Anwendung. Jedoch können auch Mischungen aus einer Verbindung I und einer Verbindung II und einer weiteren Komponente C gegen Schadpilze oder gegen andere Schädlinge wie Insekten, Spinntiere oder Nematoden oder auch herbizide- oder wachstumsregulierende Wirkstoffe oder Düngemittel, besondere Vorteile bieten.

Als weitere Komponente C im voranstehenden Sinne kommen besonders die in Tabelle 9 genannten Wirkstoffe in Betracht. Dabei können ein oder mehrere der genannten Wirkstoffe eingesetzt werden.

Erfindungsgemäße Mischungen aus drei Wirkstoffen (ternäre Kombinationen) enthalten z.B. eine Verbindung der Formel I, insbesondere 1-Methyl-3-trifluormethyl-1H-pyrazol-4-carbonsäure-N-(2'-fluor-4'-chlor-5'-methylbiphenyl-2-yl)-amid, 1-Methyl-3-trifluormethyl-1 H-pyrazol-4-carbonsäure-N-(3',4',5'-trifluorbiphenyl-2-yl)-amid, 1-Methyl-3-difluormethyl-1 H-pyrazol-4-carbonsäure-N-(3',4',5'-trifluorbiphenyl-2-yl)-amid, 1-Methyl-3-difluormethyl-1 H-pyrazol-4-carbonsäure-N-(2',4',5'-trifluorbiphenyl-2-yl)-amid oder 3-Chlorfluor-methyl-1-methyl-1H-pyrazol-4-carbonsäure-N-(3',4',5'-trifluorbiphenyl-2-yl)-amid, eine Verbindung der Formel II, insbesondere 6-(3,4-Dichlor-phenyl)-5-methyl-[1,2,4]triazolo[1,5-a]pyrimidin-7-ylamin, 6-(4-tert-Butyl-phenyl)-5-methyl-[1,2,4]tri-azolo[1,5-a]pyrimidin-7-ylamin, 5-Methyl-6-(3,5,5-trimethyl-hexyl)-[1,2,4]triazolo[1,5-a]pyrimidin-7-ylamin, 5-Methyl-6-octyl-[1,2,4]triazolo[1,5-a]pyrimidin-7-ylamin, 5-Ethyl-6-octyl-[1,2,4]triazolo[1,5-a]pyrimidin-2,7-diamin, 6-Ethyl-5-octyl-[1,2,4]triazolo[1,5-a]pyrimidin-7-ylamin, 5-Ethyl-6-octyl-[1,2,4]triazolo[1,5-a]pyrimidin-7-ylamin, 5-Ethyl-6-(3,5,5-trimethyl-hexyl)-[1,2,4]triazolo[1,5-a]pyrimidin-7-ylamin, 6-Octyl-5-propyl-[1,2,4]triazolo[1,5-a]pyrimidin-7-ylamin, 5-Methoxymethyl-6-octyl-[1,2,4]triazolo[1,5-a]pyrimidin-7-ylamin, 6-Octyl-5-trifluormethyl-[1,2,4]triazolo[1,5-a]pyrimidin-7-ylamin und 5-Trifluormethyl-6-(3,5,5-trimethyl-hexyl)-[1,2,4]triazolo[1,5-a]pyrimidin-7-ylamin, und einen Wirkstoff der Tabelle 9.

Verbindungen I und Verbindungen II werden üblicherweise in einem Gewichtsverhältnis von 100:1 bis 1:100, vorzugsweise 20:1 bis 1:20, insbesondere 10:1 bis 1:10, verwendet.

Sofern eine weitere Komponente C in der erfindungsgemäßen Mischung enthalten ist, wird diese bevorzugt im Verhältnis von 20:1 bis 1:20, bezogen auf die Verbindung I, eingesetzt.

Die Aufwandmengen der erfindungsgemäßen Mischungen liegen je nach Art der Verbindung und des gewünschten Effekts im Bereich von 5 g/ha bis 2000 g/ha, vorzugsweise von 50 bis 900 g/ha, insbesondere von 50 bis 750 g/ha.

Die Aufwandmengen für die Verbindungen I und II und gegebenenfalls der Komponente C, liegen im Bereich von 1 bis 1000 g/ha, vorzugsweise von 10 bis 900 g/ha, insbesondere von 20 bis 750 g/ha.

Bei der Saatgutbehandlung, z.B. durch Bestäuben, Beschichten oder Tränken von Saatgut, werden im allgemeinen Aufwandmengen an Mischung von 1 bis 1000g/100 kg Saatgut, vorzugsweise von 1 bis 750g/100 kg, insbesondere von 5 bis 500g/100 kg Saatgut verwendet.

Das Verfahren zur Bekämpfung von Schadpilzen erfolgt erfindungsgemäß durch die getrennte oder gemeinsame Applikation von mindestens einer Verbindung I und mindestens einer Verbindung II durch Besprühen oder Bestäuben der Samen, der Pflanzen oder der Böden vor oder nach der Aussaat der Pflanzen oder vor oder nach dem Auflaufen der Pflanzen.

Eine weitere Ausgestaltung des erfindungsgemäßen Verfahrens zur Bekämpfung von Schadpilzen erfolgt durch Applikation einer erfindungsgemäßen Mischung, enthaltend insbesondere Komponenten A und B sowie einen weiteren Wirkstoff der Tabelle 9 als Komponente C, durch Besprühen oder Bestäuben der Samen, der Pflanzen oder der Böden vor oder nach der Aussaat der Pflanzen oder vor oder nach dem Auflaufen der Pflanzen.

Die erfindungsgemäßen Mischungen, insbesondere solche, die eine Verbindung I und eine Verbindung II enthalten, können in die üblichen Formulierungen übergeführt werden, z.B. Lösungen, Emulsionen, Suspensionen, Stäube, Pulver, Pasten oder Granulate. Die Anwendungsform richtet sich nach dem jeweiligen Verwendungszweck; sie soll eine möglichst feine und gleichmäßige Verteilung der erfindungsgemäßen Mischung gewährleisten.

Die Formulierungen werden in bekannter Weise hergestellt, z.B. durch Verstrecken der Wirkstoffe mit Lösungsmitteln und Trägerstoffen beziehungsweise Lösungsmitteln oder Trägerstoffen, gewünschtenfalls unter Verwendung weiterer Hilfsstoffe wie von Emulgiermitteln oder Dispergiermitteln. Dabei können einzelne Stoffe auch unterschiedliche Funktionen erfüllen. Als Lösungsmittel, Trägerstoffe oder Hilfsstoffe kommen dafür im wesentlichen in Betracht:

- Wasser, aromatische Lösungsmittel (z.B. Solvesso® Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden.

- Trägerstoffe wie natürliche Gesteinsmehle (z.B. Kaoline, Tonerden, Talkum, Kreide) oder synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die erfindungsgemäßen Zusammensetzungen können in fester Form oder in flüssiger Form formuliert sein. Je nach Ausgestaltung können die erfindungsgemäßen Zusammensetzungen noch Hilfsmittel und/oder Träger enthalten, wie sie in Pflanzenschutzmitteln oder in Mitteln für den Materialschutz üblich sind. Zu den Hilfsmitteln zählen insbesondere konventionelle oberflächenaktive Substanzen und sonstige im Pflanzen- und Materialschutz übliche Additive und Trägerstoffe, die fest oder flüssig sein können. Zu den oberflächenaktiven Substanzen zählen insbesondere Tenside, insbesondere solche, die Netzmitteleigenschaften haben. Zu den sonstigen Hilfsmitteln (Additive) zählen insbesondere Verdickungsmittel, Entschäumer, Konservierungsmittel, Frostschutzmittel, Stabilisierungsmittel, Anticaking-Mittel bzw. Rieselhilfsmittel und Puffer.
Prinzipiell brauchbare konventionelle oberflächenaktive Substanzen sind anionische, nichtionische und amphotere Tenside, einschließlich Polymer-Tenside, wobei das Molekulargewicht der Tenside typischerweise einen Wert von 2000 Dalton und insbesondere 1000 Dalton nicht überschreiten wird (Zahlenmittel).
Zu den anionischen Tensiden gehören beispielsweise Carboxylate, insbesondere Alkali-, Erdalkali- und Ammoniumsalze von Fettsäuren, z. B. Kaliumstearat, die üblicherweise auch als Seifen bezeichnet werden; Acylglutamate; Sarkosinate, z. B. Natriumlauroylsarkosinat; Taurate; Methylcellulosen; Alkylphosphate, insbesondere Mono- und Diphosphorsäurealkylester; Sulfate, insbesondere Alkylsulfate und Alkylethersulfate; Sulfonate, weitere Alkyl- und Alkylarylsulfonate, insbesondere Alkali-, Erdalkali- und Ammoniumsalze von Arylsulfonsäuren sowie alkylsubstituierten Arylsulfonsäuren, Al-kylbenzolsulfonsäuren, wie beispielsweise Lignin- und Phenolsulfonsäure, Naphthalin- und Dibutylnaphthalinsulfonsäuren, oder Dodecylbenzolsulfonate, Alkylnaphthalinsulfonate, Alkylmethylestersulfonate, Kondensationsprodukte von sulfoniertem Naphthalin und Derivaten davon mit Formaldehyd, Kondensationsprodukte von Naphthalinsulfonsäuren, Phenol- und/oder Phenolsulfonsäuren mit Formaldehyd oder mit Formaldehyl und Harnstoff, Mono- oder Dialkylbernsteinsäureestersulfonate; sowie Eiweißhydrolysate und Lignin-Sulfitablaugen. Die zuvor genannten Sulfonsäuren werden vorteilhafterweise in Form ihrer neutralen oder gegebenenfalls basischen Salze verwendet.
Zu den nichtionischen Tensiden gehören beispielsweise:

- Fettalkoholalkoxylate und Oxoalkoholalkoxylate, insbesondere Ethoxylate und Propoxylate mit Alkoxilierungsgraden von üblicherweise 2 bis 100 und insbesondere 3 bis 50, z. B. Alkoxylate von $C_8$-$C_{30}$-Alkanolen oder Alk(adi)enolen, z. B. von iso-Tridecylalkohol, Laurylalkohol, Oleylalkohol oder Stearylalkohol sowie deren $C_1$-$C_4$-Alkylether und $C_1$-$C_4$-Alkylester z.B. deren Acetate;
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Zuckertenside, Sorbitester, wie beispielsweise Sorbitanfettsäureester (Sorbitanmonooleat, Sorbitantristearat), Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Zu den amphoteren Tensiden gehören beispielsweise Sulfobetaine, Carboxybetaine und Alkyldimethylaminoxide, z. B. Tetradecyldimethylaminoxid.
Weitere Tenside, die hier beispielhaft genannt werden sollen, sind Perfluortenside, Silikontenside, Phospholipide, wie beispielsweise Lecithin oder chemisch modifizierte Lecithine, Aminosäuretenside, z. B. N-Lauroylglutamat.
Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht. Pulver-, Streu- und Stäubemittel können durch Mischen oder gemeinsames Vermahlen der aktiven Komponenten A und B sowie, soweit vorhanden, weiteren Wirkstoffen mit mindestens einem festen Trägerstoff hergestellt werden.
Granulate, z.B. Umhüllungs-, Imprägnierungs- und Homogengranulate, können durch Bindung der Wirkstoffe an mindestens einen festen Trägerstoff hergestellt werden. Feste Trägerstoffe sind z.B. Mineralerden, wie Kieselgele,

Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen der erfindungsgemäßen Mischungen enthalten im allgemeinen 0,01 bis 95 Gew.-%, vorzugsweise 0,1 bis 90 Gew.-% der Verbindungen I und II. Die Wirkstoffe werden dabei bevorzugt in einer Reinheit von 90% bis 100%, vorzugsweise 95% bis 100% eingesetzt.

Für die Saatgutbehandlung ergeben die betreffenden Formulierungen nach zwei- bis zehnfacher Verdünnung Wirkstoffkonzentrationen von 0,01 bis 60 Gew.-%, bevorzugt 0,1 bis 40 Gew.-% in den fertig verwendbaren Zubereitungen.

Beispiele für Formulierungen sind:

1. Produkte zur Verdünnung in Wasser

F1) Wasserlösliche Konzentrate (SL)

10 Gew.-Teile einer erfindungsgemäßen Mischung werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser lösen sich die Wirkstoffe. Die Formulierung hat einen Wirkstoffgehalt von 10 Gew.-%.

F2) Dispergierbare Konzentrate (DC)

20 Gew.-Teile einer erfindungsgemäßen Mischung werden in 70 Gew.-Teilen Cyclohexanon unter Zusatz von 10 Gew.-Teilen eines Dispergiermittels, z.B. Polyvinylpyrrolidon, gelöst. Bei Verdünnung in Wasser ergibt sich eine Dispersion. Die Formulierung hat einen Wirkstoffgehalt von 20 Gew.-%.

F3) Emulgierbare Konzentrate (EC)

15 Gew.-Teile einer erfindungsgemäßen Mischung werden in 75 Gew.-Teilen Xylol unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat einen Wirkstoffgehalt von 15 Gew.-%.

F4) Emulsionen (EW, EO)

25 Gew.-Teile einer erfindungsgemäßen Mischung werden in 35 Gew.-Teilen Xylol unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturrax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

F5) Suspensionen (SC, OD)

20 Gew.-Teile einer erfindungsgemäßen Mischung werden unter Zusatz von 10 Gew.-Teilen Dispergier- und Netzmitteln und 70 Gew.-Teilen Wasser oder einem organischen Lösungsmittel z.B. in einer Rührwerkskugelmühle zu einer feinen Wirkstoffsuspen-sion zerkleinert. Bei der Verdünnung in Wasser ergibt sich eine stabile Suspension der Wirkstoffe. Die Formulierung hat einen Wirkstoffgehalt von 20 Gew.-%.

F6) Wasserdispergierbare und wasserlösliche Granulate (WG, SG)

50 Gew.-Teile einer erfindungsgemäßen Mischung werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung der Wirkstoffe Die Formulierung hat einen Wirkstoffgehalt von 50 Gew.-%.

F7) Wasserdispergierbare und wasserlösliche Pulver (WP, SP)

75 Gew.-Teile einer erfindungsgemäßen Mischung werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung der Wirkstoffe. Die Formulierung hat einen Wirkstoffgehalt von 75 Gew.-%.

2. Produkte für die Direktapplikation

F8) Stäube (DP)

5 Gew.-Teile einer erfindungsgemäßen Mischung werden fein gemahlen und mit 95 Gew.-Teilen feinteiligem Kaolin innig vermischt. Die Stäubemittel haben einen Wirkstoffgehalt von 5 Gew.-%.

F9) Granulate (GR, FG, GG, MG)

0,5 Gew-Teile einer erfindungsgemäßen Mischung werden fein gemahlen und mit 99,5 Gew.-Teilen Trägerstoffe verbunden. Gängige Verfahren sind dabei die Extrusion, die Sprühtrocknung oder die Wirbelschicht. Die Granulate für die Direktapplikation haben einen Wirkstoffgehalt von 0,5 Gew.-%.

F10) ULV- Lösungen (UL)

10 Gew.-Teile einer erfindungsgemäßen Mischung werden in 90 Gew.-Teilen eines organischen Lösungsmittel, z.B. Xylol, gelöst. Die Produkte für die Direktapplikation haben einen Wirkstoffgehalt von 10 Gew.-%.

Für die Saatgutbehandlung werden üblicherweise wasserlösliche Konzentrate (LS), Suspensionen (FS), Stäube (DS), wasserdispergierbare und wasserlösliche Pulver (WS, SS), Emulsionen (ES), emulgierbare Konzentrate (EC) und Gelformulierungen (GF) verwendet. Diese Formulierungen können auf das Saatgut unverdünnt oder, bevorzugt, verdünnt angewendet werden. Die Anwendung kann vor der Aussaat erfolgen.

Bevorzugt werden Formulierungen in Suspensionen für die Saatgutbehandlung verwendet. Üblicherweise enthalten solche Formulierungen 1 bis 800 g/l Wirkstoff, 1 bis 200 g/l Tenside, 0 bis 200 g/l Frostschutzmittel, 0 bis 400 g/l Bindemittel, 0 bis 200 g/l Farbstoffe und Lösungsmittel, vorzugsweise Wasser.

Analoge Formulierungen F1 bis F10 der erfindungsgemäßen Mischungen enthaltend Verbindungen I und II, oder gegebenenfalls eines weiteren Wirkstoffs enthalten die jeweilige Menge der Einzelwirkstoffe. Sie werden üblicherweise direkt vor Applikation beim Verdünnen auf die anwendungsfertige Wirkstoffkonzentration gemischt (Tankmix).

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten möglichst die feinste Verteilung der Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die aktiven Komponenten sowie gegebenenfalls weitere Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, andere Schädlingsbekämpfungsmittel, Bakterizide, gewünschtenfalls auch erst unmittelbar vor der Anwendung (Tankmix), zugesetzt werden. Diese Mittel werden üblicherweise zu den erfindungsgemäßen Mischungen im Gewichtsverhältnis 1:100 bis 100:1, bevorzugt 1:10 bis 10:1, zugemischt werden.

Als Adjuvants in diesem Sinne kommen insbesondere in Betracht: organisch modifizierte Polysiloxane, z.B. Break Thru® S 240; Alkoholalkoxylate, z. B. Atplus® 245, Atplus MBA® 1303, Plurafac LF® 300 und Lutensol ON® 30; EO-PO-Blockpolymerisate, z. B. Pluronic RPE® 2035 und Genapol B®; Alkoholethoxylate, z. B. Lutensol XP® 80; und Natriumdioctylsulfosuccinat, z. B. Leophen RA®.

Die erfindungsgemäßen Mischungen enthaltend Verbindungen I und II, beziehungsweise die entsprechenden Formulierungen werden angewendet, indem man die Schadpilze, deren Lebensraum oder die von ihnen freizuhaltenden Pflanzen, Samen, Böden, Flächen, Materialien oder Räume mit einer fungizid wirksamen Menge der erfindungsgemäßen Mischung behandelt. Die Anwendung kann vor oder nach dem Befall durch die Schadpilze erfolgen.

Anwendungsbeispiele

Die fungizide Wirkung der Einzelverbindungen und der erfindungsgemäßen Mischungen ließ sich durch die folgenden Versuche zeigen.

Die zu erwartenden Wirkungsgrade der Wirkstoffkombinationen wurden nach der Colby-Formel (Colby, S. R. "Calculating synergistic and antagonistic responses of herbicide Combinations", Weeds 15, S. 20 - 22, 1967) ermittelt und mit den beobachteten Wirkungsgraden verglichen.

Colby Formel: $$E = x + y - x \cdot y / 100$$

E zu erwartender Wirkungsgrad, ausgedrückt in % der unbehandelten Kontrolle, beim Einsatz der Mischung aus den Wirkstoffen A und B in den Konzentrationen a und b;

x der Wirkungsgrad, ausgedrückt in % der unbehandelten Kontrolle, beim Einsatz des Wirkstoffs A in der Konzentration a;

y der Wirkungsgrad, ausgedrückt in % der unbehandelten Kontrolle, beim Einsatz des Wirkstoffs B in der Konzentration b.

Mikrotests

Die Wirkstoffe wurden getrennt als Stammlösung formuliert mit einer Konzentration von 10000 ppm in DMSO.

Anwendungsbeispiel 1 - Aktivität gegen den Verursacher der Krautfäule *Phytophthora infestans* im Mikrotiter-Test

Die Stammlösung wird in eine Mikrotiterplatte (MTP) pipettiert und mit einem wässrigen Pilznährmedium auf Erbsensaftbasis auf die angegebene Wirkstoffkonzentration verdünnt. Anschließend erfolgte die Zugabe einer wässrigen Zoosporensuspension von *Phytophthora infestans.* Die Platten wurden in einer wasserdampf-gesättigten Kammer bei Temperaturen von 18°C aufgestellt. Mit einem Absorbtionsphotometer wurden die MTPs am 7. Tag nach der Inokulation bei 405nm gemessen.
Die gemessenen Parameter wurden mit dem Wachstum der wirkstofffreien Kontrollvariante und dem pilz- und wirkstofffreien Leerwert verrechnet, um das relative Wachstum in % der Pathogene in den einzelnen Wirkstoffen zu ermitteln.

| Nr. | Wirkstoff / Wirkstoffkombination | Konz. [ppm] | Verhältnis | Beobachtete Wirkung (%) | Berechnete Wirkung nach Colby (%) |
|---|---|---|---|---|---|
| 1 | 3-1 | 16<br>4<br>1 | | 4<br>0<br>0 | |
| 2 | 3-3 | 4<br>1<br>0,25 | | 0<br>0<br>0 | |
| 3 | 3-6 | 16<br>4<br>1 | | 18<br>3<br>0 | |
| 4 | 3-7 | 4<br>1 | | 4<br>1 | |
| 5 | 3-8 | 16<br>4 | | 20<br>10 | |
| 6 | 3-9 | 4 | | 9 | |
| 7 | 3-10 | 4<br>1 | | 6<br>1 | |
| 8 | 3-11 | 16<br>1 | | 9<br>7 | |

(fortgesetzt)

| Nr. | Wirkstoff / Wirkstoffkombination | Konz. [ppm] | Verhältnis | Beobachtete Wirkung (%) | Berechnete Wirkung nach Colby (%) |
|---|---|---|---|---|---|
| 9 | 3-12 | 1 | | 11 | |
| 10 | 3-13 | 16<br>4<br>1 | | 4<br>4<br>1 | |
| 11 | 3-14 | 16<br>4<br>1 | | 18<br>11<br>3 | |
| 12 | 7-3 | 1 | | 31 | |
| 13 | 7-4 | 4 | | 45 | |
| 14 | 7-5 | 4<br>0,25 | | 73<br>27 | |
| 15 | 7-9 | 4<br>1 | | 58<br>20 | |
| 16 | 7-12 | 16 | | 66 | |
| | | | | | |
| 17 | 3-1 + 7-3 | 1 + 1 | 1 : 1 | 67 | 31 |
| 18 | 3-3 + 7-3 | 1 + 1 | 1 : 1 | 74 | 31 |
| 19 | 3-6 + 7-3 | 1 + 1 | 1 : 1 | 51 | 31 |
| 20 | 3-10 + 7-3 | 1 + 1 | 1 : 1 | 56 | 32 |
| 21 | 3-11 + 7-3 | 1 + 1 | 1 : 1 | 62 | 36 |
| 22 | 3-13 + 7-3 | 1 + 1 | 1 : 1 | 64 | 32 |
| 23 | 3-14 + 7-3 | 1 + 1 | 1 : 1 | 75 | 34 |
| 24 | 3-1 + 7-4 | 4 + 4 | 1 : 1 | 98 | 45 |
| 25 | 3-3 + 7-4 | 4 + 4 | 1 : 1 | 80 | 45 |
| 26 | 3-6 + 7-4 | 4 + 4 | 1 : 1 | 88 | 46 |
| 27 | 3-7 + 7-4 | 4 + 4 | 1 : 1 | 84 | 47 |
| 28 | 3-8 + 7-4 | 4 + 4 | 1 : 1 | 75 | 50 |
| 29 | 3-9 + 7-4 | 4 + 4 | 1 : 1 | 76 | 49 |
| 30 | 3-10 + 7-4 | 4 + 4 | 1 : 1 | 68 | 48 |
| 31 | 3-12 + 7-4 | 4 + 4 | 1 : 1 | 74 | 47 |
| 32 | 3-13 + 7-4 | 4 + 4 | 1 : 1 | 75 | 47 |
| 33 | 3-14 + 7-4 | 4 + 4 | 1 : 1 | 82 | 51 |
| 34 | 3-1 + 7-5 | 4 + 4 | 1 : 1 | 94 | 73 |
| 35 | 3-3 + 7-5 | 0,25 + 0,25 | 1 : 1 | 26 | 0 |
| 36 | 3-14 + 7-5 | 4 + 4 | 1 : 1 | 97 | 76 |
| 37 | 3-1 + 7-9 | 4 + 4 | 1 : 1 | 85 | 58 |
| 38 | 3-3 + 7-9 | 1 + 1 | 1: 1 | 89 | 20 |
| 39 | 3-6 + 7-9 | 1 + 1 | 1 : 1 | 81 | 20 |

(fortgesetzt)

| Nr. | Wirkstoff / Wirkstoffkombination | Konz. [ppm] | Verhältnis | Beobachtete Wirkung (%) | Berechnete Wirkung nach Colby (%) |
|---|---|---|---|---|---|
| 40 | 3-7 + 7-9 | 1 + 1 | 1 : 1 | 57 | 21 |
| 41 | 3-8 + 7-9 | 4 + 4 | 1 : 1 | 87 | 62 |
| 42 | 3-10 + 7-9 | 1 + 1 | 1 : 1 | 56 | 32 |
| 43 | 3-12 + 7-9 | 1 + 1 | 1 : 1 | 48 | 29 |
| 44 | 3-13 + 7-9 | 1 + 1 | 1 : 1 | 53 | 21 |
| 45 | 3-14 + 7-9 | 1 + 1 | 1 : 1 | 48 | 23 |
| 46 | 3-1 + 7-12 | 16 + 16 | 1 : 1 | 35 | 7 |
| 47 | 3-6 + 7-12 | 16 + 16 | 1 : 1 | 100 | 72 |
| 48 | 3-8 + 7-12 | 16 + 16 | 1 : 1 | 100 | 72 |
| 49 | 3-11 + 7-12 | 16 + 16 | 1 : 1 | 100 | 69 |
| 50 | 3-12 + 7-12 | 16 + 16 | 1 : 1 | 100 | 68 |
| 51 | 3-13 + 7-12 | 16 + 16 | 1 : 1 | 100 | 67 |
| 52 | 3-14 + 7-12 | 16 + 16 | 1 : 1 | 100 | 72 |

Gewächshaus

Die Wirkstoffe wurden getrennt oder gemeinsam als eine Stammlösung aufbereitet mit 25 mg Wirkstoff, welcher mit einem Gemisch aus Aceton und/oder Dimethylsulfoxid und dem Emulgator Uniperol® EL (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser Gemisch zu der unten angegebenen Wirkstoffkonzentration verdünnt.

Anwendungsbeispiel 2 - Kurative Wirksamkeit gegen Weizenbraunrost verursacht durch *Puccinia recondita*

Blätter von in Töpfen gewachsenen Weizensämlingen wurden mit einer Sporensuspension des Braunrostes (*Puccinia recondita*) inokuliert. Danach wurden die Töpfe für 24 Stunden in eine Kammer mit hoher Luftfeuchtigkeit (90 bis 95 %) und 20 bis 22°C gestellt. Während dieser Zeit keimten die Sporen aus und die Keimschläuche drangen in das Blattgewebe ein. Die infizierten Pflanzen wurden am nächsten Tag mit der oben beschriebenen Wirkstofflösung in der unten angegebenen Wirkstoffkonzentration bis zur Tropfnässe besprüht. Nach dem Antrocknen des Spritzbelages wurden die Versuchspflanzen im Gewächshaus bei Temperaturen zwischen 20 und 22°C und 65 bis 70 % relativer Luftfeuchte für 7 Tage kultiviert. Dann wurde das Ausmaß der Rostpilzentwicklung auf den Blättern ermittelt.

Die visuell ermittelten Werte für den Prozentanteil befallener Blattflächen wurden in Wirkungsgrade als % der unbehandelten Kontrolle umgerechnet.

Der Wirkungsgrad (W) wird nach der Formel von Abbot wie folgt berechnet:

$$\text{Abbot Formel:} \qquad W = (1 - \alpha/\beta) \circ 100$$

$\alpha$ entspricht dem Pilzbefall der behandelten Pflanzen in % und

β entspricht dem Pilzbefall der unbehandelten (Kontroll-) Pflanzen in %

Bei einem Wirkungsgrad von 0 entspricht der Befall der behandelten Pflanzen demjenigen der unbehandelten Kontrollpflanzen; bei einem Wirkungsgrad von 100 weisen die behandelten Pflanzen keinen Befall auf.

| Nr. | Wirkstoff / Wirkstoffkombination | Konz. [ppm] | Verhältnis | Beobachtete Wirkung (%) | Berechnete Wirkung nach Colby (%) |
|---|---|---|---|---|---|
| 53 | - (Kontrolle) | - | | 0 (90% Befall) | |
| 54 | 3-9 | 0,25 | | 0 | |
| 55 | 7-5 | 1 | | 0 | |
| 56 | 7-6 | 1 | | 0 | |
| 57 | 7-8 | 1 | | 0 | |
| | | | | | |
| 58 | 3-9 + 7-5 | 0,25 + 1 | 1 : 4 | 67 | 0 |
| 59 | 3-9 + 7-6 | 0,25 + 1 | 1 : 4 | 72 | 0 |
| 60 | 3-9 + 7-8 | 0,25 + 1 | 1 : 4 | 44 | 0 |

Anwendungsbeispiel 3 - Protektive Wirksamkeit gegen *Puccinia recondita* an Weizen (Weizenbraunrost)

Blätter von in Töpfen gewachsenen Weizensämlingen wurden mit einer wässriger Suspension in der unten angegebenen Wirkstoffkonzentration bis zur Tropfnässe besprüht. Am nächsten Tag wurden die behandelten Pflanzen mit einer Sporensuspension des Weizenbraunrostes (P*uccinia recondita*) inokuliert. Anschließend wurden die Pflanzen für 24 Stunden in eine Kammer mit hoher Luftfeuchtigkeit (90 bis 95 %) bei 20 bis 22°C gestellt. Während dieser Zeit keimten die Sporen aus und die Keimschläuche drangen in das Blattgewebe ein. Am folgenden Tag wurden die Versuchspflanzen ins Gewächshaus zurückgestellt und bei Temperaturen zwischen 20 und 22°C und 65 bis 70 % relativer Luftfeuchte für weitere 7 Tage kultiviert. Dann wurde das Ausmaß der Rostpilzentwicklung auf den Blättern visuell ermittelt.

| Nr. | Wirkstoff / Wirkstoffkombination | Konz. [ppm] | Verhältnis | Beobachtete Wirkung (%) | Berechnete Wirkung nach Colby (%) |
|---|---|---|---|---|---|
| 61 | - (Kontrolle) | - | | 0 (80% Befall) | |
| 62 | 3-9 | 0,25 | | 50 | |
| 63 | 7-1 | 1 | | 0 | |
| | | | | | |
| 64 | 3-9 + 7-1 | 0,25 + 1 | 1 : 4 | 88 | 50 |

Anwendungsbeispiel 4 - Wirksamkeit gegen die Dürrfleckenkrankheit der Tomate verursacht durch *Alternaria solani*

Blätter von getopften Tomatenpflanzen wurden mit einer wässriger Suspension in der unten angegebenen Wirkstoffkonzentration bis zur Tropfnässe besprüht. Am folgenden Tag wurden die Blätter mit einer wässrigen Sporenaufschwemmung von *Alternaria solani* in 2 % Biomalzlösung mit einer Dichte von 0.17 x $10^6$ Sporen/ml infiziert. Anschließend wurden die Pflanzen in einer wasserdampf-gesättigten Kammer bei Temperaturen zwischen 20 und 22°C aufgestellt. Nach 5 Tagen hatte sich die Krankheit auf den unbehandelten, jedoch infizierten Kontrollpflanzen so stark entwickelt, dass der Befall visuell in % ermittelt werden konnte.

Anwendungsbeispiel 5 - Wirksamkeit gegen den Grauschimmel an Paprikablättern verursacht durch *Botrytis cinerea* bei 1 Tag protektiver Anwendung

Paprikasämlinge der Sorte "Neusiedler Ideal Elite" wurden, nachdem sich 2 - 3 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer Sporensuspension von *Botrytis cinerea,* die $1,7 \times 10^6$ Sporen/ml in einer 2%igen wässrigen Biomalzlösung enthielt, inokuliert. Anschließend wurden die Versuchspflanzen in eine Klimakammer mit 22 bis 24°C, Dunkelheit und hoher Luftfeuchtigkeit gestellt. Nach 5 Tagen konnte das Ausmaß des Pilzbefalls auf den Blättern visuell in % ermittelt werden.

Aus den Ergebnissen der Versuche geht hervor, dass die erfindungsgemäßen Mischungen aufgrund des Synergismus erheblich besser wirksam sind, als nach der Col-by-Formel vorausberechnet.

## Patentansprüche

**1.** Fungizide Mischungen, enthaltend:

A) mindestens ein 1-Methylpyrazol-4-ylcarbonsäureanilid der Formel I

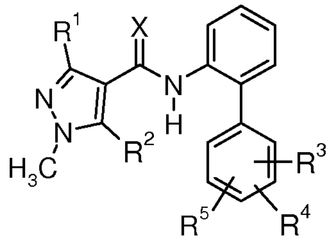

I

in der die Substituenten folgende Bedeutungen haben:

X Sauerstoff oder Schwefel;
$R^1$ $C_1$-$C_4$-Alkyl oder $C_1$-$C_4$-Halogenalkyl;
$R^2$ Wasserstoff oder Halogen;
$R^3$, $R^4$ und $R^5$ unabhängig voneinander Cyano, Nitro, Halogen, $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio;

und
B) mindestens ein Azolopyrimidinylamin der Formel II

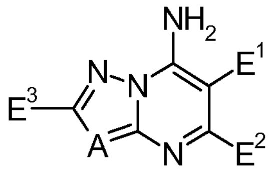

II

in der die Substituenten folgende Bedeutung haben:

$E^1$ $C_3$-$C_{12}$-Alkyl, $C_2$-$C_{12}$-Alkenyl, $C_5$-$C_{12}$-Alkoxyalkyl, $C_3$-$C_6$-Cycloalkyl, Phenyl oder Phenyl-$C_1$-$C_4$-alkyl;
$E^2$ $C_1$-$C_{12}$-Alkyl, $C_2$-$C_{12}$-Alkenyl, $C_1$-$C_4$-Halogenalkyl oder $C_1$-$C_4$-Alkoxy-$C_1$-$C_4$-alkyl;
wobei die aliphatischen Ketten in $E^1$ und/oder $E^2$ durch eine bis vier gleiche oder verschiedene Gruppen $R^a$ substituiert sein können:
$R^a$ Halogen, Cyano, Hydroxy, Mercapto, $C_1$-$C_{10}$-Alkyl, $C_1$-$C_{10}$-Halogenalkyl, $C_3$-$C_8$-Cycloalkyl, $C_2$-$C_{10}$-Alkenyl, $C_2$-$C_{10}$-Alkinyl, $C_1$-$C_6$-Alkoxy, $C_1$-$C_6$-Alkylthio, $C_1$-$C_6$-Alkoxy-$C_1$-$C_6$-alkyl oder $NR^AR^B$;
$R^A$, $R^B$ unabhängig voneinander Wasserstoff oder $C_1$-$C_6$-Alkyl;
wobei die cyclischen Gruppen in $E^1$ und/oder $R^a$ durch eine bis vier Gruppen $R^b$ substituiert sein können:
$R^b$ Halogen, Cyano, Hydroxy, Mercapto, Nitro, $NR^AR^B$, $C_1$-$C_{10}$-Alkyl, $C_1$-$C_6$-Halogenalkyl, $C_2$-$C_6$-Alkenyl, $C_2$-$C_6$-Alkinyl oder $C_1$-$C_6$-Alkoxy;

$E^3$ Wasserstoff, Halogen, Cyano, $NR^AR^B$, Hydroxy, Mercapto, $C_1$-$C_6$-Alkyl, $C_1$-$C_6$-Halogenalkyl, $C_3$-$C_8$-Cycloalkyl, $C_1$-$C_6$-Alkoxy, $C_1$-$C_6$-Alkylthio, $C_3$-$C_8$-Cycloalkoxy, $C_3$-$C_8$-Cycloalkylthio, Carboxyl, Formyl, $C_1$-$C_{10}$-Alkyl-carbonyl, $C_1$-$C_{10}$-Alkoxycarbonyl, $C_2$-$C_{10}$-Alkenyloxycarbonyl, $C_2$-$C_{10}$-Alkinyloxycarbonyl, Phenyl, Phenoxy, Phenylthio, Benzyloxy, Benzylthio oder $C_1$-$C_6$-Alkyl-$S(O)_m$-;
m 0, 1 oder 2;
A CH oder N;

in einer synergistisch wirksamen Menge.

**2.** Fungizide Mischungen nach Anspruch 1, enthaltend Komponente A und Komponente B in einem Gewichtsverhältnis von 100:1 bis 1:100.

**3.** Fungizide Mischungen nach Anspruch 1 oder 2, enthaltend als Komponente A mindestens ein 1-Methylpyrazol-4-ylcarbonsäureanilid der Formel I, wobei $R^1$ für Methyl oder Halogenmethyl, $R^2$ für Wasserstoff, Fluor oder Chlor und $R^3$, $R^4$ und $R^5$ jeweils für Halogen stehen.

**4.** Fungizide Mischungen nach Anspruch 1 oder 2, enthaltend als Komponente A mindestens eine Verbindung I, ausgewählt aus der folgenden Gruppe: 3-Trifluormethyl-1-methyl-1H-pyrazol-4-carbonsäure-N-(2'-fluor-4'-chlor-5'-methoxybiphenyl-2-yl)-amid, 3-Trifluormethyl-1-methyl-1H-pyrazol-4-carbonsäure-N-(2'-fluor-4'-chlor-5'-methylbiphenyl-2-yl)-amid, 3-Trifluormethyl-1-methyl-1 H-pyrazol-4-carbonsäure-N-(3',4',5'-trifluorbiphenyl-2-yl)-amid, 3-Trifluormethyl-1-methyl-1H-pyrazol-4-carbonsäure-N-(2',4',5'-trifluorbiphenyl-2-yl)-amid, 3-Trifluormethyl-1-methyl-1H-pyrazol-4-carbon-säure-N-(2',3',4'-trifluorbiphenyl-2-yl)-amid, 3-Difluormethyl-1-methyl-1 H-pyrazol-4-carbonsäure-N-(2'-fluor-4'-chlor-5'-methoxybiphenyl-2-yl)-amid, 3-Difluormethyl-1-methyl-1H-pyrazol-4-carbonsäure-N-(2'-fluor-4'-chlor-5'-methylbiphenyl-2-yl)-amid, 3-Difluormethyl-1-methyl-1H-pyrazol-4-carbonsäure-N-(3',4',5'-trifluorbiphenyl-2-yl)-amid, 3-Di-fluormethyl-1-methyl-1H-pyrazol-4-carbonsäure-N-(2',4',5'-trifluorbiphenyl-2-yl)-amid, 3-Fluormethyl-1-methyl-1H-pyrazol-4-carbon-säure-N-(3',4',5'-trifluorbi-phenyl-2-yl)-amid, 3-Chlordifluormethyl-1-methyl-1H-pyrazol-4-carbonsäure-N-(3',4',5'-trifluorbiphenyl-2-yl)-amid, 3-Chlorfluormethyl-1-methyl-1 H-pyrazol-4-carbonsäure-N-(3',4',5'-trifluorbiphenyl-2-yl)-amid, 3-Fluormethyl-1-methyl-1 H-pyrazol-4-carbonsäure-N-(2',3',4'-trifluorbiphenyl-2-yl)-amid und 3-Fluormethyl-1-methyl-1H-pyrazol-4-carbonsäure-N-(2',4',5'-trifluorbiphenyl-2-yl)-amid.

**5.** Fungizide Mischungen nach einem der Ansprüche 1 bis 4, enthaltend als Komponente B mindestens eine Verbindung der Formel II, in der die Gruppe A Stickstoff ist (Formel IIa)

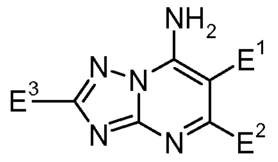

IIa

in der die Substituenten folgende Bedeutungen haben:

$E^1$ $C_3$-$C_{12}$-Alkyl, $C_5$-$C_{12}$-Alkoxyalkyl, Phenyl oder Phenyl-$C_1$-$C_4$-alkyl, wobei Phenyl substituiert sein kann durch eine bis drei Gruppen $R^b$;
$E^2$ $C_1$-$C_{12}$-Alkyl, $C_1$-$C_4$-Halogenalkyl oder $C_1$-$C_4$-Alkoxy-$C_1$-$C_4$-alkyl;
$E^3$ Wasserstoff oder $NH_2$ bedeutet.

**6.** Fungizide Mischungen nach einem der Ansprüche 1 bis 5, enthaltend als Komponente B mindestens eine Verbindung II ausgewählt aus 6-(3,4-Dichlor-phenyl)-5-methyl-[1,2,4]triazolo[1,5-a]pyrimidin-7-ylamin, 6-(4-tert-Butyl-phenyl)-5-methyl-[1,2,4]triazolo[1,5-a]pyrimidin-7-ylamin, 5-Methyl-6-(3,5,5-trimethyl-hexyl)-[1,2,4]triazolo[1,5-a]pyrimidin-7-ylamin, 5-Methyl-6-octyl-[1,2,4]triazolo[1,5-a]pyrimidin-7-ylamin, 6-Methyl-5-octyl-[1,2,4]triazolo[1,5-a]pyrimidin-7-ylamin, 6-Ethyl-5-octyl-[1,2,4]triazolo[1,5-a]pyrimidin-7-ylamin, 5-Ethyl-6-octyl-[1,2,4]triazolo[1,5-a]pyrimidin-7-ylamin, 5-Ethyl-6-(3,5,5-trimethyl-hexyl)-[1,2,4]triazolo[1,5-a]pyrimidin-7-ylamin, 6-Octyl-5-propyl-[1,2,4]triazolo[1,5-a]pyrimidin-7-ylamin, 5-Methoxymethyl-6-octyl-[1,2,4]triazolo[1,5-a]pyrimidin-7-ylamin, 6-Octyl-5-trifluormethyl-[1,2,4]triazolo[1,5-a]pyrimidin-7-ylamin und 5-Trifluormethyl-6-(3,5,5-trimethyl-hexyl)-[1,2,4]triazolo[1,5-a]pyrimidin-7-ylamin.

**7.** Fungizides Mittel, enthaltend mindestens einen flüssigen oder festen Trägerstoff und eine Mischung gemäß einem der Ansprüche 1 bis 6.

**8.** Verfahren zur Bekämpfung von pflanzenpathogenen Schadpilzen, **dadurch gekennzeichnet, dass** man die Pilze, deren Lebensraum oder die vor Pilzbefall zu schützenden Pflanzen, den Boden, Saatgüter, Flächen, Materialien oder Räume mit einer synergistisch wirksamen Menge mindestens einer Verbindung I und mindestens einer Verbindung II gemäß einem der Ansprüche 1 bis 6 behandelt.

**9.** Verfahren nach Anspruch 8, **dadurch gekennzeichnet, dass** man die Verbindungen I und II gemäß einem der Ansprüche 1 bis 6 gleichzeitig, und zwar gemeinsam oder getrennt, oder nacheinander ausbringt.

**10.** Verfahren nach Anspruch 8 oder 9, **dadurch gekennzeichnet, dass** man die Verbindungen I und II oder die Mischungen gemäß den Ansprüchen 1 bis 6 in einer Menge von 5 g/ha bis 2000 g/ha aufwendet.

**11.** Verfahren nach Anspruch 8 oder 9, **dadurch gekennzeichnet, dass** man die Verbindungen I und II oder die Mischungen gemäß einem der Ansprüche 1 bis 6 in einer Menge von 1 g bis 1000 g pro 100 kg Saatgut anwendet.

**12.** Verfahren nach einem der Ansprüche 8 bis 11, **dadurch gekennzeichnet, dass** Schadpilze aus der Klasse der *Oomyceten* bekämpft werden.

**13.** Saatgut, enthaltend die Mischung gemäß einem der Ansprüche 1 bis 6 in einer Menge von 1 g bis 1000 g pro 100 kg Saatgut.

**14.** Verfahren zur Herstellung eines Mittels nach Anspruch 7 durch Verstrecken mindestens einer Verbindung I und mindestens einer Verbindung II gemäß einem der Ansprüche 1 bis 6 mit mindestens einem flüssigen oder festen Trägerstoff.

**Claims**

**1.** A fungicidal mixture comprising:

A) at least one 1-methylpyrazol-4-ylcarboxanilide of the formula I

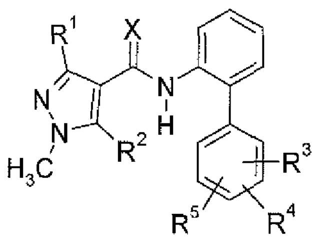

I

in which the substituents have the following meanings:

X is oxygen or sulfur:
$R^1$ is $C_1$-$C_4$-alkyl or $C_1$-$C_4$-haloalkyl;
$R^2$ is hydrogen or halogen;
$R^3$, $R^4$ and $R^5$ independently of one another are cyano, nitro, halogen, $C_1$-$C_4$-alkyl, $C_1$-$C_4$-haloalkyl, $C_1$-$C_4$-alkoxy, $C_1$-$C_4$-haloalkoxy or $C_1$-$C_4$-alkylthio;

and
B) at least one azolopyrimidinylamine of the formula II

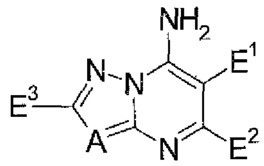

II

in which the substituents have the following meaning:

$E^1$ is $C_3$-$C_{12}$-alkyl, $C_2$-$C_{12}$-alkenyl, $C_5$-$C_{12}$-alkoxyalkyl, $C_3$-$C_6$-cycloalkyl, phenyl or phenyl-$C_1$-$C_4$-alkyl;
$E^2$ is $C_1$-$C_{12}$-alkyl, $C_2$-$C_{12}$-alkenyl, $C_1$-$C_4$-haloalkyl or $C_1$-$C_4$-alkoxy-$C_1$-$C_4$-alkyl;
where the aliphatic chains in $E^1$ and/or $E^2$ may be substituted by one to four identical or different groups $R^a$:
$R^a$ is halogen, cyano, hydroxyl, mercapto, $C_1$-$C_{10}$-alkyl, $C_1$-$C_{10}$-haloalkyl, $C_3$-$C_8$-cycloalkyl, $C_2$-$C_{10}$-alkenyl, $C_2$-$C_{10}$-alkynyl, $C_1$-$C_6$-alkoxy, $C_1$-$C_6$-alkylthio, $C_1$-$C_6$-alkoxy-$C_1$-$C_6$-alkyl or $NR^AR^B$;
$R^A$, $R^B$ independently of one another are hydrogen or $C_1$-$C_6$-alkyl;
where the cyclic groups in $E^1$ and/or $R^a$ may be substituted by one to four groups $R^b$ :
$R^b$ is halogen, cyano, hydroxyl, mercapto, nitro, $NR^AR^B$, $C_1$-$C_{10}$-alkyl, $C_1$-$C_6$-haloalkyl, $C_2$-$C_6$-alkenyl, $C_2$-$C_6$-alkynyl or $C_1$-$C_6$-alkoxy;
$E^3$ is hydrogen, halogen, cyano, $NR^AR^B$, hydroxyl, mercapto, $C_1$-$C_6$-alkyl, $C_1$-$C_6$-haloalkyl, $C_3$-$C_8$-cycloalkyl, $C_1$-$C_6$-alkoxy, $C_1$-$C_6$-alkylthio, $C_3$-$C_8$-cycloalkoxy, $C_3$-$C_8$-cycloalkylthio, carboxyl, formyl, $C_1$-$C_{10}$-alkyl- carbonyl, $C_1$-$C_{10}$-alkoxycarbonyl, $C_2$-$C_{10}$-alkenyloxycarbonyl, $C_2$-$C_{10}$-alkynyloxycarbonyl, phenyl, phenoxy, phenylthio, benzyloxy, benzylthio or $C_1$-$C_6$-alkyl-$S(O)_m$-;
m is 0, 1 or 2;
A is CH or N;

in a synergistically effective amount.

**2.** The fungicidal mixture according to claim 1 comprising component A and component B in a weight ratio of from 100:1 to 1:100.

**3.** The fungicidal mixture according to claim 1 or 2 comprising, as component A, at least one 1-methylpyrazol-4-ylcarboxanilide of the formula I where $R^1$ is methyl or halomethyl, $R^2$ is hydrogen, fluorine or chlorine and $R^3$, $R^4$ and $R^5$ are each halogen.

**4.** The fungicidal mixture according to claim 1 or 2 comprising, as component A, at least one compound I selected from the following group:

N-(2'-fluoro-4'-chloro-5'-methoxybiphenyl-2-yl)-3-trifluoromethyl-1-methyl-1H-pyrazole-4-carboxamide,
N-(2'-fluoro-4'-chloro-5'-methylbiphenyl-2-yl)-3-trifluoromethyl-1-methyl-1H-pyrazole-4-carboxamide,
N-(3',4',5'-trifluorobiphenyl-2-yl)-3-trifluoromethyl-1-methyl-1H-pyrazole-4-carboxamide,
N-(2',4',5'-trifluorobiphenyl-2-yl)-3-trifluoromethyl-1-methyl-1H-pyrazole-4-carboxamide,
N-(2',3',4'-trifluorobiphenyl-2-yl)-3-trifluoromethyl-1-methyl-1-pyrazole-4-carboxamide,
N-(2'-fluoro-4'-chloro-5'-methoxybiphenyl-2-yl)-3-difluoromethyl-1-methyl-1H-pyrazole-4-carboxamide,
N-(2'-fluro-4'-chloro-5'-methylbiphenyl-2-yl), 3-difluromethyl-1-methyl-1H-pyrazole-4-carboxamide,
N-(3',4',5'-trifluorobiphenyl-2-yl)-3-difluoromethyl-1-methyl-1H-pyrazole-4-carboxamide,
N-(21,41,5'-trifluorobiphenyl-2-yl)-3-difluoromethyl-1-methyl-1H-pyrazole-4-carboxamide,
N-(3',4',5'-trifluorobiphenyl-2-yl)-3-fluoromethyl-1-methyl-1H-pyrazole-4-carboxamide, N-(3',4',5'-trifluorobiphenyl-2-yl)-3-chlorodifluoromethyl-1-methyl-1H-pyrazole-4-carboxamide, N-(3',4',5'-trifluorobiphenyl-2-yl)-3-chlorofluoromethyl-1-methyl-1H-pyrazole-4-carboxamide, N-(2' ,3' ,4'-trifluorobiphenyl-2-yl)-3-fluoromethyl-1-methyl-1H-pyrazole-4-carboxamide and N-(2',4',5'-trifluorobiphenyl-2-yl)-3-fluoromethyl-1-methyl-1H-pyrazole-4-carboxamide.

**5.** The fungicidal mixture accroding to any of claims 1 to 4 comprising, as component B, at least one compound of the formula II in which group A is nitrogen (formula IIa)

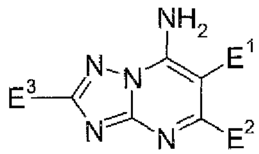

IIa

in which the substituents have the following meanings:

$E^1$ is $C_3$-$C_{12}$-alkyl, $C_5$-$C_{12}$-alkoxyalkyl, phenyl or phenyl-$C_1$-$C_4$-alkyl, where phenyl may be substituted by one to three groups $R^b$;
$E^2$ is $C_1$-$C_{12}$-alkyl, $C_1$-$C_4$-haloalkyl or $C_1$-$C_4$-alkoxy-$C_1$-$C_4$-alkyl;
$E^3$ is hydrogen or $NH_2$.

**6.** The fungicidal mixture according to any of claims 1 to 5 comprising, as component B, at least one compound II selected from the group consisting of 6-(3,4-dichlorophenyl)-5-methyl-[1,2,4]triazolo[1,5-a]pyrimidin-7-ylamine, 6-(4-tert-butylphenyl)-5-methyl-[1,2,4]triazolo[1,5-a]pyrimidin-7-ylamine, 5-methyl-6-(3,5,5-trimethylhexyl)-[1,2,4]triazolo[1,5-a]pyrimidin-7-ylamine, 5-methyl-6-octyl-[1,2,4]triazolo[1,5-a]pyrimidin-7-ylamine, 6-methyl-5-octyl-[1,2,4]triazolo[1,5-a]pyrimidin-7-ylamine, 6-ethyl-5-octyl-[1,2,4]triazolo[1,5-a]pyrimidin-7-ylamine,5-ethyl-6-octyl-[1,2,4]triazolo[1,5-a]pyrimidin-7-ylamine, 5-ethyl-6-(3,5,5-trimethylhexyl)-[1,2,4]triazolo[1,5-a]pyrimidin-7-ylamine, 6-octyl-5-propyl-[1,2,4]triazolo[2,5-a]pyrimidin-7-ylamine, 5-methoxymethyl-6-octyl-[1,2,4]triazolo[1,5-a]pyrimidin-7-ylamine, 6-octyl-5-trifluoromethyl-[1,2,4]triazolo[1,5-a]pyrimidin-7-ylamine and 5-trifluoromethyl-6-(3,5,5-trimethylhexyl)-[1,2,4]triazolo[1,5-a]pyrimidin-7-ylamine.

**7.** A fungicidal composition comprising at least one liquid or solid carrier and a mixture according to any of claims 1 to 6.

**8.** A method for controlling phytopathogenic harmful fungi wherein the fungi, their habitat or the plants, the soil, seed, areas, materials or spaces to be protected against fungal attack are treated with a synergistically effective amount of at least one compound I and at least one compound II according to any of claims 1 to 6.

**9.** The method according to claim 8 wherein the compounds I and II according to any of claims 1 to 6 are applied simultaneously, that is jointly or seperately, or in succession.

**10.** The method according to claim 8 or 9 wherein the compounds I and II or the mixtures according to claims 1 to 6 are applied in an amount of from 5 g/ha to 2000 g/ha.

**11.** The method according to claim 8 or 9 wherein the compounds I and II or the mixtures according to claims 1 to 6 are applied in an amount of from 1 g to
1000 g per 100 kg of seed.

**12.** The method according to any of claims 8 to 11 wherein harmful fungi from the class of the *Oomycetes* are controlled.

**13.** Seed comprising a mixture according to any of claims 1 to 6 in an amount of from 1 g to 1000 g per 100 kg of seed.

**14.** A process for preparing a composition according to claim 7 by extending at least one compound I and at least one compound II according to any of claims 1 to 6 with at least one solid or liquid carrier.

## Revendications

**1.** Mélanges fongicides, contenant

A) au moins un anilide de l’acide 1-méthylpyrazol-4-ylcarboxylique de formule I

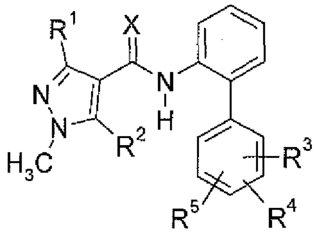

I

dans laquelle les substituants présentent les significations suivantes :

X oxygène ou azote ;
$R^1$ $C_1$-$C_4$-alkyle ou $C_1$-$C_4$-halogénoalkyle ;
$R^2$ hydrogène ou halogène ;
$R^3$, $R^9$ et $R^5$, indépendamment l' un de l' autre, cyano, nitro, halogène, $C_1$-$C_4$-alkyle, $C_1$-$C_4$-halogénoalkyle, $C_1$-$C_4$-alcoxy, $C_1$-$C_4$-halogénoalcoxy ou $C_1$-$C_4$-alkylthio ;

et
B) au moins une azolopyrimidinylamine de formule II

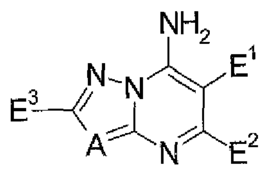

II

dans laquelle les substituants présentent la signification suivante :

$E^1$ $C_3$-$C_{12}$-alkyle, $C_2$-$C_{12}$-alcényle, $C_5$-$C_{12}$-alcoxyalkyle, $C_3$-$C_6$-cycloalkyle, phényle ou phényl-$C_1$-$C_4$-alkyle ;
$E^2$ $C_1$-$C_{12}$-alkyle, $C_2$-$C_{12}$-alcényle, $C_1$-$C_4$-halogénoalkyle ou $C_1$-$C_4$-alcoxy-$C_1$-$C_4$-alkyle ;
où les chaînes aliphatiques dans $E^1$ et/ou $E^2$ peuvent être substituées par un à quatre groupes $R^a$ identiques ou différents :
$R^a$ halogène, cyano, hydroxy, mercapto, $C_1$-$C_{10}$-alkyle, $C_1$-$C_{10}$-halogénoalkyle, $C_3$-$C_8$-cycloalkyle, $C_2$-$C_{10}$-alcényle, $C_2$-$C_{10}$-alcynyle, $C_1$-$C_6$-alcoxy, $C_1$-$C_6$-alkylthio, $C_1$-$C_6$-alcoxy-$C_1$-$C_6$-alkyle ou $NR^AR^B$ ;
$R^A$, $R^B$ indépendamment l'un de l'autre, hydrogène ou $C_1$-$C_6$-alkyle ;
où les groupes cycliques dans $E^1$ et/ou
$R^a$ peuvent être substitués par un à quatre groupes $R^b$ :
$R^b$ halogène, cyano, hydroxy, mercapto, nitro, $NR^AR^B$, $C_1$-$C_{10}$-alkyle, $C_1$-$C_6$-halogénoalkyle, $C_2$-$C_6$-alcényle, $C_2$-$C_6$-alcynyle ou $C_1$-$C_6$-alcoxy ;
$E^3$ hydrogène, halogène, cyano, $NR^AR^B$, hydroxy, mercapto, $C_1$-$C_6$-alkyle, $C_1$-$C_6$-halogénoalkyle, $C_3$-$C_8$-cycloalkyle, $C_1$-$C_6$-alcoxy, $C_1$-$C_6$-alkylthio, $C_3$-$C_8$-cycloalcoxy, $C_3$-$C_8$-cycloalkylthio, carboxyle, formule, $C_1$-$C_{10}$-alkylcarbonyle, $C_1$-$C_{10}$-alcoxycarbonyle, $C_2$-$C_{10}$-alcényloxycarbonyle, $C_2$-$C_{10}$-alcynyloxycarbonyle, phényle, phénoxy, phénylthio, benzyloxy, benzylthio ou $C_1$-$C_6$-alkyl-$S(O)_m$- ;
m 0, 1 ou 2 ;
A CH ou N ;

en une quantité synergiquement active.

2. Mélanges fongicides selon la revendication 1, contenant le composant A et le composant B en un rapport pondéral de 100 :1 à 1 :100.

3. Mélanges fongicides selon la revendication 1 ou 2, contenant comme composant A au moins un anilide de l'acide 1-méthylpyrazol-4-ylcarboxylique de formule I, où $R^1$ représente méthyle ou halogénométhyle, $R^2$ représente hydrogène, fluor ou chlore et $R^3$, $R^4$ et $R^5$ représentent à chaque fois halogène.

4. Mélanges fongicides selon la revendication 1 ou 2, contenant comme composant A au moins un composé I, choisi

dans le groupe suivant : N-(2'-fluoro-4'-chloro-5'-méthoxybiphényl-2-yl)-amide de l'acide 3-trifluorométhyl-1-méthyl-1H-pyrazole-4-carboxylique, N-(2'-fluoro-4'-chloro-5'-méthylbiphényl-2-yl)-amide de l'acide 3-trifluorométhyl-1-méthyl-1H-pyrazole-4-carboxylique, N-(3',4',5'-trifluorobiphényl-2-yl)-amide de l'acide 3-trifluorométhyl-1-méthyl-1H-pyrazole-4-carboxylique, N-(2',4',5'-trifluorobiphényl-2-yl)-amide de l'acide 3-trifluorométhyl-1-méthyl-1H-pyrazole-4-carboxylique, N-(2',3',4'-trifluorobiphényl-2-yl)-amide de l'acide 3-trifluorométhyl-1-méthyl-1H-pyrazole-4-carboxylique, N-(2'-fluoro-4'-chloro-5'-méthoxybiphényl-2-yl)-amide de l'acide 3-difluorométhyl-1-méthyl-1H-pyrazole-4-carboxylique, N-(2'-fluoro-4'-chloro-5'-méthylbiphényl-2-yl)-amide de l'acide 3-difluorométhyl-1-méthyl-1H-pyrazol-4-carboxylique, N-(3',4',5'-trifluorobiphényl-2-yl)-amide de l'acide 3-difluorométhyl-1-méthyl-1H-pyrazole-4-carboxylique, N-(2',4',5'-trifluorobiphényl-2-yl)-amide de l'acide 3-difluorométhyl-1-méthyl-1H-pyrazole-4-carboxylique, N-(3',4',5'-trifluorobiphényl-2-yl)-amide de l'acide 3-fluorométhyl-1-méthyl-1H-pyrazole-4-carboxylique, N-(3',4',5'-trifluorobiphényl-2-yl)-amide de l'acide 3-chlorodifluorométhyl-1-méthyl-1H-pyrazol-4-carboxylique, N-(3',4',5'-trifluorobiphényl-2-yl)-amide de l'acide 3-chlorofluorométhyl-1-méthyl-1H-pyrazol-4-carboxylique, N-(2',3',4'-trifluorobiphényl-2-yl)-amide de l'acide 3-fluorométhyl-1-méthyl-1H-pyrazole-4-carboxylique et N-(2',4',5'-trifluorobiphényl-2-yl)-amide de l'acide 3-fluorométhyl-1-méthyl-1H-pyrazole-4-carboxylique.

**5.** Mélanges fongicides selon l'une quelconque des revendications 1 à 4, contenant comme composant B au moins un composé de formule II, dans laquelle le groupe A représente azote (formule IIa)

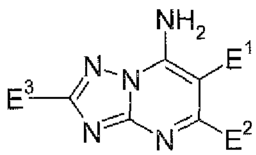

IIa

dans laquelle les substituants présentent les significations suivantes :

$E^1$ $C_3$-$C_{12}$-alkyle, $C_5$-$C_{12}$-alcoxyalkyle, phényle ou phényl-$C_1$-$C_4$-alkyle, où phényle peut être substitué par un à trois groupes $R^b$ ;
$E^2$ $C_1$-$C_{12}$-alkyle, $C_1$-$C_4$-halogénoalkyle ou $C_1$-$C_4$-alcoxy-$C_1$-$C_4$-alkyle ;
$E^3$ hydrogène ou $NH_2$,

**6.** Mélanges fongicides selon l'une quelconque des revendications 1 à 5, contenant comme composant B au moins un composé II choisi parmi 6-(3,4-dichloro-phényl)-5-méthyl-[1,2,4]triazolo[1,5-a]pyrimidin-7-ylamine, 6-(4-tert-butyl-phényl)-5-méthyl-[1,2,4]triazolo[1,5-a]pyrimidin-7-ylamine, 5-méthyl-6-(3,5,5-triméthyl-hexyl)-[1,2,4]triazolo[1,5-a]pyrimidin-7-ylamine, 5-méthyl-6-octyl-[1,2,4]triazolo[1,5-a]pyrimidin-7-ylamine, 6-méthyl-5-octyl-[1,2,4]triazolo[1,5-a]pyrimidin-7-ylamine, 6-éthyl-5-octyl-[1,2,4]triazolo[1,5-a]pyrimidin-7-ylamine, 5-éthyl-6-octyl-[1,2,4]triazolo[1,5-a]pyrimidin-7-ylamine, 5-éthyl-6-(3,5,5-triméthylhexyl)-[1,2,4]triazolo[1,5-a]pyrimidin-7-ylamine, 6-octyl-5-propyl-[1,2,4]triazolo[1,5-a]pyrimidin-7-ylamine, 5-mëthoxyméthyl-6-octyl-[1,2,4]triazolo[1,5-a]pyrimidin-7-ylamine, 6-octyl-5-trifluorométhyl-[1,2,4]triazolo[1,5-a]pyrimidin-7-ylamine et 5-trifluorométhyl-6-(3,5,5-triméthylhexyl)-[1,2,4]triazolo[1,5-a]pyrimidin-7-ylamine.

**7.** Agent fongicide contenant au moins un support liquide ou solide et un mélange selon l'une quelconque des revendications 1 à 6.

**8.** Procédé pour lutter contre des champignons nuisibles phytopathogènes, **caractérisé en ce qu'**on traite les champignons, leur espace de vie ou les plantes, le sol, les semences, les surfaces, les matériaux ou les espaces à protéger contre une attaque par les champignons avec une quantité synergiquement active d'au moins un composé I et d'au moins un composé II selon l'une quelconque des revendications 1 à 6.

**9.** Procédé selon la revendication 8, **caractérisé en ce qu'**on applique les composés I et II selon l'une quelconque des revendications 1 à 6 simultanément, et ce ensemble ou séparément, ou consécutivement.

**10.** Procédé selon la revendication 8 ou 9, **caractérisé en ce qu'**on applique les composés I et II ou les mélanges selon les revendications 1 à 6 en une quantité de 5 g/ha à 2000 g/ha.

**11.** Procédé selon la revendication 8 ou 9, **caractérisé en ce qu'**on applique les composés I et II ou les mélanges selon l'une quelconque des revendications 1 à 6 en une quantité de 1 à 1000 g par 100 kg de semences.

**12.** Procédé selon l'une quelconque des revendications 8 à 11, **caractérisé en ce qu'**on lutte contre des champignons nuisibles de la classe des *oomycètes.*

**13.** Semences, contenant le mélange selon l'une quelconque des revendications 1 à 6 en une quantité de 1 à 1000 g/100 kg de semences.

**14.** Procédé pour la préparation d'un agent selon la revendication 7 par allongement d'au moins un composé I et d'au moins un composé II selon l'une quelconque des revendications 1 à 6 avec au moins un support liquide ou solide.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 06087343 A
- EP 2006064907 W
- EP 2006064463 W


### In der Beschreibung aufgeführte Nicht-Patentliteratur


- **COLBY, S. R.** Calculating synergistic and antagonistic responses of herbicide Combinations. *Weeds,* 1967, vol. 15, 20-22